(19) Europäisches Patentamt
European Patent Office
Office européen des brevets

(11) EP 3 212 789 B1

(12) EUROPEAN PATENT SPECIFICATION

(45) Date of publication and mention of the grant of the patent:
**22.04.2020 Bulletin 2020/17**

(21) Application number: **15813148.2**

(22) Date of filing: **30.10.2015**

(51) Int Cl.:
***C12N 15/10*** *(2006.01)*

(86) International application number:
**PCT/US2015/058304**

(87) International publication number:
**WO 2016/070037 (06.05.2016 Gazette 2016/18)**

(54) **MASSIVELY PARALLEL COMBINATORIAL GENETICS FOR CRISPR**

MASSIV PARALLELE KOMBINATORISCHE GENETIK FÜR CRISPR

GÉNÉTIQUE COMBINATOIRE MASSIVEMENT PARALLÈLE POUR CRISPR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.10.2014 US 201462073126 P**
**26.05.2015 US 201562166302 P**

(43) Date of publication of application:
**06.09.2017 Bulletin 2017/36**

(73) Proprietor: **Massachusetts Institute of Technology Cambridge, MA 02139 (US)**

(72) Inventors:
- **LU, Timothy, Kuan-Ta Charlestown, MA 02129 (US)**
- **WONG, Alan Siu, Lun Ma On Shan (HK)**
- **CHOI, Ching, Gee Cambridge, MA 02139 (US)**

(74) Representative: **Hoffmann Eitle Patent- und Rechtsanwälte PartmbB Arabellastraße 30 81925 München (DE)**

(56) References cited:
**WO-A1-2013/176772 WO-A1-2014/093694**
**WO-A1-2015/153940 WO-A2-2014/005042**

- **NISSIM LIOR ET AL: "Multiplexed and Programmable Regulation of Gene Networks with an Integrated RNA and CRISPR/Cas Toolkit in Human Cells", MOLECULAR CELL, vol. 54, no. 4, 22 May 2014 (2014-05-22), pages 698-710, XP029028594, ISSN: 1097-2765, DOI: 10.1016/J.MOLCEL.2014.04.022**
- **YUEXIN ZHOU ET AL: "High-throughput screening of a CRISPR/Cas9 library for functional genomics in human cells", NATURE, vol. 509, no. 7501, 9 April 2014 (2014-04-09), pages 487-491, XP055234634, United Kingdom ISSN: 0028-0836, DOI: 10.1038/nature13166**
- **GILBERT, L. A. ET AL.: "Genome-Scale CRISPR-Mediated Control of Gene Repression and Activation", CELL, vol. 159, 23 October 2014 (2014-10-23), pages 647-661, XP002754118, cited in the application**
- **S. K. UPADHYAY ET AL: "RNA-Guided Genome Editing for Target Gene Mutations in Wheat", G3: GENES|GENOMES|GENETICS, vol. 3, no. 12, 9 December 2013 (2013-12-09), pages 2233-2238, XP055195466, ISSN: 2160-1836, DOI: 10.1534/g3.113.008847 cited in the application**
- **O. SHALEM ET AL: "Genome-Scale CRISPR-Cas9 Knockout Screening in Human Cells", SCIENCE, vol. 343, no. 6166, 12 December 2013 (2013-12-12), pages 84-87, XP055115506, ISSN: 0036-8075, DOI: 10.1126/science.1247005 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- T. WANG ET AL: "Genetic Screens in Human Cells Using the CRISPR-Cas9 System", SCIENCE, vol. 343, no. 6166, 12 December 2013 (2013-12-12), pages 80-84, XP055115509, ISSN: 0036-8075, DOI: 10.1126/science.1246981 -& Tim Wang et al.,: "Supplementary Material for Genetic screens in human cells using the CRISPR-Cas9 system.", Science 343:80-84,2014 , 12 December 2013 (2013-12-12), pages 1-28, XP002754119, DOI: 10.1126/science.1246981 Retrieved from the Internet: URL:http://science.sciencemag.org/content/sci/suppl/2013/12/11/science.1246981.DC1/Wang.SM.pdf [retrieved on 2016-02-08]
- Jia-Wang Wang ET AL: "CRISPR/Cas9 nuclease cleavage combined with Gibson assembly for seamless cloning", BioTechniques, April 2015 (2015-04), page 161, XP055213773, England DOI: 10.2144/000114261 Retrieved from the Internet: URL:http://www.ncbi.nlm.nih.gov/pubmed/25861928 [retrieved on 2016-02-08]
- KENNEDY EDWARD M ET AL: "Optimization of a multiplex CRISPR/Cas system for use as an antiviral therapeutic.", METHODS (SAN DIEGO, CALIF.) DEC 2015, vol. 91, December 2015 (2015-12), pages 82-86, XP002754120, ISSN: 1095-9130
- XUE HUI-YING ET AL: "CRISPR-Cas9 for medical genetic screens: applications and future perspectives.", JOURNAL OF MEDICAL GENETICS FEB 2016, vol. 53, no. 2, 8 February 2016 (2016-02-08), pages 91-97, XP002754121, ISSN: 1468-6244

SEQUENCE LISTING

<110> Massachusetts Institute of Technology

<120> MASSIVELY PARALLEL COMBINATORIAL GENETICS FOR CRISPR

<130> M0656.70342TW00

<140> Not Yet Assigned
<141> Concurrently Herewith

<150> US 62/166,302
<151> 2015-05-26

<150> US 62/073,126
<151> 2014-10-31

<160> 387

<170> PatentIn version 3.5

<210> 1
<211> 47
<212> DNA
<213> Artificial Sequence

<220>
<223> Synthetic Polynucleotide

<400> 1
aatgatacgg cgaccaccga gatctacacg gatccgcaac ggaattc 47

<210> 2
<211> 52

<212> DNA
<213> Artificial Sequence

<220>
<223> Synthetic Polynucleotide

<220>
<221> misc_feature
<222> (25)..(32)
<223> n is a, c, g, or t

<400> 2
caagcagaag acggcatacg agatnnnnnn nnggttgcgt cagcaaacac ag 52

<210> 3
<211> 20
<212> DNA
<213> Artificial Sequence

<220>
<223> Synthetic Polynucleotide

<400> 3
aatgatacgg cgaccaccga 20

<210> 4
<211> 21
<212> DNA
<213> Artificial Sequence

<220>
<223> Synthetic Polynucleotide

<400> 4
caagcagaag acggcatacg a 21

<210> 5
<211> 34
<212> DNA
<213> Artificial Sequence

<220>
<223> Synthetic Polynucleotide

<400> 5
ccaccgagat ctacacggat ccgcaacgga attc 34

<210> 6
<211> 35
<212> DNA
<213> Artificial Sequence

<220>
<223> Synthetic Polynucleotide

<400> 6
gtggcgtggt gtgcactgtg tttgctgacg caacc 35

<210> 7
<211> 20
<212> DNA
<213> Artificial Sequence

<220>
<223> Synthetic Polynucleotide

<400> 7
gggcgaggag ctgttcaccg 20

<210> 8
<211> 20
<212> DNA
<213> Artificial Sequence

<220>
<223> Synthetic Polynucleotide

<400> 8
cacccagacc atgaagatca 20

<210> 9
<211> 20
<212> DNA
<213> Artificial Sequence

<220>
<223> Synthetic Polynucleotide

<400> 9
ccacttcaag tgcacatccg 20

<210> 10
<211> 20
<212> DNA
<213> Artificial Sequence

<220>
<223> Synthetic Polynucleotide

<400> 10
atcgtttccg cttaacggcg 20

<210> 11
<211> 20
<212> DNA
<213> Artificial Sequence

<220>
<223> Synthetic Polynucleotide

<400> 11
aaacggtacg acagcgtgtg 20

<210> 12
<211> 20
<212> DNA

<213> Artificial Sequence

<220>
<223> Synthetic Polynucleotide

<400> 12
ccatcaccga tcgtgagcct 20

<210> 13
<211> 20
<212> DNA
<213> Artificial Sequence

<220>
<223> Synthetic Polynucleotide

<400> 13
ctagacgtcc attcacttcc 20

<210> 14
<211> 20
<212> DNA
<213> Artificial Sequence

<220>
<223> Synthetic Polynucleotide

<400> 14
tttccaaacc tcgcacgccc 20

<210> 15
<211> 20
<212> DNA
<213> Artificial Sequence

<220>
<223> Synthetic Polynucleotide

<400> 15
acgtaaagaa gaattatccg 20

<210> 16
<211> 20
<212> DNA
<213> Artificial Sequence

<220>
<223> Synthetic Polynucleotide

<400> 16
ccgccccacc ttccgtgccg 20

<210> 17
<211> 20
<212> DNA
<213> Artificial Sequence

<220>

<223> Synthetic Polynucleotide

<400> 17
tggcgctcct ccttgccacg 20

<210> 18
<211> 20
<212> DNA
<213> Artificial Sequence

<220>
<223> Synthetic Polynucleotide

<400> 18
ccgctccgca gcagagctgc 20

<210> 19
<211> 20
<212> DNA
<213> Artificial Sequence

<220>
<223> Synthetic Polynucleotide

<400> 19
agagtcgcga gcttgatctt 20

<210> 20
<211> 20
<212> DNA
<213> Artificial Sequence

<220>
<223> Synthetic Polynucleotide

<400> 20
atccgcaccc cggagatcag 20

<210> 21
<211> 20
<212> DNA
<213> Artificial Sequence

<220>
<223> Synthetic Polynucleotide

<400> 21
gaagactcga tcctcgtcaa 20

<210> 22
<211> 20
<212> DNA
<213> Artificial Sequence

<220>
<223> Synthetic Polynucleotide

<400> 22

agggatctgc gccccatgta 20

<210> 23
<211> 20
<212> DNA
<213> Artificial Sequence

<220>
<223> Synthetic Polynucleotide

<400> 23
actcaccttc tatatttcgc 20

<210> 24
<211> 20
<212> DNA
<213> Artificial Sequence

<220>
<223> Synthetic Polynucleotide

<400> 24
caccaaaatc acgtccatgc 20

<210> 25
<211> 20
<212> DNA
<213> Artificial Sequence

<220>
<223> Synthetic Polynucleotide

<400> 25
tcacccgtag gcaacgtcgc 20

<210> 26
<211> 20
<212> DNA
<213> Artificial Sequence

<220>
<223> Synthetic Polynucleotide

<400> 26
gtgtccagcg acgttgccta 20

<210> 27
<211> 20
<212> DNA
<213> Artificial Sequence

<220>
<223> Synthetic Polynucleotide

<400> 27
acgttgtgca aagactgtcg 20

<210> 28

<211> 20
<212> DNA
<213> Artificial Sequence

<220>
<223> Synthetic Polynucleotide

<400> 28
cggcgactcc gccatagagc 20

<210> 29
<211> 20
<212> DNA
<213> Artificial Sequence

<220>
<223> Synthetic Polynucleotide

<400> 29
ggagccggtc cctttcccgt 20

<210> 30
<211> 20
<212> DNA
<213> Artificial Sequence

<220>
<223> Synthetic Polynucleotide

<400> 30
agtcttgaaa gcgcatgcca 20

<210> 31
<211> 20
<212> DNA
<213> Artificial Sequence

<220>
<223> Synthetic Polynucleotide

<400> 31
agcggctcta gtatcaaccc 20

<210> 32
<211> 20
<212> DNA
<213> Artificial Sequence

<220>
<223> Synthetic Polynucleotide

<400> 32
gaatcacatg acgcattgtc 20

<210> 33
<211> 20
<212> DNA
<213> Artificial Sequence

<220>
<223> Synthetic Polynucleotide

<400> 33
cccgcaaatg actggtcacg 20

<210> 34
<211> 20
<212> DNA
<213> Artificial Sequence

<220>
<223> Synthetic Polynucleotide

<400> 34
ctgtcagaat tgctgcgatc 20

<210> 35
<211> 20
<212> DNA
<213> Artificial Sequence

<220>
<223> Synthetic Polynucleotide

<400> 35
cttggcaaga cttgcctgac 20

<210> 36
<211> 20
<212> DNA
<213> Artificial Sequence

<220>
<223> Synthetic Polynucleotide

<400> 36
tagttcccct aacctcaata 20

<210> 37
<211> 20
<212> DNA
<213> Artificial Sequence

<220>
<223> Synthetic Polynucleotide

<400> 37
acaccattcg taacgttgcc 20

<210> 38
<211> 20
<212> DNA
<213> Artificial Sequence

<220>
<223> Synthetic Polynucleotide

<400> 38
tcactcgaga tgcgcttgtc 20

<210> 39
<211> 20
<212> DNA
<213> Artificial Sequence

<220>
<223> Synthetic Polynucleotide

<400> 39
agaatgctgc cgcacgcacc 20

<210> 40
<211> 20
<212> DNA
<213> Artificial Sequence

<220>
<223> Synthetic Polynucleotide

<400> 40
tccgtaatgt tgctcgatgt 20

<210> 41
<211> 20
<212> DNA
<213> Artificial Sequence

<220>
<223> Synthetic Polynucleotide

<400> 41
tccaacatcg agcaacatta 20

<210> 42
<211> 20
<212> DNA
<213> Artificial Sequence

<220>
<223> Synthetic Polynucleotide

<400> 42
tacaacagat cgtgtaatga 20

<210> 43
<211> 20
<212> DNA
<213> Artificial Sequence

<220>
<223> Synthetic Polynucleotide

<400> 43
gttgactgtg aagctgtacg 20

<210> 44
<211> 20
<212> DNA
<213> Artificial Sequence

<220>
<223> Synthetic Polynucleotide

<400> 44
aacaggttgc gggaatccaa 20

<210> 45
<211> 20
<212> DNA
<213> Artificial Sequence

<220>
<223> Synthetic Polynucleotide

<400> 45
tacccagaac atagacacgc 20

<210> 46
<211> 20
<212> DNA
<213> Artificial Sequence

<220>
<223> Synthetic Polynucleotide

<400> 46
ctcgccgttc gcgtcgccga 20

<210> 47
<211> 20
<212> DNA
<213> Artificial Sequence

<220>
<223> Synthetic Polynucleotide

<400> 47
cccgtactca cggctgtaga 20

<210> 48
<211> 20
<212> DNA
<213> Artificial Sequence

<220>
<223> Synthetic Polynucleotide

<400> 48
gggccacgta ccgttgggtg 20

<210> 49
<211> 20
<212> DNA

<213> Artificial Sequence

<220>
<223> Synthetic Polynucleotide

<400> 49
acaggcttca ttgactgaac 20

<210> 50
<211> 20
<212> DNA
<213> Artificial Sequence

<220>
<223> Synthetic Polynucleotide

<400> 50
agctgatcaa taactatgat 20

<210> 51
<211> 20
<212> DNA
<213> Artificial Sequence

<220>
<223> Synthetic Polynucleotide

<400> 51
cctcatctga gtactgattc 20

<210> 52
<211> 20
<212> DNA
<213> Artificial Sequence

<220>
<223> Synthetic Polynucleotide

<400> 52
acacgcttcc gccaacaaac 20

<210> 53
<211> 20
<212> DNA
<213> Artificial Sequence

<220>
<223> Synthetic Polynucleotide

<400> 53
tgcgactgag acagctcaag 20

<210> 54
<211> 20
<212> DNA
<213> Artificial Sequence

<220>

<223> Synthetic Polynucleotide

<400> 54
aaaacttcat ctcccatata 20

<210> 55
<211> 20
<212> DNA
<213> Artificial Sequence

<220>
<223> Synthetic Polynucleotide

<400> 55
gtacaaattg tacgacggag 20

<210> 56
<211> 20
<212> DNA
<213> Artificial Sequence

<220>
<223> Synthetic Polynucleotide

<400> 56
gacccctcgg cggtttatag 20

<210> 57
<211> 20
<212> DNA
<213> Artificial Sequence

<220>
<223> Synthetic Polynucleotide

<400> 57
tatattgcga ccaccaaact 20

<210> 58
<211> 20
<212> DNA
<213> Artificial Sequence

<220>
<223> Synthetic Polynucleotide

<400> 58
cagagagcac aacgccgcac 20

<210> 59
<211> 20
<212> DNA
<213> Artificial Sequence

<220>
<223> Synthetic Polynucleotide

<400> 59

ggaaccgctg gcagtcgcgc 20

<210> 60
<211> 20
<212> DNA
<213> Artificial Sequence

<220>
<223> Synthetic Polynucleotide

<400> 60
ctcccgctgc ccgtgtagac 20

<210> 61
<211> 20
<212> DNA
<213> Artificial Sequence

<220>
<223> Synthetic Polynucleotide

<400> 61
ctggctcgag atttagcgca 20

<210> 62
<211> 20
<212> DNA
<213> Artificial Sequence

<220>
<223> Synthetic Polynucleotide

<400> 62
aatctgttca tgcgcttacg 20

<210> 63
<211> 20
<212> DNA
<213> Artificial Sequence

<220>
<223> Synthetic Polynucleotide

<400> 63
gattccagta ccagtacatt 20

<210> 64
<211> 20
<212> DNA
<213> Artificial Sequence

<220>
<223> Synthetic Polynucleotide

<400> 64
ctcaccgtgg tctaacttgt 20

<210> 65

<211> 20
<212> DNA
<213> Artificial Sequence

<220>
<223> Synthetic Polynucleotide

<400> 65
ggatgtcatg tcctcagcgt 20

<210> 66
<211> 20
<212> DNA
<213> Artificial Sequence

<220>
<223> Synthetic Polynucleotide

<400> 66
tttgactcct acgcacactt 20

<210> 67
<211> 20
<212> DNA
<213> Artificial Sequence

<220>
<223> Synthetic Polynucleotide

<400> 67
cgtggatgag tacgaccccg 20

<210> 68
<211> 20
<212> DNA
<213> Artificial Sequence

<220>
<223> Synthetic Polynucleotide

<400> 68
tcttctgtgc acactatgcg 20

<210> 69
<211> 20
<212> DNA
<213> Artificial Sequence

<220>
<223> Synthetic Polynucleotide

<400> 69
ctgtcccaga agtgaatcgc 20

<210> 70
<211> 20
<212> DNA
<213> Artificial Sequence

<220>
<223> Synthetic Polynucleotide

<400> 70
gccatgtgct cgttagcgtc 20

<210> 71
<211> 20
<212> DNA
<213> Artificial Sequence

<220>
<223> Synthetic Polynucleotide

<400> 71
gcctgacgct aacgagcaca 20

<210> 72
<211> 20
<212> DNA
<213> Artificial Sequence

<220>
<223> Synthetic Polynucleotide

<400> 72
gaattcatgt actcaactgt 20

<210> 73
<211> 20
<212> DNA
<213> Artificial Sequence

<220>
<223> Synthetic Polynucleotide

<400> 73
cggaatgcgg ggtccgaact 20

<210> 74
<211> 20
<212> DNA
<213> Artificial Sequence

<220>
<223> Synthetic Polynucleotide

<400> 74
cagcatacag ctttatccgc 20

<210> 75
<211> 20
<212> DNA
<213> Artificial Sequence

<220>
<223> Synthetic Polynucleotide

<400> 75
atgaactccc tcttgaaacg 20

<210> 76
<211> 20
<212> DNA
<213> Artificial Sequence

<220>
<223> Synthetic Polynucleotide

<400> 76
attgtccggc gaggacgtgc 20

<210> 77
<211> 20
<212> DNA
<213> Artificial Sequence

<220>
<223> Synthetic Polynucleotide

<400> 77
cttcgccacg cgctgtctca 20

<210> 78
<211> 20
<212> DNA
<213> Artificial Sequence

<220>
<223> Synthetic Polynucleotide

<400> 78
gacggtactg gacgtgggcg 20

<210> 79
<211> 20
<212> DNA
<213> Artificial Sequence

<220>
<223> Synthetic Polynucleotide

<400> 79
caatccgacc acggggtctg 20

<210> 80
<211> 20
<212> DNA
<213> Artificial Sequence

<220>
<223> Synthetic Polynucleotide

<400> 80
gaggttcaaa ccgcctgcta 20

<210> 81
<211> 20
<212> DNA
<213> Artificial Sequence

<220>
<223> Synthetic Polynucleotide

<400> 81
taaagtcggc tggtgacacc 20

<210> 82
<211> 20
<212> DNA
<213> Artificial Sequence

<220>
<223> Synthetic Polynucleotide

<400> 82
agttcttctc ggtgtccaaa 20

<210> 83
<211> 20
<212> DNA
<213> Artificial Sequence

<220>
<223> Synthetic Polynucleotide

<400> 83
gactatcagt tccagagata 20

<210> 84
<211> 20
<212> DNA
<213> Artificial Sequence

<220>
<223> Synthetic Polynucleotide

<400> 84
aacttacgaa ggaaggtctt 20

<210> 85
<211> 20
<212> DNA
<213> Artificial Sequence

<220>
<223> Synthetic Polynucleotide

<400> 85
ttaccttcgc ccgcttgcgc 20

<210> 86
<211> 20
<212> DNA

<213> Artificial Sequence

<220>
<223> Synthetic Polynucleotide

<400> 86
ccggccctac tgtcgtgcct 20

<210> 87
<211> 20
<212> DNA
<213> Artificial Sequence

<220>
<223> Synthetic Polynucleotide

<400> 87
agagccgact tcctcatgac 20

<210> 88
<211> 20
<212> DNA
<213> Artificial Sequence

<220>
<223> Synthetic Polynucleotide

<400> 88
cataccgcat cgataagtct 20

<210> 89
<211> 20
<212> DNA
<213> Artificial Sequence

<220>
<223> Synthetic Polynucleotide

<400> 89
atagccaaga cttatcgatg 20

<210> 90
<211> 20
<212> DNA
<213> Artificial Sequence

<220>
<223> Synthetic Polynucleotide

<400> 90
gaacatacct tctgtagtaa 20

<210> 91
<211> 20
<212> DNA
<213> Artificial Sequence

<220>

<223> Synthetic Polynucleotide

<400> 91
acgctactat gagaccccag 20

<210> 92
<211> 20
<212> DNA
<213> Artificial Sequence

<220>
<223> Synthetic Polynucleotide

<400> 92
tatggcaggg agtcgtcgca 20

<210> 93
<211> 20
<212> DNA
<213> Artificial Sequence

<220>
<223> Synthetic Polynucleotide

<400> 93
gtaacgaatc ctttcttctt 20

<210> 94
<211> 20
<212> DNA
<213> Artificial Sequence

<220>
<223> Synthetic Polynucleotide

<400> 94
cctcgttctc gtcgtatcgc 20

<210> 95
<211> 20
<212> DNA
<213> Artificial Sequence

<220>
<223> Synthetic Polynucleotide

<400> 95
gcgttactac gagacgcccg 20

<210> 96
<211> 20
<212> DNA
<213> Artificial Sequence

<220>
<223> Synthetic Polynucleotide

<400> 96

cttggtcaag cgtccgactg 20

<210> 97
<211> 20
<212> DNA
<213> Artificial Sequence

<220>
<223> Synthetic Polynucleotide

<400> 97
taaatgccga gagtgtcgct 20

<210> 98
<211> 20
<212> DNA
<213> Artificial Sequence

<220>
<223> Synthetic Polynucleotide

<400> 98
gtctgtcaaa accgacttcc 20

<210> 99
<211> 20
<212> DNA
<213> Artificial Sequence

<220>
<223> Synthetic Polynucleotide

<400> 99
gatactgctt ggctgtactg 20

<210> 100
<211> 20
<212> DNA
<213> Artificial Sequence

<220>
<223> Synthetic Polynucleotide

<400> 100
tcttgtatgg gcgccccgtg 20

<210> 101
<211> 20
<212> DNA
<213> Artificial Sequence

<220>
<223> Synthetic Polynucleotide

<400> 101
gccttgactg ttaccggctc 20

<210> 102

<211> 20
<212> DNA
<213> Artificial Sequence

<220>
<223> Synthetic Polynucleotide

<400> 102
tcctgagccg gtaacagtca 20

<210> 103
<211> 20
<212> DNA
<213> Artificial Sequence

<220>
<223> Synthetic Polynucleotide

<400> 103
actccgcaca gttaaaacca 20

<210> 104
<211> 20
<212> DNA
<213> Artificial Sequence

<220>
<223> Synthetic Polynucleotide

<400> 104
gcggaactct cgaacagtca 20

<210> 105
<211> 20
<212> DNA
<213> Artificial Sequence

<220>
<223> Synthetic Polynucleotide

<400> 105
ttccactcac ttatcgctat 20

<210> 106
<211> 20
<212> DNA
<213> Artificial Sequence

<220>
<223> Synthetic Polynucleotide

<400> 106
ccccgcgtac ttctcgctgt 20

<210> 107
<211> 20
<212> DNA
<213> Artificial Sequence

<220>
<223> Synthetic Polynucleotide

<400> 107
gtatgatgac atcgacgacg 20

<210> 108
<211> 20
<212> DNA
<213> Artificial Sequence

<220>
<223> Synthetic Polynucleotide

<400> 108
tcaccaggta ctgtaccccg 20

<210> 109
<211> 20
<212> DNA
<213> Artificial Sequence

<220>
<223> Synthetic Polynucleotide

<400> 109
cctttgcaag acccgcacga 20

<210> 110
<211> 20
<212> DNA
<213> Artificial Sequence

<220>
<223> Synthetic Polynucleotide

<400> 110
agtaggcttc gtgtgatcaa 20

<210> 111
<211> 20
<212> DNA
<213> Artificial Sequence

<220>
<223> Synthetic Polynucleotide

<400> 111
gtctaaagga gcccatcgtg 20

<210> 112
<211> 20
<212> DNA
<213> Artificial Sequence

<220>
<223> Synthetic Polynucleotide

<400> 112
catgaacccc aacgtgctaa 20

<210> 113
<211> 20
<212> DNA
<213> Artificial Sequence

<220>
<223> Synthetic Polynucleotide

<400> 113
ctgggattca aataactcgg 20

<210> 114
<211> 20
<212> DNA
<213> Artificial Sequence

<220>
<223> Synthetic Polynucleotide

<400> 114
tctctggtat gaaagtgccg 20

<210> 115
<211> 20
<212> DNA
<213> Artificial Sequence

<220>
<223> Synthetic Polynucleotide

<400> 115
gtccgcgaac tcttcccagc 20

<210> 116
<211> 20
<212> DNA
<213> Artificial Sequence

<220>
<223> Synthetic Polynucleotide

<400> 116
tcgaagaccg ggcactcggg 20

<210> 117
<211> 20
<212> DNA
<213> Artificial Sequence

<220>
<223> Synthetic Polynucleotide

<400> 117
ggacttattt cagcttaata 20

<210> 118
<211> 20
<212> DNA
<213> Artificial Sequence

<220>
<223> Synthetic Polynucleotide

<400> 118
cttaccgcca tgacacactt 20

<210> 119
<211> 20
<212> DNA
<213> Artificial Sequence

<220>
<223> Synthetic Polynucleotide

<400> 119
gataaacaat gcgttcgtag 20

<210> 120
<211> 20
<212> DNA
<213> Artificial Sequence

<220>
<223> Synthetic Polynucleotide

<400> 120
gggctacccg agcccaccga 20

<210> 121
<211> 20
<212> DNA
<213> Artificial Sequence

<220>
<223> Synthetic Polynucleotide

<400> 121
gatttactcc tcgcgtccaa 20

<210> 122
<211> 20
<212> DNA
<213> Artificial Sequence

<220>
<223> Synthetic Polynucleotide

<400> 122
aaagacttac cgcgggtggg 20

<210> 123
<211> 20
<212> DNA

<213> Artificial Sequence

<220>
<223> Synthetic Polynucleotide

<400> 123
taaggcccga catggaaccg 20

<210> 124
<211> 20
<212> DNA
<213> Artificial Sequence

<220>
<223> Synthetic Polynucleotide

<400> 124
actgtaaact gtagtacctc 20

<210> 125
<211> 20
<212> DNA
<213> Artificial Sequence

<220>
<223> Synthetic Polynucleotide

<400> 125
cagcattatc tgcataccag 20

<210> 126
<211> 20
<212> DNA
<213> Artificial Sequence

<220>
<223> Synthetic Polynucleotide

<400> 126
agactatgag tctagtttaa 20

<210> 127
<211> 20
<212> DNA
<213> Artificial Sequence

<220>
<223> Synthetic Polynucleotide

<400> 127
taccacagcg cccttcgata 20

<210> 128
<211> 20
<212> DNA
<213> Artificial Sequence

<220>

<223> Synthetic Polynucleotide

<400> 128
atccccctcc tcgtagcgca 20

<210> 129
<211> 20
<212> DNA
<213> Artificial Sequence

<220>
<223> Synthetic Polynucleotide

<400> 129
caaaggcttc ccgtgcagcg 20

<210> 130
<211> 20
<212> DNA
<213> Artificial Sequence

<220>
<223> Synthetic Polynucleotide

<400> 130
ttctgcacgg gcttgacgtc 20

<210> 131
<211> 20
<212> DNA
<213> Artificial Sequence

<220>
<223> Synthetic Polynucleotide

<400> 131
gacgtcaagc ccgtgcagaa 20

<210> 132
<211> 20
<212> DNA
<213> Artificial Sequence

<220>
<223> Synthetic Polynucleotide

<400> 132
cagtgacgtc gagaactacg 20

<210> 133
<211> 20
<212> DNA
<213> Artificial Sequence

<220>
<223> Synthetic Polynucleotide

<400> 133

tctgacgaac gtagggctcc 20

<210> 134
<211> 20
<212> DNA
<213> Artificial Sequence

<220>
<223> Synthetic Polynucleotide

<400> 134
ggcttagtga aaaaacgccg 20

<210> 135
<211> 20
<212> DNA
<213> Artificial Sequence

<220>
<223> Synthetic Polynucleotide

<400> 135
cctcgccatc attcactgtg 20

<210> 136
<211> 20
<212> DNA
<213> Artificial Sequence

<220>
<223> Synthetic Polynucleotide

<400> 136
aacgtgtatt gttcgttacc 20

<210> 137
<211> 20
<212> DNA
<213> Artificial Sequence

<220>
<223> Synthetic Polynucleotide

<400> 137
tcctacctta tattcagtag 20

<210> 138
<211> 20
<212> DNA
<213> Artificial Sequence

<220>
<223> Synthetic Polynucleotide

<400> 138
aaaggtttac catcagcaga 20

<210> 139

<211> 20
<212> DNA
<213> Artificial Sequence

<220>
<223> Synthetic Polynucleotide

<400> 139
caccgtgttc tatagagccg 20

<210> 140
<211> 20
<212> DNA
<213> Artificial Sequence

<220>
<223> Synthetic Polynucleotide

<400> 140
cggcgcgagg tggacagcat 20

<210> 141
<211> 20
<212> DNA
<213> Artificial Sequence

<220>
<223> Synthetic Polynucleotide

<400> 141
cgactcaccg gctgcgatcc 20

<210> 142
<211> 20
<212> DNA
<213> Artificial Sequence

<220>
<223> Synthetic Polynucleotide

<400> 142
cgacgtgacg tttgcagtga 20

<210> 143
<211> 20
<212> DNA
<213> Artificial Sequence

<220>
<223> Synthetic Polynucleotide

<400> 143
caaaggtcgg aagccggctg 20

<210> 144
<211> 20
<212> DNA
<213> Artificial Sequence

<220>
<223> Synthetic Polynucleotide

<400> 144
catcactgca aacgtcacgt 20

<210> 145
<211> 20
<212> DNA
<213> Artificial Sequence

<220>
<223> Synthetic Polynucleotide

<400> 145
actagcatgt ctgcggagag 20

<210> 146
<211> 20
<212> DNA
<213> Artificial Sequence

<220>
<223> Synthetic Polynucleotide

<400> 146
tctagtccat cccccattac 20

<210> 147
<211> 20
<212> DNA
<213> Artificial Sequence

<220>
<223> Synthetic Polynucleotide

<400> 147
gggaacaata aagaagcgct 20

<210> 148
<211> 20
<212> DNA
<213> Artificial Sequence

<220>
<223> Synthetic Polynucleotide

<400> 148
gagatcgacg cgaaatacca 20

<210> 149
<211> 20
<212> DNA
<213> Artificial Sequence

<220>
<223> Synthetic Polynucleotide

<400> 149
tataaatccg cgcccgaaag 20

<210> 150
<211> 20
<212> DNA
<213> Artificial Sequence

<220>
<223> Synthetic Polynucleotide

<400> 150
cccataccag ttattgcgct 20

<210> 151
<211> 20
<212> DNA
<213> Artificial Sequence

<220>
<223> Synthetic Polynucleotide

<400> 151
gcagcagcaa ctgtactcgt 20

<211> 20
<212> DNA
<213> Artificial Sequence

<220>
<223> Synthetic Polynucleotide

<400> 152
gcagcgactc cacgcactca 20

<210> 153
<211> 20
<212> DNA
<213> Artificial Sequence

<220>
<223> Synthetic Polynucleotide

<400> 153
gatcttcaag aagaccccgc 20

<210> 154
<211> 20
<212> DNA
<213> Artificial Sequence

<220>
<223> Synthetic Polynucleotide

<400> 154
tctcgcgcat ttccgtgaag 20

<210> 155

<211> 20
<212> DNA
<213> Artificial Sequence

<220>
<223> Synthetic Polynucleotide

<400> 155
cttcacggaa atgcgcgaga 20

<210> 156
<211> 20
<212> DNA
<213> Artificial Sequence

<220>
<223> Synthetic Polynucleotide

<400> 156
tcgatactgc atttgtaatc 20

<210> 157
<211> 20
<212> DNA
<213> Artificial Sequence

<220>
<223> Synthetic Polynucleotide

<400> 157
gctgctcgtg ctcgttccaa 20

<210> 158
<211> 20
<212> DNA
<213> Artificial Sequence

<220>
<223> Synthetic Polynucleotide

<400> 158
cctagaaggc cggactcaaa 20

<210> 159
<211> 20
<212> DNA
<213> Artificial Sequence

<220>
<223> Synthetic Polynucleotide

<400> 159
ggcactactc atatactcag 20

<210> 160
<211> 20
<212> DNA
<213> Artificial Sequence

<220>
<223> Synthetic Polynucleotide

<400> 160
gatctgcttc aaagcgcgcc 20

<210> 161
<211> 20
<212> DNA
<213> Artificial Sequence

<220>
<223> Synthetic Polynucleotide

<400> 161
cttccagctg atgcgagagc 20

<210> 162
<211> 20
<212> DNA
<213> Artificial Sequence

<220>
<223> Synthetic Polynucleotide

<400> 162
gaagttcctc tgaagttcgc 20

<210> 163
<211> 21
<212> DNA
<213> Artificial Sequence

<220>
<223> Synthetic Polynucleotide

<400> 163
cgagggcgac ttaaccttag g 21

<210> 164
<211> 21
<212> DNA
<213> Artificial Sequence

<220>
<223> Synthetic Polynucleotide

<400> 164
aaatcttcgt aatccaagta t 21

<210> 165
<211> 21
<212> DNA
<213> Artificial Sequence

<220>
<223> Synthetic Polynucleotide

<400> 165
gtaataccgg gtgttccgat g 21

<210> 166
<211> 21
<212> DNA
<213> Artificial Sequence

<220>
<223> Synthetic Polynucleotide

<400> 166
attaatccac acgaggtctc c 21

<210> 167
<211> 21
<212> DNA
<213> Artificial Sequence

<220>
<223> Synthetic Polynucleotide

<400> 167
tatagtaatc agggaggttc a 21

<210> 168
<211> 21
<212> DNA
<213> Artificial Sequence

<220>
<223> Synthetic Polynucleotide

<400> 168
tttagacttg attgtgctca t 21

<210> 169
<211> 23
<212> DNA
<213> Artificial Sequence

<220>
<223> Synthetic Polynucleotide

<400> 169
agaaattaaa cggctaccct cca 23

<210> 170
<211> 22
<212> DNA
<213> Artificial Sequence

<220>
<223> Synthetic Polynucleotide

<400> 170
tccatagtgt cttgagcacc ac 22

<210> 171
<211> 22
<212> DNA
<213> Artificial Sequence

<220>
<223> Synthetic Polynucleotide

<400> 171
cacttgctga tgccagtagg ag 22

<210> 172
<211> 22
<212> DNA
<213> Artificial Sequence

<220>
<223> Synthetic Polynucleotide

<400> 172
gtgaatagct tgggaatgtg gg 22

<210> 173
<211> 20
<212> DNA
<213> Artificial Sequence

<220>
<223> Synthetic Polynucleotide

<400> 173
gccacactct acatgggagc 20

<210> 174
<211> 22
<212> DNA
<213> Artificial Sequence

<220>
<223> Synthetic Polynucleotide

<400> 174
gacttttcca tcagggacac ct 22

<210> 175
<211> 20
<212> DNA
<213> Artificial Sequence

<220>
<223> Synthetic Polynucleotide

<400> 175
ggtggacaaa cacattcggc 20

<210> 176
<211> 22
<212> DNA

<213> Artificial Sequence

<220>
<223> Synthetic Polynucleotide

<400> 176
cctatcattg ccccaaggag tc 22

<210> 177
<211> 22
<212> DNA
<213> Artificial Sequence

<220>
<223> Synthetic Polynucleotide

<400> 177
ctaggcctcc gacagttgta at 22

<210> 178
<211> 20
<212> DNA
<213> Artificial Sequence

<220>
<223> Synthetic Polynucleotide

<400> 178
agccaccctt ggttggtttt 20

<210> 179
<211> 20
<212> DNA
<213> Artificial Sequence

<220>
<223> Synthetic Polynucleotide

<400> 179
ggtaagggaa actctggggc 20

<210> 180
<211> 20
<212> DNA
<213> Artificial Sequence

<220>
<223> Synthetic Polynucleotide

<400> 180
ctccctccct tcctaaggct 20

<210> 181
<211> 23
<212> DNA
<213> Artificial Sequence

<220>

<223> Synthetic Polynucleotide

<400> 181
attgccttaa gtcgacacct gat 23

<210> 182
<211> 22
<212> DNA
<213> Artificial Sequence

<220>
<223> Synthetic Polynucleotide

<400> 182
gactgtagag ttgccggaac ag 22

<210> 183
<211> 22
<212> DNA
<213> Artificial Sequence

<220>
<223> Synthetic Polynucleotide

<400> 183
gttggtacaa agtggtgaag gc 22

<210> 184
<211> 22
<212> DNA
<213> Artificial Sequence

<220>
<223> Synthetic Polynucleotide

<400> 184
acgtggcttc attgtacatc ct 22

<210> 185
<211> 22
<212> DNA
<213> Artificial Sequence

<220>
<223> Synthetic Polynucleotide

<400> 185
aagcacatgc ttcaggctaa ca 22

<210> 186
<211> 22
<212> DNA
<213> Artificial Sequence

<220>
<223> Synthetic Polynucleotide

<400> 186

tcatctgctc ttacgcttag cc 22

<210> 187
<211> 20
<212> DNA
<213> Artificial Sequence

<220>
<223> Synthetic Polynucleotide

<400> 187
ctcggcaacc ctccatacat 20

<210> 188
<211> 24
<212> DNA
<213> Artificial Sequence

<220>
<223> Synthetic Polynucleotide

<400> 188
aaccatgcac agaatccaga ttta 24

<210> 189
<211> 20
<212> DNA
<213> Artificial Sequence

<220>
<223> Synthetic Polynucleotide

<400> 189
aagagttctt ggcgcagaca 20

<210> 190
<211> 23
<212> DNA
<213> Artificial Sequence

<220>
<223> Synthetic Polynucleotide

<400> 190
tcgtccaagt tacagtcatc aca 23

<210> 191
<211> 22
<212> DNA
<213> Artificial Sequence

<220>
<223> Synthetic Polynucleotide

<400> 191
tcctctggtg cacagaaaag tc 22

<210> 192

<211> 20
<212> DNA
<213> Artificial Sequence

<220>
<223> Synthetic Polynucleotide

<400> 192
ttctctccag acactgccct 20

<210> 193
<211> 20
<212> DNA
<213> Artificial Sequence

<220>
<223> Synthetic Polynucleotide

<400> 193
gtgcccagaa ctactgccat 20

<210> 194
<211> 20
<212> DNA
<213> Artificial Sequence

<220>
<223> Synthetic Polynucleotide

<400> 194
gaggtgagtt ccagcttccc 20

<210> 195
<211> 20
<212> DNA
<213> Artificial Sequence

<220>
<223> Synthetic Polynucleotide

<400> 195
caccttacag gcactgcgtt 20

<210> 196
<211> 22
<212> DNA
<213> Artificial Sequence

<220>
<223> Synthetic Polynucleotide

<400> 196
ctccctccct agaggctatg ag 22

<210> 197
<211> 20
<212> DNA
<213> Artificial Sequence

<220>
<223> Synthetic Polynucleotide

<400> 197
gttgatgtga ttgccggctc 20

<210> 198
<211> 20
<212> DNA
<213> Artificial Sequence

<220>
<223> Synthetic Polynucleotide

<400> 198
cgtacgggtt catgcaagtt 20

<210> 199
<211> 20
<212> DNA
<213> Artificial Sequence

<220>
<223> Synthetic Polynucleotide

<400> 199
cccctcaccg cctatcagta 20

<210> 200
<211> 20
<212> DNA
<213> Artificial Sequence

<220>
<223> Synthetic Polynucleotide

<400> 200
ttaggcagga cctgtttcgg 20

<210> 201
<211> 21
<212> DNA
<213> Artificial Sequence

<220>
<223> Synthetic Polynucleotide

<400> 201
cgtttgctta agagcacctc c 21

<210> 202
<211> 20
<212> DNA
<213> Artificial Sequence

<220>
<223> Synthetic Polynucleotide

<400> 202
tcttgaacaa gtcggggtcg 20

<210> 203
<211> 61
<212> DNA
<213> Homo sapiens

<400> 203
tttcaacagt ttcctacctt atatcagtag tggtctggtc ttgtgaactt ggacatcaca 60

a 61

<210> 204
<211> 62
<212> DNA
<213> Homo sapiens

<400> 204
tgactcacaa tctctcatgt ttttctggct tagtgaaaaa acgccgtgga tcttcaaagg 60

gg 62

<210> 205
<211> 44
<212> DNA
<213> Artificial Sequence

<220>
<223> Synthetic Polynucleotide

<400> 205
tttcaacagt ttcctacctt atattgtgaa cttggacatc acaa 44

<210> 206
<211> 63
<212> DNA
<213> Artificial Sequence

<220>
<223> Synthetic Polynucleotide

<400> 206
tgactcacaa tctctcatgt ttttctggct tagtgaaaaa acggccgtgg atcttcaaag 60

ggg 63

<210> 207
<211> 59
<212> DNA
<213> Artificial Sequence

<220>
<223> Synthetic Polynucleotide

<400> 207
tgactcacaa tctctcatgt ttttctggct tagtgaaaaa acgtggatct tcaaagggg 59

<210> 208
<211> 59
<212> DNA
<213> Artificial Sequence

<220>
<223> Synthetic Polynucleotide

<400> 208
tttcaacagt ttcctacctt atattcagtg gtctggtctt gtgaacttgg acatcacaa 59

<210> 209
<211> 13
<212> DNA
<213> Artificial Sequence

<220>
<223> Synthetic Polynucleotide

<400> 209
ttcaacatca caa 13

<210> 210
<211> 59
<212> DNA
<213> Artificial Sequence

<220>
<223> Synthetic Polynucleotide

<400> 210
tgactcacaa tctctcatgt ttttctggct tagtgaaaaa acgtggatct tcaaagggg 59

<210> 211
<211> 60
<212> DNA
<213> Homo sapiens

<400> 211
tagacatttg ggaagtttct agtccatccc ccattactgg cagatttctc aatctcgtcc 60

<210> 212
<211> 59
<212> DNA
<213> Homo sapiens

<400> 212
gaaggataat cacctttgca agacccgcac gatgggctcc tttagactcc atgtatgca 59

<210> 213
<211> 57
<212> DNA
<213> Artificial Sequence

<220>
<223> Synthetic Polynucleotide

<400> 213

gaaggataat cacctttgca agacccacga tgggctcctt tagactccat gtatgca 57

<210> 214
<211> 57
<212> DNA
<213> Artificial Sequence

<220>
<223> Synthetic Polynucleotide

<400> 214
gaaggataat cacctttgca agacccgcga tgggctcctt tagactccat gtatgca 57

<210> 215
<211> 52
<212> DNA
<213> Artificial Sequence

<220>
<223> Synthetic Polynucleotide

<400> 215
tagacatttg ggaagtttct agtccatcct ggcagatttc tcaatctcgt cc 52

<210> 216
<211> 58
<212> DNA
<213> Artificial Sequence

<220>
<223> Synthetic Polynucleotide

<400> 216
gaaggataat cacctttgca agacccgccg atgggctcct ttagactcca tgtatgca 58

<210> 217
<211> 34
<212> DNA
<213> Artificial Sequence

<220>
<223> Synthetic Polynucleotide

<400> 217
gaaggataat cacctttgca aatccatgta tgca 34

<210> 218
<211> 50
<212> DNA
<213> Artificial Sequence

<220>
<223> Synthetic Polynucleotide

<400> 218
gaaggataat cacctttgca agatgggctc ctttagactc catgtatgca 50

<210> 219

<211> 49
<212> DNA
<213> Artificial Sequence

<220>
<223> Synthetic Polynucleotide

<400> 219
tttcaacagt ttcctacctt atattcagtt gtgaacttgg acatcacaa 49

<210> 220
<211> 65
<212> DNA
<213> Artificial Sequence

<220>
<223> Synthetic Polynucleotide

<400> 220

**tgactcacaa tctctcatgt ttttctggct tagtgaaaaa acaaaaccgt ggatcttcaa 60**

**agggg 65**

<210> 221
<211> 56
<212> DNA
<213> Artificial Sequence

<220>
<223> Synthetic Polynucleotide

<400> 221
tttcaacagt ttcctacctt attagtggtc tggtcttgtg aacttggaca tcacaa 56

<210> 222
<211> 52
<212> DNA
<213> Artificial Sequence

<220>
<223> Synthetic Polynucleotide

<400> 222
tttcaacagt ttcctacctt atattctggt cttgtgaact tggacatcac aa 52

<210> 223
<211> 60
<212> DNA
<213> Artificial Sequence

<220>
<223> Synthetic Polynucleotide

<400> 223
tttcaacagt ttcctacctt atattctagt ggtctggtct tgtgaacttg gacatcacaa 60

<210> 224
<211> 55
<212> DNA
<213> Artificial Sequence

<220>
<223> Synthetic Polynucleotide

<400> 224
tttcaacagt ttcctacctt ttagtggtct ggtcttgtga acttggacat cacaa 55

<210> 225
<211> 90
<212> DNA
<213> Artificial Sequence

<220>
<223> Synthetic Polynucleotide

<400> 225

tacaactcca ataataatta caaacaagga atttcaacag tttcctacct tatattcagg 60

tcttgtgaac ttggacatca caaataggac 90

<210> 226
<211> 90
<212> DNA
<213> Artificial Sequence

<220>
<223> Synthetic Polynucleotide

<400> 226

cgttctgact cacaatctct catgtttttc tggcttagtg aaaaaacttc aaaggggcat 60

gatacacaca aggggaaacc agtgaagacc 90

<210> 227
<211> 91
<212> DNA
<213> Artificial Sequence

<220>
<223> Synthetic Polynucleotide

<400> 227

tacaactcca ataataatta caaacaagga atttcaacag tttcctacct tataggtctg 60

gtcttgtgaa cttggacatc acaaatagga c 91

<210> 228
<211> 97

<212> DNA
<213> Artificial Sequence

<220>
<223> Synthetic Polynucleotide

<400> 228

tacaactcca ataataatta caaacaagga atttcaacag tttcctacct tatattcagt 60

ggtctggtct tgtgaacttg gacatcacaa ataggac 97

<210> 229
<211> 99
<212> DNA
<213> Artificial Sequence

<220>
<223> Synthetic Polynucleotide

<400> 229

cgttctgact cacaatctct catgtttttc tggcttagtg aaaaacgccg tggatcttca 60

aaggggcatg atacacacaa ggggaaacca gtgaagacc 99

<210> 230
<211> 95
<212> DNA
<213> Artificial Sequence

<220>
<223> Synthetic Polynucleotide

<400> 230

cgttctgact cacaatctct catgtttttc tggcttagtg aaaaactgga tcttcaaagg 60

ggcatgatac acacaagggg aaaccagtga agacc 95

<210> 231
<211> 100
<212> DNA
<213> Artificial Sequence

<220>
<223> Synthetic Polynucleotide

<400> 231

actccaataa taattacaaa caaggaattt caacagtttc ctaccttata ttctgtgatc 60

tgtggtctgg tcttgtgaac ttggacatca caaataggac 100

<210> 232
<211> 97
<212> DNA
<213> Artificial Sequence

<220>
<223> Synthetic Polynucleotide

<400> 232

cgttctgact cacaatctct catgtttttc tggcttagtg aaaaaacgtg gatcttcaaa 60

ggggcatgat acacacaagg ggaaaccagt gaagacc 97

<210> 233
<211> 100
<212> DNA
<213> Homo sapiens

<400> 233

tacaactcca ataataatta caaacaagga atttcaacag tttcctacct tatattcagt 60

agtggtctgg tcttgtgaac ttggacatca caaataggac 100

<210> 234
<211> 100
<212> DNA
<213> Homo sapiens

<400> 234

cgttctgact cacaatctct catgtttttc tggcttagtg aaaaaacgcc gtggatcttc 60

aaaggggcat gatacacaca aggggaaacc agtgaagacc 100

<210> 235
<211> 100
<212> DNA
<213> Artificial Sequence

<220>
<223> Synthetic Polynucleotide

<400> 235

atttcaacag tttcctacct tatatactat actatatata tatatatata ctatatatat 60

agtggtctgg tcttgtgaac ttggacatca caaataggac 100

<210> 236
<211> 100
<212> DNA
<213> Artificial Sequence

<220>
<223> Synthetic Polynucleotide

<400> 236

```
ctctcatgtt tttctggctt agtgaaaaaa cttcactaag tttttactta gtggatcttc    60

aaaggggcat gatacacaca aggggaaacc agtgaagacc                         100
```

<210> 237
<211> 95
<212> DNA
<213> Artificial Sequence

<220>
<223> Synthetic Polynucleotide

<400> 237

```
tacaactcca ataataatta caaacaagga atttcaacag tttcctacct tatattctgg    60

tctggtcttg tgaacttgga catcacaaat aggac                                95
```

<210> 238
<211> 76
<212> DNA
<213> Artificial Sequence

<220>
<223> Synthetic Polynucleotide

<400> 238

```
tacaactcca ataataatta caaacaagga atttcaacag tttcctactt gtgaacttgg    60

acatcacaaa taggac                                                     76
```

<210> 239
<211> 96
<212> DNA
<213> Artificial Sequence

<220>
<223> Synthetic Polynucleotide

<400> 239

```
cgttctgact cacaatctct catgtttttc tggcttagtg aaaaacatgg atcttcaaag    60

gggcatgata cacacaaggg gaaaccagtg aagacc                               96
```

<210> 240
<211> 97

<212> DNA
<213> Artificial Sequence

<220>
<223> Synthetic Polynucleotide

<400> 240

cgttctgact cacaatctct catgtttttc tggcttagtg aaaaaacgtg gatcttcaaa 60

ggggcatgat acacacaagg ggaaaccagt gaagacc 97

<210> 241
<211> 90
<212> DNA
<213> Artificial Sequence

<220>
<223> Synthetic Polynucleotide

<400> 241

tacaactcca ataataatta caaacaagga atttcaacag tttcctacct agtggtctgg 60

tcttgtgaac ttggacatca caaataggac 90

<210> 242
<211> 97
<212> DNA
<213> Artificial Sequence

<220>
<223> Synthetic Polynucleotide

<400> 242

tacaactcca ataataatta caaacaagga atttcaacag tttcctacct tatattctgt 60

ggtctggtct tgtgaacttg gacatcacaa ataggac 97

<210> 243
<211> 100
<212> DNA
<213> Artificial Sequence

<220>
<223> Synthetic Polynucleotide

<400> 243

cgttctgact cacaatctct catgtttttc tggcttagtg aaaaaactaa gtggatcttc 60

aaaggggcat gatacacaca aggggaaacc agtgaagacc 100

<210> 244
<211> 89
<212> DNA
<213> Artificial Sequence

<220>
<223> Synthetic Polynucleotide

<400> 244

tacaactcca ataataatta caaacaagga atttcaacag tttcctacta gtggtctggt 60

cttgtgaact tggacatcac aaataggac 89

<210> 245
<211> 87
<212> DNA
<213> Artificial Sequence

<220>
<223> Synthetic Polynucleotide

<400> 245

cgttctgact cacaatctct catgtttttc tggcttagtg aatcttcaaa ggggcatgat 60

acacacaagg ggaaaccagt gaagacc 87

<210> 246
<211> 86
<212> DNA
<213> Artificial Sequence

<220>
<223> Synthetic Polynucleotide

<400> 246

tacaactcca ataataatta caaacaagga atttcaacag tttccaagtg gtctggtctt 60

gtgaacttgg acatcacaaa taggac 86

<210> 247
<211> 97
<212> DNA
<213> Artificial Sequence

<220>
<223> Synthetic Polynucleotide

<400> 247

cgttctgact cacaatctct catgtttttc tggcttagtg aaaaaacgtg gatcttcaaa 60

ggggcatgat acacacaagg ggaaaccagt gaagacc 97

<210> 248
<211> 100
<212> DNA
<213> Artificial Sequence

<220>
<223> Synthetic Polynucleotide

<400> 248

acaactccaa taataattac aaacaaggaa tttcaacagt ttcctacctt atattcagat 60

agtggtctgg tcttgtgaac ttggacatca caaataggac 100

<210> 249
<211> 86
<212> DNA
<213> Artificial Sequence

<220>
<223> Synthetic Polynucleotide

<400> 249

tacaactcca ataataatta caaacaagga atttcaacag tttcctagtg gtctggtctt 60

gtgaacttgg acatcacaaa taggac 86

<210> 250
<211> 97
<212> DNA
<213> Artificial Sequence

<220>
<223> Synthetic Polynucleotide

<400> 250

tacaactcca ataataatta caaacaagga atttcaacag tttcctacct tatattctag 60

ggtctggtct tgtgaacttg gacatcacaa ataggac 97

<210> 251
<211> 87
<212> DNA
<213> Artificial Sequence

<220>
<223> Synthetic Polynucleotide

<400> 251

tacaactcca ataataatta caaacaagga atttcaacag tttcctacct tatattcagt 60

tgtgaacttg gacatcacaa ataggac 87

<210> 252
<211> 95
<212> DNA
<213> Artificial Sequence

<220>
<223> Synthetic Polynucleotide

<400> 252

tacaactcca ataataatta caaacaagga atttcaacag tttcctacct tatatagtgg 60

tctggtcttg tgaacttgga catcacaaat aggac 95

<210> 253
<211> 100
<212> DNA
<213> Artificial Sequence

<220>
<223> Synthetic Polynucleotide

<400> 253

tctgactcac aatctctcat gtttttctgg cttagtgaaa aacatgcccc gtggatcttc 60

aaaggggcat gatacacaca aggggaaacc agtgaagacc 100

<210> 254
<211> 91
<212> DNA
<213> Artificial Sequence

<220>
<223> Synthetic Polynucleotide

<400> 254

tacaactcca ataataatta caaacaagga atttcaacag tttcctacct tattggtctg 60

gtcttgtgaa cttggacatc acaaatagga c 91

<210> 255
<211> 78
<212> DNA
<213> Artificial Sequence

<220>
<223> Synthetic Polynucleotide

<400> 255

tacaactcca ataataatta caaacaagga atttcaacag tggtctggtc ttgtgaactt 60

ggacatcaca aataggac 78

<210> 256
<211> 100
<212> DNA
<213> Artificial Sequence

<220>
<223> Synthetic Polynucleotide

<400> 256

tctcatgttt ttctggctta gtgaaatcta agcttagtga aatctaagcc gtggatcttc 60

aaaggggcat gatacacaca aggggaaacc agtgaagacc 100

<210> 257
<211> 100
<212> DNA
<213> Artificial Sequence

<220>
<223> Synthetic Polynucleotide

<400> 257

tacaactcca ataataatta caaacaagga atttcaacag tttcctacct tatattctta 60

tatggtctgg tcttgtgaac ttggacatca caaataggac 100

<210> 258
<211> 97
<212> DNA
<213> Artificial Sequence

<220>
<223> Synthetic Polynucleotide

<400> 258

tacaactcca ataataatta caaacaagga atttcaacag tttcctacct tatatatagt 60

ggtctggtct tgtgaacttg gacatcacaa ataggac 97

<210> 259
<211> 82
<212> DNA
<213> Artificial Sequence

<220>
<223> Synthetic Polynucleotide

<400> 259

tacaactcca ataataatta caaacaagga atttcaacag tttcctacct tatattcaga 60

acttggacat cacaaatagg ac 82

<210> 260
<211> 100
<212> DNA
<213> Artificial Sequence

<220>
<223> Synthetic Polynucleotide

<400> 260

gttctgactc acaatctctc atgtttttct ggcttagtga aaaaaacgcc gtggatcttc 60

aaaggggcat gatacacaca aggggaaacc agtgaagacc 100

<210> 261
<211> 100
<212> DNA
<213> Artificial Sequence

<220>
<223> Synthetic Polynucleotide

<400> 261

taattacaaa caaggaattt caacagtttc ctaccttata ttcagtataa tatattcatt 60

agtggtctgg tcttgtgaac ttggacatca caaataggac 100

<210> 262
<211> 87
<212> DNA
<213> Artificial Sequence

<220>
<223> Synthetic Polynucleotide

<400> 262

cgttctgact cacaatctct catgtttttc tggcttagtg gatcttcaaa ggggcatgat 60

acacacaagg ggaaaccagt gaagacc 87

<210> 263
<211> 98
<212> DNA

<213> Artificial Sequence

<220>
<223> Synthetic Polynucleotide

<400> 263

tacaactcca ataataatta caaacaagga atttcaacag tttcctacct tatattctag 60

tggtctggtc ttgtgaactt ggacatcaca aataggac 98

<210> 264
<211> 100
<212> DNA
<213> Artificial Sequence

<220>
<223> Synthetic Polynucleotide

<400> 264

tacaactcca ataataatta caaacaagga atttcaacag tttcctacct tatattatat 60

agtggtctgg tcttgtgaac ttggacatca caaataggac 100

<210> 265
<211> 100
<212> DNA
<213> Artificial Sequence

<220>
<223> Synthetic Polynucleotide

<400> 265

taattacaaa caaggaattt caacagtttc ctaccttata ttcaggtagt gaatctgaat 60

agtggtctgg tcttgtgaac ttggacatca caaataggac 100

<210> 266
<211> 85
<212> DNA
<213> Artificial Sequence

<220>
<223> Synthetic Polynucleotide

<400> 266

tacaactcca ataataatta caaacaagga atttcaacag tttcctacct tctggtcttg 60

tgaacttgga catcacaaat aggac 85

<210> 267
<211> 100
<212> DNA
<213> Artificial Sequence

<220>
<223> Synthetic Polynucleotide

<400> 267

acaatctctc atgtttttct ggcttagtga atcttcaaag ggatcttcaa aaggatcttc 60

aaaggggcat gatacacaca aggggaaacc agtgaagacc 100

<210> 268
<211> 82
<212> DNA
<213> Artificial Sequence

<220>
<223> Synthetic Polynucleotide

<400> 268

tacaactcca ataataatta caaacaagga atttcaacag tttcctacct tatattgtga 60

acttggacat cacaaatagg ac 82

<210> 269
<211> 97
<212> DNA
<213> Artificial Sequence

<220>
<223> Synthetic Polynucleotide

<400> 269

tacaactcca ataataatta caaacaagga atttcaacag tttcctacct tatattcagt 60

ggtctggtct tgtgaacttg gacatcacaa ataggac 97

<210> 270
<211> 20
<212> DNA
<213> Artificial Sequence

<220>
<223> Synthetic Polynucleotide

<400> 270
gttgtgctca gtactgactt 20

<210> 271
<211> 20

<212> DNA
<213> Artificial Sequence

<220>
<223> Synthetic Polynucleotide

<400> 271
tttcagcttc caataaaaac 20

<210> 272
<211> 20
<212> DNA
<213> Artificial Sequence

<220>
<223> Synthetic Polynucleotide

<400> 272
attccacggg aaggagatct 20

<210> 273
<211> 20
<212> DNA
<213> Artificial Sequence

<220>
<223> Synthetic Polynucleotide

<400> 273
aggattgaag ctgacgttct 20

<210> 274
<211> 20
<212> DNA
<213> Artificial Sequence

<220>
<223> Synthetic Polynucleotide

<400> 274
ggcttagtga aaaaacgccg 20

<210> 275
<211> 20
<212> DNA
<213> Artificial Sequence

<220>
<223> Synthetic Polynucleotide

<400> 275
tcctacctta tattcagtag 20

<210> 276
<211> 20
<212> DNA
<213> Artificial Sequence

<220>
<223> Synthetic Polynucleotide

<400> 276
gctttgcccg aaccgcacga 20

<210> 277
<211> 20
<212> DNA
<213> Artificial Sequence

<220>
<223> Synthetic Polynucleotide

<400> 277
cctaggccag acctgcacga 20

<210> 278
<211> 20
<212> DNA
<213> Artificial Sequence

<220>
<223> Synthetic Polynucleotide

<400> 278
gctctggaag acccgcacca 20

<210> 279
<211> 20
<212> DNA
<213> Artificial Sequence

<220>
<223> Synthetic Polynucleotide

<400> 279
ccttatcaag acccacacca 20

<210> 280
<211> 20
<212> DNA
<213> Artificial Sequence

<220>
<223> Synthetic Polynucleotide

<400> 280
atccccctcc tcgtagcgca 20

<210> 281
<211> 20
<212> DNA
<213> Artificial Sequence

<220>
<223> Synthetic Polynucleotide

<400> 281
ctgctcctcc tcgtagcgct 20

<210> 282
<211> 20
<212> DNA
<213> Artificial Sequence

<220>
<223> Synthetic Polynucleotide

<400> 282
tgcgccctcc tcctagcgca 20

<210> 283
<211> 20
<212> DNA
<213> Artificial Sequence

<220>
<223> Synthetic Polynucleotide

<400> 283
ctcctcctcc gcgtagcgct 20

<210> 284
<211> 20
<212> DNA
<213> Artificial Sequence

<220>
<223> Synthetic Polynucleotide

<400> 284
ctgcccctcc tggtagcgcc 20

<210> 285
<211> 20
<212> DNA
<213> Artificial Sequence

<220>
<223> Synthetic Polynucleotide

<400> 285
aaccagctcc tcgtagctca 20

<210> 286
<211> 20
<212> DNA
<213> Artificial Sequence

<220>
<223> Synthetic Polynucleotide

<400> 286
accgccctcc tcctagctca 20

<210> 287
<211> 20
<212> DNA
<213> Artificial Sequence

<220>
<223> Synthetic Polynucleotide

<400> 287
agcccgctcc tcgtggggca 20

<210> 288
<211> 20
<212> DNA
<213> Artificial Sequence

<220>
<223> Synthetic Polynucleotide

<400> 288
gggaacaata aagaagcgct 20

<210> 289
<211> 20
<212> DNA
<213> Artificial Sequence

<220>
<223> Synthetic Polynucleotide

<400> 289
tggaaaaaca aagaagagct 20

<210> 290
<211> 20
<212> DNA
<213> Artificial Sequence

<220>
<223> Synthetic Polynucleotide

<400> 290
gggaagtata aggaagagct 20

<210> 291
<211> 20
<212> DNA
<213> Artificial Sequence

<220>
<223> Synthetic Polynucleotide

<400> 291
gtgagcaata aagcagccct 20

<210> 292
<211> 20
<212> DNA

<213> Artificial Sequence

<220>
<223> Synthetic Polynucleotide

<400> 292
tctagtccat cccccattac 20

<210> 293
<211> 20
<212> DNA
<213> Artificial Sequence

<220>
<223> Synthetic Polynucleotide

<400> 293
aatattccat tccccattac 20

<210> 294
<211> 20
<212> DNA
<213> Artificial Sequence

<220>
<223> Synthetic Polynucleotide

<400> 294
tgttgtccat acctcattac 20

<210> 295
<211> 20
<212> DNA
<213> Artificial Sequence

<220>
<223> Synthetic Polynucleotide

<400> 295
tctaggtcat gcaccattac 20

<210> 296
<211> 20
<212> DNA
<213> Artificial Sequence

<220>
<223> Synthetic Polynucleotide

<400> 296
cccattcctt cccccattac 20

<210> 297
<211> 20
<212> DNA
<213> Artificial Sequence

<220>

<223> Synthetic Polynucleotide

<400> 297
tccacaccct cccccattac 20

<210> 298
<211> 59
<212> DNA
<213> Artificial Sequence

<220>
<223> Synthetic Polynucleotide

<400> 298
acactctttc cctacacgac gctcttccga tctggtatct gtggttgatg cagttttcc 59

<210> 299
<211> 56
<212> DNA
<213> Artificial Sequence

<220>
<223> Synthetic Polynucleotide

<400> 299
acactctttc cctacacgac gctcttccga tctgcacaga gctgctgctt ggagtg 56

<210> 300
<211> 56
<212> DNA
<213> Artificial Sequence

<220>
<223> Synthetic Polynucleotide

<400> 300
acactctttc cctacacgac gctcttccga tctcgctttc ctgcctcagg atgaac 56

<210> 301
<211> 58
<212> DNA
<213> Artificial Sequence

<220>
<223> Synthetic Polynucleotide

<400> 301
acactctttc cctacacgac gctcttccga tctttggaag agaaggatct gctgaggc 58

<210> 302
<211> 56
<212> DNA
<213> Artificial Sequence

<220>
<223> Synthetic Polynucleotide

<400> 302

acactctttc cctacacgac gctcttccga tctgctaccc tccacaaagc acacac 56

<210> 303
<211> 58
<212> DNA
<213> Artificial Sequence

<220>
<223> Synthetic Polynucleotide

<400> 303
acactctttc cctacacgac gctcttccga tctccttcag aaacaatgtc ccaaatcg 58

<210> 304
<211> 55
<212> DNA
<213> Artificial Sequence

<220>
<223> Synthetic Polynucleotide

<400> 304
acactctttc cctacacgac gctcttccga tctagccttt ctgagagcgg gctag 55

<210> 305
<211> 56
<212> DNA
<213> Artificial Sequence

<220>
<223> Synthetic Polynucleotide

<400> 305
acactctttc cctacacgac gctcttccga tctgcctcct ctcatcctct cgcttc 56

<210> 306
<211> 58
<212> DNA
<213> Artificial Sequence

<220>
<223> Synthetic Polynucleotide

<400> 306
acactctttc cctacacgac gctcttccga tctgattggc tccaagcggc catcaaac 58

<210> 307
<211> 59
<212> DNA
<213> Artificial Sequence

<220>
<223> Synthetic Polynucleotide

<400> 307
acactctttc cctacacgac gctcttccga tctttgaact caaggctcag ccaacaggc 59

<210> 308

<211> 55
<212> DNA
<213> Artificial Sequence

<220>
<223> Synthetic Polynucleotide

<400> 308
acactctttc cctacacgac gctcttccga tctcaactca ggctggatgc atcgg 55

<210> 309
<211> 57
<212> DNA
<213> Artificial Sequence

<220>
<223> Synthetic Polynucleotide

<400> 309
acactctttc cctacacgac gctcttccga tctttggtgg ccgctgagtg tgtgtac 57

<210> 310
<211> 53
<212> DNA
<213> Artificial Sequence

<220>
<223> Synthetic Polynucleotide

<400> 310
acactctttc cctacacgac gctcttccga tctcagcatg ttgacatagc ggc 53

<210> 311
<211> 55
<212> DNA
<213> Artificial Sequence

<220>
<223> Synthetic Polynucleotide

<400> 311
acactctttc cctacacgac gctcttccga tcttgagagg agatgagtcg gggtc 55

<210> 312
<211> 56
<212> DNA
<213> Artificial Sequence

<220>
<223> Synthetic Polynucleotide

<400> 312
acactctttc cctacacgac gctcttccga tctgacgggt caaagcctca ggagag 56

<210> 313
<211> 57
<212> DNA
<213> Artificial Sequence

<220>
<223> Synthetic Polynucleotide

<400> 313
acactctttc cctacacgac gctcttccga tctttagctg cccagctcac agctacc 57

<210> 314
<211> 53
<212> DNA
<213> Artificial Sequence

<220>
<223> Synthetic Polynucleotide

<400> 314
acactctttc cctacacgac gctcttccga tctgagcccc aagagcgaga caa 53

<210> 315
<211> 54
<212> DNA
<213> Artificial Sequence

<220>
<223> Synthetic Polynucleotide

<400> 315
acactctttc cctacacgac gctcttccga tctggcgctc agaaggctgt gcag 54

<210> 316
<211> 56
<212> DNA
<213> Artificial Sequence

<220>
<223> Synthetic Polynucleotide

<400> 316
acactctttc cctacacgac gctcttccga tctcctaggt gacactggac ttttgc 56

<210> 317
<211> 58
<212> DNA
<213> Artificial Sequence

<220>
<223> Synthetic Polynucleotide

<400> 317
acactctttc cctacacgac gctcttccga tctaggttca cctcaggctg ctcagaag 58

<210> 318
<211> 57
<212> DNA
<213> Artificial Sequence

<220>
<223> Synthetic Polynucleotide

<400> 318
acactctttc cctacacgac gctcttccga tctcgtctct ctccatgtga gcttgtg 57

<210> 319
<211> 59
<212> DNA
<213> Artificial Sequence

<220>
<223> Synthetic Polynucleotide

<400> 319
acactctttc cctacacgac gctcttccga tctggaaaga tcatctgatc aggcccatc 59

<210> 320
<211> 57
<212> DNA
<213> Artificial Sequence

<220>
<223> Synthetic Polynucleotide

<400> 320
acactctttc cctacacgac gctcttccga tctcttgtta gggttggagg tctctgg 57

<210> 321
<211> 57
<212> DNA
<213> Artificial Sequence

<220>
<223> Synthetic Polynucleotide

<400> 321
acactctttc cctacacgac gctcttccga tctaaggccc gtaaagggca agttcag 57

<210> 322
<211> 57
<212> DNA
<213> Artificial Sequence

<220>
<223> Synthetic Polynucleotide

<400> 322
acactctttc cctacacgac gctcttccga tctggtgaca ggaagctgtc ggaacat 57

<210> 323
<211> 58
<212> DNA
<213> Artificial Sequence

<220>
<223> Synthetic Polynucleotide

<400> 323
acactctttc cctacacgac gctcttccga tctctcactg tgatctgaca ccaaacac 58

<210> 324
<211> 58
<212> DNA
<213> Artificial Sequence

<220>
<223> Synthetic Polynucleotide

<400> 324
acactctttc cctacacgac gctcttccga tctctagttg ccttcatgcc ttacagac 58

<210> 325
<211> 55
<212> DNA
<213> Artificial Sequence

<220>
<223> Synthetic Polynucleotide

<400> 325
acactctttc cctacacgac gctcttccga tctctgctcc cactccagac taccc 55

<210> 326
<211> 56
<212> DNA
<213> Artificial Sequence

<220>
<223> Synthetic Polynucleotide

<400> 326
gtgactggag ttcagacgtg tgctcttccg atctagtagt gaggccgctt ataacc 56

<210> 327
<211> 57
<212> DNA
<213> Artificial Sequence

<220>
<223> Synthetic Polynucleotide

<400> 327
gtgactggag ttcagacgtg tgctcttccg atcttaacaa ggagatgccc tggctgg 57

<210> 328
<211> 57
<212> DNA
<213> Artificial Sequence

<220>
<223> Synthetic Polynucleotide

<400> 328
gtgactggag ttcagacgtg tgctcttccg atctaggtca tgaaggcaaa ctcagcc 57

<210> 329
<211> 58
<212> DNA

<213> Artificial Sequence

<220>
<223> Synthetic Polynucleotide

<400> 329
gtgactggag ttcagacgtg tgctcttccg atctcacctg tcaaaagtcc tactccgg 58

<210> 330
<211> 58
<212> DNA
<213> Artificial Sequence

<220>
<223> Synthetic Polynucleotide

<400> 330
gtgactggag ttcagacgtg tgctcttccg atctcctgga gaccagcaag tattgtcc 58

<210> 331
<211> 57
<212> DNA
<213> Artificial Sequence

<220>
<223> Synthetic Polynucleotide

<400> 331
gtgactggag ttcagacgtg tgctcttccg atctgtcctc tgaaccccag ctgtaag 57

<210> 332
<211> 58
<212> DNA
<213> Artificial Sequence

<220>
<223> Synthetic Polynucleotide

<400> 332
gtgactggag ttcagacgtg tgctcttccg atctcaaaca gaggccaaag ggtgtccc 58

<210> 333
<211> 58
<212> DNA
<213> Artificial Sequence

<220>
<223> Synthetic Polynucleotide

<400> 333
gtgactggag ttcagacgtg tgctcttccg atctacagga ggtcgtggtg cagttctc 58

<210> 334
<211> 59
<212> DNA
<213> Artificial Sequence

<220>

<223> Synthetic Polynucleotide

<400> 334
gtgactggag ttcagacgtg tgctcttccg atctttgtgt gaggaacgtt gacgctacc 59

<210> 335
<211> 57
<212> DNA
<213> Artificial Sequence

<220>
<223> Synthetic Polynucleotide

<400> 335
gtgactggag ttcagacgtg tgctcttccg atctgagcgt aggtcctctg catggag 57

<210> 336
<211> 58
<212> DNA
<213> Artificial Sequence

<220>
<223> Synthetic Polynucleotide

<400> 336
gtgactggag ttcagacgtg tgctcttccg atctccacag aatgacagga acccatgg 58

<210> 337
<211> 57
<212> DNA
<213> Artificial Sequence

<220>
<223> Synthetic Polynucleotide

<400> 337
gtgactggag ttcagacgtg tgctcttccg atctactgag cagagcctag gaggcag 57

<210> 338
<211> 56
<212> DNA
<213> Artificial Sequence

<220>
<223> Synthetic Polynucleotide

<400> 338
gtgactggag ttcagacgtg tgctcttccg atcttgttgc cagatccaga ggcgtc 56

<210> 339
<211> 58
<212> DNA
<213> Artificial Sequence

<220>
<223> Synthetic Polynucleotide

<400> 339

gtgactggag ttcagacgtg tgctcttccg atctaactgg cccgagtagt cggagcag 58

<210> 340
<211> 59
<212> DNA
<213> Artificial Sequence

<220>
<223> Synthetic Polynucleotide

<400> 340
gtgactggag ttcagacgtg tgctcttccg atcttcctca gagtgtgtgg aagtgctgg 59

<210> 341
<211> 57
<212> DNA
<213> Artificial Sequence

<220>
<223> Synthetic Polynucleotide

<400> 341
gtgactggag ttcagacgtg tgctcttccg atctaagagc tcctagggga ggatcag 57

<210> 342
<211> 56
<212> DNA
<213> Artificial Sequence

<220>
<223> Synthetic Polynucleotide

<400> 342
gtgactggag ttcagacgtg tgctcttccg atcttggcag gagcacagcc taagga 56

<210> 343
<211> 57
<212> DNA
<213> Artificial Sequence

<220>
<223> Synthetic Polynucleotide

<400> 343
gtgactggag ttcagacgtg tgctcttccg atctacaccc gcctcggaga tcaacac 57

<210> 344
<211> 58
<212> DNA
<213> Artificial Sequence

<220>
<223> Synthetic Polynucleotide

<400> 344
gtgactggag ttcagacgtg tgctcttccg atctccaccc ctacatctca ccttgttg 58

<210> 345

<211> 55
<212> DNA
<213> Artificial Sequence

<220>
<223> Synthetic Polynucleotide

<400> 345
gtgactggag ttcagacgtg tgctcttccg atctgtgagg tttccacgtg ccagc 55

<210> 346
<211> 60
<212> DNA
<213> Artificial Sequence

<220>
<223> Synthetic Polynucleotide

<400> 346
gtgactggag ttcagacgtg tgctcttccg atctccaaca attccaggta tgaaactccc 60

<210> 347
<211> 59
<212> DNA
<213> Artificial Sequence

<220>
<223> Synthetic Polynucleotide

<400> 347
gtgactggag ttcagacgtg tgctcttccg atctctcccc acttgtaggt tcctaatcc 59

<210> 348
<211> 58
<212> DNA
<213> Artificial Sequence

<220>
<223> Synthetic Polynucleotide

<400> 348
gtgactggag ttcagacgtg tgctcttccg atctgtagag tgcctggtga agaatgtg 58

<210> 349
<211> 57
<212> DNA
<213> Artificial Sequence

<220>
<223> Synthetic Polynucleotide

<400> 349
gtgactggag ttcagacgtg tgctcttccg atcttccaga ctgttgttca gtcctgt 57

<210> 350
<211> 58
<212> DNA
<213> Artificial Sequence

<220>
<223> Synthetic Polynucleotide

<400> 350
gtgactggag ttcagacgtg tgctcttccg atctctctgg atttgcccac acctagtc 58

<210> 351
<211> 58
<212> DNA
<213> Artificial Sequence

<220>
<223> Synthetic Polynucleotide

<400> 351
gtgactggag ttcagacgtg tgctcttccg atctgcatgc ttgctttctg aaggtggc 58

<210> 352
<211> 59
<212> DNA
<213> Artificial Sequence

<220>
<223> Synthetic Polynucleotide

<400> 352
gtgactggag ttcagacgtg tgctcttccg atctttccaa gcaagtgagc ttcagcacc 59

<210> 353
<211> 54
<212> DNA
<213> Artificial Sequence

<220>
<223> Synthetic Polynucleotide

<400> 353
gtgactggag ttcagacgtg tgctcttccg atctccaccc atgacacagg aggg 54

<210> 354
<211> 57
<212> DNA
<213> Artificial Sequence

<220>
<223> Synthetic Polynucleotide

<400> 354
gtctcgtggg ctcggagatg tgtataagag acaggctacc ctccacaaag cacacac 57

<210> 355
<211> 59
<212> DNA
<213> Artificial Sequence

<220>
<223> Synthetic Polynucleotide

<400> 355
gtctcgtggg ctcggagatg tgtataagag acagttggaa gagaaggatc tgctgaggc 59

<210> 356
<211> 57
<212> DNA
<213> Artificial Sequence

<220>
<223> Synthetic Polynucleotide

<400> 356
tcgtcggcag cgtcagatgt gtataagaga cagcctggag accagcaagt attgtcc 57

<210> 357
<211> 57
<212> DNA
<213> Artificial Sequence

<220>
<223> Synthetic Polynucleotide

<400> 357
tcgtcggcag cgtcagatgt gtataagaga cagcacctgt caaaagtcct actccgg 57

<210> 358
<211> 59
<212> DNA
<213> Artificial Sequence

<220>
<223> Synthetic Polynucleotide

<220>
<221> misc_feature
<222> (6)..(24)
<223> n is a, c, g, or t

<220>
<221> misc_feature
<222> (51)..(58)
<223> n is a, c, g, or t

<400> 358
caccgnnnnn nnnnnnnnnn nnnngtttgg gtcttcgaga agacctattc nnnnnnnnc 59

<210> 359
<211> 59
<212> DNA
<213> Artificial Sequence

<220>
<223> Synthetic Polynucleotide

<220>
<221> misc_feature
<222> (6)..(13)
<223> n is a, c, g, or t

<220>
<221> misc_feature
<222> (40)..(58)
<223> n is a, c, g, or t

<400> 359
aattgnnnnn nnngaatagg tcttctcgaa gacccaaacn nnnnnnnnnn nnnnnnnnc 59

<210> 360
<211> 63
<212> DNA
<213> Artificial Sequence

<220>
<223> Synthetic Polynucleotide

<220>
<221> misc_feature
<222> (6)..(24)
<223> n is a, c, g, or t

<220>
<221> misc_feature
<222> (51)..(58)
<223> n is a, c, g, or t

<400> 360

**caccgnnnnn nnnnnnnnnn nnnngtttgg gtcttcgaga agacctattc nnnnnnnnca 60**

**att 63**

<210> 361
<211> 63
<212> DNA
<213> Artificial Sequence

<220>
<223> Synthetic Polynucleotide

<220>
<221> misc_feature
<222> (6)..(13)
<223> n is a, c, g, or t

<220>
<221> misc_feature
<222> (40)..(58)
<223> n is a, c, g, or t

<400> 361

**aattgnnnnn nnngaatagg tcttctcgaa gacccaaacn nnnnnnnnnn nnnnnnnncg 60**

**gtg 63**

<210> 362

<211> 23
<212> DNA
<213> Artificial Sequence

<220>
<223> Synthetic Polynucleotide

<400> 362
cctttgcaag acccgcacga tgg 23

<210> 363
<211> 23
<212> DNA
<213> Artificial Sequence

<220>
<223> Synthetic Polynucleotide

<400> 363
gctttgcccg aaccgcacga gag 23

<210> 364
<211> 23
<212> DNA
<213> Artificial Sequence

<220>
<223> Synthetic Polynucleotide

<400> 364
cctaggccag acctgcacga tgg 23

<210> 365
<211> 23
<212> DNA
<213> Artificial Sequence

<220>
<223> Synthetic Polynucleotide

<400> 365
gctctggaag acccgcacca ggg 23

<210> 366
<211> 23
<212> DNA
<213> Artificial Sequence

<220>
<223> Synthetic Polynucleotide

<400> 366
ccttatcaag acccacacca gag 23

<210> 367
<211> 23
<212> DNA
<213> Artificial Sequence

<220>
<223> Synthetic Polynucleotide

<400> 367
atccccctcc tcgtagcgca tgg 23

<210> 368
<211> 23
<212> DNA
<213> Artificial Sequence

<220>
<223> Synthetic Polynucleotide

<400> 368
tgcgccctcc tcctagcgca tgg 23

<210> 369
<211> 23
<212> DNA
<213> Artificial Sequence

<220>
<223> Synthetic Polynucleotide

<400> 369
ctgcccctcc tggtagcgcc tgg 23

<210> 370
<211> 23
<212> DNA
<213> Artificial Sequence

<220>
<223> Synthetic Polynucleotide

<400> 370
ctgctcctcc tcgtagcgct ggg 23

<210> 371
<211> 23
<212> DNA
<213> Artificial Sequence

<220>
<223> Synthetic Polynucleotide

<400> 371
aaccagctcc tcgtagctca ggg 23

<210> 372
<211> 23
<212> DNA
<213> Artificial Sequence

<220>
<223> Synthetic Polynucleotide

<400> 372
agcccgctcc tcgtggggca cgg 23

<210> 373
<211> 23
<212> DNA
<213> Artificial Sequence

<220>
<223> Synthetic Polynucleotide

<400> 373
accgccctcc tcctagctca ggg 23

<210> 374
<211> 23
<212> DNA
<213> Artificial Sequence

<220>
<223> Synthetic Polynucleotide

<400> 374
ctcctcctcc gcgtagcgct tgg 23

<210> 375
<211> 23
<212> DNA
<213> Artificial Sequence

<220>
<223> Synthetic Polynucleotide

<400> 375
ctcctcctcc tcctaccgca agg 23

<210> 376
<211> 23
<212> DNA
<213> Artificial Sequence

<220>
<223> Synthetic Polynucleotide

<400> 376
attcctctcc tggtaccgca agg 23

<210> 377
<211> 23
<212> DNA
<213> Artificial Sequence

<220>
<223> Synthetic Polynucleotide

<400> 377
ctcaccctcc tcctagcaca tag 23

<210> 378
<211> 23
<212> DNA
<213> Artificial Sequence

<220>
<223> Synthetic Polynucleotide

<400> 378
gggaacaata aagaagcgct tgg 23

<210> 379
<211> 23
<212> DNA
<213> Artificial Sequence

<220>
<223> Synthetic Polynucleotide

<400> 379
tggaaaaaca aagaagagct tgg 23

<210> 380
<211> 23
<212> DNA
<213> Artificial Sequence

<220>
<223> Synthetic Polynucleotide

<400> 380
gggaagtata aggaagagct cag 23

<210> 381
<211> 23
<212> DNA
<213> Artificial Sequence

<220>
<223> Synthetic Polynucleotide

<400> 381
gtgagcaata aagcagccct aag 23

<210> 382
<211> 23
<212> DNA
<213> Artificial Sequence

<220>
<223> Synthetic Polynucleotide

<400> 382
tctagtccat cccccattac tgg 23

<210> 383
<211> 23
<212> DNA

<213> Artificial Sequence

<220>
<223> Synthetic Polynucleotide

<400> 383
aatattccat tccccattac tgg 23

<210> 384
<211> 23
<212> DNA
<213> Artificial Sequence

<220>
<223> Synthetic Polynucleotide

<400> 384
tgttgtccat acctcattac tgg 23

<210> 385
<211> 23
<212> DNA
<213> Artificial Sequence

<220>
<223> Synthetic Polynucleotide

<400> 385
tctaggtcat gcaccattac tgg 23

<210> 386
<211> 23
<212> DNA
<213> Artificial Sequence

<220>
<223> Synthetic Polynucleotide

<400> 386
tccacaccct cccccattac tag 23

<210> 387
<211> 23
<212> DNA
<213> Artificial Sequence

<220>
<223> Synthetic Polynucleotide

<400> 387
cccattcctt cccccattac cag 23

## Description

### FIELD OF INVENTION

The invention relates to methods and compositions for the rapid generation of high-order combinations of genetic elements comprising a CRISPR guide sequence and scaffold sequence, and the identification of said genetic elements. The invention also relates to compositions of inhibitors that target epigenetic genes to inhibit cell proliferation and related methods.

### BACKGROUND

The clustered regularly interspaced short palindromic repeats (CRISPR) / Cas system was initially discovered in bacterial and archaeal species as a defense mechanism against foreign genetic material (e.g., plasmids and bacteriophages). The naturally occurring CRISPR / Cas systems rely on expression of three components: a guide RNA sequence that is complementary to a target sequence, scaffold RNA that aids in recruiting the third component, an endonuclease, to the site. Though in many bacterial and archaeal species CRISPR / Cas systems are used to degrade foreign genetic material, the system has been adapted for use in a wide variety of prokaryotic and eukaryotic organisms and have been used for many methods including gene knockout, mutagenesis, and expression activation or repression (Hsu, et al. Cell (2014) 157(6):1262-1278). In genetically engineered CRISPR / Cas systems, the requirement for three independent components can be circumvented by expression of a small guide RNA (sgRNA) that contains both the CRISPR guide RNA sequence for binding a target sequence and the scaffold RNA that together mimics the structure formed by the individual guide RNA sequence and scaffold sequence and is sufficient to recruit the endonuclease to the appropriate target site (Jinek, et al. Science (2012) 337(6096):816-821).

Yuexin Zhou et al., Nature, vol. 509, no. 7501, 9 April 2014 (2014-04-09), pages 487-491, concerns high-throughput screening of a CRISPR/Cas9 library for functional genomics in human cells.

T. Wang et al., Science, vol. 343, no. 6166, 12 December 2013, pages 80-84, concerns genetic Screens in Human Cells Using the CRISPR-Cas9 System.

The present invention is defined by the claims.

### SUMMARY

Generation of vectors and genetic elements for the expression of multiple CRISPR systems comprising more than one sgRNA (guide sequence and scaffold sequence) is very laborious, and the complexity of libraries of CRISPR systems built using traditional cloning methods is very limited. The methods described herein allow for the generation of vectors comprising multiple sgRNAs each comprising a CRISPR guide sequences and a scaffold sequence, and concatenated barcode elements that can be detected and used as indicators of the identity of the CRISPR guide sequence(s). The methods also provide simple and rapid generation of highly complex libraries of vectors.

Aspects of the present description provide genetic constructs comprising a first DNA element comprising a CRISPR guide sequence and a scaffold sequence; a first compatible end element and a second compatible end element flanking the first DNA element, wherein the first and second compatible end elements are capable of annealing to each other; a barcode element; a third compatible end element and a fourth compatible end element flanking the barcode element, wherein the third and fourth compatible end elements are capable of annealing to each other but are not capable of annealing to the first or second compatible end elements; and a separation site located between the fourth compatible end element and the first compatible end element, wherein the DNA element, first compatible end element, and second compatible end element are on one side of the separation site, and the barcode element, the third compatible end element, and the fourth compatible end element are on the other side of the separation site.

In some embodiments, the genetic construct further comprises a promoter element upstream of the first DNA element.

Aspects provide vectors comprising any of the genetic constructs provided herein.

Other aspects provide genetic constructs comprising a plurality of DNA elements, wherein each DNA element of the plurality of DNA element comprises a CRISPR guide sequence and a scaffold sequence; a first compatible end element and a second compatible end element flanking the plurality of DNA elements, wherein the first and second compatible end elements are capable of annealing to each other; a plurality of barcode elements; a third compatible end element and a fourth compatible end element flanking the plurality of barcode elements, wherein the third and fourth compatible end elements are capable of annealing to each other but are not capable of annealing to the first or second compatible end elements; and a separation site located between the plurality of DNA elements and the plurality of barcode elements.

Other aspects provide vectors comprising any of the genetic constructs provided herein and a promoter sequence located upstream of each of the CRISPR guide sequences.

Yet other aspects provide methods for generating a combinatorial vector, comprising (a) providing a vector containing a first genetic construct comprising a CRISPR guide sequence; a second compatible end element and a first recognition site for a first restriction enzyme flanking the CRISPR guide sequence; a barcode element; and a third compatible end element and a second recognition site for a second restriction enzyme flanking the barcode element; (b) cleaving the first genetic construct at the first recognition site, resulting in a fifth compatible end element, and cleaving the vector at the second recognition site, resulting in a sixth compatible end element; (c) providing a scaffold element comprising a scaffold sequence; a separation site comprising a first compatible end element and a fourth compatible end element; and a seventh compatible end element and an eighth compatible end element flanking the scaffold element, wherein the seventh compatible end element is capable of annealing to the fifth compatible end element and the eighth compatible end element is capable of annealing to the sixth compatible end element; and (d) annealing the scaffold element to the cleaved first genetic construct, wherein the annealing occurs at compatible end elements within the vector and the scaffold element that are capable of annealing to each other, and wherein after the annealing, the scaffold element is integrated between the CRISPR guide sequence and the barcode element, and wherein the separation site is located between the scaffold sequence and the barcode element, creating a combinatorial vector.

In some embodiments, the method further comprises (a) providing any of the combinatorial vector as described herein; (b) cleaving the vector at the separation site within the scaffold element, resulting in a first compatible end element and a fourth compatible end element; (c) providing a second genetic construct comprising a CRISPR guide sequence; a scaffold sequence; a barcode element; and a second compatible end element and a third compatible end element flanking the second genetic construct, wherein the second compatible end element of the second genetic construct is capable of annealing with the first compatible end element of the vector and the third compatible end element of the second genetic construct is capable of annealing to the fourth compatible end element of the vector; and (d) annealing the second genetic construct to the cleaved vector, wherein the annealing occurs at compatible end elements within the second genetic construct and the vector that are capable of annealing to each other, and wherein after annealing, the second genetic construct is integrated into the vector, creating a combinatorial vector comprising concatenated barcode elements and concatenated CRISPR guide and scaffold sequences.

In some embodiments, the combinatorial vector further comprises one or more promoter upstream of the CRISPR guide sequence. In some embodiments, the method is iterative. In some embodiments, the first recognition site and the second recognition sites have the same recognition site sequence, and the first restriction enzyme and the second restriction enzyme are the same restriction enzymes.

Other aspects of the description provide genetic constructs comprising at least two CRISPR guide sequences; a barcode element; and a restriction recognition site located between each CRISPR guide sequence and between the barcode element and the CRISPR guide sequence nearest to the barcode element.

Other aspects provide genetic constructs comprising a plurality of DNA elements, each comprising a CRISPR guide sequence and a scaffold sequence; a barcode element; and a promoter sequence located upstream of each of the DNA elements of the plurality of DNA elements. In some embodiments, the barcode element is located at the 5' end of the genetic construct. In some embodiments, the barcode element is located at the 3' end of the genetic construct.

Other aspects provide vectors comprising any of the genetic constructs described herein.

Yet other aspects provide methods for generating a combinatorial vector, comprising (a) providing a vector comprising: a plurality of CRISPR guide sequences; a barcode element, wherein the barcode element is located downstream of the plurality of CRISPR guide sequences; optionally a promoter sequence located upstream of at least one of the plurality of CRISPR guide sequences; and a plurality of recognition sites for a plurality of restriction enzymes, wherein each of the plurality of recognition sites is located downstream of one of the plurality of CRISPR guide sequences; (b) cleaving the vector at at least one of the plurality of recognition sites with at least one of the plurality of restriction enzymes, resulting in a first compatible end element and a second compatible end element; (c) providing a first scaffold element comprising: a scaffold sequence, optionally a promoter sequence, and a third compatible end element and fourth compatible end element flanking the first scaffold element, wherein the third compatible end element is capable of annealing to the first compatible end element of the cleaved vector and the fourth compatible end element is capable of annealing to the second compatible end element of the cleaved vector; and (d) annealing the first scaffold element to the cleaved vector, wherein the annealing occurs at compatible end elements within the first scaffold element and the cleaved vector, and wherein after annealing, the first scaffold element is integrated downstream of one of the plurality of CRISPR guide sequences, thereby producing a combinatorial vector. In some embodiments, the method is iterative.

Other aspects provide methods for generating a combinatorial vector comprising (a) providing a vector comprising: a plurality of CRISPR guide sequences, a barcode element, wherein the barcode element is located upstream of the plurality of CRISPR guide sequences; optionally a promoter sequence located upstream of at least one of the plurality of CRISPR guide sequences; and a plurality of recognition sites for a plurality of restriction enzymes, wherein each of the plurality of recognition sites is located upstream of one of the plurality of CRISPR guide sequences; (b) cleaving the vector at least one of the plurality of recognition sites with at least one of the plurality of restriction enzymes, resulting in a first compatible end element and a second compatible end element; (c) providing a first scaffold element comprising optionally a scaffold sequence, a promoter sequence, and a third compatible end element and fourth compatible end element flanking the first scaffold element, wherein the third compatible end element is capable of annealing to the first compatible end element of the cleaved vector and the fourth compatible end element is capable of annealing to the second compatible end element of the cleaved vector; (d) annealing the first scaffold element to the cleaved vector, wherein the annealing occurs at compatible end elements within the first scaffold element and the cleaved vector, and wherein after annealing, the first scaffold element is integrated upstream of one of the plurality of CRISPR guide sequences, thereby producing a combinatorial vector. In some embodiments, the method is iterative.

Aspects of the escription provide compositions comprising two or more inhibitors targeting two or more epigenetic genes selected from the combinations of epigenetic genes set forth in Table 2. In some embodiments, each of the two or more inhibitors reduce or prevent expression of an epigenetic gene or reduce or prevent activity of a protein encoded by the epigenetic gene. In some embodiments, each of the inhibitors is selected from the group consisting of a CRISPR guide sequence and scaffold sequence; an shRNA; and a small molecule. In some embodiments, at least one of the inhibitors is a CRISPR guide sequence and scaffold sequence; and the composition further comprises or encodes a Cas9 endonuclease. In some embodiments, the CRISPR guide sequence or shRNA is expressed from a recombinant expression vector. In some embodiments, the combination of epigenetic genes comprises BRD4 and KDM4C or BRD4 and KDM6B. In some embodiments, the inhibitor of BRD4 is JQ1 ((6S)-4-(4-Chlorophenyl)-2,3,9-trimethyl-6H-thieno[3,2-f][1,2,4]triazolo[4,3-a][1,4]diazepine-6-acetic acid 1,1-dimethylethyl ester). In some embodiments, the inhibitor of KDM4C is SD70 (N-(furan-2-yl(8-hydroxyquinolin-7-yl)methyl)isobutyramide). In some embodiments, the inhibitor of KDM6B is GSK-J4 (ethyl 3-((6-(4,5-dihydro-1H-benzo[d]azepin-3(2H)-yl)-2-(pyridin-2-yl)pyrimidin-4-yl)amino)propanoate, monohydrochloride).

Other aspects provide methods for reducing proliferation of a cell, comprising contacting the cell with a combination of two or more inhibitors targeting two or more epigenetic genes selected from the combinations of epigenetic genes set forth in Table 2. In some embodiments, the cell is a cancer cell. In some embodiments, the cancer cell is an ovarian cancer cell. In some embodiments, each of the two or more inhibitors reduce or prevent expression of an epigenetic gene or reduce or prevent activity of a protein encoded by the epigenetic gene. In some embodiments, each of the inhibitors is selected from the group consisting of a CRISPR guide sequence and scaffold sequence; an shRNA; and a small molecule. In some embodiments, at least one of the inhibitors is a CRISPR guide sequence and scaffold sequence; and the composition further comprises or encodes a Cas9 endonuclease. In some embodiments, the CRISPR guide sequence or shRNA is expressed from a recombinant expression vector. In some embodiments, the combination of epigenetic genes comprises BRD4 and KDM4C or BRD4 and KDM6B. In some embodiments, the inhibitor of BRD4 is JQ1 ((6S)-4-(4-Chlorophenyl)-2,3,9-trimethyl-6H-thieno[3,2-f][1,2,4]triazolo[4,3-a][1,4]diazepine-6-acetic acid 1,1-dimethylethyl ester). In some embodiments, the inhibitor of KDM4C is SD70 (N-(furan-2-yl(8-hydroxyquinolin-7-yl)methyl)isobutyramide). In some embodiments, the inhibitor of KDM6B is GSK-J4 (ethyl 3-((6-(4,5-dihydro-1H-benzo[d]azepin-3(2H)-yl)-2-(pyridin-2-yl)pyrimidin-4-yl)amino)propanoate, monohydrochloride).

Other aspects provide methods for treating cancer in a subject comprising administering to the subject a combination of two or more inhibitors targeting two or more epigenetic genes selected from the combinations of epigenetic genes set forth in Table 2, wherein each of the two or more inhibitors are administered in an effective amount. In some embodiments, each of the inhibitors is selected from the group consisting of a CRISPR guide sequence, an shRNA, and a small molecule. In some embodiments, the effective amount of each of the two or more inhibitors administered in the combination is less than the effective amount of the inhibitor when not administered in the combination. In some embodiments, each of the two or more inhibitors reduce or prevent expression of an epigenetic gene or reduce or prevent activity of a protein encoded by the epigenetic gene. In some embodiments, each of the inhibitors is selected from the group consisting of a CRISPR guide sequence and scaffold sequence; an shRNA; and a small molecule. In some embodiments, at least one of the inhibitors is a CRISPR guide sequence and scaffold sequence; and the composition further comprises or encodes a Cas9 endonuclease. In some embodiments, the CRISPR guide sequence or shRNA is expressed from a recombinant expression vector. In some embodiments, the combination of epigenetic genes comprises BRD4 and KDM4C or BRD4 and KDM6B. In some embodiments, the inhibitor of BRD4 is JQ1 ((6S)-4-(4-Chlorophenyl)-2,3,9-trimethyl-6H-thieno[3,2-f][1,2,4]triazolo[4,3-a][1,4]diazepine-6-acetic acid 1,1-dimethylethyl ester). In some embodiments, the inhibitor of KDM4C is SD70 (N-(furan-2-yl(8-hydroxyquinolin-7-yl)methyl)isobutyramide). In some embodiments, the inhibitor of KDM6B is GSK-J4 (ethyl 3-((6-(4,5-dihydro-1H-benzo[d]azepin-3(2H)-yl)-2-(pyridin-2-yl)pyrimidin-4-yl)amino)propanoate, monohydrochloride).

Other aspects provide methods for identifying a combination of inhibitors of epigenetic genes that reduces proliferation of a cell comprising contacting a first population of cells and a second population of cells with a plurality of combinations of two or more CRISPR guide sequences and scaffold sequences and a Cas9 endonuclease; culturing the first population of cells and the second population of cells such that the second population of cells is cultured for a longer duration compared to the first population of cells; identifying the combinations of two or more CRISPR guide sequences and scaffold sequences in the first population of cells and the combinations of two or more CRISPR guide sequences and scaffold sequences in the second population of cells; comparing the abundance of each combination of two or more CRISPR guide sequences and scaffold sequences in the first population of cells to the abundance of each combination of two or more CRISPR guide sequences and scaffold sequences in the second population of cells; and identifying a combination of two or more CRISPR guide sequences and scaffold sequences that is absent from or in reduced abundance in the second population of cells but present in or in increased abundance in the first population of cells as a combination of CRISPR guide sequences and scaffold sequences that reduces cell proliferation.

Yet other aspects provide methods for identifying a combination of genes to be inhibited to reduce proliferation of a cell comprising contacting a first population of cells and a second population of cells with a plurality of combinations of two or more CRISPR guide sequences and scaffold sequences and a Cas9 endonuclease; culturing the first population of cells and the second population of cells such that the second population of cells is cultured for a longer duration compared to the first population of cells; identifying the combinations of two or more CRISPR guide sequences and scaffold sequences in the first population of cells and the combinations of two or more CRISPR guide sequences and scaffold sequences in the second population of cells; comparing the abundance of each combination of two or more CRISPR guide sequences and scaffold sequences in the first population of cells to the abundance of each combination of two or more CRISPR guide sequences and scaffold sequences in the second population of cells; and identifying a combination of two or more CRISPR guide sequences and scaffold sequences that is absent from or in reduced abundance in the second population of cells but present in or in increased abundance in the first population of cells as a combination of genes to be inhibited to reduce proliferation.

These and other aspects of the invention, as well as various embodiments thereof, will become more apparent in reference to the drawings and detailed description of the invention.

Each of the limitations of the invention can encompass various embodiments of the invention. It is, therefore, anticipated that each of the limitations of the invention involving any one element or combination of elements can be included in each aspect of the invention. This invention is not limited in its application to the details of construction and the arrangement of components set forth in the following description or illustrated in the drawings. The invention is capable of other embodiments and of being practiced or of being carried out in various ways.

## BRIEF DESCRIPTION OF THE DRAWINGS

The accompanying drawings are not intended to be drawn to scale. For purposes of clarity, not every component may be labeled in every drawing. In the drawings:

FIG. 1 presents a schematic depicting a non-limiting embodiment of the invention. In steps 1 and 2, an oligonucleotide library is synthesized and corresponding oligonucleotide pairs are annealed together. Each oligonucleotide contains a CRISPR guide sequence, two BbsI restriction recognition sites, and a barcode element. In step 3, the oligonucleotides are ligated into a storage vector in a one-pot ligation reaction resulting in a vector containing the oligonucleotide. In step 4, the vector is digested at the BbsI restriction recognition sites to allow for insertion of a scaffold sequence as well as a separation site formed by BamHI and EcoRI restriction recognition sites. The barcoded guide RNA library can be iteratively digested at the separation site for insertion of additional elements containing a CRISPR guide sequence, scaffold sequence, separation site, and barcode element, resulting in a complex guide RNA library with concatenated barcode elements. The sequences, from top to bottom, correspond to SEQ ID NOs: 364-366.

FIGs. 2A and 2B present schematics depicting non-limiting embodiments of the invention. In step 1 of FIG. 2A, oligonucleotides are synthesized, each containing multiple CRISPR guide sequences and a single barcode element downstream of the CRISPR guide sequences. Restriction recognition sites (RE) are present following each of the CRISPR guide sequences. In step 2, the pooled synthesized oligonucleotides are ligated into a destination vector in a one-pot ligation reaction. As shown in step 3, the vector can be sequentially digested at each of the restriction recognition sites following each CRISPR guide sequence with different restriction enzymes, allowing for insertion of a scaffold element, and in some cases a promoter element to drive expression of a downstream CRISPR guide sequence, resulting in a barcoded combinatorial guide RNA library encoding multiple CRISPR guide sequences and scaffold sequences with a single barcode element. In step 1 of FIG. 2B, oligonucleotides are synthesized, each containing multiple CRISPR guide sequences and a single barcode element upstream of the CRISPR guide sequences. Restriction recognition sites (RE) are present following each of the CRISPR guide sequences. In step 2, the pooled synthesized oligonucleotides are ligated into a destination vector in a one-pot ligation reaction. As shown in step 3, the vector can be sequentially digested at each of the restriction recognition sites following each CRISPR guide sequence with different restriction enzymes, allowing for insertion of a scaffold element, and in some cases a promoter element to drive expression of a downstream CRISPR guide sequence, resulting in a barcoded combinatorial guide RNA library encoding multiple CRISPR guide sequences and scaffold sequences with a single barcode element.

FIGs. 3A-3D show generation of a high-coverage combinatorial gRNA library and efficient delivery of the library to human cells. FIG. 3A presents the cumulative distributions of barcode reads for a one-wise gRNA library in the plasmid pool extracted from *E. coli* indicating full coverage for all expected combinations. FIG. 3B presents the two-wise gRNA library in both the plasmid pool and the lentivirus-infected OVCAR8-ADR-Cas9 cell pool indicating near-full coverage for all expected combinations. Most barcoded gRNA combinations were detected within a 5-fold range from the mean barcode reads per combination (highlighted by the shaded areas and indicated by the arrows). FIG. 3C shows a high correlation between barcode representations ($log_2$ values of normalized barcode counts) within the plasmid pool and the infected OVCAR8-ADR-Cas9 cell pool, indicating efficient lentiviral delivery of the two-wise library into human cells. FIGs. 3D shows high reproducibility for barcode representations between two biological replicates in OVCAR8-ADR-Cas9 cells cultured for 5-days post-infection with the two-wise gRNA library. R is the Pearson correlation coefficient.

FIGs. 4A-4C show identification of gRNA combinations that inhibit cancer cell proliferation using a high-throughput screening. FIG. 4A shows a schematic of the high-throughput screen in which OVCAR8-ADR-Cas9 cells were infected with the barcoded two-wise gRNA library and cultured for 15 or 20 days. Barcode representations within the cell pools were identified and quantified using Illumina HiSeq and compared between the two pools. FIG. 4B (right panel) shows two-wise gRNA combinations that were found to modulate cell proliferation ranked by $log_2$ ratios between the normalized barcode count in 20-day versus 15-day cultured cells. FIG. 4B (left panel) shows the same gRNAs paired with control gRNAs. Combinations with control gRNA pairs are highlighted in open triangles. The anti-proliferative effects of gRNA combinations that were confirmed in another biological replicate are highlighted in open circles (see FIG. 12). The labeled gRNA combinations were further validated. FIG. 4C presents validation of two-wise combinations that modulate cancer cell proliferation. OVCAR8-ADR-Cas9 cell populations were infected with the indicated two-wise gRNA combinations and cultured for 15 days. Equal numbers of cells were then re-plated and cultured for additional time periods as indicated. Cell viability was measured using the MTT assay and characterized by absorbance measurements ($OD_{570}$ - $OD_{650}$) (n = 3). Data represent the mean ± standard deviation (SD).

FIGs. 5A-5D show combinatorial inhibition of KDM4C and BRD4 or KDM6B and BRD4 inhibits human ovarian cancer cell growth. FIG. 5A shows the fold change in cell viability of OVCAR8-ADR-Cas9 cells infected with lentiviruses expressing the indicated single or combinatorial gRNAs relative to cells infected with lentiviruses expressing control gRNA. Cells were cultured for 15 days, then equal numbers of infected cells were then re-plated and cultured for 5 additional days. FIG. 5B shows the fold change in cell viability of OVCAR8-ADR cells co-infected with lentiviruses expressing the indicated shRNAs relative to cells infected with lentiviruses expressing control shRNA. Cells were cultured for 9 days, then equal numbers of infected cells were re-plated and cultured for 4 additional days. FIG. 5C shows the percentage of cell growth inhibition of OVCAR8-ADR cells treated with SD70 and JQ1 at the indicated concentrations for 5 days relative control cells that did not receive drug. The calculated excess inhibition over the predicted Bliss independence and HSA models are also shown for the combination of SD70 and JQ1 (center and right panels). FIG. 5D shows the percentage of cell growth inhibition of OVCAR8-ADR cells treated with GSK-J4 and JQ1 at the indicated concentrations for 7 days relative to control cells that did not receive drug. The calculated excess inhibition over the predicted Bliss independence and HSA models are also shown for the combination of GSK-J4 and JQ1 (center and right panels). Cell viability was determined by MTT assay. Data represent mean ± SD (n =3 for (FIG. 5A); n = 6 for (FIGs. 5B-5D)). The asterisk (*P < 0.05) and hash (#P < 0.05) represent significant differences between the indicated samples and between drug-treated versus no drug control samples, respectively.

FIGs. 6A-6E show lentiviral delivery of combinatorial gRNA expression constructs provides efficient target gene repression. FIG. 6A presents a schematic of a strategy for testing lentiviral combinatorial gRNA expression constructs in human cells. Lentiviruses were generated that contained genes encoding RFP and GFP expressed under control of UBC and CMV promoters, respectively, and tandem U6 promoter-driven expression cassettes of gRNAs targeting RFP (RFP-sg1 or RFP-sg2) and GFP (GFP-sg1) sequences. The lentiviruses were used to infect OVCAR8-ADR or OVCAR8-ADR-Cas9 cells, and GFP and RFP expression were assessed using flow cytometry and fluorescence microscopy. FIG. 6B shows flow cytometry scatter plots assessing GFP and RFP expression in cells infected with lentiviruses encoding the indicated gRNA expression constructs for 4 days. Lentiviruses encoding combinatorial gRNA expression constructs reduced the percentage of cells positive for RFP and GFP fluorescence in OVCAR8-ADR-Cas9 cells but not OVCAR8-ADR cells. FIG. 6C presents the percentage of cells positive for GFP (left columns) and RFP (right columns) at day 4 post-infection with lentiviruses encoding the indicated gRNA expression constructs. FIG. 6D presents the percentage of cells positive for GFP (left columns) and RFP (right columns) at day 8 post-infection with lentiviruses encoding the indicated gRNA expression constructs. Limited cross-reactivity between gRNAs targeting RFP and GFP was detected. Data in FIG. 6B represents flow cytometry measurements for cells infected for 4 days, while quantifications in FIGs. 6C and 6D represent the mean ± standard deviation (n = 3). FIG. 6E presents representative fluorescence micrographs demonstrating that combinatorial gRNA expression constructs effectively repressed RFP and GFP fluorescence levels in OVCAR8-ADR-Cas9 cells but not in OVCAR8-ADR cells at day 3 post-infection.

FIGs. 7A-7C show the cleavage efficiency of gRNAs of targeted genes in OVCAR8-ADR-Cas9 cells. FIG. 7A presents a summary table showing the indel percentages detected in OVCAR8-ADR-Cas9 cells, using the Surveyor assay. Cells were infected with 8 different gRNAs randomly-selected from the screening library for 8 or 12 days. The expected sizes of the uncleaved and cleaved PCR products detected for the Surveyor assay are listed in base pairs. FIGs. 7B and 7C present agarose gels showing the Surveyor assay results for DNA cleavage efficiency in OVCAR8-ADR-Cas9 cells that were either uninfected or infected with the indicated gRNAs for 8 or 12 days.

FIGs. 8A and 8B show the cleavage efficiency of dual-gRNA expression constructs at targeted genes in OVCAR8-ADR-Cas9 cells. FIG. 8A presents the expected sizes of the uncleaved and cleaved PCR products detected for the Surveyor assay listed in base pairs (upper panel). The agarose gel shows the indel percentages detected in OVCAR8-ADR-Cas9 cells infected with the indicated single or dual-gRNA expression constructs for 12 days using the Surveyor assay (lower panel). FIG. 8B is an immunoblot analysis showing protein levels in OVCAR8-ADR-Cas9 cells that were either infected with vector control, or the indicated single- or dual- gRNA constructs for 15 days.

FIGs. 9A-9C present DNA alignments of targeted alleles for single-cell-derived OVCAR8-ADR-Cas9 clones infected with dual-gRNA expression constructs. FIG. 9A shows alignments of sequences from OVCAR8-ADR-Cas9 cells infected with lentiviruses expressing sgRNAs targeting BMI1 and PHF8. The sequences, from top to bottom, correspond to SEQ ID NOs: 203, 204, 203, 203, 204, 204, 203, 205, 206, 206, 203, 203, 204, 207, 208, 209, 204, 210, 208, 208, 210, 210, 203, 219, 206, 206, 208, 208, 220, 220, 221, 222, 204, 204, 223, 224, 204, 204, 203, 203, 204, and 204, respectively. FIG. 9B shows alignments of sequences from OVCAR8-ADR-Cas9 cells infected with lentiviruses expressing gRNAs targeting BRD4 and KDM4C. The OVCAR8-ADR-Cas9 cells were infected with lentiviruses for 12 days and plated as single cells. Genomic DNA for each single cell-expanded clone was extracted. The targeted alleles were amplified by PCR and inserted into the TOPO vector by TA cloning for Sanger sequencing. The sequences for the two alleles of each clone are shown. Mutations and insertions of nucleotides are in bold, while deletions are indicated as "-". Wildtype (WT) sequences for the targeted genes are shown as references, with the 20 bp gRNA target underlined and PAM sequences in bold italics. The sequences, from top to bottom, correspond to SEQ ID NOs: 211,212, 211, 211, 213,213,211,211,214,214,211,215,212,216,211,211,217,218,211,211,212,212, 211,211,225,226,211,211,226,227,211,211,212,212,211,228,226,229,211,211, 229, 226, 211, 211, 230, and 226, respectively. FIG. 9C is a Venn diagram showing the frequency of single- and dual- gene-edited cells. OVCAR8-ADR-Cas9 cells harboring the indicated dual-gRNA expression constructs were plated as single cells by FACS. The targeted alleles were sequenced from 40 whole genome-amplified single cells with Illumina MiSeq. 75% (i.e., 30/40) and 80% (i.e., 32/40) of the single cells harbored at least one mutant allele at the targeted BMI1 and PHF8 loci, respectively. 62.5% (i.e., 25/40) of the single cells contained at least one mutant allele in both BMI1 and PHF8 genes. The sequences for the two alleles of each single cell are shown in Table 6. Similar mutant allele frequencies determined from the single-cell-derived clones by Sanger sequencing (FIG. 9B) and whole-genome-amplified single cells by Illumina MiSeq (FIG. 9C) were observed.

FIGs. 10A and 10B show high reproducibility of barcode quantitation between biological replicates for the combinatorial gRNA screen. FIG. 10A presents a scatter plot comparing barcode representations ($log_2$ number of normalized barcode counts) between two biological replicates for OVCAR8-ADR-Cas9 cells cultured for 15 days post-infection with the two-wise gRNA library. FIG. 10B presents a scatter plot comparing barcode representations ($log_2$ number of normalized barcode counts) between two biological replicates for OVCAR8-ADR-Cas9 cells cultured for 20 days post-infection with the two-wise gRNA library. R is the Pearson correlation coefficient.

FIG. 11 shows consistent fold-changes in barcodes quantitation among the same gRNA combinations arranged in different orders within the expression constructs. The coefficient of variation (CV; defined as SD /mean of the fold changes of normalized barcode counts for 20-day versus 15-day cultured OVCAR8-ADR-Cas9 cells) was determined for each two-wise gRNA combination arranged in different orders (i.e., sgRNA-A + sgRNA-B and sgRNA-B + sgRNA-A). Over 82% of the two-wise gRNA combinations had a CV of <0.2, and 95% of two-wise gRNA combinations had a CV of <0.4, respectively, in the cell-proliferation screen.

FIGs. 12A-12C show biological replicates for the combinatorial screen identifying gRNA pairs that inhibit cancer cell proliferation. FIG. 12A shows $log_2$ fold change for OVCAR8-ADR-Cas9 cells infected with the same two-wise gRNA library used in FIG. 4B. Combinations of guide RNA pairs (right panel) and their gRNA + control counterparts (i.e., gene-targeting gRNA + control gRNA; left panel) that modulated proliferation were ranked by the $log_2$ ratios of the normalized barcode count for 20-day compared to 15-day cultured cells. The anti-proliferative effects of gRNA combinations that were confirmed in another biological replicate are highlighted in open circles (FIG. 4B), while combinations with control gRNA pairs are highlighted in open triangles. Labeled gRNA combinations were further validated in FIG. 4C. FIG. 12B presents a scatter plot showing the $log_2$ ratios of the normalized barcode counts for 20-day versus 15-day cultured cell between two biological replicates of OVCAR8-ADR-Cas9 cells infected with the two-wise gRNA library. FIG. 12C shows the frequency distribution of $log_2$ ratios for the gRNA combinations in the pooled screen. $Log_2$ ratios shown were calculated form the mean of two biological replicates.

FIG. 13 shows high consistency between individual hits in the pooled screen and in the validation data. For each two-wise gRNA combination, the fold-change in the normalized barcode count for 20-day versus 15-day cultured cells, obtained from the pooled screening data ('Screen phenotype') was plotted against its relative cell viability compared to the vector control determined from the individual cell-proliferation assays ('Validation phenotype') (R = 0.932). Data for the screen phenotype are the mean of two biological replicates; the individual validation phenotype represents the mean of three independent experiments. R is the Pearson correlation coefficient.

FIGs. 14A-14F show shRNA-mediated knockdown of targeted genes in OVCAR8-ADR cells. FIG. 14A presents the relative mRNA levels of KDM4C in OVCAR8-ADR cells expressing control shRNA or shRNA targeting KDM4C. FIG. 14B presents the relative mRNA levels of BRD4 in OVCAR8-ADR cells expressing control shRNA or shRNA targeting BRD4. FIG. 14C presents the relative mRNA levels of KDM6B in OVCAR8-ADR cells expressing control shRNA or shRNA targeting KDM6B. mRNA levels were quantified by qRT-PCR and normalized to actin mRNA levels. Data represent the mean $\pm$ SD (n = 3). FIGs. 14D-14F show Western blot analysis of relative protein levels in OVCAR8-ADR cells expressing control shRNA or shRNAs targeting *KDM4C, BRD4,* or *KDM6B.* Measured protein levels were normalized to actin levels, normalized to the control shRNA samples, and plot as the relative protein level in the graphs below. The asterisk (*P < 0.05) represents a significant difference in mRNA or protein levels between cells expressing the gene-targeting shRNA versus control shRNA.

FIG. 15 shows a strategy for assembling barcoded combinatorial gRNA libraries. Barcoded gRNA oligo pairs were synthesized, annealed, and cloned in storage vectors in pooled format. Oligos with the gRNA scaffold sequence were inserted into the pooled storage vector library to create the barcoded sgRNA library. Detailed assembly steps are shown in FIG. 1. The CombiGEM strategy was used to build the combinatorial gRNA library. Pooled barcoded sgRNA inserts prepared from the sgRNA library with BglII and MfeI digestion were ligated via compatible overhangs generated in the destination vectors with BamHI and EcoRI digestion. Iterative one-pot ligation created (n)-wise gRNA libraries with unique barcodes corresponding to the gRNAs concatenated at one end, thus enabling tracking of individual combinatorial members within pooled populations via next-generation sequencing.

FIGs. 16A-16E present the results from deep sequencing for indel analysis at gRNA-at targeted genomic loci in OVCAR8-ADR-Cas9 cells. Cells were infected with the indicated sgRNAs for 15 days and then subjected to deep sequencing. FIG. 16A presents the indel frequency. FIG. 16B shows the percentage of frameshift and in-frame mutations. FIG. 16C shows the distribution of indel sizes. FIG. 16D shows the distribution of indels analyzed by deep sequencing of the targeted genomic loci in OVCAR8-ADR-Cas9 cells that were infected for 15 days with either the single sgRNAs (KDM4C or BRD4), top graphs, or dual-gRNA expression constructs (KDM4 and BRD4), bottom graphs. FIG. 16E shows the distribution of indels analyzed by deep sequencing of the targeted genomic loci in OVCAR8-ADR-Cas9 cells that were infected for 15 days with either the single sgRNAs (PHF8 or BMI1), top graphs, or dual-gRNA expression constructs (PHF8 and BMI1), bottom graphs.

FIGs. 17A-17C show mathematical modeling of the frequency of a pro-proliferative gRNA and an anti-proliferative gRNA within a mixed cell population. FIG. 17A shows simulation of the relative frequencies of a pro-proliferative gRNA and an anti-proliferative gRNA in a cell population with different fractions (*i.e.*, 2, 5, or 10%) of cells that contain the anti-proliferative gRNA ($f_s$) and the pro-proliferative ($f_f$) gRNA initially. The relative frequency is defined as the barcode abundance at a given time compared to the initial time point. In this example, the fraction of cells with the modified growth rate due to genetic perturbations by the CRISPR-Cas9 system (p) is set as 1.0 (i.e., 100%), and the doubling time of the anti-proliferative clone ($T_{doubling,m}$) is 48 hours. FIGs. 17B and 17C show modeled relative frequencies of an anti-proliferative gRNA in a mixed cell population with regard to variations in the parameters: $p$, $T_{doubling,m}$, $f_s$, and $f_f$. In each graph of FIGs. 17B and 17C, lines represent p=0.2, p=.4, p=0.6, p=0.8, and p=1.0, from top to bottom. In FIGs. 17A-17C, the doubling time of the pro-proliferative clone is set as 12 hours. Detailed definitions are described in Example 3.

FIG. 18 shows pooled screen and validation data for individual gRNA combinations. For each gRNA combination, the fold-change in the normalized barcode count for 20-day versus 15-day cultured cells obtained from the pooled screening data ('Screen phenotype') was plotted against its relative cell viability compared to the vector control determined from the individual cell-proliferation assays ('Validation phenotype'). The Screen phenotype of each individual sgRNA was averaged from the fold-change of the corresponding sgRNA paired with each of the three control sgRNAs. Data for the screening data are the mean of two biological replicates, while the individual validation data represent the mean $\pm$ SD ($n \geq 3$).

FIG. 19 presents the measurement of on-target and off-target indel generation rates for gRNAs targeting KDM4C, KDM6B, and BRD4. Each row represents a genomic locus corresponding to a 20 bp guide sequence (in black) followed by a 3 bp PAM sequence (in gray). Sequences in bold black font represent the gRNA's on-target genomic sequence. Below each dashed line for KDM4C-sg1, KDM6B-sg2, and BRD4-sg3 are all the predicted exonic off-target genomic sequences identified using the CRISPR design (Ran, et al. Nature Protocols (2013) 8:2281-2308) and CCTop (Stemmer, PLoS One (2015) 10:e0124633) tools. Five exonic/intronic off-target sites predicted for BRD4-sg2 were also evaluated. Underlined nucleotides highlight the differences in the off-target sequences from the on-target sequence. Each genomic locus was PCR amplified from -10,000 cells and deep sequenced with >4.2 million reads. n.d. indicates that PCR of the genomic sequence failed to provide specific amplicons for sequencing. The sequences, from top to bottom, correspond to SEQ ID NOs: 368-393.

FIGs. 20A-20B show the reduced growth in OVCAR8-ADR-Cas9 cells harboring both KDM4C and BRD4 frameshift mutations. FIG. 20A depicts a cell growth assay on a single-cell-expanded OVCAR8-ADR-Cas9 mutant clone with both *KDM4C* and *BRD4* frameshift mutations (i.e., derived from Clone #3 shown in FIG. 9B). Equal numbers of cells were plated and cultured for 5 days before MTT assay. Data represent mean $\pm$ SD (n = 3) of absorbance measurements ($OD_{570}$ - $OD_{650}$) relative to control OVCAR8-ADR-Cas9 cells. The asterisk (*P < 0.01) represents significant difference between the control and mutant cells. FIG. 20B presents an immunoblot analysis of protein levels in the control and mutant cells from FIG. 20A.

FIGs. 21A-21B show RNA-sequencing analysis of OVCAR8-ADR-Cas9 cells infected with gRNA expression constructs. FIG. 21A presents representative heatmaps showing the relative expression levels of each gene transcript (rows) in each sample (column) for OVCAR8-ADR-Cas9 cells targeted by the respective single or dual gRNAs. Transcripts that were identified as significantly differentially expressed in OVCAR8-ADR-Cas9 cells infected with the indicated gRNA(s), when compared to the vector control, are included in the heatmaps. Values are $log_2$-transformed FPKM measured using RNA-Seq, and mean-centered by the transcript. Hierarchical clustering of transcripts and samples was performed based on the Pearson's correlation. FIG. 21B shows the top ten enriched gene sets of biological processes for the differentially expressed genes identified in OVCAR8-ADR-Cas9 cells infected with the indicated gRNAs when compared to the vector control (Q-value <0.05). Subsets of the differentially expressed genes (x-axes) that are associated with the gene sets (y-axes) are shaded in gray in the tables.

FIGs. 22A-22B show the effect of KDM4C and BRD4, as well as KDM6B and BRD4, on cell growth for additional cancer cell lines. FIG. 22A shows that combinatorial gRNA expression constructs effectively repressed targeted fluorescence genes in breast cancer MDA-MB231-Cas9 and pancreatic cancer Bx-PC3-Cas9 cells. Lentiviral vectors that contained RFP and GFP genes expressed from constitutive promoters, with or without tandem U6 promoter-driven expression cassettes of gRNAs targeting RFP and GFP sequences, were delivered to MDA-MB231-Cas9 and Bx-PC3-Cas9 cells for analysis of GFP and RFP expression under flow cytometry. Detailed strategy is described in FIG. 6. Lentiviruses encoding combinatorial gRNA expression constructs reduced the percentage of cells positive for RFP and GFP fluorescence at day 4 post-infection. FIG. 22B shows MDA-MB231-Cas9 and Bx-PC3-Cas9 cells infected with lentiviruses expressing the indicated single or combinatorial gRNAs were cultured for 14 days. Equal numbers of infected cells were then re-plated and cultured for additional 5 days. Cell viabilities relative to control sgRNA were determined by the MTT assay. Data represent mean $\pm$ SD (n =6) from biological replicates. The asterisk (*P < 0.05) represents significant differences between the indicated samples. These results indicate that combinatorial gRNA targeting of epigenetic genes can have variable phenotypes depending on the cellular background.

## DETAILED DESCRIPTION

Generation of vectors and genetic elements for the expression of multiple CRISPR systems comprising more than one sgRNA (guide sequence and scaffold sequence) is very laborious, and the complexity of libraries of CRISPR systems built using traditional cloning methods is very limited. The methods described herein result in vectors with concatenated barcodes and CRISPR guide sequences and scaffold sequences. The methods are potentially highly efficient for building large libraries for combinatorial genetic screening and leverage the fact that large numbers of oligonucleotides can be readily printed and that guide sequences with target specificity determining regions can be printed onto these oligonucleotides because the guide sequences and barcode elements are of short lengths.

The Massively Parallel Combinatorial Genetics approach to generating CRISPR constructs and vectors described herein allows the rapid generation of combinatorial sets of genetic constructs comprising components of the CRISPR system (CRISPR guide sequences and scaffold sequences) capable of targeting nucleic acid of a host cell. The methods also enable the pooled screening of multiple combination orders (*e.g.*, pairwise, tri-wise, and n-wise combination can be pooled and screened together simultaneously), identifying minimal combinations needed for a given application. Combinatorial sets of genetic constructs, such as those generated using the methods described herein, may be useful for the identification of genes and genetic pathways that interact synergistically to regulate a cellular process or phenotype, such as cancer cell growth. Also described herein are novel combinations of epigenetic genes identified, using combinatorial CRISPR constructs described herein that, when inhibited together, have anti-cancer effects, such as reducing proliferation of cells.

Aspects of the present disclosure relate to genetic constructs, vectors comprising genetic constructs, combinatorial vectors, and methods of generating combinatorial vectors using in the Massively Parallel Combinatorial Genetics approach, which can be found in PCT Publication No. WO2014/005042. As used herein, a "genetic construct" refers to one or more DNA element(s) comprising a CRISPR guide sequence and a scaffold sequence and a barcode element, such that each DNA element is associated with a barcode element. As used herein, association between a specific DNA element and a barcode element means that a specific DNA element and a barcode element are always contained within the same genetic construct. Accordingly, the presence or detection of a specific barcode element within a genetic construct indicates that the associated specific DNA element(s) is also present within the same genetic construct.

In a host cell, the DNA element comprising a CRISPR guide sequence and a scaffold sequence is transcribed and forms a CRISPR small-guide RNA (sgRNA) that functions to recruit an endonuclease to a specific target nucleic acid in a host cell, which may result in site-specific CRISPR activity. As used herein, a "CRISPR guide sequence" refers to a nucleic acid sequence that is complementary to a target nucleic acid sequence in a host cell. The CRISPR guide sequence targets the sgRNA to a target nucleic acid sequence, also referred to as a target site. The CRISPR guide sequence that is complementary to the target nucleic acid may be between 15-25 nucleotides, 18-22 nucleotides, or 19-21 nucleotides in length. In some embodiments, the CRISPR guide sequence that is complementary to the target nucleic acid is 15, 16, 17, 18, 19, 20, 21, 22, 23, 24, or 25 nucleotides in length. In some embodiments, the CRISPR guide sequence that is complementary to the target nucleic acid is 20 nucleotides in length.

It will be appreciated that a CRISPR guide sequence is complementary to a target nucleic acid in a host cell if the CRISPR guide sequence is capable of hybridizing to the target nucleic acid. In some embodiments, the CRISPR guide sequence is at least 50%, 55%, 60%, 65%, 70%, 75%, 80%, 85%, 90%, 95%, 96%, 97%, 98%, 99%, or at least 100% complementary to a target nucleic acid (see also US Patent 8,697,359, which is referred to for its teaching of complementarity of a CRISPR guide sequence with a target polynucleotide sequence). It has been demonstrated that mismatches between a CRISPR guide sequence and the target nucleic acid near the 3' end of the target nucleic acid may abolish nuclease cleavage activity (Upadhyay, et al. Genes Genome Genetics (2013) 3(12):2233-2238). In some embodiments, the CRISPR guide sequence is at least 50%, 55%, 60%, 65%, 70%, 75%, 80%, 85%, 90%, 95%, 96%, 97%, 98%, 99%, or at least 100% complementary to the 3' end of the target nucleic acid (*e.g.*, the last 5, 6, 7, 8, 9, or 10 nucleotides of the 3' end of the target nucleic acid).

The CRISPR guide sequence may be obtained from any source known in the art. For example, the CRISPR guide sequence may be any nucleic acid sequence of the indicated length present in the nucleic acid of a host cell *(e.g.,* genomic nucleic acid and/or extra-genomic nucleic acid). In some embodiments, CRISPR guide sequences may be designed and synthesized to target desired nucleic acids, such as nucleic acids encoding transcription factors, signaling proteins, transporters, etc. In some embodiments, the CRISPR guide sequences are designed and synthesized to target epigenetic genes. For example, the CRISPR guide sequences may be designed to target any of the combinations of epigenetic genes presented in Table 2. In some embodiments, the CRISPR guide sequences comprise any of the example CRISPR guide sequences provided in Table 1.

As used herein, a "scaffold sequence," also referred to as a tracrRNA, refers to a nucleic acid sequence that recruits an endonuclease to a target nucleic acid bound (hybridized) to a complementary CRISPR guide sequence. Any scaffold sequence that comprises at least one stem loop structure and recruits an endonuclease may be used in the genetic elements and vectors described herein. Exemplary scaffold sequences will be evident to one of skill in the art and can be found for example in Jinek, et al. Science (2012) 337(6096):816-821, Ran, et al. Nature Protocols (2013) 8:2281-2308, PCT Application No. WO2014/093694, and PCT Application No. WO2013/176772.

The terms "target nucleic acid," "target site, " and "target sequence" may be used interchangeably throughout and refer to any nucleic acid sequence in a host cell that may be targeted by the CRISPR guide sequences described herein. The target nucleic acid is flanked downstream (on the 3' side) by a protospacer adjacent motif (PAM) that may interact with the endonuclease and be further involved in targeting the endonuclease activity to the target nucleic acid. It is generally thought that the PAM sequence flanking the target nucleic acid depends on the endonuclease and the source from which the endonuclease is derived. For example, for Cas9 endonucleases that are derived from *Streptococcus pyogenes,* the PAM sequence is NGG. For Cas9 endonucleases derived from *Staphylococcus aureus,* the PAM sequence is NNGRRT. For Cas9 endonucleases that are derived from *Neisseria meningitidis,* the PAM sequence is NNNNGATT. For Cas9 endonucleases derived from *Streptococcus thermophilus*, the PAM sequence is NNAGAA. For Cas9 endonuclease derived from *Treponema denticola*, the PAM sequence is NAAAAC. For a Cpf1 nuclease, the PAM sequence is TTN.

In some embodiments, the CRISPR guide sequence and the scaffold sequence are expressed as separate transcripts. In such embodiments, the CRISPR guide sequence further comprises an additional sequence that is complementary to a portion of the scaffold sequence and functions to bind (hybridize) the scaffold sequence and recruit the endonuclease to the target nucleic acid. In other embodiments, the CRISPR guide sequence and the scaffold sequence are expressed as a single transcript, as a chimeric RNA that may be referred to as a single guide RNA (sgRNA). An sgRNA has the dual function of both binding (hybridizing) to the target nucleic acid and recruiting the endonuclease to the target nucleic acid. In such embodiments, the scaffold sequence may further comprise a linker loop sequence.

The barcode elements can be used as identifiers for a genetic construct and may indicate the presence of one or more specific CRISPR guide sequences in a vector or genetic element. Members of a set of barcode elements have a sufficiently unique nucleic acid sequence such that each barcode element is readily distinguishable from the other barcode elements of the set. Barcode elements may be any length of nucleotide but are preferably less than 30 nucleotides in length. In some embodiments, the barcode element is 6, 7, 8, 9, 10, 11, 12, 13, 14, 15, 16, 17, 18, 19, 20, 21, 22, 23, 25, 26, 27, 28, 29, or 30 or more nucleotides in length. Detecting barcode elements and determining the nucleic acid sequence of a barcode element or plurality of barcode elements are used to determine the presence of an associated DNA element of a genetic construct. Barcode elements as described herein can be detected by any method known in the art, including sequencing or microarray methods.

FIG. 1 shows several schematics of non-limiting examples of genetic constructs associated with the invention. In FIG. 1 step 4, a DNA element comprising a CRISPR guide sequence, designated "guide sequence," and a scaffold sequence, designated "scaffold," is flanked by a first compatible end element, indicated with "BamHI," and a second compatible end element, indicated with "BglII," which are capable of annealing to each other. The genetic construct also contains a barcode element, designated as "barcode," which is flanked by a third compatible end element, indicated with "EcoRI," and a fourth compatible end element, indicated with "MfeI," which are capable of annealing to each other, but are not capable of annealing to the first and second compatible end elements. The genetic construct also contains a separation site, such that the barcode element is located on one side of the separation site and the DNA element is located on the other side of the separation site. FIG. 1 also depicts a promoter element upstream (5' relative to) the DNA element that allows for expression (transcription) of the DNA element. While FIG. 1 depicts the DNA element as being upstream (5' relative to) the barcode element, this arrangement can also be reversed.

Compatible ends can be created in a variety of ways that will be evident to one of skill in the art and can consist of a variety of different sequences. As used herein, "compatible end elements" refer to regions of DNA that are capable of ligating or annealing to each other. Compatible end elements that are capable of ligating or annealing to each other will be apparent to one of skill in the art and refers to end elements that are complementary in nucleotide sequence to one another and therefore, are capable of base-pairing to one another. In several non-limiting embodiments, compatible end elements can be composed of restriction sites with compatible overhangs, Gibson assembly sequences, or functional elements of any other DNA assembly method, including recombinases, meganucleases, TAL Effector/Zinc-finger nucleases, trans-cleaving ribozymes/DNAzymes or integrases.

In some embodiments, Gibson assembly is used to generate compatible overhangs. Gibson assembly refers to an isothermal DNA end-linking technique whereby multiple DNA fragments can be joined in a single reaction. This method is described further in, Gibson et al. (2009) Nature Methods 6:343-5.

In other embodiments, restriction digestion is used to generate compatible ends, as depicted in FIG. 1. Using this method, two unique restriction enzymes generate compatible overhangs. When these overhangs are ligated, a scar is created that is no longer recognized by either enzyme. It should be appreciated that any restriction enzymes that generate compatible overhangs can be used. In some non-limiting embodiments, standard biological parts such as BIOBRICKS ® (The BioBricks Foundation) or BglBricks (Anderson et al. (2010) Journal of Biological Engineering 4:1), and enzymes associated with such standard biological parts, are used. The use of standard biological parts such as BIOBRICKS ® or BglBricks is routine to one of ordinary skill in the art. It should be appreciated that while classical restriction enzymes can be used (such as Type I, II or III restriction enzymes), other DNA-cleaving molecules can also be used. For example, targeted ribozymes can be used for cleavage of specific target sites. Meganucleases can also be utilized to minimize the possibility of interference with the inserted DNA elements. TALE or ZF nucleases can also be used to target long DNA sites to minimize the probability of internal cleavage within inserted DNA elements. Furthermore, TOPO® cloning can be used to accomplish restriction digestions and ligations.

In some embodiments, the first compatible end element is generated by recognition and cleavage with the restriction enzyme BamHI, and the second compatible end element is generated by recognition and cleavage with the restriction enzyme BglII. In some embodiments, the third compatible end element is generated by recognition and cleavage with the restriction enzyme MfeI, and the fourth compatible end element is generated by recognition and cleavage with the restriction enzyme EcoRI.

As used herein, a "separation site" of a genetic construct refers to a region that allows linearization of the construct. It should be appreciated that the separation site is a site within the nucleic acid of a construct at which cleavage linearizes the construct and may allow for insertion of additional genetic elements. In some embodiments, the separation site is a restriction enzyme recognition site. For example, in FIG. 1 the separation site is formed by the first and fourth compatible end elements, indicated by the BamHI and EcoRI recognition sites, respectively. Cleavage of the construct using the corresponding restriction enzymes (BamHI and EcoRI) linearizes the construct, and allows for insertion of additional genetic constructs. In some embodiments, the separation site is formed by one recognition site. In some embodiments, the separation site is formed by more than one recognition site.

Aspects of the description relate to methods for producing a combinatorial vector comprising genetic constructs described herein. As depicted in step 3 of FIG. 1, the methods involve providing a vector containing a first genetic construct comprising a CRISPR guide sequence denoted "20 bp guide sequence," flanked by a second compatible end element indicated by "BglII," and a first recognition site for a first restriction enzyme denoted as "BbsI;" a barcode element, denoted "barcode," flanked by a third compatible end element indicated by "MfeI" and a second recognition site for a second restriction enzyme. In some embodiments, the vector may be generated by annealing and ligating a first genetic construct containing compatible ends with a cleaved vector, as shown in step 2 of FIG. 1. In some embodiments, the first genetic construct is synthesized, for example by oligonucleotide array synthesis. The first genetic construct can be cleaved at the first recognition site, resulting in a fifth compatible end element, and cleaved at the second recognition site, resulting in a sixth compatible end element. A scaffold element is provided comprising a scaffold sequence and a separation site, indicated by "BamHI" and "EcoRI," flanked by a seventh compatible end element that is capable of annealing to the fifth compatible end element of the cleaved vector and an eight compatible end element that is capable of annealing to the sixth compatible end element of the cleaved vector. The scaffold element is annealed to the cleaved first genetic construct of the vector using the compatible end elements. After annealing, the scaffold element is integrated between the CRISPR guide sequence and the barcode element, and the separation site is located between the scaffold sequence and the barcode element.

It should be appreciated that a variety of different enzyme combinations can be used to cleave the first and second recognition sites. In some embodiments, the two recognition sites located outside of the CRISPR guide sequence and the barcode element are recognized by the same restriction enzyme, which produces compatible ends with the scaffold element. In other embodiments, the two restriction sites located outside of the CRISPR guide sequence and the barcode element are recognized by two different restriction enzymes, each of which produces compatible ends with the scaffold element.

Further aspects of the description relate to combinatorial constructs, and methods for producing combinatorial constructs. As used herein, a "combinatorial construct" refers to a genetic construct that contains a plurality of DNA elements. As used herein, a plurality of DNA elements refers to more than one DNA element, each of the DNA elements comprising a CRISPR guide sequence and a scaffold sequence. As shown in step 5 of FIG. 1, the generation of a combinatorial construct can involve the linearization of a vector that contains a first genetic construct associated with the invention, by cleaving the vector at the separation site within the genetic construct. A second genetic construct associated with the invention may be inserted into the cleaved vector and annealed and ligated to the vector. As used herein, an "insert" refers to a genetic construct that is intended to be inserted into a cleaved vector. In some embodiments, the insert is purified from a vector, such as by PCR or restriction digestion. The insert can be ligated to the cleaved vector through the annealing of terminal compatible end elements within the insert and their compatible components within the linearized vector.

The (n)-wise guide RNA library of step 5 of FIG. 1 depicts a post-combination combinatorial construct that contains a plurality of DNA elements and a plurality of corresponding barcode elements. In the non-limiting example depicted in step 5 of FIG. 1, the genetic construct contains four different DNA elements and four corresponding barcode elements. The combinatorial construct further contains a separation site, located between the plurality of barcode elements and the plurality of DNA elements.

The methods described herein for generating combinatorial constructs can be iterative. For example, the combinatorial vector depicted in FIG. 1 can be cleaved again at the separation site, and one or more further inserts can be ligated into the combinatorial construct, while maintaining a separation site for further insertions. Significantly, throughout the iterative process, as the number of DNA elements within the genetic construct continues to increase, the unique barcodes associated with each DNA element are maintained within the same genetic construct as their associated DNA elements. In some embodiments, the combination process is repeated at least 2, 3, 4, 5, 6, 7, 8, 9, 10, 11, 12, 13, 14, 15, 16, 17, 18, 19, 20, times or more than 20 times. In some embodiments, the process is repeated an nth number of times, where n can be 1, 2, 3, 4, 5, 6, 7, 8, 9, 10, 11, 12, 13, 14, 15, 16, 17, 18, 19, 20, or a number greater than 20.

It should be appreciated that combinatorial constructs can contain any number of DNA elements and associated barcode elements. In some embodiments a combinatorial construct contains 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more than 100 DNA elements and associated barcode elements.

Another aspect of the present description relates to genetic constructs and vectors comprising more than one CRISPR guide sequence associated with a single barcode element. FIG. 2 shows several schematics of non-limiting examples of genetic constructs associated with the invention. Step 1 of FIG. 2A and 2B shows a genetic construct containing three CRISPR guide sequences, denoted "20 bp guide sequence A," "20 bp guide sequence B," and "20 bp guide sequence C," a barcode element indicated by "barcode" and a recognition site located between each CRISPR guide sequence and between the barcode element and the CRISPR guide sequence nearest to the barcode element. In some embodiments, the barcode element may be located downstream of the CRISPR guide sequences, as shown in FIG. 2A. In other embodiments, the barcode element may be located upstream of the CRISPR guide sequences, as shown in FIG. 2B. In some embodiments, the recognition sites located between the CRISPR guide sequences and between the barcode element and the CRISPR guide sequence nearest to the barcode element are each different recognition sites for different restriction enzymes. In some embodiments, the genetic construct comprising the at least two CRISPR guide sequences, barcode element, and recognition sites are synthesized by any method known in the art, such as by oligonucleotide array synthesis.

Also within the scope of the present description are genetic constructs comprising a plurality of DNA elements and one barcode element. In step 3 of FIG. 2, a genetic construct comprises three DNA elements each of which contain a CRISPR guide sequence and a scaffold sequence, a barcode element, and a promoter sequence located upstream of each of the DNA elements. In some embodiments, the barcode element may be located downstream of the CRISPR guide sequences, as shown in FIG. 2A. In other embodiments, the barcode element may be located upstream of the CRISPR guide sequences, as shown in FIG. 2B.

Aspects of the description relate to methods for producing a combinatorial vector comprising the genetic constructs described herein. In some embodiments, the methods involve providing a vector containing a plurality of CRISPR guide sequences and a barcode element located downstream of the plurality of CRISPR guide sequences. As shown in step 1 of FIG. 2A and 2B, three CRISPR guide sequences are denoted "20 bp guide sequence A," "20 bp guide sequence B," and "20 bp guide sequence C," and the barcode element is indicated by "barcode." The vector also contains a plurality of recognition sites for a plurality of restriction enzymes. In step 1 of FIG. 2A, each of the recognition sites is located downstream of a CRISPR guide sequence and indicated by "RE1," "RE2," and "RE3." In step 1 of FIG. 2B, each of the recognition sites is located upstream of a CRISPR guide sequence and indicated by "RE1," "RE2," and "RE3." In some embodiments, the vector also contains a promoter sequence located upstream of at least one of the CRISPR guide sequences. Compatible end elements downstream of at least one of the CRISPR guide sequences are generated by any method known in the art. In some embodiments, the vector is cleaved at at least one of the recognition sites with a restriction enzyme resulting in a first compatible end element and a second compatible end element. A scaffold element is provided comprising a scaffold sequence, optionally a promoter sequence, and a third compatible end element and fourth compatible end element that are capable of annealing to the first compatible end element and second compatible end element of the cleaved vector, respectively. The scaffold element is annealed to the cleaved vector through the annealing of terminal compatible end elements within the scaffold element and their compatible components within the cleaved vector. The methods described herein may be iterative resulting in a combinatorial vector containing a plurality of CRISPR guide sequences and scaffold sequences and one barcode element.

It should be appreciated that combinatorial vectors can contain any number of DNA elements associated with one barcode element. In some embodiments a combinatorial construct contains 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more than 100 DNA elements and one barcode element. The number of DNA elements associated with one barcode element may depend on the length of the genetic construct containing at the CRISPR guide sequences, barcode and recognition sites that is capable of being synthesized.

In any of the constructs or vectors described herein, one or more RNA domains may be inserted into one or more CRISPR guide sequences. In some embodiments, the CRISPR guide sequence is fused to one or more RNA domain. In some embodiments, the RNA is a non-coding RNA or fragment thereof. In such embodiments, the RNA domain may be targeted to a DNA loci. Such constructs or vectors may be used for CRISPR display.

Further aspects of the description relate to methods for identifying one or more DNA elements within a genetic construct or vector. After a combination event, a unique barcode that is associated with a specific DNA element(s) remains within the same genetic construct as the specific DNA element. Accordingly, identification of a barcode element or plurality of barcode elements allows for the identification of the associated DNA element or plurality of DNA elements within the same genetic construct. In some embodiments, the sequence of a barcode element and/or a DNA element is determined by sequencing or by microarray analysis. It should be appreciated that any means of determining DNA sequence is compatible with identifying one or more barcode elements and corresponding DNA elements. Significantly, in a combinatorial construct, such as is depicted in step 5 of FIG. 1, the plurality of barcode elements are within close proximity to each other allowing for the rapid identification of multiple barcode elements, and accordingly multiple DNA elements, simultaneously through methods such as DNA sequencing.

Further aspects of the description relate to libraries comprising two or more genetic constructs as described herein that are compatible with methods for Massively Parallel Combinatorial Genetics. As used herein, a library of genetic constructs refers to a collection of two or more genetic constructs. In some embodiments, a library of genetic constructs is generated in which each unique DNA element is on a plasmid. This plasmid library can be pooled to form a vector library. An insert library can be generated, for example, by conducting PCR on the vector library. In a first combination event, all of the vectors can be paired with all of the inserts, generating a full combinatorial set of pairwise combinations. Further reactions between this pairwise library and an insert library can lead to a tri-wise, quad-wise or more than quad-wise library arising from a single vector library. Libraries of combinatorial constructs can used to conduct screens of host cells expressing said libraries of combinatorial constructs. In some embodiments, the libraries of combinatorial constructs contain DNA elements or combinations of DNA elements with CRISPR guide sequences that target epigenetic genes, such as the example CRISPR guide sequences presented in Table 1.

It should be appreciated that since the combinatorial step is conducted in vitro, this technology can be scaled to any host cell or organism that can receive DNA. In some embodiments, the host cell is a bacterial cell. In some embodiments, the organism is bacteria and the constructs are carried on plasmids or phages. In some embodiments, the host cell is a yeast cell. In other embodiments, the organism is yeast and the constructs are carried on plasmids or shuttle vectors. In other embodiments, the host cell is a mammalian cell, such as a human cell. In such embodiments, the genetic constructs described herein can be carried on plasmids or delivered by viruses such as lentiviruses or adenoviruses.

The genetic constructs and vectors described herein relate to the expression of components of a CRISPR system including a CRISPR guide sequence and scaffold sequence. The host cell in which the CRISPR system is expressed may express one or more additional CRISPR components, such as an endonuclease. In some embodiments, the host cell also expresses an endonuclease, such as a Cas endonuclease. In some embodiments, the Cas endonuclease is Cas1, Cas2, or Cas9 endonuclease. In some embodiments, the host cell expresses a Cas9 endonuclease derived from *Streptococcus pyogenes, Staphylococcus aureus, Neisseria meningitidis, Streptococcus thermophilus,* or *Treponema denticola.* In some embodiments, the nucleotide sequence encoding the Cas9 endonuclease may be codon optimized for expression in a host cell or organism. In some embodiments, the endonuclease is a Cas9 homology or ortholog.

In some embodiments, the nucleotide sequence encoding the Cas9 endonuclease is further modified to alter the activity of the protein. In some embodiments, the Cas9 endonuclease is a catalytically inactive Cas9. For example, dCas9 contains mutations of catalytically active residues (D10 and H840) and does not have nuclease activity. Alternatively or in addition, the Cas9 endonuclease may be fused to another protein or portion thereof. In some embodiments, dCas9 is fused to a repressor domain, such as a KRAB domain. In some embodiments, such dCas9 fusion proteins are used with the constructs described herein for multiplexed gene repression (e.g. CRISPR interference (CRISPRi)). In some embodiments, dCas9 is fused to an activator domain, such as VP64 or VPR. In some embodiments, such dCas9 fusion proteins are used with the constructs described herein for multiplexed gene activation (e.g. CRISPR activation (CRISPRa)). In some embodiments, dCas9 is fused to an epigenetic modulating domain, such as a histone demethylase domain or a histone acetyltransferase domain. In some embodiments, dCas9 is fused to a LSD1 or p300, or a portion thereof. In some embodiments, the dCas9 fusion is used for CRISPR-based epigenetic modulation. In some embodiments, dCas9 or Cas9 is fused to a Fok1 nuclease domain. In some embodiments, Cas9 or dCas9 fused to a Fok1 nuclease domain is used for multiplexed gene editing. In some embodiments, Cas9 or dCas9 is fused to a fluorescent protein *(e.g.,* GFP, RFP, mCherry, etc). In some embodiments, Cas9/dCas9 proteins fused to fluorescent proteins are used for multiplexed labeling and/or visualization of genomic loci.

Alternatively or in addition, the endonuclease is a Cpfl nuclease. In some embodiments, the host cell expresses a Cpf1 nuclease derived from *Provetella* spp. or *Francisella* spp. In some embodiments, the nucleotide sequence encoding the Cpfl nuclease may be codon optimized for expression in a host cell or organism.

The description encompasses any cell type in which DNA can be introduced, including prokaryotic and eukaryotic cells. In some embodiments the cell is a bacterial cell, such as *Escherichia* spp., *Streptomyces* spp., *Zymonas* spp., *Acetobacter* spp., *Citrobacter* spp., *Synechocystis* spp., *Rhizobium* spp., *Clostridium* spp., *Corynebacterium* spp., *Streptococcus* spp., *Xanthomonas* spp., *Lactobacillus* spp., *Lactococcus* spp., *Bacillus* spp., *Alcaligenes* spp., *Pseudomonas* spp., *Aeromonas* spp., *Azotobacter* spp., *Comamonas* spp., *Mycobacterium* spp., *Rhodococcus* spp., *Gluconobacter* spp., *Ralstonia* spp., *Acidithiobacillus* spp., *Microlunatus* spp., *Geobacter* spp., *Geobacillus* spp., *Arthrobacter* spp., *Flavobacterium* spp., *Serratia* spp., *Saccharopolyspora* spp., *Thermus* spp., *Stenotrophomonas* spp., *Chromobacterium* spp., *Sinorhizobium* spp., *Saccharopolyspora* spp., *Agrobacterium* spp. and *Pantoea* spp. The bacterial cell can be a Gram-negative cell such as an *Escherichia coli* (*E. coli*) cell, or a Gram-positive cell such as a species of *Bacillus.*

In other embodiments, the cell is a fungal cell such as a yeast cell, e.g., *Saccharomyces* spp., *Schizosaccharomyces* spp., *Pichia* spp., *Phaffia* spp., *Kluyveromyces* spp., *Candida* spp., *Talaromyces* spp., *Brettanomyces* spp., *Pachysolen* spp., *Debaryomyces* spp., *Yarrowia* spp. and industrial polyploid yeast strains. Preferably the yeast strain is a *S. cerevisiae* strain. Other examples of fungi include *Aspergillus* spp., *Penicillium* spp., *Fusarium* spp., *Rhizopus* spp., *Acremonium* spp., *Neurospora* spp., *Sordaria* spp., *Magnaporthe* spp., *Allomyces* spp., *Ustilago* spp., *Botrytis* spp., and *Trichoderma* spp.

In other embodiments, the cell is an algal cell, a plant cell, an insect cell, a rodent cell or a mammalian cell, including a rodent cell or a human cell (e.g., a human embryonic kidney cell (e.g., HEK293T cell), a human dermal fibroblast, a human cancer cells, such as a OVCAR8 cell or a OVCAR8-ADR cell. In some embodiments, the cell is a human cancer cell, such as a human ovarian cancer cell.

Also provided herein are compositions comprising inhibitors targeting epigenetic genes. As used herein, the term "epigenetic gene" refers to any gene that affects epigenetic regulation of another molecule or process in a cell. In some embodiments, the epigenetic gene encodes a protein that is involved in epigenetic regulation. In some embodiments, the epigenetic gene encodes a nucleic acid, such as an RNA *(e.g.,* a microRNA), that affects epigenetic regulation. In general, epigenetics refers to any alteration to a molecule or process that does not involve mutation of the genomic DNA of the cell (Jaenisch and Bird Nat. Gene. (2003) 33: 245-254). Epigenetic regulation involves DNA-mediated processes in a cell, such as transcription, DNA repair, and replication through mechanisms including DNA methylation, histone modification, nucleosome remodeling, and RNA-mediating targeting (Dawson and Kouzarides Cell (2012) 150(1): 12-27). Non-limiting examples of epigenetic genes include: DNMT1, DNMT3A, DNMT3B, DNMT3L, MBD1, MBD2, CREBBP, EP300, HDAC1, HDAC2, SIRT1, CARM1, EZH1, EZH2, MLL, MLL2, NSD1, PRMT1, PRMT2, PRMT3, PRMT5, PRMT6, PRMT7, SETD2, KDM1A-, KDM1B, KDM2A, KDM2B, KDM3A, KDM3B, KDM4A, KDM4B, KDM4C, KDM5A, KDM5B, KDM5C, KDM5D, KDM6A, KDM6B, PHF2, PHF8, BMI1, BRD1, BRD3, BRD4, ING1, ING2, ING3, ING4, and ING5.

As used herein, the term "inhibitor" refers to any molecule, such as a protein, nucleic acid, or small molecule that reduces or prevents expression of an epigenetic gene or reduces or prevents activity of a protein encoded by the epigenetic gene. In some embodiments, the combination of two or more inhibitors of epigenetic genes reduces expression of an epigenetic gene by at least 10%, 15%, 20%, 25%, 30%, 35%, 40%, 45%, 50%, 55%, 60%, or at least 65% as compared to expression of the epigenetic gene in the absence of the combination of inhibitors. In some embodiments, the combination of two or more inhibitors of epigenetic genes reduces activity of a protein encoded by an epigenetic gene by at least 10%, 15%, 20%, 25%, 30%, 35%, 40%, 45%, 50%, 55%, 60%, or at least 65% as compared to activity of the protein encoded by the epigenetic gene in the absence of the combination of inhibitors.

In some embodiments, the combination of inhibitors of epigenetic genes comprises at least 2, 3, 4, 5, 6, 7, 8, 9, or at least 10 or more inhibitors of epigenetic genes. In some embodiments, the combination of inhibitors of epigenetic genes comprises two inhibitors of epigenetic genes. In some embodiments, the combination of inhibitors inhibit 2, 3, 4, 5, 6, 7, 8, 9, 10 or more epigenetic genes. In some embodiments, the combination of inhibitors of epigenetic genes comprises two inhibitors that target and inhibit two epigenetic genes.

In some embodiments, at least one inhibitor of the combination of inhibitors is a protein that directly or indirectly reduces or prevents expression of an epigenetic gene or reduces or prevents activity of a protein encoded by the epigenetic gene. For example, the protein may be a repressor that reduces or prevents expression of the epigenetic gene or an allosteric inhibitor of a protein encoded by the epigenetic gene. In some embodiments, two or more inhibitors are proteins targeting two or more epigenetic genes. In some embodiments, the two or more inhibitors are proteins that target any of the combinations of epigenetic genes presented in Table 2.

In some embodiments, at least one inhibitor of the combination of inhibitors is a nucleic acid that reduces or prevents expression of an epigenetic gene or reduces or prevents activity of a protein encoded by the epigenetic gene. In some embodiments, the nucleic acid is a CRISPR guide sequence that, along with a scaffold sequence, recruits an endonuclease to the epigenetic gene. In some embodiments, two or more inhibitors are CRISPR guide sequences targeting two or more epigenetic genes. In some embodiments, the two or more inhibitors are CRISPR guide sequences that target any of the combinations of epigenetic genes presented in Table 2. In some embodiments, the two or more inhibitors are CRISPR guide sequences selected from the example CRISPR guide sequences targeting epigenetic genes provided in Table 1. In some embodiments, the combination of epigenetic genes comprises BRD4 and KDM4C. In some embodiments, the combination of epigenetic genes comprises BRD4 and KDM6B.

In some embodiments, the nucleic acid is a shRNA that is processed by the RNA interference (RNAi) pathway of the cell to silence expression of the target gene *(e.g.,* reduce mRNA levels and/or protein production). In some embodiments, two or more inhibitors are shRNAs targeting two or more epigenetic genes. In some embodiments, the two or more inhibitors are shRNAs that target any of the combinations of epigenetic genes presented in Table 2. In some embodiments, the two or more inhibitors are shRNAs selected from the example shRNAs targeting epigenetic genes provided in Table 4. In some embodiments, the combination of epigenetic genes comprises BRD4 and KDM4C. In some embodiments, the combination of epigenetic genes comprises BRD4 and KDM6B.

In some embodiments, at least one inhibitor of the combination of inhibitors is a small molecule that reduces or prevents expression of an epigenetic gene or reduces or prevents activity of a protein encoded by the epigenetic gene. In some embodiments, two or more inhibitors are small molecules targeting two or more epigenetic genes. In some embodiments, the two or more inhibitors are small molecules that target any of the combinations of epigenetic genes presented in Table 2. In some embodiments, the combination of epigenetic genes comprises BRD4 and KDM4C. In some embodiments, the combination of epigenetic genes comprises BRD4 and KDM6B.

Any small molecule that reduces or prevents expression of BRD4 or reduces or prevents activity of a protein encoded by BRD4 may be compatible with the compositions and methods described herein. Examples of BRD4 inhibitors include, without limitation, JQ1 ((6S)-4-(4-Chlorophenyl)-2,3,9-trimethyl-6H-thieno[3,2-f][1,2,4]triazolo[4,3-a][1,4]diazepine-6-acetic acid 1,1-dimethylethyl ester), MS417 (methyl [(6S)-4-(4-chlorophenyl)-2,3,9-trimethyl-6H-thieno[3,2-*f*][1,2,4]triazolo[4,3-*a*][1,4]diazepin-6-yl]acetate), or RVX-208 (2-[4-(2-Hydroxyethoxy)-3,5-dimethylphenyl]-5,7-dimethoxy-4(3H)-quinazolinone). In some embodiments, the BRD4 inhibitor is JQ1. Additional BRD4 inhibitors will be evident to one of skill in the art and can be found, for example, in PCT Publication WO 2014/154760 A1 and Vidler et al. J. Med. Chem. (2013) 56: 8073-8088.

Any small molecule that reduces or prevents expression of KDM4C (also referred to as JMJD2) or reduces or prevents activity of a protein encoded by KDM4C may be compatible with the compositions and methods described herein. In some embodiments, the KDM4C inhibitor is SD70 (N-(furan-2-yl(8-hydroxyquinolin-7-yl)methyl)isobutyramide) or caffeic acid. Additional KDM4C inhibitors will be evident to one of skill in the art and can be found, for example, in Leurs et al. Bioorg. & Med. Chem. Lett. (2012) 22(12): 5811-5813 and Hamada et al. Bioorg. & Med. Chem. Lett. (2009) 19: 2852-2855).

Any small molecule that reduces or prevents expression of KDM6B (also referred to as JMJD3) or reduces or prevents activity of a protein encoded by KDM6B may be compatible with the compositions and methods described herein. Examples of KDM6B inhibitors include, without limitation, GSK-J4 (ethyl 3-((6-(4,5-dihydro-1H-benzo[d]azepin-3(2H)-yl)-2-(pyridin-2-yl)pyrimidin-4-yl)amino)propanoate, monohydrochloride), GSK-J1 (N-[2-(2-Pyridinyl)-6-(1,2,4,5-tetrahydro-3H-3-benzazepin-3-yl)-4-pyrimidinyl]-β-alanine), and IOX1 (8-Hydroxy-5-quinolinecarboxylic acid; 8-Hydroxy-5-quinolinecarboxylic acid). In some embodiments, the KDM6B inhibitor is GSK-J4. Additional KDM4C inhibitors will be evident to one of skill in the art.

The combination of two or more inhibitors may comprise two or more protein inhibitors, two or more CRISPR guide sequences, two or more shRNAs, or two or more small molecule inhibitors. In some embodiments, the two or more inhibitors are different types of inhibitors (e.g., proteins, nucleic acids, small molecules). In some embodiments, the combination comprises a protein inhibitor and one or more additional inhibitors (e.g., CRISPR guide sequences, shRNAs, and/or small molecule inhibitors). In other embodiments, the combination comprises a CRISPR guide sequence and one or more additional inhibitors (e.g., proteins, shRNAs, and/or small molecule inhibitors). In other embodiments, the combination comprises a shRNA and one or more additional inhibitors (e.g., proteins, CRISPR guide sequences, and/or small molecule inhibitors). In other embodiments, the combination comprises a small molecule inhibitor and one or more additional inhibitors (e.g., proteins, shRNAs, and/or shRNAs).

The methods and compositions described herein may be useful for reducing proliferation of a cell, such as a cancer cell or other cell for which reduced proliferation is desired. In some embodiments, contacting a cell with a combination of two or more inhibitors of epigenetic genes (e.g., combinations of inhibitors targeting epigenetic genes presented in Table 2) partially or completely reduces proliferation of the cell. In some embodiments, contacting a cell with a combination of two or more inhibitors of epigenetic genes partially or completely reduces proliferation of the cell as compared to a cell that is not contacted with the combination of inhibitors. In some embodiments, contacting cells with a combination of two or more inhibitors of epigenetic genes reduces proliferation of the cells by at least 10%, 15%, 20%, 25%, 30%, 35%, 40%, 45%, 50%, 55%, 60%, or at least 65% as compared to cells that were not contacted with the combination of inhibitors. In some embodiments, contacting a cell with a combination of two or more inhibitors of epigenetic genes partially or completely reduces proliferation of a cancer cell as compared to a non-cancer cell that is contacted with the combination of inhibitors. Cell proliferation may be assessed and quantified by any method known in the art, for example using cell viability assays, MTT assays, or BrdU cell proliferation assays.

Other aspects of the description relate to methods and compositions for treating cancer in a subject. Cancer is a disease characterized by uncontrolled or aberrantly controlled cell proliferation and other malignant cellular properties. As used herein, the term "cancer" refers to any type of cancer known in the art, including without limitation, breast cancer, biliary tract cancer, bladder cancer, brain cancer, cervical cancer, choriocarcinoma, colon cancer, endometrial cancer, esophageal cancer, gastric cancer, hematological neoplasms, T-cell acute lymphoblastic leukemia/lymphoma, hairy cell leukemia, chronic myelogenous leukemia, multiple myeloma, AIDS-associated leukemias and adult T-cell leukemia/lymphoma, intraepithelial neoplasms, liver cancer, lung cancer, lymphomas, neuroblastomas, oral cancer, ovarian cancer, pancreatic cancer, prostate cancer, rectal cancer, sarcomas, skin cancer, testicular cancer, thyroid cancer, and renal cancer. The cancer cell may be a cancer cell *in vivo* (i.e., in an organism), *ex vivo* (i.e., removed from an organism and maintained in vitro), or *in vitro*.

The methods involve administering to a subject a combination of two or more inhibitors of epigenetic genes in an effective amount. In some embodiments, the subject is a subject having, suspected of having, or at risk of developing cancer. In some embodiments, the subject is a mammalian subject, including but not limited to a dog, cat, horse, cow, pig, sheep, goat, chicken, rodent, or primate. In some embodiments, the subject is a human subject, such as a patient. The human subject may be a pediatric or adult subject. Whether a subject is deemed "at risk" of having a cancer may be determined by a skilled practitioner.

As used herein "treating" includes ameliorating, curing, preventing it from becoming worse, slowing the rate of progression, or preventing the disorder from re-occurring (i.e., to prevent a relapse). An effective amount of a composition refers to an amount of the composition that results in a therapeutic effect. For example, in methods for treating cancer in a subject, an effective amount of a combination of inhibitors targeting epigenetic genes is any amount that provides an anti-cancer effect, such as reduces or prevents proliferation of a cancer cell or is cytotoxic towards a cancer cell. In some embodiments, the effective amount of an inhibitor targeting an epigenetic gene is reduced when an inhibitor is administered concomitantly or in combination with one or more additional inhibitors targeting epigenetic genes as compared to the effective amount of the inhibitor when administered in the absence of one or more additional inhibitors targeting epigenetic genes. In some embodiments, the inhibitor targeting an epigenetic gene does not reduce or prevent proliferation of a cancer cell when administered in the absence of one or more additional inhibitors targeting epigenetic genes.

Inhibitors targeting epigenetic genes or combinations of inhibitors targeting epigenetic genes (*e.g.*, combinations of epigenetic genes presented in Table 2) may be administered to a subject using any method known in the art. In some embodiments, the inhibitors are administered by a topical, enteral, or parenteral route of administration. In some embodiments, the inhibitors are administered intravenously, intramuscularly, or subcutaneously.

Any of the inhibitors of epigenetic genes described herein may be administered to a subject, or delivered to or contacted with a cell by any methods known in the art. In some embodiments, the inhibitors of epigenetic genes are delivered to the cell by a nanoparticle, cell-permeating peptide, polymer, liposome, or recombinant expression vector. In other embodiments, the inhibitors of epigenetic genes are conjugated to one or more nanoparticle, cell-permeating peptide, and/or polymer. In other embodiments, the inhibitors of epigenetic genes are contained within a liposome.

Also provided are methods for identifying combinations of inhibitors of epigenetic genes that reduce or prevent proliferation of a cell or population of cells. As depicted in FIG. 4A, the methods involve contacting two populations of cells with a combinatorial library of CRISPR guide sequences targeting epigenetic genes and scaffold sequences (e.g., a barcoded CRISPR library) and a Cas9 endonuclease. The two populations of cells are cultured for different durations of time. For example, one population of cells may be cultured for 15 days and the other population of cells is cultured for 20 days. The identification of the combinations of two or more CRISPR guide sequences and scaffold sequences are determined for each population of cells, *e.g.* by sequencing methods. For example, the CRISPR guide sequences and scaffold sequences may be identified by sequencing a barcode that is a unique identifier of the CRISPR guide sequence. The abundance of each combination of CRISPR guide sequences and scaffold sequences in the population of cells that was cultured for a longer duration of time is compared to the abundance of each combination of CRISPR guide sequences and scaffold sequences in the population of cells that was cultured for the shorter duration of time. Combinations of CRISPR guide sequences and scaffold sequences that reduced proliferation of the cells will be less abundant in the population of cells that was cultured for the longer duration of time compared to the abundance of the CRISPR guide sequence in the population of cells that was cultured for the shorter duration of time. Such combinations are identified as combinations of inhibitors of epigenetic genes that reduce cell proliferation.

Other methods are provided for identifying combinations of epigenetic genes that when inhibited reduce or prevent proliferation of a cell or population of cells. As depicted in FIG. 4A, the methods involve contacting two populations of cells with a combinatorial library of CRISPR guide sequences targeting epigenetic genes and scaffold sequences (e.g., a barcoded CRISPR library) and a Cas9 endonuclease. The two populations of cells are cultured for different durations of time. For example, one population of cells may be cultured for 15 days and the other population of cells is cultured for 20 days. The identification of the combinations of two or more CRISPR guide sequences and scaffold sequences are determined for each population of cells, *e.g.* by sequencing methods. For example, the CRISPR guide sequences and scaffold sequences may be identified by sequencing a barcode that is a unique identifier of the CRISPR guide sequence. The abundance of each combination of CRISPR guide sequences and scaffold sequences in the population of cells that was cultured for a longer duration of time is compared to the abundance of each combination of CRISPR guide sequences and scaffold sequences in the population of cells that was cultured for the shorter duration of time. Combinations of CRISPR guide sequences and scaffold sequences that reduced proliferation of the cells will be less abundant in the population of cells that was cultured for the longer duration of time. Such combinations are identified as combinations of epigenetic genes that may be target by inhibitors to reduce or prevent cell proliferation.

In some embodiments, one or more of the genes or inhibitors targeting an epigenetic gene associated with the invention is expressed in a recombinant expression vector. As used herein, a "vector" may be any of a number of nucleic acids into which a desired sequence or sequences may be inserted by restriction and ligation (*e.g.*, using the CombiGEM method) or by recombination for transport between different genetic environments or for expression in a host cell *(e.g.,* a cancer cell). Vectors are typically composed of DNA, although RNA vectors are also available. Vectors include, but are not limited to: plasmids, fosmids, phagemids, virus genomes and artificial chromosomes. In some embodiments, the vector is a lentiviral vector. In some embodiments, two or more genes or inhibitors targeting epigenetic genes are expressed on the same recombinant expression vector. In some embodiments, two or more genes or inhibitors targeting epigenetic genes are expressed on two or more recombinant expression vectors.

A cloning vector is one which is able to replicate autonomously or integrated in the genome in a host cell, and which is further characterized by one or more endonuclease restriction sites at which the vector may be cut in a determinable fashion and into which a desired DNA sequence may be ligated or recombination sites at which an insert with compatible ends can be integrated such that the new recombinant vector retains its ability to replicate in the host cell. In the case of plasmids, replication of the desired sequence may occur many times as the plasmid increases in copy number within the host cell such as a host bacterium or just a single time per host before the host reproduces by mitosis. In the case of phage, replication may occur actively during a lytic phase or passively during a lysogenic phase.

An expression vector is one into which a desired DNA sequence may be inserted by restriction and ligation or recombination such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (e.g., β-galactosidase, luciferase or alkaline phosphatase), and genes which visibly affect the phenotype of transformed or transfected cells, hosts, colonies or plaques (e.g., green fluorescent protein, red fluorescent protein). Preferred vectors are those capable of autonomous replication and expression of the structural gene products present in the DNA segments to which they are operably joined.

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript can be translated into the desired protein or polypeptide.

When the nucleic acid molecule is expressed in a cell, a variety of transcription control sequences (e.g., promoter/enhancer sequences) can be used to direct its expression. The promoter can be a native promoter, i.e., the promoter of the gene in its endogenous context, which provides normal regulation of expression of the gene. In some embodiments the promoter can be constitutive, i.e., the promoter is unregulated allowing for continual transcription of its associated gene. A variety of conditional promoters also can be used, such as promoters controlled by the presence or absence of a molecule. In some embodiments, the promoter is a RNA polymerase II promoter, such as a mammalian RNA polymerase II promoter. In some embodiments, the promoter is a human ubiquitin C promoter (UBCp). In some embodiments, the promoter is a viral promoter. In some embodiments, the promoter is a human cytomegalovirus promoter (CMVp). In some embodiments, the promoter is a RNA polymerase III promoter. Examples of RNA polymerase III promoters include, without limitation, HI promoter, U6 promoter, mouse U6 promoter, swine U6 promoter. In some embodiments, the promoter is a U6 promoter (U6p).

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. In particular, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

Expression vectors containing all the necessary elements for expression are commercially available and known to those skilled in the art. See, e.g., Sambrook et al., Molecular Cloning: A Laboratory Manual, Fourth Edition, Cold Spring Harbor Laboratory Press, 2012. Cells are genetically engineered by the introduction into the cells of heterologous DNA (RNA). That heterologous DNA (RNA) is placed under operable control of transcriptional elements to permit the expression of the heterologous DNA in the host cell.

A nucleic acid molecule associated with the invention can be introduced into a cell or cells using methods and techniques that are standard in the art. For example, nucleic acid molecules can be introduced by standard protocols such as transformation including chemical transformation and electroporation, viral transduction, particle bombardment, etc. In some embodiments, the viral transduction is achieved using a lentivirus. Expressing the nucleic acid molecule may also be accomplished by integrating the nucleic acid molecule into the genome.

The invention is not limited in its application to the details of construction and the arrangement of components set forth in the following description or illustrated in the drawings. The invention is capable of other embodiments and of being practiced or of being carried out in various ways. Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

The present invention is further illustrated by the following Examples, which in no way should be construed as further limiting.

EXAMPLES

Example 1

As shown in FIG. 1, a high complexity combinatorial library of barcoded CRISPR molecules can be made using the methods described herein. In step 1, a massive CRISPR guide RNA (gRNA) library was generated using array-based oligonucleotide synthesis, including forward and reverse oligonucleotides for each CRISPR guide sequence (e.g, Oligo F-A and Oligo R-A; Oligo F-B and Oligo R-B). The length of the oligonucleotide synthesized is independent of the complexity of the end-product library. In step 2, the pair of oligonucleotides for a gRNA are then isolated and annealed. An oligonucleotide against each gRNA contains the 20-base pair CRISPR guide sequence, two BbsI sites, and a barcode element. The oligonucleotide may also contain 5' and 3' single stranded overhang regions for ligation of the oligonucleotide into a storage vector. In step 3, annealed oligo pairs containing BbsI and MfeI overhangs were pooled together for a one-pot ligation reaction to insert the gRNA library into the AWp28 storage vector digested with BbsI and MfeI. This results in a library of AWp28 storage vectors each containing a CRISPR guide sequence, two BbsI sites, and a barcode element.

In step 4, the pooled library of storage vectors then underwent a single-pot digestion using BbsI to open the vector between the CRISPR guide sequence and the barcode element to allow for the insertion of gRNA scaffold element in between the CRISPR guide sequence and the barcode element. Since the insert of gRNA scaffold element contains a separation site formed by the restriction recognition sites BamHI and EcoRI at the 3'end of the scaffold element, the library of gRNA storage vectors can undergo iterative cloning steps to generate progressively more complex n-wise barcoded library encompassing multiple gRNA expression cassettes that can mediate combinatorial gene knockout, activation or repression using Cas9 nuclease or effectors, as shown in step 5. Briefly, the barcoded guide RNA library is digested using restriction enzyme complementary to the restriction recognition sites at the separation site (e.g., BamHI and EcoRI). Digestion allows for insertion of additional segments encoding a CRISPR guide sequence, scaffold sequence, separation site, and barcode element. Because the same sets of restriction enzymes can be used for the multiple rounds of cloning to build an (n)-wise library, one of the advantages of this strategy is that the (n+1)-wise library does not increase the set of restriction enzymes required.

To build a (n)-wise library of (m) gRNA members, it takes (n + 2) rounds of cloning steps. An additional advantage of this strategy is that the same CRISPR guide libraries can be used to generate higher order complexity libraries.

Example 2:

As shown in FIG. 2A, a complex combinatorial libraries of barcoded CRISPR molecules can be made using the methods described herein. In step 1, multiple CRISPR guide sequences and a single barcode element were synthesized on a single oligonucleotide to generate a massive combinatorial gRNA library. Restriction recognition sites were present following each of the CRISPR guide sequences. Guide sequences and the barcode are linked together by the different restriction enzyme sites. The genetic construct and vector of FIG. 2A shows an exemplary oligonucleotide containing three CRISPR guide sequences and a barcode element located downstream of the CRISPR guide sequences.

In step 2, the pooled synthesized oligonucleotides were ligated into a destination vector in a single-pot assembly. The destination vector may contain an promoter to drive expression of at least one of the CRISPR guide sequences. As shown in step 3, the vector was sequentially digested at each of the restriction recognition sites following each CRISPR guide sequence with different restriction enzymes, allowing for insertion of a scaffold element, and in some cases a promoter element to drive expression of a downstream CRISPR guide sequence. The method resulted in the generation of a barcoded combinatorial guide RNA library encoding multiple CRISPR guide sequences and scaffold sequences with a single barcode element for combinatorial gene knockout, activation or repression using Cas9 nuclease or effectors.

FIG. 2B depicts an alternative strategy to generate a complex combinatorial libraries of barcoded CRISPR molecules. In step 1, multiple CRISPR guide sequences and a single barcode element were synthesized on a single oligonucleotide to generate a massive combinatorial gRNA library. Restriction recognition sites were present following each of the CRISPR guide sequences as well as a restriction recognition site upstream of the first CRISPR guide sequence for insertion of a promoter element. Guide sequences and the barcode are linked together by the different restriction enzyme sites. The genetic construct and vector of FIG. 2B shows an exemplary oligonucleotide containing three CRISPR guide sequences and a barcode element located upstream of the CRISPR guide sequences.

In step 2, the pooled synthesized oligonucleotides were ligated into a destination vector in a single-pot assembly. The destination vector may contain an promoter to drive expression of at least one of the CRISPR guide sequences. As shown in step 3, the vector was sequentially digested at each of the restriction recognition sites following each CRISPR guide sequence with different restriction enzymes, allowing for insertion of a scaffold element, and in some cases a promoter element to drive expression of a downstream CRISPR guide sequence. The method resulted in the generation of a barcoded combinatorial guide RNA library encoding multiple CRISPR guide sequences and scaffold sequences with a single barcode element for combinatorial gene knockout, activation or repression using Cas9 nuclease or effectors.

Using the strategies depicted in FIGs. 2A and 2B, a (n)-wise library of (m) gRNA members can be built with (n+1) rounds of cloning steps. The complexity of the library generated is dependent on the length of the oligonucleotide synthesized in step 1. Because the restriction recognition sites that allow for insertion of the scaffold sequence are different for each CRISPR guide sequence as well as promoter elements, increasing the complexity of the oligonucleotide (*i.e.*, number of CRISPR guide sequences) also increases the number of restriction enzymes necessary for the digestion steps.

Example 3:

The CombiGEM-based DNA assembly method was used for the efficient and scalable assembly of barcoded combinatorial gRNA libraries. The libraries were delivered into human cells by lentiviruses in order to create genetically ultra-diverse cell populations harboring unique gRNA combinations that may be tracked via barcode sequencing in pooled assays. This strategy, termed CombiGEM-CRISPR, uses simple one-pot cloning steps to enable the scalable assembly of high-order combinatorial gRNA libraries, thus simplifying and accelerating the workflow towards systematic analysis of combinatorial gene functions.

To create the initial barcoded sgRNA library, an array of oligo pairs encoding a library of barcoded gRNA target sequences was first synthesized, annealed, and pooled in equal ratios for cloning downstream of a U6 promoter in the storage vector (FIG. 1). Subsequently, the scaffold sequence for the gRNAs was inserted into the storage vector library in a single-pot ligation reaction. The CombiGEM method was applied for scalable assembly of higher-order combinatorial gRNA libraries (FIG. 1). Within the barcoded sgRNA construct, BamHI and EcoRI sites were positioned in between the gRNA sequence and its barcode, while BglII and MfeI sites were located at the ends. Strategic positioning of these restriction enzyme sites results in the segregation of the barcode from its gRNA sequence upon enzymatic digestion and the concatenation of barcodes representing their respective gRNAs upon ligation of inserts. To construct the one-wise library, pooled inserts of the barcoded sgRNA expression units were prepared by restriction digestion of the storage vectors with BglII and MfeI and joined to their compatible DNA ends in the lentiviral destination vector, which was digested with BamHI and EcoRI. The one-wise library then served as the destination vector for the next round of pooled insertion of the barcoded sgRNA expression units to generate the two-wise library, in which barcodes representing each sgRNA were localized to one end of each lentiviral construct. This process may be iteratively repeated to generate higher-order barcoded combinatorial gRNA libraries. The identity of the combinatorial gRNAs can be tracked by detection of the concatenated barcodes, which are unique for each combination (*e.g.*, by high-throughput sequencing).

To evaluate the functionality of our lentiviral combinatorial gRNA expression system, gRNA combinations were constructed targeting sequences encoding green fluorescent protein (GFP) and red fluorescent protein (RFP) (Table 1). The combinatorial gene perturbation phenotypes were determined by using flow cytometry (FIGs. 6A and 6B) and fluorescence microscopy (FIG. 6E). Lentiviruses carrying dual RFP and GFP reporters together with the barcoded combinatorial gRNA expression units were used to infect human ovarian cancer cells (OVCAR8-ADR) (Honma, et al. Nat. Med. (2008) 14: 939-948) stably expressing human codon-optimized Cas9 nuclease (OVCAR8-ADR-Cas9) (FIG. 6A). It was anticipated that active gRNAs would target the sequences encoding GFP and RFP, and generate indels to knockout the expression of GFP and RFP. Efficient repression of GFP and RFP fluorescence levels was observed, as the GFP and RFP double-negative population was the major population observed in cells carrying Cas9 nuclease and gRNA expression units targeting both RFP and GFP at both day 4 and 8 post-infection (-83 to 97% of the total population), compared with < 0.7% in the vector control (FIGs. 6C and 6D). This repression was not observed in control cell lines expressing the gRNAs targeting GFP and/or RFP but without Cas9 nuclease (FIG. 6B). The specificity of gene perturbation was confirmed, as cells only harboring GFP-targeting sgRNA exhibited loss of the GFP signal but not the RFP signal. Similarly, cells containing the RFP-targeting sgRNAs exhibited a reduction in RFP expression, but there was not effect on GFP expression (FIG. 6E). These results demonstrate the ability of lentiviral vectors to encode combinatorial gRNA constructs that can repress the expression of multiple genes simultaneously within a single human cell.

Diverse epigenetic modifications tend to act cooperatively to regulate gene expression patterns (Wang, et al. Nat. Genet. (2008) 40:897-903), and combinatorial epigenetic modulation is emerging as a promising strategy for effective cancer therapeutics (Dawson, et al. Cell (2012) 150: 12-27; Juergens, et al. Cancer Discov. (2011) 1: 598-607). Using the CombiGEM-CRISPR methods and compositions described herein, the combinatorial effects of epigenetic gene perturbations on anti-cancer phenotypes were systematically evaluated. A library was constructed containing 153 barcoded sgRNAs targeting a set of 50 epigenetic genes (3 sgRNAs per gene) and 3 control sgRNAs based on the GeCKOv2 library (Shalem, et al. Science (2014) 343:84-87)(Table 1).

Table 1: sgRNA target sequences

| sgRNA ID | sgRNA target sequence |
|---|---|
| GFP-sg1 | GGGCGAGGAGCTGTTCACCG (SEQ ID NO: 7) |
| RFP-sg1 | CACCCAGACCATGAAGATCA (SEQ ID NO: 8) |
| RFP-sg2 | CCACTTCAAGTGCACATCCG (SEQ ID NO: 9) |
| Control-sg1 | ATCGTTTCCGCTTAACGGCG (SEQ ID NO: 10) |
| Control-sg2 | AAACGGTACGACAGCGTGTG (SEQ ID NO: 11) |
| Control-sg3 | CCATCACCGATCGTGAGCCT (SEQ ID NO: 12) |
| DNMT1-sg1 | CTAGACGTCCATTCACTTCC (SEQ ID NO: 13) |
| DNMT1-sg2 | TTTCCAAACCTCGCACGCCC (SEQ ID NO: 14) |
| DNMT1-sg3 | ACGTAAAGAAGAATTATCCG (SEQ ID NO: 15) |
| DNMT3A-sg1 | CCGCCCCACCTTCCGTGCCG (SEQ ID NO: 16) |
| DNMT3A-sg2 | TGGCGCTCCTCCTTGCCACG (SEQ ID NO: 17) |
| DNMT3A-sg3 | CCGCTCCGCAGCAGAGCTGC (SEQ ID NO: 18) |
| DNMT3B-sg1 | AGAGTCGCGAGCTTGATCTT (SEQ ID NO: 19) |
| DNMT3B-sg2 | ATCCGCACCCCGGAGATCAG (SEQ ID NO: 20) |
| DNMT3B-sg3 | GAAGACTCGATCCTCGTCAA (SEQ ID NO: 21) |
| DNMT3L-sg1 | AGGGATCTGCGCCCCATGTA (SEQ ID NO: 22) |
| DNMT3L-sg2 | ACTCACCTTCTATATTTCGC (SEQ ID NO: 23) |
| DNMT3L-sg3 | CACCAAAATCACGTCCATGC (SEQ ID NO: 24) |
| MBD1-sg1 | TCACCCGTAGGCAACGTCGC (SEQ ID NO: 25) |
| MBD1-sg2 | GTGTCCAGCGACGTTGCCTA (SEQ ID NO: 26) |
| MBD1-sg3 | ACGTTGTGCAAAGACTGTCG (SEQ ID NO: 27) |
| MBD2-sg1 | CGGCGACTCCGCCATAGAGC (SEQ ID NO: 28) |
| MBD2-sg2 | GGAGCCGGTCCCTTTCCCGT (SEQ ID NO: 29) |
| MBD2-sg3 | AGTCTTGAAAGCGCATGCCA (SEQ ID NO: 30) |
| CREBBP-sg1 | AGCGGCTCTAGTATCAACCC (SEQ ID NO: 31) |
| CREBBP-sg2 | GAATCACATGACGCATTGTC (SEQ ID NO: 32) |
| CREBBP-sg3 | CCCGCAAATGACTGGTCACG (SEQ ID NO: 33) |
| EP300-sg1 | CTGTCAGAATTGCTGCGATC (SEQ ID NO: 34) |

(continued)

| sgRNA ID | sgRNA target sequence |
|---|---|
| EP300-sg2 | CTTGGCAAGACTTGCCTGAC (SEQ ID NO: 35) |
| EP300-sg3 | TAGTTCCCCTAACCTCAATA (SEQ ID NO: 36) |
| HDAC1-sg1 | ACACCATTCGTAACGTTGCC (SEQ ID NO: 37) |
| HDAC1-sg2 | TCACTCGAGATGCGCTTGTC (SEQ ID NO: 38) |
| HDAC1-sg3 | AGAATGCTGCCGCACGCACC (SEQ ID NO: 39) |
| HDAC2-sg1 | TCCGTAATGTTGCTCGATGT (SEQ ID NO: 40) |
| HDAC2-sg2 | TCCAACATCGAGCAACATTA (SEQ ID NO: 41) |
| HDAC2-sg3 | TACAACAGATCGTGTAATGA (SEQ ID NO: 42) |
| SIRT1-sg1 | GTTGACTGTGAAGCTGTACG (SEQ ID NO: 43) |
| SIRT1-sg2 | AACAGGTTGCGGGAATCCAA (SEQ ID NO: 44) |
| SIRT1-sg3 | TACCCAGAACATAGACACGC (SEQ ID NO: 45) |
| CARM1-sg1 | CTCGCCGTTCGCGTCGCCGA (SEQ ID NO: 46) |
| CARM1-sg2 | CCCGTACTCACGGCTGTAGA (SEQ ID NO: 47) |
| CARM1-sg3 | GGGCCACGTACCGTTGGGTG (SEQ ID NO: 48) |
| EZH1-sg1 | ACAGGCTTCATTGACTGAAC (SEQ ID NO: 49) |
| EZH1-sg2 | AGCTGATCAATAACTATGAT (SEQ ID NO: 50) |
| EZH1-sg3 | CCTCATCTGAGTACTGATTC (SEQ ID NO: 51) |
| EZH2-sg1 | ACACGCTTCCGCCAACAAAC (SEQ ID NO: 52) |
| EZH2-sg2 | TGCGACTGAGACAGCTCAAG (SEQ ID NO: 53) |
| EZH2-sg3 | AAAACTTCATCTCCCATATA (SEQ ID NO: 54) |
| MLL-sg1 | GTACAAATTGTACGACGGAG (SEQ ID NO: 55) |
| MLL-sg2 | GACCCCTCGGCGGTTTATAG (SEQ ID NO: 56) |
| MLL-sg3 | TATATTGCGACCACCAAACT (SEQ ID NO: 57) |
| MLL2-sg1 | CAGAGAGCACAACGCCGCAC (SEQ ID NO: 58) |
| MLL2-sg2 | GGAACCGCTGGCAGTCGCGC (SEQ ID NO: 59) |
| MLL2-sg3 | CTCCCGCTGCCCGTGTAGAC (SEQ ID NO: 60) |
| NSD1-sg1 | CTGGCTCGAGATTTAGCGCA (SEQ ID NO: 61) |
| NSD1-sg2 | AATCTGTTCATGCGCTTACG (SEQ ID NO: 62) |
| NSD1-sg3 | GATTCCAGTACCAGTACATT (SEQ ID NO: 63) |
| PRMT1-sg1 | CTCACCGTGGTCTAACTTGT (SEQ ID NO: 64) |
| PRMT1-sg2 | GGATGTCATGTCCTCAGCGT (SEQ ID NO: 65) |
| PRMT1-sg3 | TTTGACTCCTACGCACACTT (SEQ ID NO: 66) |
| PRMT2-sg1 | CGTGGATGAGTACGACCCCG (SEQ ID NO: 67) |
| PRMT2-sg2 | TCTTCTGTGCACACTATGCG (SEQ ID NO: 68) |
| PRMT2-sg3 | CTGTCCCAGAAGTGAATCGC (SEQ ID NO: 69) |
| PRMT3-sg1 | GCCATGTGCTCGTTAGCGTC (SEQ ID NO: 70) |
| PRMT3-sg2 | GCCTGACGCTAACGAGCACA (SEQ ID NO: 71) |
| PRMT3-sg3 | GAATTCATGTACTCAACTGT (SEQ ID NO: 72) |

(continued)

| sgRNA ID | sgRNA target sequence |
|---|---|
| PRMT5-sg1 | CGGAATGCGGGGTCCGAACT (SEQ ID NO: 73) |
| PRMT5-sg2 | CAGCATACAGCTTTATCCGC (SEQ ID NO: 74) |
| PRMT5-sg3 | ATGAACTCCCTCTTGAAACG (SEQ ID NO: 75) |
| PRMT6-sg1 | ATTGTCCGGCGAGGACGTGC (SEQ ID NO: 76) |
| PRMT6-sg2 | CTTCGCCACGCGCTGTCTCA (SEQ ID NO: 77) |
| PRMT6-sg3 | GACGGTACTGGACGTGGGCG (SEQ ID NO: 78) |
| PRMT7-sg1 | CAATCCGACCACGGGGTCTG (SEQ ID NO: 79) |
| PRMT7-sg2 | GAGGTTCAAACCGCCTGCTA (SEQ ID NO: 80) |
| PRMT7-sg3 | TAAAGTCGGCTGGTGACACC (SEQ ID NO: 81) |
| SETD2-sg1 | AGTTCTTCTCGGTGTCCAAA (SEQ ID NO: 82) |
| SETD2-sg2 | GACTATCAGTTCCAGAGATA (SEQ ID NO: 83) |
| SETD2-sg3 | AACTTACGAAGGAAGGTCTT (SEQ ID NO: 84) |
| KDM1A-sg1 | TTACCTTCGCCCGCTTGCGC (SEQ ID NO: 85) |
| KDM1A-sg2 | CCGGCCCTACTGTCGTGCCT (SEQ ID NO: 86) |
| KDM1A-sg3 | AGAGCCGACTTCCTCATGAC (SEQ ID NO: 87) |
| KDM1B-sg1 | CATACCGCATCGATAAGTCT (SEQ ID NO: 88) |
| KDM1B-sg2 | ATAGCCAAGACTTATCGATG (SEQ ID NO: 89) |
| KDM1B-sg3 | GAACATACCTTCTGTAGTAA (SEQ ID NO: 90) |
| KDM2A-sg1 | ACGCTACTATGAGACCCCAG (SEQ ID NO: 91) |
| KDM2A-sg2 | TATGGCAGGGAGTCGTCGCA (SEQ ID NO: 92) |
| KDM2A-sg3 | GTAACGAATCCTTTCTTCTT (SEQ ID NO: 93) |
| KDM2B-sg1 | CCTCGTTCTCGTCGTATCGC (SEQ ID NO: 94) |
| KDM2B-sg2 | GCGTTACTACGAGACGCCCG (SEQ ID NO: 95) |
| KDM2B-sg3 | CTTGGTCAAGCGTCCGACTG (SEQ ID NO: 96) |
| KDM3A-sg1 | TAAATGCCGAGAGTGTCGCT (SEQ ID NO: 97) |
| KDM3A-sg2 | GTCTGTCAAAACCGACTTCC (SEQ ID NO: 98) |
| KDM3A-sg3 | GATACTGCTTGGCTGTACTG (SEQ ID NO: 99) |
| KDM3B-sg1 | TCTTGTATGGGCGCCCCGTG (SEQ ID NO: 100) |
| KDM3B-sg2 | GCCTTGACTGTTACCGGCTC (SEQ ID NO: 101) |
| KDM3B-sg3 | TCCTGAGCCGGTAACAGTCA (SEQ ID NO: 102) |
| KDM4A-sg1 | ACTCCGCACAGTTAAAACCA (SEQ ID NO: 103) |
| KDM4A-sg2 | GCGGAACTCTCGAACAGTCA (SEQ ID NO: 104) |
| KDM4A-sg3 | TTCCACTCACTTATCGCTAT (SEQ ID NO: 105) |
| KDM4B-sg1 | CCCCGCGTACTTCTCGCTGT (SEQ ID NO: 106) |
| KDM4B-sg2 | GTATGATGACATCGACGACG (SEQ ID NO: 107) |
| KDM4B-sg3 | TCACCAGGTACTGTACCCCG (SEQ ID NO: 108) |
| KDM4C-sg1 | CCTTTGCAAGACCCGCACGA (SEQ ID NO: 109) |
| KDM4C-sg2 | AGTAGGCTTCGTGTGATCAA (SEQ ID NO: 110) |

(continued)

| sgRNA ID | sgRNA target sequence |
|---|---|
| KDM4C-sg3 | GTCTAAAGGAGCCCATCGTG (SEQ ID NO: 111) |
| KDM5A-sg1 | CATGAACCCCAACGTGCTAA (SEQ ID NO: 112) |
| KDM5A-sg2 | CTGGGATTCAAATAACTCGG (SEQ ID NO: 113) |
| KDM5A-sg3 | TCTCTGGTATGAAAGTGCCG (SEQ ID NO: 114) |
| KDM5B-sg1 | GTCCGCGAACTCTTCCCAGC (SEQ ID NO: 115) |
| KDM5B-sg2 | TCGAAGACCGGGCACTCGGG (SEQ ID NO: 116) |
| KDM5B-sg3 | GGACTTATTTCAGCTTAATA (SEQ ID NO: 1 17) |
| KDM5C-sg1 | CTTACCGCCATGACACACTT (SEQ ID NO: 118) |
| KDM5C-sg2 | GATAAACAATGCGTTCGTAG (SEQ ID NO: 119) |
| KDM5C-sg3 | GGGCTACCCGAGCCCACCGA (SEQ ID NO: 120) |
| KDM5D-sg1 | GATTTACTCCTCGCGTCCAA (SEQ ID NO: 121) |
| KDM5D-sg2 | AAAGACTTACCGCGGGTGGG (SEQ ID NO: 122) |
| KDM5D-sg3 | TAAGGCCCGACATGGAACCG (SEQ ID NO: 123) |
| KDM6A-sg1 | ACTGTAAACTGTAGTACCTC (SEQ ID NO: 124) |
| KDM6A-sg2 | CAGCATTATCTGCATACCAG (SEQ ID NO: 125) |
| KDM6A-sg3 | AGACTATGAGTCTAGTTTAA (SEQ ID NO: 126) |
| KDM6B-sg1 | TACCACAGCGCCCTTCGATA (SEQ ID NO: 127) |
| KDM6B-sg2 | ATCCCCCTCCTCGTAGCGCA (SEQ ID NO: 128) |
| KDM6B-sg3 | CAAAGGCTTCCCGTGCAGCG (SEQ ID NO: 129) |
| PHF2-sg1 | TTCTGCACGGGCTTGACGTC (SEQ ID NO: 130) |
| PHF2-sg2 | GACGTCAAGCCCGTGCAGAA (SEQ ID NO: 131) |
| PHF2-sg3 | CAGTGACGTCGAGAACTACG (SEQ ID NO: 132) |
| PHF8-sg1 | TCTGACGAACGTAGGGCTCC (SEQ ID NO: 133) |
| PHF8-sg2 | GGCTTAGTGAAAAAACGCCG (SEQ ID NO: 134) |
| PHF8-sg3 | CCTCGCCATCATTCACTGTG (SEQ ID NO: 135) |
| BMI1-sg1 | AACGTGTATTGTTCGTTACC (SEQ ID NO: 136) |
| BMI1-sg2 | TCCTACCTTATATTCAGTAG (SEQ ID NO: 137) |
| BMI1-sg3 | AAAGGTTTACCATCAGCAGA (SEQ ID NO: 138) |
| BRD1-sg1 | CACCGTGTTCTATAGAGCCG (SEQ ID NO: 139) |
| BRD1-sg2 | CGGCGCGAGGTGGACAGCAT (SEQ ID NO: 140) |
| BRD1-sg3 | CGACTCACCGGCTGCGATCC (SEQ ID NO: 141) |
| BRD3-sg1 | CGACGTGACGTTTGCAGTGA (SEQ ID NO: 142) |
| BRD3-sg2 | CAAAGGTCGGAAGCCGGCTG (SEQ ID NO: 143) |
| BRD3-sg3 | CATCACTGCAAACGTCACGT (SEQ ID NO: 144) |
| BRD4-sg1 | ACTAGCATGTCTGCGGAGAG (SEQ ID NO: 145) |
| BRD4-sg2 | TCTAGTCCATCCCCCATTAC (SEQ ID NO: 146) |
| BRD4-sg3 | GGGAACAATAAAGAAGCGCT (SEQ ID NO: 147) |
| ING1-sg1 | GAGATCGACGCGAAATACCA (SEQ ID NO: 148) |

(continued)

| sgRNA ID | sgRNA target sequence |
|---|---|
| ING1-sg2 | TATAAATCCGCGCCCGAAAG (SEQ ID NO: 149) |
| ING1-sg3 | CCCATACCAGTTATTGCGCT (SEQ ID NO: 150) |
| ING2-sg1 | GCAGCAGCAACTGTACTCGT (SEQ ID NO: 151) |
| ING2-sg2 | GCAGCGACTCCACGCACTCA (SEQ ID NO: 152) |
| ING2-sg3 | GATCTTCAAGAAGACCCCGC (SEQ ID NO: 153) |
| ING3-sg1 | TCTCGCGCATTTCCGTGAAG (SEQ ID NO: 154) |
| ING3-sg2 | CTTCACGGAAATGCGCGAGA (SEQ ID NO: 155) |
| ING3-sg3 | TCGATACTGCATTTGTAATC (SEQ ID NO: 156) |
| ING4-sg1 | GCTGCTCGTGCTCGTTCCAA (SEQ ID NO: 157) |
| ING4-sg2 | CCTAGAAGGCCGGACTCAAA (SEQ ID NO: 158) |
| ING4-sg3 | GGCACTACTCATATACTCAG (SEQ ID NO: 159) |
| ING5-sg1 | GATCTGCTTCAAAGCGCGCC (SEQ ID NO: 160) |
| ING5-sg2 | CTTCCAGCTGATGCGAGAGC (SEQ ID NO: 161) |
| ING5-sg3 | GAAGTTCCTCTGAAGTTCGC (SEQ ID NO: 162) |

Expression of these 50 epigenetic genes was evaluated in OVCAR8-ADR cells using qRT-PCR. A two-wise (153 x 153 sgRNAs = 23,409 total combinations) pooled barcoded gRNA library was generated using the CombiGEM method. Lentiviral pools were produced to deliver the library into OVCAR8-ADR-Cas9 cells. Genomic DNA from the pooled cell populations was isolated for unbiased barcode amplification by polymerase chain reaction (PCR). The representation of individual barcoded combinations in the plasmid pools stored in *Escherichia coli* and also in the infected human cell pools was quantified using Illumina HiSeq sequencing (FIGs. 3A-3D). Near-full coverage for the two-wise library was achieved within both the plasmid and infected cell pools with between -23 to 34 million reads per sample (FIG. 3B), and a relatively even distribution of barcoded gRNA combinations was observed (FIGs. 3A and 3B). Furthermore, there was a high correlation between barcode representation in the plasmid and infected cell pools (FIG. 3C), as well as high reproducibility in barcodes represented in biological replicates for infected cell pools (FIG. 3D). Thus, CombiGEM-CRISPR can be used to efficiently assemble and deliver barcoded combinatorial gRNA libraries into human cells.

To confirm the function of gRNAs to edit endogenous genes in OVCAR8-ADR-Cas9 cells, Surveyor assays were performed to estimate the cleavage efficiency at 8 randomly picked loci targeted by the gRNAs from the library. Indel generation efficiencies ranging from 1.9% to 26.2% were observed at day 12 post-infection (FIGs. 7A-7C). Cleavage of DNA mismatches for all of the gRNA-targeted loci at day 12 post-infection were detected (FIGs. 7A and 8A). The simultaneous cleavage efficiency was determined at multiple loci in our dual-gRNA system, and comparable levels of cleavage were observed in cells expressing individual gRNAs or double gRNAs (FIGs. 7A and 8A). Depletion of targeted protein levels in individual gRNA- and double gRNA-expressing cells was also detected (FIG. 8B). These results indicated that the multiplexed system did not hamper the activity of the gRNAs. To distinguish dual-cleavage events directed by double gRNAs within a single cell from the whole infected population, clones derived from single cells infected with the double gRNAs were isolated and cells with insertions, deletions, or mutations in both targeted genomic loci were detected using Sanger sequencing (FIGs. 9A and 9B).

Indel generation efficiency was also estimated by performing deep sequencing at targeted genomic loci. Large variations in the rates of generating indels were observed (i.e., 14 to 93%; FIG. 16A) and frameshift mutations (i.e., 52 to 95% out of all indels; FIG. S5B) among different gRNAs. In addition, gRNAs that were validated in a previous study with A375 melanoma cells (Shalem et al. 2014 Science 343:84-7) displayed reduced activity *(e.g.,* for NF1-sg4 and MED12-sg1 sgRNAs) and differential indel generation preferences *(e.g.,* for the NF1-sg1 sgRNA) in OVCAR8-ADR-Cas9 cells (Fig. 16C). Such discrepancies may be partially due to variations in chromatin accessibility at target loci (Wu et al. (2014) Nat. Biotechnol. 32: 670-6) and DNA break repair mechanisms (Ghezraoui et al (2014) Mol Cell 55:829-42) that can vary among cell types. Continual efforts in gRNA design optimization, including improving on-target cleavage rates (Donesch et al (2014) Nat Biotechnol 32:1262-7) and minimizing off-target cleavage, should enable the creation of more efficient gRNA sets that will improve their applicability for large-scale genetic perturbation screening in a broad range of cell types. Indel generation was further assessed by gRNAs in the multiplexed system. The deep sequencing analysis detected largely comparable indel generation frequencies and preferences for the same gRNA expressed under the sgRNA or double gRNA systems (FIG. 16D). To distinguish dual-cleavage events directed by double gRNAs within a single cell from cleavage events distributed across the population, clones derived from single cells infected with double gRNA constructs were isolated. Cells with insertions, deletions, or mutations in both targeted genomic loci were detected (Fig. 9A-9C; Table 6). Our results indicate that the CombiGEM combinatorial gRNA library can be used to generate double genetic mutants in OVCAR8-ADR-Cas9 cells.

A pooled combinatorial genetic screen with OVCAR8-ADR-Cas9 cells was initiated to identify gRNA combinations that regulate cancer cell proliferation. A mathematical model was constructed to map out how relative changes in abundances of each library member within a population depend on various parameters (see Methods below; FIGs. 17A and 17B). Populations containing heterogeneous subpopulations that harbor different gRNA combinations were simulated. Specifically, specific percentages of the overall population were defined at the start of the simulation as harboring subpopulations with anti-proliferative ($f_s$) and pro-proliferative ($f_f$) gRNA combinations. Within each subpopulation, a fraction of cells was mutated by the CRISPR-Cas9 system ($p$) at the start of the simulation, resulting in a modified doubling time ($T_{doubling,m}$). The model indicated that the representation of barcoded cells with an anti-proliferative gRNA set in the entire cell population can be depleted by about 23 to 97% under simulated conditions (i.e., $f_s$, and $f_f$ = 2, 5, or 10%; $p$ = 0.2, 0.4, 0.6, 0.8, or 1.0; $T_{doubling,m}$= 36, 48, or 60 hours) (FIG. 17B). In general, increasing mutation efficiencies, increasing doubling times for anti-proliferative cells, decreasing doubling times for pro-proliferative cells, as well as increasing the percentage of pro-proliferative combinations in the population (FIG. 17C), are expected to result in greater barcode depletion of anti-proliferative barcodes in the overall population.

In the experimental screen, to identify gRNA combinations that regulate cancer cell proliferation, the OVCAR8-ADR-Cas9 cell populations infected with the two-wise combinatorial gRNA library were cultured for 15 and 20 days, then genomic DNA was isolated from the cells for unbiased amplification and quantification of the integrated barcodes (FIGs. 4A, 10A and 10B). Comparison of the barcode abundances (normalized per million reads) between the day 20 and day 15 groups yielded $\log_2$ (barcode count ratios) values (FIGs. 4A, 10A, and 10B). Guide RNA combinations inhibiting cell proliferation were expected to yield negative $\log_2$ ratios, while those conferring cells with growth advantages were expected to have positive $\log_2$ ratios. To reduce variability, combinations with less than -100 absolute reads in the day 15 group were filtered out, and the $\log_2$ ratios of the two potential arrangements for each gRNA pair (i.e., *sgRNA-A* + *sgRNA-B* and *sgRNA-B* + *sgRNA-A)* were averaged (FIG. 11). $\text{Log}_2$ ratios for each gRNA combination were determined for two biological replicates and ranked (FIGs. 5B and 12A). The majority of the gRNA combinations did not exhibit significant changes in barcode representations between the day 15 and day 20 groups, including three control gRNAs from the GeCKOv2 library (Shalem et al (2014) Science 343: 84-7) that do not have on-target loci in the human genome as internal controls. Sixty-one gRNA combinations were shown to exert considerable anti-proliferative effects ($\log_2$ ratio < -0.90) in both biological replicates (Q-value <0.01, Table 2 and FIG. 12B), yielding potential sets of genes to investigate further for their ability to suppress the growth of cancer cells.

Table 2: Two-wise sgRNA hits that inhibit OVCAR8-ADR cell proliferation based on pooled screening

| **sgRNA-A** | **sgRNA-B** | **$\text{Log}_2$ ratio - Day 20/Day15 Replicate 1** | **$\text{Log}_2$ ratio - Day 20/Day15 Replicate 2** | **Z-score Replicate 1** | **Z-score Replicate 2** | **Q value** |
|---|---|---|---|---|---|---|
| BRD4_sg3 | MLL_sg3 | -2.61 | -3.32 | -8.45 | -10.72 | 7.01E-33 |
| BMI1_sg2 | HDAC2_sg3 | -3.91 | -1.93 | -12.62 | -6.29 | 3.41E-32 |
| BMI1_sg2 | KDM1B_sg3 | -1.34 | -3.71 | -4.39 | -11.99 | 1.20E-23 |
| ING3_sg3 | BMI1_sg1 | -3.68 | -1.11 | -11.87 | -3.64 | 4.57E-21 |
| BRD4_sg3 | KDM6A_sg2 | -2.30 | -2.20 | -7.45 | -7.15 | 1.52E-18 |
| BRD4_sg3 | PHF2_sg2 | -1.57 | -2.88 | -5.12 | -9.31 | 4.05E-18 |
| BMI1_sg2 | PRMT6_sg1 | -2.80 | -1.59 | -9.05 | -5.19 | 1.19E-17 |

(continued)

| sgRNA-A | sgRNA-B | $Log_2$ ratio - Day 20/Day15 Replicate 1 | $Log_2$ ratio - Day 20/Day15 Replicate 2 | Z-score Replicate 1 | Z-score Replicate 2 | Q value |
|---|---|---|---|---|---|---|
| ING3_sg2 | KDM5A_sg2 | -2.28 | -2.04 | -7.40 | -6.65 | 3.48E-17 |
| KDM5B_sg3 | MLL_sg3 | -3.17 | -1.05 | -10.23 | -3.47 | 2.61E-16 |
| KDM6A_sg3 | KDM6A_sg2 | -1.95 | -2.26 | -6.35 | -7.33 | 2.61E-16 |
| BRD4_sg3 | KDM4C_sg1 | -1.48 | -2.28 | -4.84 | -7.40 | 7.88E-13 |
| KDM6B_sg1 | KDM3A_sg2 | -0.98 | -2.70 | -3.23 | -8.74 | 3.00E-12 |
| ING3_sg3 | KDM6A_sg2 | -1.46 | -2.10 | -4.79 | -6.81 | 2.02E-11 |
| PRMT5_sg3 | PRMT5_sg3 | -1.54 | -1.92 | -5.04 | -6.26 | 9.13E-11 |
| BRD4_sg3 | BRD4_sg2 | -2.45 | -1.00 | -7.93 | -3.30 | 1.24E-10 |
| BMI1_sg2 | NSD1_sg3 | -2.19 | -1.20 | -7.13 | -3.94 | 2.68E-10 |
| BMI1_sg2 | MBD2_sg3 | -1.44 | -1.93 | -4.70 | -6.27 | 3.94E-10 |
| BMI1_sg2 | KDM1A_sg1 | -1.62 | -1.40 | -5.29 | -4.59 | 5.85E-08 |
| KDM3A_sg2 | PRMT5_sg3 | -1.28 | -1.72 | -4.21 | -5.62 | 6.62E-08 |
| BRD4_sg3 | EP300_sg3 | -1.28 | -1.71 | -4.22 | -5.58 | 7.28E-08 |
| BMI1_sg2 | KDM3A_sg1 | -1.47 | -1.48 | -4.79 | -4.85 | 1.32E-07 |
| BRD4_sg3 | KDM6B_sg1 | -1.20 | -1.64 | -3.94 | -5.35 | 5.58E-07 |
| PHF8_sg2 | KDM1A_sg1 | -1.11 | -1.69 | -3.65 | -5.52 | 8.77E-07 |
| KDM6A_sg3 | HDAC2_sg3 | -1.03 | -1.77 | -3.40 | -5.77 | 8.77E-07 |
| BRD4_sg3 | KDM6B_sg2 | -0.92 | -1.86 | -3.05 | -6.05 | 1.13E-06 |
| PRMT5_sg3 | HDAC1_sg1 | -1.26 | -1.43 | -4.15 | -4.68 | 3.14E-06 |
| PHF8_sg2 | PRMT5_sg3 | -1.60 | -1.08 | -5.23 | -3.57 | 3.50E-06 |
| BRD4_sg3 | PHF2_sg1 | -1.51 | -1.13 | -4.94 | -3.71 | 5.35E-06 |

(continued)

| sgRNA-A | sgRNA-B | $Log_2$ ratio - Day 20/Day15 Replicate 1 | $Log_2$ ratio - Day 20/Day15 Replicate 2 | Z-score Replicate 1 | Z-score Replicate 2 | Q value |
|---|---|---|---|---|---|---|
| BRD4_sg3 | EZH2_sg3 | -1.02 | -1.57 | -3.38 | -5.12 | 8.80E-06 |
| PRMT5_sg3 | EZH1_sg1 | -0.96 | -1.62 | -3.19 | -5.28 | 9.53E-06 |
| KDM6A_sg2 | KDM5A_sg2 | -1.48 | -1.04 | -4.86 | -3.44 | 1.72E-05 |
| BRD4_sg3 | KDM2B_sg2 | -1.00 | -1.49 | -3.30 | -4.88 | 2.59E-05 |
| KDM6B_sg3 | PRMT5_sg3 | -1.03 | -1.43 | -3.41 | -4.67 | 3.60E-05 |
| BRD4_sg1 | KDM6B_sg1 | -1.30 | -1.15 | -4.27 | -3.79 | 3.74E-05 |
| KDM1A_sg3 | PRMT5_sg3 | -1.52 | -0.91 | -4.96 | -3.01 | 4.81E-05 |
| PRMT5_sg3 | EP300_sg2 | -1.41 | -1.00 | -4.61 | -3.31 | 5.44E-05 |
| BRD4_sg3 | PRMT5_sg2 | -1.46 | -0.91 | -4.79 | -3.02 | 7.59E-05 |
| KDM5D_sg2 | MLL_sg3 | -1.01 | -1.34 | -3.35 | -4.40 | 9.28E-05 |
| BRD4_sg3 | PRMT5_sg3 | -1.03 | -1.33 | -3.39 | -4.35 | 9.28E-05 |
| PHF2_sg1 | PRMT5_sg3 | -1.19 | -1.13 | -3.92 | -3.71 | 1.32E-04 |
| PRMT5_sg2 | DNMT1_sg1 | -1.28 | -1.02 | -4.21 | -3.37 | 1.54E-04 |
| KDM6A_sg2 | KDM5C_sg1 | -1.28 | -1.01 | -4.19 | -3.34 | 1.73E-04 |
| BMI1_sg2 | KDM6A_sg2 | -0.96 | -1.30 | -3.17 | -4.25 | 2.47E-04 |
| KDM1A_sg1 | PRMT5_sg3 | -0.95 | -1.29 | -3.15 | -4.24 | 2.68E-04 |
| BRD4_sg2 | KDM2B_sg1 | -1.06 | -1.17 | -3.51 | -3.85 | 2.82E-04 |
| KDM4A_sg2 | PRMT5_sg3 | -1.07 | -1.16 | -3.52 | -3.81 | 3.18E-04 |
| PRMT6_sg2 | PRMT5_sg2 | -1.02 | -1.13 | -3.37 | -3.74 | 6.20E-04 |
| KDM2B_sg3 | PRMT5_sg3 | -1.13 | -1.01 | -3.73 | -3.34 | 6.95E-04 |
| KDM6B_sg1 | MLL_sg3 | -0.92 | -1.19 | -3.05 | -3.90 | 9.39E-04 |

(continued)

| sgRNA-A | sgRNA-B | $Log_2$ ratio - Day 20/Day15 Replicate 1 | $Log_2$ ratio - Day 20/Day15 Replicate 2 | Z-score Replicate 1 | Z-score Replicate 2 | Q value |
|---|---|---|---|---|---|---|
| BRD4_sg3 | BMI1_sg2 | -0.94 | -1.14 | -3.11 | -3.76 | 1.10E-03 |
| KDM5A_sg3 | NSD1_sg3 | -0.91 | -1.14 | -3.03 | -3.75 | 1.37E-03 |
| BRD4_sg3 | KDM3A_sg2 | -0.92 | -1.13 | -3.04 | -3.74 | 1.37E-03 |
| KDM6B_sg3 | PRMT7_sg3 | -1.11 | -0.90 | -3.65 | -3.00 | 1.85E-03 |
| BRD3_sg3 | KDM4C_sg1 | -0.99 | -1.01 | -3.27 | -3.33 | 2.13E-03 |
| KDM4C_sg1 | PRMT5_sg3 | -0.93 | -1.06 | -3.08 | -3.51 | 2.15E-03 |
| KDM3B_sg1 | PRMT5_sg3 | -1.00 | -0.99 | -3.31 | -3.28 | 2.15E-03 |
| PRMT5_sg2 | MBD1_sg1 | -0.94 | -1.04 | -3.11 | -3.42 | 2.47E-03 |
| EP300_sg3 | MBD1_sg3 | -0.92 | -1.03 | -3.05 | -3.41 | 2.93E-03 |
| ING3_sg1 | BRD4_sg3 | -0.99 | -0.96 | -3.28 | -3.17 | 2.93E-03 |
| KDM1A_sg1 | HDAC2_sg3 | -0.94 | -0.95 | -3.12 | -3.14 | 4.71E-03 |
| PRMT5_sg2 | CARM1_sg1 | -0.95 | -0.91 | -3.14 | -3.02 | 5.78E-03 |

Hits from the screen were validated by evaluating the ability of the gRNA pair to inhibit the proliferation of OVCAR8-ADR-Cas9 cells (i.e., by ~33% within 5 days) in individual (non-pooled) cell growth assays using the corresponding gRNA pairs delivered via lentiviruses (FIG. 4C). There was high consistency between data collected from the pooled screen and individual validation assays (FIG. 13). Collectively, the methods described herein provide an experimental pipeline for the systematic screening of barcoded combinatorial gRNAs that are capable of exerting anti-proliferative effects on ovarian cancer cells.

Many gRNAs targeting epigenetic genes exhibited stronger anti-proliferative effects when used in combination with other epigenetic-gene-targeting gRNAs than when used in combination with control gRNAs (FIGs. 4B and 12A).

Off-target activity of the gRNAs was assessed by deep sequencing, which revealed a low indel generation rate (*i.e.*, 0.15 to 0.38%) at all exonic off-target genomic loci computationally predicted by the CRISPR design and CCTop tools for the two gRNAs (FIG. 19). Collectively, an experimental pipeline was established and validated for the systematic screening of barcoded combinatorial gRNAs that are capable of exerting anti-proliferative effects on ovarian cancer cells.

The gRNA pairs were confirmed with validation assays (FIGs. 5A and 8) and shRNA pairs (FIGs. 5B and 14) targeting KDM4C and BRD4 simultaneously led to synergistic reductions in cancer cell growth. Furthermore, co-treatment with the small-molecule KDM4C inhibitor SD70 (Jin, et al. PNAS (2014) 111:9235-9240) and small-molecule BRD4 inhibitor JQ1 (Asangani, et al. Nature (2014) 510:278-282)(FIG. 5C) inhibited the proliferation of OVCAR8-ADR cells synergistically. Similarly, gRNA pairs (FIGs. 5A and 8) and shRNA pairs (FIGs. 5B and 14) that simultaneously targeted KDM6B and BRD4 exhibited synergy, as did co-treatment with the KDM6B/6A inhibitor GSK-J4 (Kruidenier, et al. Nature (2012) 488: 404-408) and JQ1 (FIG. 5D). Synergy between both of these pairwise combinations of small-molecule drugs was confirmed by both the Bliss independence (Bliss Ann. Appl. Biol. (1939) 6: 585-615) and the Highest Single Agent (Borisy, et al. PNAS (2003) 100: 7977-7982) models (FIGs. 5C and 5D).

The methods described herein allow for the identification of novel epigenetic target gene pairs that inhibit cancer cell proliferation and the potential development of synergistic drug therapies. The methods also expand the utility of CRISPR-Cas9-based systems for performing systemic multiplexed genetic perturbation screens in a high-throughput capacity.

These methods can also help identify new areas for biological inquiry, such as studies into the mechanisms that underlie observed phenotypes. For example, gene expression patterns were evaluated in cell populations infected with lentiviruses encoding gRNAs targeting both KDM4C and BRD4, or KDM6B and BRD4 (FIG. 21A). Significantly perturbed genes were associated with gene sets involved in cancer-related pathways, including TNF$\alpha$/NF$\kappa$B signaling, p53 pathways, and apoptosis (Fig. 21B). In addition, the combinatorial effects of epigenetic perturbations are complex and can vary across different cell types (FIGs. 22A and 22B).

Methods

**Vector construction**

The vectors were constructed using standard molecular cloning techniques, including restriction enzyme digestion, ligation, PCR, and Gibson assembly (Table 3). Custom oligonucleotides were purchased from Integrated DNA Technologies. The vector constructs were transformed into *E. coli* strain DH5$\alpha$, and 50 $\mu$g/ml of carbenicillin (Teknova) was used to isolate colonies harboring the constructs. DNA was extracted and purified using Plasmid Mini or Midi Kits (Qiagen). Sequences of the vector constructs were verified with Genewiz's DNA sequencing service.

Table 3: Constructs

| **Construct ID** | **Design** |
|---|---|
| pAWp28 | pBT264-U6p-{2xBbsI}-sgRNA scaffold-{MfeI} |
| pAWp28-1 | pBT264-U6p-GFP-sg1 |
| pAWp28-2 | pBT264-U6p-RFP-sg1 |
| pAWp28-3 | pBT264-U6p-RFP-sg2 |
| pAWp28-4 | pBT264-U6p-KDM4C-sg1 |
| pAWp28-5 | pBT264-U6p-PHF2-sg1 |
| pAWp28-6 | pBT264-U6p-KDM6B-sg2 |
| pAWp28-7 | pBT264-U6p-PHF2-sg2 |
| pAWp28-8 | pBT264-U6p-DNMT1-sg1 |
| pAWp28-9 | pBT264-U6p-DNMT3B-sg1 |
| pAWp28-10 | pBT264-U6p-PRMT2-sg3 |
| pAWp28-11 | pBT264-U6p-HDAC2-sg1 |
| pAWp28-12 | pBT264-U6p-ING4-sg1 |
| pAWp28-13 | pBT264-U6p-KDM1B-sg3 |
| pAWp28-14 | pBT264-U6p-KDM2A-sg3 |
| pAWp28-15 | pBT264-U6p-PRMT6-sg1 |
| pAWp28-16 | pBT264-U6p-BMI1-sg2 |
| pAWp28-17 | pBT264-U6p-PHF8-sg2 |
| pAWp9 | pFUGW-UBCp-*RFP*-CMVp-*GFP*-{BamHI+EcoRI} |
| pAWp9-1 | pFUGW-UBCp-*RFP*-CMVp-*GFP*-U6p-GFP-sg1 |
| pAWp9-2 | pFUGW-UBCp-*RFP*-CMVp-*GFP*-U6p-RFP-sg 1 |
| pAWp9-3 | pFUGW-UBCp-*RFP*-CMVp-*GFP*-U6p-RFP-sg2 |
| pAWp9-4 | pFUGW-UBCp-*RFP*-CMVp-*GFP*-U6p-RFP-sg1-U6p-GFP-sg1 |
| pAWp9-5 | pFUGW-UBCp-*RFP*-CMVp-*GFP*-U6p-RFP-sg2-U6p-GFP-sg1 |

(continued)

| Construct ID | Design |
|---|---|
| pAWp11 | pFUGW-CMVp |
| pAWp12 | pFUGW-CMVp-*GFP* |
| pAWp12-1 | pFUGW-CMVp-*GFP*-[U6p-BRD4-sg3]-[U6p-PHF2-sg1] |
| pAWp12-2 | pFUGW-CMVp-*GFP*-[U6p-BRD4-sg3]-[U6p-KDM6B-sg2] |
| pAWp12-3 | pFUGW-CMVp-*GFP*-[U6p-BRD4-sg3]-[U6p-KDM4C-sg1] |
| pAWp12-4 | pFUGW-CMVp-*GFP*-[U6p-BRD4-sg3]-[U6p-PHF2-sg2] |
| pAWp12-5 | pFUGW-CMVp-*GFP*-[U6p-BRD4-sg3] |
| pAWp12-6 | pFUGW-CMVp-*GFP*-[U6p-KDM4C-sg1] |
| pA Wp12-7 | pFUGW-CMVp-*GFP*-[U6p-KDM6B-sg2] |
| pAWp12-8 | pFUGW-CMVp-*GFP*-[U6p-DNMT1-sg1] |
| pAWp12-9 | pFUGW-CMVp-*GFP*-[U6p-DNMT3B-sg1] |
| pAWp12-10 | pFUGW-CMVp-*GFP*-[U6p-PRMT2-sg3] |
| pAWp12-11 | pFUGW-CMVp-*GFP*-[U6p-HDAC2-sg1] |
| pAWp12-12 | pFUGW-CMVp-*GFP*-[U6p-ING4-sg1] |
| pAWp12-13 | pFUGW-CMVp-*GFP*-[U6p-KDM1B-sg3] |
| pAWp12-14 | pFUGW-CMVp-*GFP*-[U6p-KDM2A-sg3] |
| pAWp12-15 | pFUGW-CMVp-*GFP*-[U6p-PRMT6-sg1] |
| pAWp12-16 | pFUGW-CMVp-*GFP*-[U6p-BMI1-sg2]-[U6p-PHF8-sg2] |
| pAWp21 | pLKO.1-Control sh |
| pAWp21-1 | pLKO.1-KDM4C-sh1 |
| pAWp21-2 | pLKO.1-KDM4C-sh2 |
| pAWp21-3 | pLKO.1-KDM6B-sh |
| pAWp21-4 | pLKO.1-BRD4-sh1 |
| pAWp21-5 | pLKO.1-BRD4-sh2 |
| pAWp30 | pFUGW-EFSp-*Cas9-P2A-Zeo* |

To generate a lentiviral vector encoding an shRNA that targeted a specific gene, oligonucleotide pairs harboring the sense and antisense sequences were synthesized, annealed, and cloned in the AgeI- and EcoRI-digested pLKO.1 vector29 (Addgene plasmid #10879) by ligation. The shRNA sense and antisense sequences were designed and constructed based on the siRNA Selection Program (sirna.wi.mit.edu/) (Table 4).

Table 4: shRNA antisense sequences used for individual validation assays

| shRNA ID | shRNA antisense sequence |
|---|---|
| Control-sh | CGAGGGCGACTTAACCTTAGG (SEQ ID NO: 163) |
| KDM4C-sh1 | AAATCTTCGTAATCCAAGTAT (SEQ ID NO: 164) |
| KDM4C-sh2 | GTAATACCGGGTGTTCCGATG (SEQ ID NO: 165) |
| KDM6B-sh | ATTAATCCACACGAGGTCTCC (SEQ ID NO: 166) |
| BRD4-sh1 | TATAGTAATCAGGGAGGTTCA (SEQ ID NO: 167) |
| BRD4-sh2 | TTTAGACTTGATTGTGCTCAT (SEQ ID NO: 168) |

To generate the pAWp30 lentiviral expression vector encoding Cas9 protein and Zeocin resistance as the selection marker, the EFS promoter and Cas9 sequences were amplified from Addgene plasmid #49535, while the Zeocin sequence was amplified from Addgene plasmid #25736, by PCR using Phusion DNA polymerase (New England Biolabs). The PCR products were cloned into the pAWp11 lentiviral vector backbone using Gibson Assembly Master Mix (New England Biolabs).

To construct a storage vector containing U6 promoter (U6p)-driven expression of sgRNA that targeted a specific gene, oligo pairs with the 20 bp sgRNA target sequences were synthesized, annealed, and cloned in the BbsI-digested pAWp28 vector using T4 ligase (New England Biolabs). To construct a lentiviral vector for U6p-driven expression of single or combinatorial sgRNA(s), U6p-sgRNA expression cassettes were prepared from digestion of the storage vector with BglII and MfeI enzymes (Thermo Scientific), and inserted into the pAWp12 vector backbone or the single sgRNA expression vector, respectively, using ligation via the compatible sticky ends generated by digestion of the vector with BamHI and EcoRI enzymes (Thermo Scientific). To express the sgRNAs together with the dual RFP and GFP fluorescent protein reporters, the U6p-driven sgRNA expression cassettes were inserted into the pAWp9, instead of pAWp12, lentiviral vector backbone using the same strategy described above. The pAWp9 vector was modified from the pAWp7 vector backbone by introducing unique BamHI and EcoRI sites into the vector to enable the insertion of the U6p-sgRNA expression cassettes.

**Assembly of the barcoded combinatorial sgRNA library pool**

An array of 153 oligo pairs (Oligo F-(x) and Oligo R-(x), where x = 1 to 153) harboring the barcoded sgRNA sequences were synthesized, and annealed to generate double-stranded inserts harboring the 20 bp sgRNA target sequences, two BbsI restriction sites, 8 bp barcodes unique to each sgRNA while differed from each other by at least two bases, and 5' overhangs at their ends. To generate the pooled storage vector library, the 153 annealed inserts were mixed at equal ratios and cloned in the pAWp28 storage vector (digested with BbsI and MfeI) via a single pot of ligation reaction via their compatible ends. To build the barcoded sgRNA library, another one-pot ligation reaction was performed with the pooled storage vector library digested with BbsI, and an insert containing the sgRNA scaffold sequence, BamHI and EcoRI restriction sites, and 5' overhangs at their ends that was prepared via synthesis and annealing of an oligo pair S1 and S2. The pooled storage vector and the barcoded sgRNA libraries were both prepared in Endura competent cells (Lucigen) and purified by the Plasmid Midi kit (Qiagen).

Pooled lentiviral vector libraries harboring single or combinatorial gRNA(s) were constructed with same strategy as for the generation of single and combinatorial sgRNA constructs described above, except that the assembly was performed with pooled inserts and vectors, instead of individual ones. Briefly, the pooled U6p-sgRNA inserts were generated by a single-pot digestion of the pooled storage vector library with BglII and MfeI. The destination lentiviral vector (pAWp12) was digested with BamHI and EcoRI. The digested inserts and vectors were ligated via their compatible ends (i.e., BamHI + BglII & EcoRI + MfeI) to create the pooled one-wise sgRNA library (153 sgRNAs) in lentiviral vector. The one-wise sgRNA vector library was digested again with BamHI and EcoRI, and ligated with the same U6p-sgRNA insert pool to assemble the two-wise sgRNA library (153 x 153 sgRNAs = 23,409 total combinations). After the pooled assembly steps, the sgRNAs were localized to one end of the vector construct and their respective barcodes were concatenated at the other end. The lentiviral sgRNA library pools were prepared in XL10-Gold ultracompetent cells (Agilent Technologies) and purified by Plasmid Midi kit (Qiagen).

**Cell culture**

HEK293T cells were obtained from ATC, and were cultured in DMEM supplemented with 10% heat-inactivated fetal bovine serum and IX antibiotic-antimycotic (Life Technologies) at 37°C with 5% $CO_2$. OVCAR8-ADR cells were a gift from T. Ochiya (Japanese National Cancer Center Research Institute, Japan). The identity of the OVCAR8-ADR cells was authenticated (Genetica DNA Laboratories). OVCAR8-ADR cells stably expressing Cas9 protein (OVCAR-ADR-Cas9) were generated by lentiviral infection of OVCAR8-ADR cells with the pAWp30 vector and selected for three weeks in the presence of 200 μg/ml Zeocin (Life Technologies). OVCAR8-ADR and OVCAR8-ADR-Cas9 cells were cultured in RPMI supplemented with 10% heat-inactivated fetal bovine serum and IX antibiotic-antimycotic at 37°C with 5% $CO_2$. For drug treatment, SD70 (Xcessbio #M60194), GSK-J4 (Cayman Chemical #12073), and/or (+)-JQ1 (Cayman Chemical #11187) were used to treat OVCAR8-ADR cells at indicated drug doses prior to the cell viability assays.

**Lentivirus production and transduction**

Lentiviruses were produced and packaged in HEK283T cells in 6-well format. HEK293T cells were maintained at -70% confluency before transfection. FuGENE HD transfection reagents (Promega) were mixed with 0.5 μg of lentiviral vector, 1 μg of pCMV-dR8.2-dvpr vector, and 0.5 μg of pCMV-VSV-G vector in 100 μl of OptiMEM medium (Life Technologies), and were incubated for 15 minutes at room temperature before adding to cell culture. Culture medium was replaced the next day. Supernatant containing newly produced viruses were collected at 48-hour and 96-hour post-transfection, and filtered through a 0.45 μm polyethersulfone membrane (Pall). 500 μl of filtered viral supernatant was used to infect 250,000 cells in the presence of 8 μg/ml polybrene (Sigma) overnight for transduction with individual vector constructs. For pooled lentiviral library production used in the screens, lentivirus production and transduction were scaled up using the same experimental procedures. Filtered viral supernatant was concentrated using Amicon Ultra Centrifugal Filter Unit (Millipore). Cells were infected in the presence of 8 μg/ml polybrene at a multiplicity of infection of 0.3 to 0.5 to ensure single copy integration in most cells, which corresponded to an infection efficiency of 30-40%. The total number of cells used in the screening was approximately 300-fold more than the library sizes in order to maintain library coverage and reduce any spurious effects due to random lentiviral integration into the genome. Cell culture medium was replaced the next day after infection and cultured for indicated time periods prior to experiments.

**Sample preparation for barcode sequencing**

To prepare samples from cultured cells for barcode sequencing, genomic DNA was extracted and prepared using DNeasy Blood & Tissue Kit (Qiagen) according to the manufacturer's protocol. For the barcoded sgRNA plasmid libraries, plasmid DNA transformed into *E. coli* was extracted using the Plasmid Midi Kit (Qiagen). DNA concentrations were determined using Quant-iT PicoGreen dsDNA Assay Kit (Life Technologies).

A -360 bp fragment containing unique barcode representing each combination within the pooled vector and infected cell libraries was PCR amplified from the plasmid/genomic DNA samples using Kapa Hotstart Ready Mix (Kapa Biosystems). For plasmid DNA, 1 ng of DNA template was added for a 25-μl PCR reaction. For genomic DNA, 800 ng of DNA was added for a 50-μl PCR reaction and a total of 64 PCR reactions were performed for each genomic DNA sample to ensure that the number of cell genomes being amplified was more than 100 times the library size. Moreover, the PCR parameters were optimized to ensure that PCR amplification steps were maintained in the exponential phase to avoid PCR bias. The Illumina anchor sequences and an 8 base-pair indexing barcode were added during the PCR for multiplexed sequencing. The primer pair sequences used to amplify barcode sequence were: 5' -AATGATACGGCGACCACCGAGATCTACACGGATCCGCAACGGAATTC-3' (SEQ ID NO: 1) and 5'CAAGCAGAAGACGGCATACGAGATNNNNNNNNGGTTGCGTCAGCAAACACAG-3' (SEQ ID NO: 2), where NNNNNNNN indicates a specific indexing barcode assigned for each experimental sample.

The PCR products containing the barcode sequences were then purified based on fragment size by running on a 1.5% agarose gel and further extracted using the QIAquick Gel Extraction Kit (Qiagen). The PCR product concentrations were determined by quantitative PCR using KAPA SYBR Fast qPCR Master Mix (Kapa Biosystems) and the Illumina Library Quantification Kit (Kapa Biosystems). The forward and reverse primer used for quantitative PCR were 5'-AATGATACGGCGACCACCGA-3' (SEQ ID NO: 3) and 5'-CAAGCAGAAGACGGCATACGA-3' (SEQ ID NO: 4), respectively. The PCR products from different samples were then pooled at a desired ratio for multiplexed sample sequencing and loaded on the Illumina HiSeq system with CombiGEM barcode primer (5'-CCACCGAGATCTACACGGATCCGCAACGGAATTC-3' (SEQ ID NO: 5)) and indexing barcode primer (5'-GTGGCGTGGTGTGCACTGTGTTTGCTGACGCAACC-3' (SEQ ID NO: 6)).

**Barcode sequencing data analysis**

Barcode reads for each sgRNA combinations were processed from the Illumina sequencing data. Barcode reads representing each combination were normalized per million reads for each sample categorized by the indexing barcodes. As measures of cell proliferation, barcode count ratios of normalized barcode reads comparing day 20 against day 15 groups were calculated as fold changes. Pro-proliferation and anti-proliferation phenotypes had fold changes of normalized barcode reads of > 1 and < 1 respectively, while no phenotypic change resulted in a fold change = 1. Barcodes that gave less than -100 absolute reads in the day 15 group were filtered out to improve data reliability. The fold changes of the different possible orders of each same sgRNA combination were averaged, and high consistency in the fold-changes was observed (i.e., coefficient of variation (CV) < 0.2 and < 0.4 for over 82% and 95% of the combinations, respectively (FIG. 11). The calculated fold change was log transformed to give the log2 ratio. Screens were performed in two biological replicates with independent infections of the same lentiviral libraries. Combinations were ranked by the log2 ratio across all experimental conditions. The set of top hits (open circles) were defined as those with a $\log_2$ ratio that was at least three standard deviations from the mean of sgRNA combinations harboring only the control sgRNAs (open triangles) in both biological replicates (FIGs. 4B, 12A, and 12B).

**Cell viability assay**

The MTT colorimetric assay was performed to assess cell viability. For each 96 well, 100 μl of MTT (3-(4,5-dimethylthiazol-2-yl)-2,5-diphenyltetrazolium bromide) solution (Sigma) was added to the cell cultures. Cells were incubated for 3 hours at 37°C with 5% $CO_2$. Viable cells convert the soluble MTT salt to insoluble blue formazan crystals. Formazan crystals were dissolved with 100 μl of solubilization buffer at 37°C. Absorbance reading at an optical density (OD) of 570nm and 650nm (reference) were measured using a Synergy H1 Microplate Reader (BioTek).

**Drug synergy quantification**

The Bliss independence (BI) model (Bliss Ann. Appl.Biol. (1939) 26: 585-615) and the Highest Single Agent (HSA) model (Borisy, et al. PNAS (2003) 100: 7977-7982) are commonly used methods to evaluate synergy between drug combinations.

Based on the BI model, the expected effect ($E_{Exp}$) is given by:

$$E_{Exp} = E_A + E_B - (E_A \text{ x } E_B),$$

where $E_A$ is the growth inhibition effect observed at a certain concentration of drug A alone, and $E_B$ is the growth inhibition effect observed at a certain concentration of drug B alone. $E_{Obs}$ is the observed growth inhibition effect for the drug combination (A + B), each at the same concentration as in $E_A$ and $E_B$, respectively. Each effect is expressed as a fractional inhibition between 0 and 1. When $E_{Obs} - E_{Exp} > 0$, the two drugs are considered to be interacting synergistically.

The HSA model is similar to the BI model except, according to the HSA model, $E_{Exp}$ is equal to the larger of the growth inhibition effect produced by the combination's single drug agents ($E_A$ or $E_B$) at the same concentrations as in the drug combination (A + B).

Two drugs were considered to be synergistic if $E_{Obs} - E_{Exp} > 0.1$ (i.e. > 10% of excess inhibition over the predicted BI and HSA models in FIGs. 5C and 5D) for at least two different concentration combinations in both models to enhance the stringency of our criteria.

**Flow cytometry**

Cells were collected at 4-day and 8-day post infection. Samples were washed and resuspended in 1x PBS supplemented with 2% fetal bovine serum. To remove any clumps of cells, the resuspended cells were passed through cell strainers before loading onto the LSRII Fortessa flow cytometer (Becton Dickinson). At least 20,000 events were acquired per sample. Proper laser sets and filters were selected based on cell samples. Forward scatter and side scatter were used to identify appropriate cell populations. Data were analyzed using manufacturer's build-in software.

**Fluorescence microscopy**

Cultured cell were directly observed under an inverted fluorescent microscope (Zeiss) three days post-lentiviral infection. Images were captured using the Zeiss built-in software.

**Immunoblot analysis**

Cells were lysed in 2X RIPA buffer supplemented with protease inhibitors. Lysates were homogenized using a pestle motor mixer (Agros) for 30 seconds, and then centrifuged at 15,000 rpm for 15 min at 4°C. Supernatants were quantified using the BCA assay (Thermo Scientific). Protein was denatured at 99°C for 5 minutes before gel electrophoresis on a 4-15% polyacrylamide gel (Bio-Rad). Proteins were transferred to nitrocellulose membranes at 80V for 2 hours at 4°C. Primary antibodies used were: anti-BRD4 (1:2,000, Cell Signaling #13440), anti-KDM4C (1:1,000, Abcam ab85454) anti-KDM6B (1:1,000, Abcam ab85392), and anti-beta-actin (1:4,000, Abcam ab6276). Secondary antibodies used were: HRP-linked anti-rabbit IgG (1:2,000, Cell Signaling #7074), and HRP-linked anti-mouse IgG (1:4,000, Cell Signaling #7076). Membranes were developed by SuperSignal West Pico chemiluminescent substrate (Thermo Scientific) and imaged using a ChemiDoc Touch imaging system (BioRad).

**Surveyor assay and sequencing analysis for genome modification**

The Surveyor assay was carried out to evaluate DNA cleavage efficiency. Genomic DNA was extracted from cell cultures using QuickExtract DNA extraction solution (Epicentre) according to the manufacturer's protocol. Amplicons harboring the targeted loci were generated by PCR using Phusion DNA polymerase and primers listed in Table 5. About 200 ng of the PCR amplicons were denatured, self-annealed, and incubated with 1.5 μl of Surveyor Nuclease (Transgenomic) at 42°C for 30 minutes. The samples were then analyzed on a 2% agarose gel. The DNA band intensities were quantified using ImageJ software, and the indel occurence was estimated with the following formula (Ran et al. Nat. Protoc. (2013) 8:2281-2308):

$$\text{Indel } (\%) = 100 \text{ x } (1\text{- square root of } (1\text{-} f_{\text{cut}})),$$

with $f_{cut} = (b + c)/(a + b + c)$, where $a$ is the band intensity for the uncleaved PCR amplicon while $b$ and $c$ are the intensities for each cleaved band. The expected uncleaved and cleaved bands for the targeted alleles are listed in FIGs. 7 and 8.

Sanger sequencing was performed to analyze the genome modifications generated by the expression of combinatorial sgRNAs. Cells infected with the combinatorial sgRNA constructs were cultured for 12 days, and re-plated in 96-well plates as single cells by serial dilution of the cultures. Genomic DNA was extracted from the isolated single cell-expanded clones after culturing for 5 to 21 days using QuickExtract DNA extraction solution, and amplicons harboring the targeted alleles were prepared by PCR as described above. The PCR amplicons were cloned into a TOPO vector using TA Cloning Kit (Life Technologies) according to the manufacturer's protocol, and the nucleotide mutations, insertions, and deletions were identified using Sanger sequencing.

Table 5: List of PCR primers used in Surveyor assay and Sanger sequencing

| Target sgRNA ID | Forward primer (5' to 3') | Reverse primer (5' to 3') |
|---|---|---|
| BMI1-sg2 | AGAAATTAAACGGCTACCCTCCA (SEQ ID NO: 169) | GTTGGTACAAAGTGGTGAAGGC (SEQ ID NO: 183) |
| BRD4-sg2 | TCCATAGTGTCTTGAGCACCAC (SEQ ID NO: 170) | ACGTGGCTTCATTGTACATCCT (SEQ ID NO: 184) |
| BRD4-sg3 | CACTTGCTGATGCCAGTAGGAG (SEQ ID NO: 171) | AAGCACATGCTTCAGGCTAACA (SEQ ID NO: 185) |
| DNMT1-sg1 | GTGAATAGCTTGGGAATGTGGG (SEQ ID NO: 172) | TCATCTGCTCTTACGCTTAGCC (SEQ ID NO: 186) |
| DNMT3B-sg1 | GCCACACTCTACATGGGAGC (SEQ ID NO: 173) | CTCGGCAACCCTCCATACAT (SEQ ID NO: 187) |
| HDAC2-sg1 | GACTTTTCCATCAGGGACACCT (SEQ ID NO: 174) | AACCATGCACAGAATCCAGATTTA (SEQ ID NO: 188) |
| ING4-sg1 | GGTGGACAAACACATTCGGC (SEQ ID NO: 175) | AAGAGTTCTTGGCGCAGACA (SEQ ID NO: 189) |
| KDM1B-sg3 | CCTATCATTGCCCCAAGGAGTC (SEQ ID NO: 176) | TCGTCCAAGTTACAGTCATCACA (SEQ ID NO: 190) |
| KDM2A-sg3 | CTAGGCCTCCGACAGTTGTAAT (SEQ ID NO: 177) | TCCTCTGGTGCACAGAAAAGTC (SEQ ID NO: 191) |
| KDM4C-sg1 | AGCCACCCTTGGTTGGTTTT (SEQ ID NO: 178) | TTCTCTCCAGACACTGCCCT (SEQ ID NO: 192) |
| KDM6B-sg2 | GGTAAGGGAAACTCTGGGGC (SEQ ID NO: 179) | GTGCCCAGAACTACTGCCAT (SEQ ID NO: 193) |
| PHF8-sg2 | CTCCCTCCCTTCCTAAGGCT (SEQ ID NO: 180) | GAGGTGAGTTCCAGCTTCCC (SEQ ID NO: 194) |
| PRMT2-sg3 | ATTGCCTTAAGTCGACACCTGAT (SEQ ID NO: 181) | CACCTTACAGGCACTGCGTT (SEQ ID NO: 195) |
| PRMT6-sg1 | GACTGTAGAGTTGCCGGAACAG (SEQ ID NO: 182) | CTCCCTCCCTAGAGGCTATGAG (SEQ ID NO: 196) |

Table 6: Sequence of the targeted alleles in OVCAR8-ADR-Cas9 single cells harboring BMI1-sg2 and PHF8-sg2 expression construct. Nucleotides in boldface indicate the PAM sequence; and underlined nucleotides refer to base pair insertions or mutations.

| Single Cell | sgRNA ID | Sequence (5' to 3') | SEQ ID NO. | Indel |
|---|---|---|---|---|
| 1 | BMI1-sg2 | TACAACTCCAATAATAATTACAAACAAGGAATTT<br>CAACAGTTTCCTACCTTATATTCAG----------<br>GTCTTGTGAACTTGGACATCACAAATAGGAC | 231 | -10bp |
| 1 | BMI1-sg2 | TACAACTCCAATAATAATTACAAACAAGGAATTT<br>CAACAGTTTCCTACCTTATATTCAG----------<br>GTCTTGTGAACTTGGACATCACAAATAGGAC | 231 | -10bp |
| 1 | PHF8-sg2 | CGTTCTGACTCACAATCTCTCATGTTTTTCTGGCT<br>TAGTGAAAAAAC----------<br>TTCAAAGGGGCATGATACACACAAGGGGAAACC<br>AGTGAAGACC | 232 | -10bp |
| 1 | PHF8-sg2 | CGTTCTGACTCACAATCTCTCATGTTTTTCTGGCT<br>TAGTGAAAAAAC----------<br>TTCAAAGGGGCATGATACACACAAGGGGAAACC<br>AGTGAAGACC | 232 | -10bp |
| 2 | BMI1-sg2 | TACAACTCCAATAATAATTACAAACAAGGAATTT<br>CAACAGTTTCCTACCTTATA---------<br>**GG**TCTGGTCTTGTGAACTTGGACATCACAAATAG<br>GAC | 233 | -9bp |
| 2 | BMI1-sg2 | TACAACTCCAATAATAATTACAAACAAGGAATTT<br>CAACAGTTTCCTACCTTATATTCAGT---<br>**GG**TCTGGTCTTGTGAACTTGGACATCACAAATAG<br>GAC | 234 | -3bp |
| 2 | PHF8-sg2 | CGTTCTGACTCACAATCTCTCATGTTTTTCTGGCT<br>TAGTGAAAAA-<br>CGCCG**TGG**ATCTTCAAAGGGGCATGATACACAC<br>AAGGGGAAACCAGTGAAGACC | 235 | -1bp |
| 2 | PHF8-sg2 | CGTTCTGACTCACAATCTCTCATGTTTTTCTGGCT<br>TAGTGAAAAA-C----<br>**TGG**ATCTTCAAAGGGGCATGATACACACAAGGG<br>GAAACCAGTGAAGACC | 236 | -5bp |
| 3 | BMI1-sg2 | ACTCCAATAATAATTACAAACAAGGAATTTCAAC<br>AGTTTCCTACCTTATATTCTGTGATCTG**TGG**TCTG<br>GTCTTGTGAACTTGGACATCACAAATAGGAC | 237 | 2mut & +4bp |
| 3 | BMI1-sg2 | ACTCCAATAATAATTACAAACAAGGAATTTCAAC<br>AGTTTCCTACCTTATATTCTGTGATCTG**TGG**TCTG<br>GTCTTGTGAACTTGGACATCACAAATAGGAC | 237 | 2mut & +4bp |
| 3 | PHF8-sg2 | CGTTCTGACTCACAATCTCTCATGTTTTTCTGGCT<br>TAGTGAAAAAAA---<br>CG**TGG**ATCTTCAAAGGGGCATGATACACACAAG<br>GGGAAACCAGTGAAGACC | 238 | -3bp |

(continued)

| Single Cell | sgRNA ID | Sequence (5' to 3') | SEQ ID NO. | Indel |
|---|---|---|---|---|
| 3 | PHF8-sg2 | CGTTCTGACTCACAATCTCTCATGTTTTTCTGGCT TAGTGAAAAAA--- CG**TGG**ATCTTCAAAGGGGCATGATACACACAAG GGGAAACCAGTGAAGACC | 238 | -3bp |
| 4 | BMI1-sg2 | TACAACTCCAATAATAATTACAAACAAGGAATTT CAACAGTTTCCTACCTTATATTCAGTAG**TGG**TCT GGTCTTGTGAACTTGGACATCACAAATAGGAC | 239 | WT |
| 4 | BMI1-sg2 | TACAACTCCAATAATAATTACAAACAAGGAATTT CAACAGTTTCCTACCTTATATTCAGTAG**TGG**TCT GGTCTTGTGAACTTGGACATCACAAATAGGAC | 239 | WT |
| 4 | PHF8-sg2 | CGTTCTGACTCACAATCTCTCATGTTTTTCTGGCT TAGTGAAAAAACGCCG**TGG**ATCTTCAAAGGGGC ATGATACACACAAGGGGAAACCAGTGAAGACC | 240 | WT |
| 4 | PHF8-sg2 | CGTTCTGACTCACAATCTCTCATGTTTTTCTGGCT TAGTGAAAAAACGCCG**TGG**ATCTTCAAAGGGGC ATGATACACACAAGGGGAAACCAGTGAAGACC | 240 | WT |
| 5 | BMI1-sg2 | ATTTCAACAGTTTCCTACCTTATATACTATACTAT ATATATATATATATACTATATATATATAG**TGG**TCTG GTCTTGTGAACTTGGACATCACAAATAGGAC | 241 | +30bp |
| 5 | BMI1-sg2 | ATTTCAACAGTTTCCTACCTTATATACTATACTAT ATATATATATATATACTATATATATATAG**TGG**TCTG GTCTTGTGAACTTGGACATCACAAATAGGAC | 241 | +30bp |
| 5 | PHF8-sg2 | CTCTCATGTTTTTCTGGCTTAGTGAAAAAACTTCA CTAAGTTTTTACTTAG**TGG**ATCTTCAAAGGGGCA TGATACACACAAGGGGAAACCAGTGAAGACC | 242 | +16bp & 2mut |
| 5 | PHF8-sg2 | CTCTCATGTTTTTCTGGCTTAGTGAAAAAACTTCA CTAAGTTTTTACTTAG**TGG**ATCTTCAAAGGGGCA TGATACACACAAGGGGAAACCAGTGAAGACC | 242 | +16bp & 2mut |
| 6 | BMI1-sg2 | TACAACTCCAATAATAATTACAAACAAGGAATTT CAACAGTTTCCTACCTTATATTC----- **TGG**TCTGGTCTTGTGAACTTGGACATCACAAATA GGAC | 243 | -5bp |
| 6 | BMI1-sg2 | TACAACTCCAATAATAATTACAAACAAGGAATTT CAACAGTTTCCTAC------------------------ TTGTGAACTTGGACATCACAAATAGGAC | 244 | -24bp |
| 6 | PHF8-sg2 | CGTTCTGACTCACAATCTCTCATGTTTTTCTGGCT TAGTGAAAAAA---- CA**TGG**ATCTTCAAAGGGGCATGATACACACAAG GGGAAACCAGTGAAGACC | 245 | -4bp & 1mut |
| 6 | PHF8-sg2 | CGTTCTGACTCACAATCTCTCATGTTTTTCTGGCT TAGTGAAAAAACG--- **TGG**ATCTTCAAAGGGGCATGATACACACAAGGG GAAACCAGTGAAGACC | 246 | -3bp |

(continued)

| Single Cell | sgRNA ID | Sequence (5' to 3') | SEQ ID NO. | Indel |
|---|---|---|---|---|
| 7 | BMI1-sg2 | TACAACTCCAATAATAATTACAAACAAGGAATTTCAACAGTTTCCTACCT----------AG**TGG**TCTGGTCTTGTGAACTTGGACATCACAAATAGGAC | 247 | -10bp |
| 7 | BMI1-sg2 | TACAACTCCAATAATAATTACAAACAAGGAATTTCAACAGTTTCCTACCT----------AG**TGG**TCTGGTCTTGTGAACTTGGACATCACAAATAGGAC | 247 | -10bp |
| 7 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 7 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 8 | BMI1-sg2 | TACAACTCCAATAATAATTACAAACAAGGAATTTCAACAGTTTCCTACCTTATATTCT---G**TGG**TCTGGTCTTGTGAACTTGGACATCACAAATAGGAC | 248 | 1mut & -3bp |
| 8 | BMI1-sg2 | TACAACTCCAATAATAATTACAAACAAGGAATTTCAACAGTTTCCTACCTTATATTCT---G**TGG**TCTGGTCTTGTGAACTTGGACATCACAAATAGGAC | 248 | 1mut & -3bp |
| 8 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 8 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 9 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 9 | BMI1-sg2 | TACAACTCCAATAATAATTACAAACAAGGAATTTCAACAGTTTCCTACCTTATATTC-----**TGG**TCTGGTCTTGTGAACTTGGACATCACAAATAGGAC | 243 | -5bp |
| 9 | PHF8-sg2 | CGTTCTGACTCACAATCTCTCATGTTTTTCTGGCTTAGTGAAAAA----CA**TGG**ATCTTCAAAGGGGCATGATACACACAAGGGGAAACCAGTGAAGACC | 245 | -4bp & 1mut |

(continued)

| Single Cell | sgRNA ID | Sequence (5' to 3') | SEQ ID NO. | Indel |
|---|---|---|---|---|
| 9 | PHF8-sg2 | CGTTCTGACTCACAATCTCTCATGTTTTTCTGGCT TAGTGAAAAAA---CG**TGG**ATCTTCAAAGGGGCATGATACACACAAG GGGAAACCAGTGAAGACC | 246 | -3bp |
| 10 | BMI1-sg2 | TACAACTCCAATAATAATTACAAACAAGGAATTT CAACAGTTTCCTACCTTATATTCAGTAG**TGG**TCT GGTCTTGTGAACTTGGACATCACAAATAGGAC | 239 | WT |
| 10 | BMI1-sg2 | TACAACTCCAATAATAATTACAAACAAGGAATTT CAACAGTTTCCTACCTTATATTCAGTAG**TGG**TCT GGTCTTGTGAACTTGGACATCACAAATAGGAC | 239 | WT |
| 10 | PHF8-sg2 | CGTTCTGACTCACAATCTCTCATGTTTTTCTGGCT TAGTGAAAAAAC<u>TAA</u>GTGGATCTTCAAAGGGGC ATGATACACACAAGGGGAAACCAGTGAAGACC | 249 | 3mut |
| 10 | PHF8-sg2 | CGTTCTGACTCACAATCTCTCATGTTTTTCTGGCT TAGTGAAAAAACGCCG**TGG**ATCTTCAAAGGGGC ATGATACACACAAGGGGAAACCAGTGAAGACC | 240 | WT |
| 11 | BMI1-sg2 | TACAACTCCAATAATAATTACAAACAAGGAATTT CAACAGTTTCCTACCTTATATTCAGTAG**TGG**TCT GGTCTTGTGAACTTGGACATCACAAATAGGAC | 239 | WT |
| 11 | BMI1-sg2 | TACAACTCCAATAATAATTACAAACAAGGAATTT CAACAGTTTCCTAC-----------TAG**TGG**TCTGGTCTTGTGAACTTGGACATCACAA ATAGGAC | 250 | -11bp |
| 11 | PHF8-sg2 | CGTTCTGACTCACAATCTCTCATGTTTTTCTGGCT TAGTGAAAAAACGCCG**TGG**ATCTTCAAAGGGGC ATGATACACACAAGGGGAAACCAGTGAAGACC | 240 | WT |
| 11 | PHF8-sg2 | CGTTCTGACTCACAATCTCTCATGTTTTTCTGGCT TAGTGAAAAAACGCCG**TGG**ATCTTCAAAGGGGC ATGATACACACAAGGGGAAACCAGTGAAGACC | 240 | WT |
| 12 | BMI1-sg2 | TACAACTCCAATAATAATTACAAACAAGGAATTT CAACAGTTTCCTACCTTATATTCAGTAG**TGG**TCT GGTCTTGTGAACTTGGACATCACAAATAGGAC | 239 | WT |
| 12 | BMI1-sg2 | TACAACTCCAATAATAATTACAAACAAGGAATTT CAACAGTTTCCTACCTTATATTCAGTAG**TGG**TCT GGTCTTGTGAACTTGGACATCACAAATAGGAC | 239 | WT |
| 12 | PHF8-sg2 | CGTTCTGACTCACAATCTCTCATGTTTTTCTGGCT TAGTGAAAAAACGCCG**TGG**ATCTTCAAAGGGGC ATGATACACACAAGGGGAAACCAGTGAAGACC | 240 | WT |
| 12 | PHF8-sg2 | CGTTCTGACTCACAATCTCTCATGTTTTTCTGGCT TAGTGAAAAAACGCCG**TGG**ATCTTCAAAGGGGC ATGATACACACAAGGGGAAACCAGTGAAGACC | 240 | WT |

(continued)

| Single Cell | sgRNA ID | Sequence (5' to 3') | SEQ ID NO. | Indel |
|---|---|---|---|---|
| 13 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 13 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 13 | PHF8-sg2 | CGTTCTGACTCACAATCTCTCATGTTTTTCTGGCTTAGTGA-------------ATCTTCAAAGGGGCATGATACACACAAGGGGAAACCAGTGAAGACC | 251 | -13bp |
| 13 | PHF8-sg2 | CGTTCTGACTCACAATCTCTCATGTTTTTCTGGCTTAGTGA-------------ATCTTCAAAGGGGCATGATACACACAAGGGGAAACCAGTGAAGACC | 251 | -13bp |
| 14 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 14 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 14 | PHF8-sg2 | CGTTCTGACTCACAATCTCTCATGTTTTTCTGGCTTAGTGAAAAAAACG---**TGG**ATCTTCAAAGGGGCATGATACACACAAGGGGAAACCAGTGAAGACC | 246 | -3bp |
| 14 | PHF8-sg2 | CGTTCTGACTCACAATCTCTCATGTTTTTCTGGCTTAGTGAAAAAAACG---**TGG**ATCTTCAAAGGGGCATGATACACACAAGGGGAAACCAGTGAAGACC | 246 | -3bp |
| 15 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 15 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 15 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 15 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |

(continued)

| Single Cell | sgRNA ID | Sequence (5' to 3') | SEQ ID NO. | Indel |
|---|---|---|---|---|
| 16 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 16 | BMI1-sg2 | TACAACTCCAATAATAATTACAAACAAGGAATTTCAACAGTTTCCTACCTTATATTC-----**TGG**TCTGGTCTTGTGAACTTGGACATCACAAATAGGAC | 243 | -5bp |
| 16 | PHF8-sg2 | CGTTCTGACTCACAATCTCTCATGTTTTTCTGGCTTAGTGAAAAA----CA**TGG**ATCTTCAAAGGGGCATGATACACACAAGGGGAAACCAGTGAAGACC | 245 | -4bp & 1mut |
| 16 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 17 | BMI1-sg2 | TACAACTCCAATAATAATTACAAACAAGGAATTTCAACAGTTTCCTACCTTATATTCA---G**TGG**TCTGGTCTTGTGAACTTGGACATCACAAATAGGAC | 234 | -3bp |
| 17 | BMI1-sg2 | TACAACTCCAATAATAATTACAAACAAGGAATTTCAACAGTTTCCTACCTTATATTCA---G**TGG**TCTGGTCTTGTGAACTTGGACATCACAAATAGGAC | 234 | -3bp |
| 17 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 17 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 18 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 18 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 18 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 18 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |

(continued)

| Single Cell | sgRNA ID | Sequence (5' to 3') | SEQ ID NO. | Indel |
|---|---|---|---|---|
| 19 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 19 | BMI1-sg2 | TACAACTCCAATAATAATTACAAACAAGGAATTTCAACAGTTTCCAA--------------G**TGG**TCTGGTCTTGTGAACTTGGACATCACAAATAGGAC | 252 | -14bp & 1mut |
| 19 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 19 | PHF8-sg2 | CGTTCTGACTCACAATCTCTCATGTTTTTCTGGCTTAGTGAAAAA----CA**TGG**ATCTTCAAAGGGGCATGATACACACAAGGGGAAACCAGTGAAGACC | 245 | -4bp & 1mut |
| 20 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 20 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 20 | PHF8-sg2 | CGTTCTGACTCACAATCTCTCATGTTTTTCTGGCTTAGTGAAAAAAC---G**TGG**ATCTTCAAAGGGGCATGATACACACAAGGGGAAACCAGTGAAGACC | 253 | -3bp |
| 20 | PHF8-sg2 | CGTTCTGACTCACAATCTCTCATGTTTTTCTGGCTTAGTGAAAAAAC---G**TGG**ATCTTCAAAGGGGCATGATACACACAAGGGGAAACCAGTGAAGACC | 253 | -3bp |
| 21 | BMI1-sg2 | ACAACTCCAATAATAATTACAAACAAGGAATTTCAACAGTTTCCTACCTTATATTCAGATAG**TGG**TCTGGTCTTGTGAACTTGGACATCACAAATAGGAC | 254 | +1bp |
| 21 | BMI1-sg2 | ACAACTCCAATAATAATTACAAACAAGGAATTTCAACAGTTTCCTACCTTATATTCAGATAG**TGG**TCTGGTCTTGTGAACTTGGACATCACAAATAGGAC | 254 | +1bp |
| 21 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 21 | PHF8-sg2 | CGTTCTGACTCACAATCTCTCATGTTTTTCTGGCTTAGTGAAAAAACG---**TGG**ATCTTCAAAGGGGCATGATACACACAAGGGGAAACCAGTGAAGACC | 246 | -3bp |

(continued)

| Single Cell | sgRNA ID | Sequence (5' to 3') | SEQ ID NO. | Indel |
|---|---|---|---|---|
| 22 | BMI1-sg2 | TACAACTCCAATAATAATTACAAACAAGGAATTTCAACAGTTTCCTA--------------G**TGG**TCTGGTCTTGTGAACTTGGACATCACAAATAGGAC | 255 | -14bp |
| 22 | BMI1-sg2 | TACAACTCCAATAATAATTACAAACAAGGAATTTCAACAGTTTCCTACCTTATATTC--TAG-**GG**TCTGGTCTTGTGAACTTGGACATCACAAATAGGAC | 256 | -3bp |
| 22 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 22 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 23 | BMI1-sg2 | TACAACTCCAATAATAATTACAAACAAGGAATTTCAACAGTTTCCTACCTTATATTCAG-------------TTGTGAACTTGGACATCACAAATAGGAC | 257 | -13bp |
| 23 | BMI1-sg2 | TACAACTCCAATAATAATTACAAACAAGGAATTTCAACAGTTTCCTACCTTATA-----TAG**TGG**TCTGGTCTTGTGAACTTGGACATCACAAATAGGAC | 258 | -5bp |
| 23 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 23 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 24 | BMI1-sg2 | TACAACTCCAATAATAATTACAAACAAGGAATTTCAACAGTTTCCTACCTTATATTCA---G**TGG**TCTGGTCTTGTGAACTTGGACATCACAAATAGGAC | 239 | -3bp |
| 24 | BMI1-sg2 | TACAACTCCAATAATAATTACAAACAAGGAATTTCAACAGTTTCCTA--------------G**TGG**TCTGGTCTTGTGAACTTGGACATCACAAATAGGAC | 255 | -14bp |
| 24 | PHF8-sg2 | TCTGACTCACAATCTCTCATGTTTTTCTGGCTTAGTGAAAAACATGCCCCG**TGG**ATCTTCAAAGGGGCATGATACACACAAGGGGAAACCAGTGAAGACC | 259 | +3bp & 2mut |

(continued)

| Single Cell | sgRNA ID | Sequence (5' to 3') | SEQ ID NO. | Indel |
|---|---|---|---|---|
| 24 | PHF8-sg2 | TCTGACTCACAATCTCTCATGTTTTTCTGGCTTAGTGAAAAACATGCCCCG**TGG**ATCTTCAAAGGGGCATGATACACACAAGGGGAAACCAGTGAAGACC | 259 | +3bp & 2mut |
| 25 | BMI1-sg2 | TACAACTCCAATAATAATTACAAACAAGGAATTTCAACAGTTTCCTACCTTATATTCA---G**TGG**TCTGGTCTTGTGAACTTGGACATCACAAATAGGAC | 239 | -3bp |
| 25 | BMI1-sg2 | TACAACTCCAATAATAATTACAAACAAGGAATTTCAACAGTTTCCTACCTTATATTCA---G**TGG**TCTGGTCTTGTGAACTTGGACATCACAAATAGGAC | 239 | -3bp |
| 25 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 25 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 26 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 26 | BMI1-sg2 | TACAACTCCAATAATAATTACAAACAAGGAATTTCAACAGTTTCCTACCTTATATTCAGT---**GG**TCTGGTCTTGTGAACTTGGACATCACAAATAGGAC | 234 | -3bp |
| 26 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 26 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 27 | BMI1-sg2 | TACAACTCCAATAATAATTACAAACAAGGAATTTCAACAGTTTCCTACCTTAT---------**TGG**TCTGGTCTTGTGAACTTGGACATCACAAATAGGAC | 260 | -9bp |
| 27 | BMI1-sg2 | TACAACTCCAATAATAATTACAAACAAGGAATTTCAACAGTTTCCTACCTTAT---------**TGG**TCTGGTCTTGTGAACTTGGACATCACAAATAGGAC | 260 | -9bp |

(continued)

| Single Cell | sgRNA ID | Sequence (5' to 3') | SEQ ID NO. | Indel |
|---|---|---|---|---|
| 27 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 27 | PHF8-sg2 | CGTTCTGACTCACAATCTCTCATGTTTTTCTGGCTTAGTGAAAAAACG---**TGG**ATCTTCAAAGGGGCATGATACACACAAGGGGAAACCAGTGAAGACC | 246 | -3bp |
| 28 | BMI1-sg2 | TACAACTCCAATAATAATTACAAACAAGGAATTTCAACA----------------------G**TGG**TCTGGTCTTGTGAACTTGGACATCACAAATAGGAC | 261 | -22bp |
| 28 | BMI1-sg2 | TACAACTCCAATAATAATTACAAACAAGGAATTTCAACA----------------------G**TGG**TCTGGTCTTGTGAACTTGGACATCACAAATAGGAC | 261 | -22bp |
| 28 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 28 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 29 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 29 | BMI1-sg2 | TACAACTCCAATAATAATTACAAACAAGGAATTTCAACAGTTTCCTACCTTATATTC-----**TGG**TCTGGTCTTGTGAACTTGGACATCACAAATAGGAC | 243 | -5bp |
| 29 | PHF8-sg2 | CGTTCTGACTCACAATCTCTCATGTTTTTCTGGCTTAGTGAAAAA----CA**TGG**ATCTTCAAAGGGGCATGATACACACAAGGGGAAACCAGTGAAGACC | 245 | -4bp & 1mut |
| 29 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 30 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |

(continued)

| Single Cell | sgRNA ID | Sequence (5' to 3') | SEQ ID NO. | Indel |
|---|---|---|---|---|
| 30 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 30 | PHF8-sg2 | TCTCATGTTTTTCTGGCTTAGTGAAATCTAAGCTTAGTGAAATCTAAGCCG**TGG**ATCTTCAAAGGGGCATGATACACACAAGGGGAAACCAGTGAAGACC | 262 | +17bp & lmut |
| 30 | PHF8-sg2 | TCTCATGTTTTTCTGGCTTAGTGAAATCTAAGCTTAGTGAAATCTAAGCCG**TGG**ATCTTCAAAGGGGCATGATACACACAAGGGGAAACCAGTGAAGACC | 262 | +17bp & 1mut |
| 31 | BMI1-sg2 | TACAACTCCAATAATAATTACAAACAAGGAATTTCAACAGTTTCCTACCTTATATTCTTATA**TGG**TCTGGTCTTGTGAACTTGGACATCACAAATAGGAC | 263 | 5mut |
| 31 | BMI1-sg2 | TACAACTCCAATAATAATTACAAACAAGGAATTTCAACAGTTTCCTA--------------G**TGG**TCTGGTCTTGTGAACTTGGACATCACAAATAGGAC | 255 | -14bp |
| 31 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 31 | PHF8-sg2 | CGTTCTGACTCACAATCTCTCATGTTTTTCTGGCTTAGTGAAAAAA---CG**TGG**ATCTTCAAAGGGGCATGATACACACAAGGGGAAACCAGTGAAGACC | 246 | -3bp |
| 32 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 32 | BMI1-sg2 | TACAACTCCAATAATAATTACAAACAAGGAATTTCAACAGTTTCCTACCTTATATA---TAG**TGG**TCTGGTCTTGTGAACTTGGACATCACAAATAGGAC | 264 | -3bp & 1mut |
| 32 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 32 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 33 | BMI1-sg2 | TACAACTCCAATAATAATTACAAACAAGGAATTTCAACAGTTTCCTACCTTATATTCAG------------------AACTTGGACATCACAAATAGGAC | 265 | -18bp |
| 33 | BMI1-sg2 | TACAACTCCAATAATAATTACAAACAAGGAATTTCAACAGTTTCCTACCTTATATTCAG------------------AACTTGGACATCACAAATAGGAC | 265 | -18bp |

(continued)

| Single Cell | sgRNA ID | Sequence (5' to 3') | SEQ ID NO. | Indel |
|---|---|---|---|---|
| 33 | PHF8-sg2 | CGTTCTGACTCACAATCTCTCATGTTTTTCTGGCT<br>TAGTGAAAAAACGCCG**TGG**ATCTTCAAAGGGGC<br>ATGATACACACAAGGGGAAACCAGTGAAGACC | 240 | WT |
| 33 | PHF8-sg2 | CGTTCTGACTCACAATCTCTCATGTTTTTCTGGCT<br>TAGTGAAAAAACGCCG**TGG**ATCTTCAAAGGGGC<br>ATGATACACACAAGGGGAAACCAGTGAAGACC | 240 | WT |
| 34 | BMI1-sg2 | TACAACTCCAATAATAATTACAAACAAGGAATTT<br>CAACAGTTTCCTACCTTATATTCAGTAG**TGG**TCT<br>GGTCTTGTGAACTTGGACATCACAAATAGGAC | 239 | WT |
| 34 | BMI1-sg2 | TACAACTCCAATAATAATTACAAACAAGGAATTT<br>CAACAGTTTCCTACCTTATATTCAGTAG**TGG**TCT<br>GGTCTTGTGAACTTGGACATCACAAATAGGAC | 239 | WT |
| 34 | PHF8-sg2 | CGTTCTGACTCACAATCTCTCATGTTTTTCTGGCT<br>TAGTGAAAAAACGCCG**TGG**ATCTTCAAAGGGGC<br>ATGATACACACAAGGGGAAACCAGTGAAGACC | 240 | WT |
| 34 | PHF8-sg2 | GTTCTGACTCACAATCTCTCATGTTTTTCTGGCTT<br>AGTGAAAAAAACGCCGTGGATCTTCAAAGGGGC<br>ATGATACACACAAGGGGAAACCAGTGAAGACC | 266 | +1bp |
| 35 | BMI1-sg2 | TAATTACAAACAAGGAATTTCAACAGTTTCCTAC<br>CTTATATTCAGTATAATATATTCATTAG**TGG**TCTG<br>GTCTTGTGAACTTGGACATCACAAATAGGAC | 267 | +14bp |
| 35 | BMI1-sg2 | TAATTACAAACAAGGAATTTCAACAGTTTCCTAC<br>CTTATATTCAGTATAATATATTCATTAG**TGG**TCTG<br>GTCTTGTGAACTTGGACATCACAAATAGGAC | 267 | +14bp |
| 35 | PHF8-sg2 | CGTTCTGACTCACAATCTCTCATGTTTTTCTGGCT<br>TAGTG-------------<br>**G**ATCTTCAAAGGGGCATGATACACACAAGGGGA<br>AACCAGTGAAGACC | 268 | -13bp |
| 35 | PHF8-sg2 | CGTTCTGACTCACAATCTCTCATGTTTTTCTGGCT<br>TAGTG-------------<br>**G**ATCTTCAAAGGGGCATGATACACACAAGGGGA<br>AACCAGTGAAGACC | 268 | -13bp |
| 36 | BMI1-sg2 | TACAACTCCAATAATAATTACAAACAAGGAATTT<br>CAACAGTTTCCTACCTTATATTC--<br>TAG**TGG**TCTGGTCTTGTGAACTTGGACATCACAA<br>ATAGGAC | 269 | -2bp |
| 36 | BMI1-sg2 | TACAACTCCAATAATAATTACAAACAAGGAATTT<br>CAACAGTTTCCTACCTTATATTC--<br>TAG**TGG**TCTGGTCTTGTGAACTTGGACATCACAA<br>ATAGGAC | 269 | -2bp |

(continued)

| Single Cell | sgRNA ID | Sequence (5' to 3') | SEQ ID NO. | Indel |
|---|---|---|---|---|
| 36 | PHF8-sg2 | CGTTCTGACTCACAATCTCTCATGTTTTTCTGGCTTAGTGAAAAAACG---**TGG**ATCTTCAAAGGGGCATGATACACACAAGGGGAAACCAGTGAAGACC | 246 | -3bp |
| 36 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 37 | BMI1-sg2 | TACAACTCCAATAATAATTACAAACAAGGAATTTCAACAGTTTCCTACCTTATATTATATAG**TGG**TCTGGTCTTGTGAACTTGGACATCACAAATAGGAC | 270 | 3mut |
| 37 | BMI1-sg2 | TACAACTCCAATAATAATTACAAACAAGGAATTTCAACAGTTTCCTACCTTATATTCAGTAG**TGG**TCTGGTCTTGTGAACTTGGACATCACAAATAGGAC | 239 | WT |
| 37 | PHF8-sg2 | CGTTCTGACTCACAATCTCTCATGTTTTTCTGGCTTAGTGAAAAA-C----**TGG**ATCTTCAAAGGGGCATGATACACACAAGGGGAAACCAGTGAAGACC | 236 | -5bp |
| 37 | PHF8-sg2 | CGTTCTGACTCACAATCTCTCATGTTTTTCTGGCTTAGTGAAAAA-C----**TGG**ATCTTCAAAGGGGCATGATACACACAAGGGGAAACCAGTGAAGACC | 236 | -5bp |
| 38 | BMI1-sg2 | TACAACTCCAATAATAATTACAAACAAGGAATTTCAACAGTTTCCTACCTTATATTCA---G**TGG**TCTGGTCTTGTGAACTTGGACATCACAAATAGGAC | 239 | -3bp |
| 38 | BMI1-sg2 | TAATTACAAACAAGGAATTTCAACAGTTTCCTACCTTATATTCAGGTAGTGAATCTGAATAG**TGG**TCTGGTCTTGTGAACTTGGACATCACAAATAGGAC | 271 | +14bp |
| 38 | PHF8-sg2 | CGTTCTGACTCACAATCTCTCATGTTTTTCTGGCTTAGTGAAAAAAC---G**TGG**ATCTTCAAAGGGGCATGATACACACAAGGGGAAACCAGTGAAGACC | 253 | -3bp |
| 38 | PHF8-sg2 | CGTTCTGACTCACAATCTCTCATGTTTTTCTGGCTTAGTGAAAAAAC---G**TGG**ATCTTCAAAGGGGCATGATACACACAAGGGGAAACCAGTGAAGACC | 253 | -3bp |
| 39 | BMI1-sg2 | TACAACTCCAATAATAATTACAAACAAGGAATTTCAACAGTTTCCTACCTT---------------CTGGTCTTGTGAACTTGGACATCACAAATAGGAC | 272 | -15bp |
| 39 | BMI1-sg2 | TACAACTCCAATAATAATTACAAACAAGGAATTTCAACAGTTTCCTACCTTATATTCA---G**TGG**TCTGGTCTTGTGAACTTGGACATCACAAATAGGAC | 239 | -3bp |

(continued)

| Single Cell | sgRNA ID | Sequence (5' to 3') | SEQ ID NO. | Indel |
|---|---|---|---|---|
| 39 | PHF8-sg2 | ACAATCTCTCATGTTTTTCTGGCTTAGTGAATCTTCAAAGGGATCTTCAAAA**GG**ATCTTCAAAGGGGCATGATACACACAAGGGGAAACCAGTGAAGACC | 273 | +11bp & 4mut |
| 39 | PHF8-sg2 | ACAATCTCTCATGTTTTTCTGGCTTAGTGAATCTTCAAAGGGATCTTCAAAA**GG**ATCTTCAAAGGGGCATGATACACACAAGGGGAAACCAGTGAAGACC | 273 | +11bp & 4mut |
| 40 | BMI1-sg2 | TACAACTCCAATAATAATTACAAACAAGGAATTTCAACAGTTTCCTACCTTATATT------------------GTGAACTTGGACATCACAAATAGGAC | 274 | -18bp |
| 40 | BMI1-sg2 | TACAACTCCAATAATAATTACAAACAAGGAATTTCAACAGTTTCCTACCTTATATTC---AG**TGG**TCTGGTCTTGTGAACTTGGACATCACAAATAGGAC | 275 | -3bp |
| 40 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |
| 40 | PHF8-sg2 | CGTTCTGACTCACAATCTCTCATGTTTTTCTGGCTTAGTGAAAAAACGCCG**TGG**ATCTTCAAAGGGGCATGATACACACAAGGGGAAACCAGTGAAGACC | 240 | WT |

**RNA extraction and quantitative RT-PCR (qRT-PCR)**

RNA were extracted from cells using TRIzol Plus RNA Purification Kit (Life Technologies) according to manufacturer's protocol and treated with PureLink on-column DNase kit (Life Technologies). RNA quality and concentration was determined using NanoDrop Spectrophotometer. RNA samples were reverse-transcribed using SuperScript III Reverse Transcriptase (Life Technologies), Random Primer Mix (New England Biolabs) and RNAse OUT (Invitrogen). To evaluate gene expression level, quantitative PCR was conducted using SYBR FAST qPCR MasterMix (KAPA) on the LightCycler480 system (Roche). Data was quantified and analyzed using LifeCyler480 SW 1.1 build-in software. PCR primers were designed and evaluated using PrimerBlast (NCBI). Primer sequences are listed in Table 7.

Table 6: PCR primers used in qRT-PCR

| Target gene ID | Forward primer (5' to 3') | Reverse primer (5' to 3') |
|---|---|---|
| BRD4 | GTTGATGTGATTGCCGGCTC (SEQ ID NO: 197) | TTAGGCAGGACCTGTTTCGG (SEQ ID NO: 200) |
| KDM4C | CGTACGGGTTCATGCAAGTT (SEQ ID NO: 198) | CGTTTGCTTAAGAGCACCTCC (SEQ ID NO: 201) |
| KDM6B | CCCCTCACCGCCTATCAGTA (SEQ ID NO: 199) | TCTTGAACAAGTCGGGGTCG (SEQ ID NO: 202) |

Table 8: PCR primers used in deep sequencing for indel detection

| Target gRNA ID | Types of target site | 20 bp gRNA targeting sequence (5' to 3') | SEQ ID NO. | Forward primer (5' to 3') | SEQ ID NO. | Reverse primer (5' to 3') | SEQ ID NO. |
|---|---|---|---|---|---|---|---|
| NF1-sg1 | On-target | GTTGT<br>GCTCA<br>GTACT<br>GACTT | 276 | ACACTCTTTCCCT<br>ACACGACGCTCTT<br>CCGATCT-<br>GGTATCTGTGGTT<br>GATGCAGTTTTCC | 304 | GTGACTGGAGTTC<br>AGACGTGTGCTCT<br>TCCGATCT-<br>AGTAGTGAGGCC<br>GCTTATAACC | 332 |
| NF1-sg4 | On-target | TTTCA<br>GCTTC<br>CAATA<br>AAAAC | 277 | ACACTCTTTCCCT<br>ACACGACGCTCTT<br>CCGATCT-<br>GGTATCTGTGGTT<br>GATGCAGTTTTCC | 304 | GTGACTGGAGTTC<br>AGACGTGTGCTCT<br>TCCGATCT-<br>AGTAGTGAGGCC<br>GCTTATAACC | 332 |
| NF2-sg2 | On-target | ATTCC<br>ACGGG<br>AAGGA<br>GATCT | 278 | ACACTCTTTCCCT<br>ACACGACGCTCTT<br>CCGATCT-<br>GCACAGAGCTGC<br>TGCTTGGAGTG | 305 | GTGACTGGAGTTC<br>AGACGTGTGCTCT<br>TCCGATCT-<br>TAACAAGGAGAT<br>GCCCTGGCTGG | 333 |
| MED1 2-sg1 | On-target | AGGAT<br>TGAAG<br>CTGAC<br>GTTCT | 279 | ACACTCTTTCCCT<br>ACACGACGCTCTT<br>CCGATCT-<br>CGCTTTCCTGCCT<br>CAGGATGAAC | 306 | GTGACTGGAGTTC<br>AGACGTGTGCTCT<br>TCCGATCT-<br>AGGTCATGAAGG<br>CAAACTCAGCC | 334 |
| PHF8-sg2 | On-target | GGCTT<br>AGTGA<br>AAAAA<br>CGCCG | 280 | ACACTCTTTCCCT<br>ACACGACGCTCTT<br>CCGATCT-<br>TTGGAAGAGAAG<br>GATCTGCTGAGG<br>C | 307 | GTGACTGGAGTTC<br>AGACGTGTGCTCT<br>TCCGATCT-<br>CACCTGTCAAAA<br>GTCCTACTCCGG | 335 |

(continued)

| Target gRNA ID | Types of target site | 20 bp gRNA targeting sequence (5' to 3') | SEQ ID NO. | Forward primer (5' to 3') | SEQ ID NO. | Reverse primer (5' to 3') | SEQ ID NO. |
|---|---|---|---|---|---|---|---|
| BMI-sg2 | On-target | TCCTA CCTTAT ATTCA GTAG | 281 | ACACTCTTTCCCT ACACGACGCTCTT CCGATCT-GCTACCCTCCACA AAGCACACAC | 308 | GTGACTGGAGTTC AGACGTGTGCTCT TCCGATCT-CCTGGAGACCAG CAAGTATTGTCC | 336 |
| KDM4 C-sg1 | On-target | CCTTTG CAAGA CCCGC ACGA | 109 | ACACTCTTTCCCT ACACGACGCTCTT CCGATCT-CCTTCAGAAACA ATGTCCCAAATCG | 309 | GTGACTGGAGTTC AGACGTGTGCTCT TCCGATCT-GTCCTCTGAACCC CAGCTGTAAG | 337 |
| KDM4 C-sg1 | Off-target | GCTTT GCCCG AACCG CACGA | 282 | ACACTCTTTCCCT ACACGACGCTCTT CCGATCT-AGCCTTTCTGAGA GCGGGCTAG | 310 | GTGACTGGAGTTC AGACGTGTGCTCT TCCGATCT-CAAACAGAGGCC AAAGGGTGTCCC | 338 |
| KDM4 C-sg1 | Off-target | CCTAG GCCAG ACCTG CACGA | 283 | ACACTCTTTCCCT ACACGACGCTCTT CCGATCT-GCCTCCTCTCATC CTCTCGCTTC | 311 | GTGACTGGAGTTC AGACGTGTGCTCT TCCGATCT-ACAGGAGGTCGT GGTGCAGTTCTC | 339 |
| KDM4 C-sg1 | Off-target | GCTCT GGAAG ACCCG CACCA | 284 | ACACTCTTTCCCT ACACGACGCTCTT CCGATCT-GATTGGCTCCAA GCGGCCATCAAA C | 312 | GTGACTGGAGTTC AGACGTGTGCTCT TCCGATCT-TTGTGTGAGGAA CGTTGACGCTACC | 340 |

(continued)

| Target gRNA ID | Types of target site | 20 bp gRNA targeting sequence (5' to 3') | SEQ ID NO. | Forward primer (5' to 3') | SEQ ID NO. | Reverse primer (5' to 3') | SEQ ID NO. |
|---|---|---|---|---|---|---|---|
| KDM4 C-sg1 | Off-target | CCTTAT CAAGA CCCAC ACCA | 285 | ACACTCTTTCCCT ACACGACGCTCTT CCGATCT-TTGAACTCAAGG CTCAGCCAACAG GC | 313 | GTGACTGGAGTTC AGACGTGTGCTCT TCCGATCT-GAGCGTAGGTCC TCTGCATGGAG | 341 |
| KDM6 B-sg2 | On-target | ATCCC CCTCCT CGTAG CGCA | 286 | ACACTCTTTCCCT ACACGACGCTCTT CCGATCT-CAACTCAGGCTG GATGCATCGG | 314 | GTGACTGGAGTTC AGACGTGTGCTCT TCCGATCT-CCACAGAATGAC AGGAACCCATGG | 342 |
| KDM6 B-sg2 | Off-target | CTGCT CCTCCT CGTAG CGCT | 287 | ACACTCTTTCCCT ACACGACGCTCTT CCGATCT-TTGGTGGCCGCTG AGTGTGTGTAC | 315 | GTGACTGGAGTTC AGACGTGTGCTCT TCCGATCT-ACTGAGCAGAGC CTAGGAGGCAG | 343 |
| KDM6 B-sg2 | Off-target | TGCGC CCTCCT CCTAG CGCA | 288 | ACACTCTTTCCCT ACACGACGCTCTT CCGATCT-CAGCATGTTGAC ATAGCGGC | 316 | GTGACTGGAGTTC AGACGTGTGCTCT TCCGATCT-TGTTGCCAGATCC AGAGGCGTC | 344 |
| KDM6 B-sg2 | Off-target | CTCCTC CTCCG CGTAG CGCT | 289 | ACACTCTTTCCCT ACACGACGCTCTT CCGATCT-TGAGAGGAGATG AGTCGGGGTC | 317 | GTGACTGGAGTTC AGACGTGTGCTCT TCCGATCT-AACTGGCCCGAG TAGTCGGAGCAG | 345 |

(continued)

| Target gRNA ID | Types of target site | 20 bp gRNA targeting sequence (5' to 3') | SEQ ID NO. | Forward primer (5' to 3') | SEQ ID NO. | Reverse primer (5' to 3') | SEQ ID NO. |
|---|---|---|---|---|---|---|---|
| KDM6 B-sg2 | Off-target | CTGCC CCTCCT GGTAG CGCC | 290 | ACACTCTTTCCCT ACACGACGCTCTT CCGATCT- GACGGGTCAAAG CCTCAGGAGAG | 318 | GTGACTGGAGTTC AGACGTGTGCTCT TCCGATCT- TCCTCAGAGTGTG TGGAAGTGCTGG | 346 |
| KDM6 B-sg2 | Off-target | AACCA GCTCC TCGTA GCTCA | 291 | ACACTCTTTCCCT ACACGACGCTCTT CCGATCT- TTAGCTGCCCAGC TCACAGCTACC | 319 | GTGACTGGAGTTC AGACGTGTGCTCT TCCGATCT- AAGAGCTCCTAG GGGAGGATCAG | 347 |
| KDM6 B-sg2 | Off-target | ACCGC CCTCCT CCTAG CTCA | 292 | ACACTCTTTCCCT ACACGACGCTCTT CCGATCT- GAGCCCCAAGAG CGAGACAA | 320 | GTGACTGGAGTTC AGACGTGTGCTCT TCCGATCT- TGGCAGGAGCAC AGCCTAAGGA | 348 |
| KDM6 B-sg2 | Off-target | AGCCC GCTCC TCGTG GGGCA | 293 | ACACTCTTTCCCT ACACGACGCTCTT CCGATCT- GGCGCTCAGAAG GCTGTGCAG | 321 | GTGACTGGAGTTC AGACGTGTGCTCT TCCGATCT- ACACCCGCCTCG GAGATCAACAC | 349 |
| BRD4-sg3 | On-target | GGGAA CAATA AAGAA GCGCT | 294 | ACACTCTTTCCCT ACACGACGCTCTT CCGATCT- CCTAGGTGACACT GGACTTTTGC | 322 | GTGACTGGAGTTC AGACGTGTGCTCT TCCGATCT- CCACCCCTACATC TCACCTTGTTG | 350 |

(continued)

| Target gRNA ID | Types of target site | 20 bp gRNA targeting sequence (5' to 3') | SEQ ID NO. | Forward primer (5' to 3') | SEQ ID NO. | Reverse primer (5' to 3') | SEQ ID NO. |
|---|---|---|---|---|---|---|---|
| BRD4-sg3 | Off-target | TGGAA AAACA AAGAA GAGCT | 295 | ACACTCTTTCCCT ACACGACGCTCTT CCGATCT- AGGTTCACCTCAG GCTGCTCAGAAG | 323 | GTGACTGGAGTTC AGACGTGTGCTCT TCCGATCT- GTGAGGTTTCCAC GTGCCAGC | 351 |
| BRD4-sg3 | Off-target | GGGAA GTATA AGGAA GAGCT | 296 | ACACTCTTTCCCT ACACGACGCTCTT CCGATCT- CGTCTCTCTCCAT GTGAGCTTGTG | 324 | GTGACTGGAGTTC AGACGTGTGCTCT TCCGATCT- CCAACAATTCCA GGTATGAAACTC CC | 352 |
| BRD4-sg3 | Off-target | GTGAG CAATA AAGCA GCCCT | 297 | ACACTCTTTCCCT ACACGACGCTCTT CCGATCT- GGAAAGATCATC TGATCAGGCCCAT C | 325 | GTGACTGGAGTTC AGACGTGTGCTCT TCCGATCT- CTCCCCACTTGTA GGTTCCTAATCC | 353 |
| BRD4-sg2 | On-target | TCTAG TCCAT CCCCC ATTAC | 298 | ACACTCTTTCCCT ACACGACGCTCTT CCGATCT- CTTGTTAGGGTTG GAGGTCTCTGG | 326 | GTGACTGGAGTTC AGACGTGTGCTCT TCCGATCT- GTAGAGTGCCTG GTGAAGAATGTG | 354 |
| BRD4-sg2 | Off-target | AATAT TCCATT CCCCA TTAC | 299 | ACACTCTTTCCCT ACACGACGCTCTT CCGATCT- AAGGCCCGTAAA GGGCAAGTTCAG | 327 | GTGACTGGAGTTC AGACGTGTGCTCT TCCGATCT- TCCAGACTGTTGT TCAGTCCTGT | 355 |

(continued)

| Target gRNA ID | Types of target site | 20 bp gRNA targeting sequence (5' to 3') | SEQ ID NO. | Forward primer (5' to 3') | SEQ ID NO. | Reverse primer (5' to 3') | SEQ ID NO. |
|---|---|---|---|---|---|---|---|
| BRD4-sg2 | Off-target | TGTTGTCCATACCTCATTAC | 300 | ACACTCTTTCCCTACACGACGCTCTTCCGATCT-GGTGACAGGAAGCTGTCGGAACAT | 328 | GTGACTGGAGTTCAGACGTGTGCTCTTCCGATCT-CTCTGGATTTGCCCACACCTAGTC | 356 |
| BRD4-sg2 | Off-target | TCTAGGTCATGCACCATTAC | 301 | ACACTCTTTCCCTACACGACGCTCTTCCGATCT-CTCACTGTGATCTGACACCAAACAC | 329 | GTGACTGGAGTTCAGACGTGTGCTCTTCCGATCT-GCATGCTTGCTTTCTGAAGGTGGC | 357 |
| BRD4-sg2 | Off-target | CCCATTCCTTCCCCCATTAC | 302 | ACACTCTTTCCCTACACGACGCTCTTCCGATCT-CTAGTTGCCTTCATGCCTTACAGAC | 330 | GTGACTGGAGTTCAGACGTGTGCTCTTCCGATCT-TTCCAAGCAAGTGAGCTTCAGCACC | 358 |
| BRD4-sg2 | Off-target | TCCACACCCTCCCCCATTAC | 303 | ACACTCTTTCCCTACACGACGCTCTTCCGATCT-CTGCTCCCACTCCAGACTACCC | 331 | GTGACTGGAGTTCAGACGTGTGCTCTTCCGATCT-CCACCCATGACACAGGAGGG | 359 |

Table 9: PCR primers used in whole genome amplification for indel detection

| Target sgRNA ID | Forward primer (5' to 3') | Reverse primer (5' to 3') |
|---|---|---|
| BMI1-sg2 | GTCTCGTGGGCTCGGAGATGTGTATAAGAGACAGGCTACCCTCCACAAAGCACACAC (SEQ ID NO: 360) | TCGTCGGCAGCGTCAGATGTGTATAAGAGACAGCCTGGAGACCAGCAAGTATTGTCC (SEQ ID NO: 362) |
| PHF8-sg2 | GTCTCGTGGGCTCGGAGATGTGTATAAGAGACAGTTGGAAGAGAAGGATCTGCTGAGGC (SEQ ID NO: 361 ) | TCGTCGGCAGCGTCAGATGTGTATAAGAGACAGCACCTGTCAAAAGTCCTACTCCGG (SEQ ID NO: 363) |

## RNA-Seq and data analysis

RNA was extracted from cells using TRIzol Plus RNA Purification Kit (Life Technologies) according to the manufacturer's protocol and treated with PureLink on-column DNase kit (Life Technologies). RNA quality and concentration was determined using NanoDrop Spectrophotometer. RNA samples were reverse-transcribed using SuperScript III Reverse Transcriptase (Life Technologies), Random Primer Mix (New England Biolabs) and RNAse OUT (Invitrogen). Sequencing libraries were prepared using the Illumina Library Prep Kit, starting with an input amount of 1 μg total RNA and following the manufacturer's recommendations. After PCR amplification, the libraries were size-selected to 300 +/- 25 bp on a 2% agarose gel (E-Gel EX, Invitrogen) and submitted to single-end sequencing on an Illumina HiSeq 2000 instrument. RNA-Seq experiments were performed in two biological replicates.

Raw single-end reads of the cDNA fragments were aligned to the human transcriptome (RefSeq, hg19) using TopHat2 (Kim, et al. Genome Biol (2013) 14:R36) and Bowtie (Langmead, et al. Genome Biol (2009) 10:R25). Differentially expressed genes between samples were called using Cuffdiff2 (Trapnell, et al. Nat Biotechnol (2013) 31:46-53) with the bias correction option, masking reads mapping to mitochondrial and ribosomal RNA transcripts. Genes were called differentially expressed if they met a minimum of 0.1 fragments per kilobase per million reads (FPKM) in at least one of the conditions tested, the absolute $\log_2$-fold-change was at least 0.5, and the P-value after multiple hypothesis correction (Q-value) was less than 0.05. Gene set enrichment analysis was performed using MSigDB database (broadinstitute.org/gsea/index.jsp) (Subramanian, et al. Proc Natl Acad Sci USA (2005) 102:15545-50).

## Mathematical modeling of cell proliferation in a mixed population

To estimate how different parameters affect results in our pooled screens, we simulated cell proliferation in a mixed cell population harboring gRNA combinations that exhibit different growth rates. At a given time (t), the total cell count ($C_N$) in the population is represented by the summation of individual cell counts containing different combinations ($C_i$; 1 to N, where N is the total number of combinations), such that $C_N(t) = \sum_{i=1}^{N} C_i(t)$.

For each individual gRNA combination, cell growth is represented by Eq. (1). Based on an exponential cell growth model, cells with each gRNA combination consist of two populations: one with a modified growth rate ($k_m$) due to gene disruption by the CRISPR-Cas9 system, and the other (unmodified cells) with the wild-type growth rate ($k_{wt}$). The former population is defined as a fraction of cells, *p*, which is limited by the cleavage efficiency of the CRISPR-Cas9 system. For simplicity, we assumed that *p* was constant throughout the duration of the assay.

$$C_i(t) = p\,C_i(t) + (1-p)C_i(t) = pC_0e^{k_m t} + (1-p)C_0e^{k_{wt} t} \qquad \text{(Eq. 1)}$$

where *p* represents the fraction of mutated cells with a modified growth rate, and $C_0$ represents the initial number of cells carrying the same barcoded gRNA combination. The cell growth rate (*k*) is evaluated from the cell's doubling time ($T_{doubling}$) following Eq. 2. The doubling time for wild-type OVCAR8-ADR-Cas9 cells was experimentally determined to be ~24 hours (data not shown).

$$k = \ln 2 \;/\; T_{doubling} \qquad \text{(Eq. 2)}$$

For simplicity in modeling, we segregated the total cell population into three sub-populations with different growth phenotypes as described in Eq. (3).

$$C_N(t) = \sum_{i=1}^{N} C_i(t) \;\approx f_{wt}N \times C_0e^{k_{wt}t} + f_sN \times C_{i,slow}(t) + f_fN \times C_{i,fast}(t) \quad \text{(Eq. 3)}$$

where $C_{i,slow}(t)$ and $C_{i,fast}(t)$ represent the average growth profiles of cells with anti-proliferative gRNAs and pro-proliferative gRNAs, respectively, which are determined by Eq. (1). At the start of the experiment, the percentages of the overall population that behave as wild type or that contain anti-proliferative gRNAs and pro-proliferative gRNAs are represented by $f_{wt}$, $f_s$ and $f_f$, respectively.

Based on this mixed cell growth model, we modeled the relative frequency (*R. F.*) of a pro-proliferative gRNA's and an anti-proliferative gRNA's representation in the whole population. The relative frequency is defined as the barcode abundance at a given time compared to the initial time point (i. e., $R.F. = \frac{F(t)}{F(t=0)}$, *where* $F = C_i(t)/C_N(t)$). The total number of combinations in a pool, $N$ and the initial number of cells, $C_0$, do not impact the relative frequency results. After running the simulation with defined parameters, we observed enrichment and depletion of a pro-proliferative gRNA and anti-proliferative gRNA in the population, respectively (FIG. 17A). The degree of enrichment and depletion was observed to change with different percentages (i.e., 2, 5, or 10%) of initial gRNA combinations defined to have an anti-proliferative ($f_s$) and pro-proliferative ($f_f$) response (FIG. 17A). We further evaluated the relative frequency of an anti-proliferative gRNA's representation by modulating the doubling time of modified cells ($T_{doubling,m}$) and the fraction of cells with the modified growth rate (p) for the anti-proliferative gRNA. Assuming that $p$ stayed constant throughout the experiment, the representation of an anti-proliferative clone in the entire cell population could be depleted by -23% to 97% under the parameter ranges shown in FIGs. 17B-17C. This model represents a simplified version of cell growth dynamics by segregating cell populations into sub-populations with average growth rates and does not account for potential interactions between cells. Based on our model, the sensitivity of our screen could be enhanced with improved gRNA efficiencies to increase the fraction of cells with modified growth rates and by increasing the assay time.

Having thus described several aspects of at least one embodiment of this invention, it is to be appreciated various alterations, modifications, and improvements will readily occur to those skilled in the art. Accordingly, the foregoing description and drawings are by way of example only.

**References**

1. Shalem, O. et al. Genome-scale CRISPR-Cas9 knockout screening in human cells. Science 343, 84-7 (2014).
2. Wang, T., Wei, J. J., Sabatini, D. M. & Lander, E. S. Genetic screens in human cells using the CRISPR-Cas9 system. Science 343, 80-4 (2014).
3. Zhou, Y. et al. High-throughput screening of a CRISPR/Cas9 library for functional genomics in human cells. Nature 509, 487-491 (2014).
4. Koike-Yusa, H., Li, Y., Tan, E.-P., Velasco-Herrera, M. D. C. & Yusa, K. Genome-wide recessive genetic screening in mammalian cells with a lentiviral CRISPR-guide RNA library. Nat. Biotechnol. 32, 267-73 (2014).
5. Gilbert, L. A. et al. Genome-Scale CRISPR-Mediated Control of Gene Repression and Activation. Cell 159, 647-661 (2014).
6. Konermann, S. et al. Genome-scale transcriptional activation by an engineered CRISPR-Cas9 complex. Nature doi:10.1038/nature14136 (2014).
7. Cong, L. et al. Multiplex genome engineering using CRISPR/Cas systems. Science 339, 819-23 (2013).
8. Mali, P. et al. RNA-guided human genome engineering via Cas9. Science 339, 823-6 (2013).
9. Gilbert, L. A. et al. CRISPR-mediated modular RNA-guided regulation of transcription in eukaryotes. Cell 154, 442-451 (2013).
10. Cheng, A. A., Ding, H. & Lu, T. K. Enhanced killing of antibiotic-resistant bacteria enabled by massively parallel combinatorial genetics. Proc. Natl. Acad. Sci. 111, 12462-7 (2014).
11. Honma, K. et al. RPN2 gene confers docetaxel resistance in breast cancer. Nat. Med. 14, 939-948 (2008).
12. Wang, Z. et al. Combinatorial patterns of histone acetylations and methylations in the human genome. Nat. Genet. 40, 897-903 (2008).
13. Dawson, M. A. & Kouzarides, T. Cancer epigenetics: From mechanism to therapy. Cell 150, 12-27 (2012).
14. Juergens, R. A. et al. Combination epigenetic therapy has efficacy in patients with refractory advanced non-small cell lung cancer. Cancer Discov. 1, 598-607 (2011).
15. Jones, P. A. & Baylin, S. B. The Epigenomics of Cancer. Cell 128, 683-692 (2007).
16. Yoo, C. B. & Jones, P. A. Epigenetic therapy of cancer: past, present and future. Nat. Rev. Drug Discov. 5, 37-50 (2006).
17. Jin, C. et al. Chem-seq permits identification of genomic targets of drugs against androgen receptor regulation selected by functional phenotypic screens. Proc. Natl. Acad. Sci. U. S. A. 111, 9235-40 (2014).
18. Asangani, I. a et al. Therapeutic targeting of BET bromodomain proteins in castration-resistant prostate cancer. Nature 510, 278-82 (2014).
19. Kruidenier, L. et al. A selective jumonji H3K27 demethylase inhibitor modulates the proinflammatory macrophage response. Nature 488, 404-408 (2012).
20. Bliss, C. I. THE TOXICITY OF POISONS APPLIED JOINTLY1. Ann. Appl. Biol. 26, 585-615 (1939).
21. Borisy, A. A. et al. Systematic discovery of multicomponent therapeutics. Proc. Natl. Acad. Sci. U. S. A. 100, 7977-7982 (2003).
22. Pattanayak, V. et al. High-throughput profiling of off-target DNA cleavage reveals RNA-programmed Cas9 nuclease specificity. Nat. Biotechnol. 31, 839-43 (2013).

23. Kuscu, C., Arslan, S., Singh, R., Thorpe, J. & Adli, M. Genome-wide analysis reveals characteristics of off-target sites bound by the Cas9 endonuclease. Nat. Biotechnol. 32, 677-683 (2014).
24. Wu, X. et al. Genome-wide binding of the CRISPR endonuclease Cas9 in mammalian cells. Nat. Biotechnol. 32, 670-676 (2014).
25. Doench, J. G. et al. Rational design of highly active sgRNAs for CRISPR-Cas9-mediated gene inactivation. Nat. Biotechnol. 32, 1262-1267 (2014).
26. Essletzbichler, P. et al. Megabase-scale deletion using CRISPR/Cas9 to generate a fully haploid human cell line. Genome Res. 24, 2059-2065 (2014).
27. Blasco, R. B. et al. Simple and rapid in vivo generation of chromosomal rearrangements using CRISPR/Cas9 technology. Cell Rep. 9, 1219-1227 (2014).
28. Choi, P. S. & Meyerson, M. Targeted genomic rearrangements using CRISPR/Cas technology. Nat. Commun. 5, 3728 (2014).
29. Moffat, J. et al. A Lentiviral RNAi Library for Human and Mouse Genes Applied to an Arrayed Viral High-Content Screen. Cell 124, 1283-1298 (2006).
30. Ran, F. A. et al. Genome engineering using the CRISPR-Cas9 system. Nat. Protoc. 8, 2281-308 (2013).
31. Kim D, et al. (2013) TopHat2: accurate alignment of transcriptomes in the presence of insertions, deletions and gene fusions. Genome Biol 14:R36.
32. Langmead B, Trapnell C, Pop M, Salzberg SL (2009) Ultrafast and memory-efficient alignment of short DNA sequences to the human genome. Genome Biol 10:R25.
33. Trapnell C, et al. (2013) Differential analysis of gene regulation at transcript resolution with RNA-seq. Nat Biotechnol 31:46-53.
34. Subramanian A, et al. (2005) Gene set enrichment analysis: a knowledge-based approach for interpreting genome-wide expression profiles. Proc Natl Acad Sci U S A 102:15545-50.
35. Stemmer M (2015) CCTop: An Intuitive, Flexible and Reliable CRISPR/Cas9 Target Prediction Tool. PLoS One 10(4):e0124633.

EQUIVALENTS

While several inventive embodiments have been described and illustrated herein, those of ordinary skill in the art will readily envision a variety of other means and/or structures for performing the function and/or obtaining the results and/or one or more of the advantages described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed.

All definitions, as defined and used herein, should be understood to control over dictionary definitions and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or," as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of" shall be closed or semi-closed transitional phrases,.

**Claims**

**1.** A genetic construct comprising
a first DNA element comprising
a CRISPR guide sequence and

a scaffold sequence that recruits an endonuclease to a target nucleic acid;

a first compatible end element and a second compatible end element flanking the first DNA element, wherein the first and second compatible end elements are capable of annealing to each other;
a barcode element;
a third compatible end element and a fourth compatible end element flanking the barcode element, wherein the third and fourth compatible end elements are capable of annealing to each other but are not capable of annealing to the first or second compatible end elements; and
a separation site located between the fourth compatible end element and the first compatible end element, wherein the DNA element, first compatible end element, and second compatible end element are on one side of the separation site, and the barcode element, the third compatible end element, and the fourth compatible end element are on the other side of the separation site.

**2.** The genetic construct of claim 1, further comprising a promoter element upstream of the first DNA element.

**3.** A vector comprising a genetic construct according to claim 1 or 2.

**4.** A genetic construct comprising:

a plurality of DNA elements, wherein each DNA element of the plurality of DNA element comprises a CRISPR guide sequence and a scaffold sequence that recruits an endonuclease to a target nucleic acid;
a first compatible end element and a second compatible end element flanking the plurality of DNA elements, wherein the first and second compatible end elements are capable of annealing to each other;
a plurality of barcode elements;
a third compatible end element and a fourth compatible end element flanking the plurality of barcode elements, wherein the third and fourth compatible end elements are capable of annealing to each other but are not capable of annealing to the first or second compatible end elements; and
a separation site located between the plurality of DNA elements and the plurality of barcode elements.

**5.** A vector comprising
a genetic construct according to claim 4 and
a promoter sequence located upstream of each of the CRISPR guide sequences.

**6.** A method for generating a combinatorial vector, comprising:

(a) providing a vector containing a first genetic construct comprising:

a CRISPR guide sequence;
a second compatible end element and a first recognition site for a first restriction enzyme flanking the CRISPR guide sequence;
a barcode element; and
a third compatible end element and a second recognition site for a second restriction enzyme flanking the barcode element;

(b) cleaving the first genetic construct at the first recognition site, resulting in a fifth compatible end element, and cleaving the vector at the second recognition site, resulting in a sixth compatible end element;
(c) providing a scaffold element comprising
a scaffold sequence that recruits an endonuclease to a target nucleic acid;
a separation site comprising a first compatible end element and a fourth compatible end element; and
a seventh compatible end element and an eighth compatible end element flanking the scaffold element, wherein the seventh compatible end element is capable of annealing to the fifth compatible end element and the eighth compatible end element is capable of annealing to the sixth compatible end element;
(d) annealing the scaffold element to the cleaved first genetic construct, wherein the annealing occurs at compatible end elements within the vector and the scaffold element that are capable of annealing to each other, and wherein after the annealing, the scaffold element is integrated between the CRISPR guide sequence and the barcode element, and wherein the separation site is located between the scaffold sequence and the barcode element, creating a combinatorial vector,
optionally wherein the first recognition site and the second recognition site have the same recognition site sequence, and the first restriction enzyme and the second restriction enzyme are the same restriction enzymes.

**7.** The method of claim 6, further comprising:

(a) providing a combinatorial vector according to claim 6;
(b) cleaving the vector at the separation site within the scaffold element, resulting in a first compatible end element and a fourth compatible end element;
(c) providing a second genetic construct comprising
a CRISPR guide sequence;
a scaffold sequence that recruits an endonuclease to a target nucleic acid;
a barcode element; and
a second compatible end element and a third compatible end element flanking the second genetic construct, wherein the second compatible end element of the second genetic construct is capable of annealing with the first compatible end element of the vector and the third compatible end element of the second genetic construct is capable of annealing to the fourth compatible end element of the vector;
(d) annealing the second genetic construct to the cleaved vector, wherein the annealing occurs at compatible end elements within the second genetic construct and the vector that are capable of annealing to each other, and wherein after annealing, the second genetic construct is integrated into the vector, creating a combinatorial vector comprising concatenated barcode elements and concatenated CRISPR guide and scaffold sequences, optionally wherein the method is iterative.

**8.** The method of claim 7, wherein the combinatorial vector further comprises a promoter element upstream of the CRISPR guide sequences.

**9.** A genetic construct comprising
at least two CRISPR guide sequences;
a scaffold sequence that recruits an endonuclease to a target nucleic acid;
a barcode element; and
a restriction recognition site located between each CRISPR guide sequence and between the barcode element and the CRISPR guide sequence nearest to the barcode element.

**10.** A genetic construct comprising
a plurality of DNA elements, each comprising
a CRISPR guide sequence and
a scaffold sequence that recruits an endonuclease to a target nucleic acid;

a barcode element; and
a promoter sequence located upstream of each of the DNA elements of the plurality of DNA elements.

**11.** The genetic construct of claim 9 or 10, wherein the barcode element is located at the 5' end of the genetic construct.

**12.** The genetic construct of claim 9 or 10, wherein the barcode element is located at the 3' end of the genetic construct.

**13.** A vector comprising any of the genetic constructs according to claims 9-12.

**14.** A method for generating a combinatorial vector, comprising

(a) providing a vector comprising:
a plurality of CRISPR guide sequences;
a barcode element, wherein the barcode element is located downstream of the plurality of CRISPR guide sequences;
optionally a promoter sequence located upstream of at least one of the plurality of CRISPR guide sequences; and
a plurality of recognition sites for a plurality of restriction enzymes, wherein each of the plurality of recognition sites is located downstream of one of the plurality of CRISPR guide sequences;
(b) cleaving the vector at at least one of the plurality of recognition sites with at least one of the plurality of restriction enzymes, resulting in a first compatible end element and a second compatible end element;
(c) providing a first scaffold element comprising:
a scaffold sequence that recruits an endonuclease to a target nucleic acid,

optionally a promoter sequence, and
a third compatible end element and fourth compatible end element flanking the first scaffold element, wherein the third compatible end element is capable of annealing to the first compatible end element of the cleaved vector and the fourth compatible end element is capable of annealing to the second compatible end element of the cleaved vector;
(d) annealing the first scaffold element to the cleaved vector, wherein the annealing occurs at compatible end elements within the first scaffold element and the cleaved vector, and wherein after annealing, the first scaffold element is integrated downstream of one of the plurality of CRISPR guide sequences, thereby producing a combinatorial vector, optionally wherein the method is iterative.

**15.** A method for generating a combinatorial vector, comprising

(a) providing a vector comprising:
a plurality of CRISPR guide sequences,
a barcode element, wherein the barcode element is located upstream of the plurality of CRISPR guide sequences;
optionally a promoter sequence located upstream of at least one of the plurality of CRISPR guide sequences; and
a plurality of recognition sites for a plurality of restriction enzymes, wherein each of the plurality of recognition sites is located upstream of one of the plurality of CRISPR guide sequences;
(b) cleaving the vector at least one of the plurality of recognition sites with at least one of the plurality of restriction enzymes, resulting in a first compatible end element and a second compatible end element;
(c) providing a first scaffold element comprising:
optionally a scaffold sequence that recruits an endonuclease to a target nucleic acid,
a promoter sequence, and
a third compatible end element and fourth compatible end element flanking the first scaffold element, wherein the third compatible end element is capable of annealing to the first compatible end element of the cleaved vector and the fourth compatible end element is capable of annealing to the second compatible end element of the cleaved vector;
(d) annealing the first scaffold element to the cleaved vector, wherein the annealing occurs at compatible end elements within the first scaffold element and the cleaved vector, and wherein after annealing, the first scaffold element is integrated upstream of one of the plurality of CRISPR guide sequences, thereby producing a combinatorial vector,
optionally wherein the method is iterative.

**Patentansprüche**

**1.** Genetisches Konstrukt, umfassend
ein erstes DNA-Element, umfassend
eine CRISPR-Führungssequenz und
eine Gerüstsequenz, die eine Endonuklease zu einer Ziel-Nukleinsäure rekrutiert;
ein erstes kompatibles Endelement und ein zweites kompatibles Endelement, die das erste DNA-Element flankieren, wobei das erste und das zweite kompatible Endelement in der Lage sind, aneinander zu annealen;
ein Strichcode-Element;
ein drittes kompatibles Endelement und ein viertes kompatibles Endelement, die das Strichcode-Element flankieren, wobei das dritte und das vierte kompatible Endelement in der Lage sind, aneinander zu annealen, jedoch nicht in der Lage sind, an das erste oder das zweite kompatible Endelement zu annealen; und
eine Trennungsstelle, die zwischen dem vierten kompatiblen Endelement und dem ersten kompatiblen Endelement lokalisiert ist, wobei das DNA-Element, das erste kompatible Endelement und das zweite kompatible Endelement auf einer Seite der Trennungsstelle liegen und das Strichcode-Element, das dritte kompatible Endelement und das vierte kompatible Endelement auf der anderen Seite der Trennungsstelle liegen.

**2.** Genetisches Konstrukt nach Anspruch 1, welches weiterhin ein Promotorelement strangaufwärts zu dem ersten DNA-Element umfasst.

**3.** Vektor, welcher ein genetisches Konstrukt gemäß Anspruch 1 oder 2 umfasst.

**4.** Genetisches Konstrukt, umfassend:

eine Mehrzahl von DNA-Elementen, wobei jedes DNA-Element aus der Mehrzahl von DNA-Elementen eine CRISPR-Führungssequenz und eine Gerüstsequenz, die eine Endonuklease zu einer Ziel-Nukleinsäure rekrutiert, umfasst;
ein erstes kompatibles Endelement und ein zweites kompatibles Endelement, die die Mehrzahl von DNA-Elementen flankieren, wobei das erste und das zweite kompatible Endelement in der Lage sind, aneinander zu annealen;
eine Mehrzahl von Strichcode-Elementen;
ein drittes kompatibles Endelement und ein viertes kompatibles Endelement, die die Mehrzahl von Strichcode-Elementen flankieren, wobei das dritte und das vierte kompatible Endelement in der Lage sind, aneinander zu annealen, jedoch nicht in der Lage sind, an das erste oder das zweite kompatible Endelement zu annealen; und
eine Trennungsstelle, die zwischen der Mehrzahl von DNA-Elementen und der Mehrzahl von Strichcode-Elementen lokalisiert ist.

**5.** Vektor, umfassend
ein genetisches Konstrukt gemäß Anspruch 4 und
eine Promotorsequenz, die strangaufwärts zu jeder der CRISPR-Führungssequenzen lokalisiert ist.

**6.** Verfahren zum Erzeugen eines kombinatorischen Vektors, umfassend:

(a) Bereitstellen eines Vektors, welcher ein erstes genetisches Konstrukt enthält, umfassend:

eine CRISPR-Führungssequenz;
ein zweites kompatibles Endelement und eine erste Erkennungsstelle für ein erstes Restriktionsenzym, die die CRISPR-Führungssequenz flankieren;
ein Strichcode-Element und
ein drittes kompatibles Endelement und eine zweite Erkennungsstelle für ein zweites Restriktionsenzym, die das Strichcode-Element flankieren;

(b) Spalten des ersten genetischen Konstrukts an der ersten Erkennungsstelle, was zu einem fünften kompatiblen Endelement führt, und Spalten des Vektors an der zweiten Erkennungsstelle, was zu einem sechsten kompatiblen Endelement führt;
(c) Bereitstellen eines Gerüstelements, umfassend
eine Gerüstsequenz, die eine Endonuklease zu einer Ziel-Nukleinsäure rekrutiert;
eine Trennungsstelle, welche ein erstes kompatibles Endelement und ein viertes kompatibles Endelement umfasst; und
ein siebtes kompatibles Endelement und ein achtes kompatibles Endelement, die das Gerüstelement flankieren, wobei das siebte kompatible Endelement in der Lage ist, an das fünfte kompatible Endelement zu annealen, und das achte kompatible Endelement in der Lage ist, an das sechste kompatible Endelement zu annealen;
(d) Annealen des Gerüstelements an das gespaltene erste genetische Konstrukt, wobei das Annealen an kompatiblen Endelementen innerhalb des Vektors und des Gerüstelements, die in der Lage sind, aneinander zu annealen, erfolgt, und wobei nach dem Annealen das Gerüstelement zwischen der CRISPR-Führungssequenz und dem Strichcode-Element integriert wird, und wobei die Trennungsstelle zwischen der Gerüstsequenz und dem Strichcode-Element lokalisiert ist, was einen kombinatorischen Vektor erzeugt,
wobei optional die erste Erkennungsstelle und die zweite Erkennungsstelle die gleiche Erkennungsstellensequenz haben und das erste Restriktionsenzym und das zweite Restriktionsenzym die gleichen Restriktionsenzyme sind.

**7.** Verfahren nach Anspruch 6, weiterhin umfassend:

(a) Bereitstellen eines kombinatorischen Vektors gemäß Anspruch 6;
(b) Spalten des Vektors an der Trennungsstelle innerhalb des Gerüstelements, was zu einem ersten kompatiblen Endelement und einem vierten kompatiblen Endelement führt;
(c) Bereitstellen eines zweiten genetischen Konstrukts, umfassend
eine CRISPR-Führungssequenz,
eine Gerüstsequenz, die eine Endonuklease zu einer Ziel-Nukleinsäure rekrutiert;
ein Strichcode-Element und
ein zweites kompatibles Endelement und ein drittes kompatibles Endelement, die das zweite genetische Konstrukt flankieren, wobei das zweite kompatible Endelement des zweiten genetischen Konstrukts in der Lage ist, an das erste kompatible Endelement des Vektors zu annealen, und das dritte kompatible Endelement des zweiten genetischen Konstrukts in der Lage ist, an das vierte kompatible Endelement des Vektors zu annealen;
(d) Annealen des zweiten genetischen Konstrukts an den gespaltenen Vektor, wobei das Annealen an kompatiblen Endelementen innerhalb des zweiten genetischen Konstrukts und des Vektors, die in der Lage sind, aneinander zu annealen, erfolgt, und wobei nach dem Annealen das zweite genetische Konstrukt in den Vektor integriert wird, was einen kombinatorischen Vektor erzeugt, welcher verkettete Strichcode-Elemente und verkettete CRISPR-Führungs- und -Gerüstsequenzen umfasst, wobei das Verfahren optional iterativ ist.

**8.** Verfahren nach Anspruch 7, wobei der kombinatorische Vektor weiterhin ein Promotorelement strangaufwärts zu den CRISPR-Führungssequenzen umfasst.

**9.** Genetisches Konstrukt, umfassend
wenigstens zwei CRISPR-Führungssequenzen;
eine Gerüstsequenz, die eine Endonuklease zu einer Ziel-Nukleinsäure rekrutiert;
ein Strichcode-Element und
eine Restriktionserkennungsstelle, die zwischen jeder CRISPR-Führungssequenz und zwischen dem Strichcode-Element und der dem Strichcode-Element am nächsten liegenden CRISPR-Sequenz lokalisiert ist.

**10.** Genetisches Konstrukt, umfassend
eine Mehrzahl von DNA-Elementen, jeweils umfassend
eine CRISPR-Führungssequenz und
eine Gerüstsequenz, die eine Endonuklease zu einer Ziel-Nukleinsäure rekrutiert;
ein Strichcode-Element und
eine Promotorsequenz, die strangaufwärts zu jedem der DNA-Elemente der Mehrzahl von DNA-Elementen lokalisiert ist.

**11.** Genetisches Konstrukt nach Anspruch 9 oder 10, wobei das Strichcode-Element am 5'-Ende des genetischen Konstrukts lokalisiert ist.

**12.** Genetisches Konstrukt nach Anspruch 9 oder 10, wobei das Strichcode-Element am 3'-Ende des genetischen Konstrukts lokalisiert ist.

**13.** Vektor, welcher irgendeines der genetischen Konstrukte gemäß den Ansprüchen 9-12 umfasst.

**14.** Verfahren zum Erzeugen eines kombinatorischen Vektors, umfassend

(a) Bereitstellen eines Vektors, umfassend:

eine Mehrzahl von CRISPR-Führungssequenzen;
ein Strichcode-Element, wobei das Strichcode-Element strangabwärts zu der Mehrzahl von CRISPR-Führungssequenzen lokalisiert ist;
optional eine Promotorsequenz, die strangaufwärts zu wenigstens einer aus der Mehrzahl von CRISPR-Führungssequenzen lokalisiert ist; und
eine Mehrzahl von Erkennungsstellen für eine Mehrzahl von Restriktionsenzymen, wobei jede aus der Mehrzahl von Erkennungsstellen strangabwärts zu einer aus der Mehrzahl von CRISPR-Führungssequenzen lokalisiert ist;

(b) Spalten des Vektors an wenigstens einer aus der Mehrzahl von Erkennungsstellen mit wenigstens einem aus der Mehrzahl von Restriktionsenzymen, was zu einem ersten kompatiblen Endelement und einem zweiten kompatiblen Endelement führt;
(c) Bereitstellen eines ersten Gerüstelements, umfassend:

eine Gerüstsequenz, die eine Endonuklease zu einer Ziel-Nukleinsäure rekrutiert,
optional eine Promotorsequenz und
ein drittes kompatibles Endelement und ein viertes kompatibles Endelement, die das erste Gerüstelement flankieren, wobei das dritte kompatible Endelement in der Lage ist, an das erste kompatible Endelement des gespaltenen Vektors zu annealen, und das vierte kompatible Endelement in der Lage ist, an das zweite kompatible Endelement des gespaltenen Vektors zu annealen;

(d) Annealen des ersten Gerüstelements an den gespaltenen Vektor, wobei das Annealen an kompatiblen Endelementen innerhalb des ersten Gerüstelements und des gespaltenen Vektors erfolgt, und wobei nach dem Annealen das erste Gerüstelement strangabwärts zu einer aus der Mehrzahl von CRISPR-Führungssequenzen integriert wird, wodurch ein kombinatorischer Vektor erzeugt wird, wobei das Verfahren optional iterativ ist.

**15.** Verfahren zum Erzeugen eines kombinatorischen Vektors, umfassend

(a) Bereitstellen eines Vektors, umfassend:

eine Mehrzahl von CRISPR-Führungssequenzen,
ein Strichcode-Element, wobei das Strichcode-Element strangaufwärts zu der Mehrzahl von CRISPR-Führungssequenzen lokalisiert ist;
optional eine Promotorsequenz, die strangaufwärts zu wenigstens einer aus der Mehrzahl von CRISPR-Führungssequenzen lokalisiert ist; und
eine Mehrzahl von Erkennungsstellen für eine Mehrzahl von Restriktionsenzymen, wobei jede aus der Mehrzahl von Erkennungsstellen strangaufwärts zu einer aus der Mehrzahl von CRISPR-Führungssequenzen lokalisiert ist;

(b) Spalten des Vektors an wenigstens einer aus der Mehrzahl von Erkennungsstellen mit wenigstens einem aus der Mehrzahl von Restriktionsenzymen, was zu einem ersten kompatiblen Endelement und einem zweiten kompatiblen Endelement führt;
(c) Bereitstellen eines ersten Gerüstelements, umfassend:

optional eine Gerüstsequenz, die eine Endonuklease zu einer Ziel-Nukleinsäure rekrutiert,
eine Promotorsequenz und
ein drittes kompatibles Endelement und ein viertes kompatibles Endelement, die das erste Gerüstelement flankieren, wobei das dritte kompatible Endelement in der Lage ist, an das erste kompatible Endelement des gespaltenen Vektors zu annealen, und das vierte kompatible Endelement in der Lage ist, an das zweite kompatible Endelement des gespaltenen Vektors zu annealen;

(d) Annealen des ersten Gerüstelements an den gespaltenen Vektor, wobei das Annealen an kompatiblen Endelementen innerhalb des ersten Gerüstelements und des gespaltenen Vektors erfolgt, und wobei nach dem Annealen das erste Gerüstelement strangaufwärts zu einer aus der Mehrzahl von CRISPR-Führungssequenzen integriert wird, wodurch ein kombinatorischer Vektor erzeugt wird, wobei das Verfahren optional iterativ ist.

## Revendications

**1.** Construction génétique comprenant
un premier élément d'ADN comprenant
une séquence de guidage CRISPR et
une séquence de squelette qui recrute une endonucléase vers un acide nucléique cible ;
un premier élément terminal compatible et un deuxième élément terminal compatible flanquant le premier élément d'ADN, dans laquelle les premier et deuxième éléments terminaux compatibles sont capables de s'hybrider l'un à l'autre ;
un élément de code à barres ;
un troisième élément terminal compatible et un quatrième élément terminal compatible flanquant l'élément de code à barres, dans laquelle les troisième et quatrième éléments terminaux compatibles sont capables de s'hybrider l'un à l'autre mais ne sont pas capables de s'hybrider au premier ou au deuxième élément terminal compatible ; et
un site de séparation situé entre le quatrième élément terminal compatible et le premier élément terminal compatible, dans laquelle l'élément d'ADN, le premier élément terminal compatible, et le deuxième élément terminal compatible sont sur un côté du site de séparation, et l'élément de code à barres, le troisième élément terminal compatible, et le quatrième élément terminal compatible sont sur l'autre côté du site de séparation.

**2.** Construction génétique selon la revendication 1, comprenant en outre un élément de promoteur en amont du premier élément d'ADN.

**3.** Vecteur comprenant une construction génétique selon la revendication 1 ou 2.

**4.** Construction génétique comprenant :

une pluralité d'éléments d'ADN, dans laquelle chaque élément d'ADN de la pluralité d'éléments d'ADN comprend une séquence de guidage CRISPR et une séquence de squelette qui recrute une endonucléase vers un acide nucléique cible ;
un premier élément terminal compatible et un deuxième élément terminal compatible flanquant la pluralité d'éléments d'ADN, dans laquelle les premier et deuxième éléments terminaux compatibles sont capables de s'hybrider l'un à l'autre ;
une pluralité d'éléments de code à barres ;
un troisième élément terminal compatible et un quatrième élément terminal compatible flanquant la pluralité d'éléments de code à barres, dans laquelle les troisième et quatrième éléments terminaux compatibles sont capables de s'hybrider l'un à l'autre mais ne sont pas capables de s'hybrider au premier ou au deuxième élément terminal compatible ; et
un site de séparation situé entre la pluralité d'éléments d'ADN et la pluralité d'éléments de code à barres.

**5.** Vecteur comprenant
une construction génétique selon la revendication 4 et
une séquence de promoteur située en amont de chacune des séquences de guidage CRISPR.

**6.** Procédé de génération d'un vecteur combinatoire, comprenant :

(a) la fourniture d'un vecteur contenant une première construction génétique comprenant :

une séquence de guidage CRISPR ;
un deuxième élément terminal compatible et un premier site de reconnaissance pour une première enzyme de restriction flanquant la séquence de guidage CRISPR ;
un élément de code à barres ; et
un troisième élément terminal compatible et un deuxième site de reconnaissance pour une deuxième enzyme de restriction flanquant l'élément de code à barres ;

(b) le clivage de la première construction génétique au niveau du premier site de reconnaissance, pour donner un cinquième élément terminal compatible, et le clivage du vecteur au niveau du deuxième site de reconnaissance, pour donner un sixième élément terminal compatible ;
(c) la fourniture d'un élément de squelette comprenant
une séquence de squelette qui recrute une endonucléase vers un acide nucléique cible ;
un site de séparation comprenant un premier élément terminal compatible et un quatrième élément terminal compatible ; et
un septième élément terminal compatible et un huitième élément terminal compatible flanquant l'élément de squelette, dans lequel le septième élément terminal compatible est capable de s'hybrider au cinquième élément terminal compatible et le huitième élément terminal compatible est capable de s'hybrider au sixième élément terminal compatible ;
(d) l'hybridation de l'élément de squelette à la première construction génétique clivée, dans lequel l'hybridation a lieu au niveau des éléments terminaux compatibles à l'intérieur du vecteur et de l'élément de squelette qui sont capables de s'hybrider les uns aux autres, et dans lequel après l'hybridation, l'élément de squelette est intégré entre la séquence de guidage CRISPR et l'élément de code à barres, et dans lequel le site de séparation est situé entre la séquence de squelette et l'élément de code à barres, créant un vecteur combinatoire,
facultativement dans lequel le premier site de reconnaissance et le deuxième site de reconnaissance présentent la même séquence de site de reconnaissance, et la première enzyme de restriction et la deuxième enzyme de restriction sont des enzymes de restriction identiques.

**7.** Procédé selon la revendication 6, comprenant en outre :

(a) la fourniture d'un vecteur combinatoire selon la revendication 6 ;
(b) le clivage du vecteur au niveau du site de séparation à l'intérieur de l'élément de squelette, donnant un premier élément terminal compatible et un quatrième élément terminal compatible ;
(c) la fourniture d'une deuxième construction génétique comprenant
une séquence de guidage CRISPR ;
une séquence de squelette qui recrute une endonucléase vers un acide nucléique cible ;

un élément de code à barres ; et
un deuxième élément terminal compatible et un troisième élément terminal compatible flanquant la deuxième construction génétique, dans lequel le deuxième élément terminal compatible de la deuxième construction génétique est capable de s'hybrider au premier élément terminal compatible du vecteur et le troisième élément terminal compatible de la deuxième construction génétique est capable de s'hybrider au quatrième élément terminal compatible du vecteur;
(d) l'hybridation de la deuxième construction génétique au vecteur clivé, dans lequel l'hybridation a lieu au niveau des éléments terminaux compatibles à l'intérieur de la deuxième construction génétique et du vecteur qui sont capables de s'hybrider les uns aux autres, et dans lequel après l'hybridation, la deuxième construction génétique est intégrée dans le vecteur, créant un vecteur combinatoire comprenant des éléments à code à barres concaténés et des séquences de guidage CRISPR et de squelette concaténées, facultativement dans lequel le procédé est itératif.

**8.** Procédé selon la revendication 7, dans lequel le vecteur combinatoire comprend en outre un élément de promoteur en amont des séquences de guidage CRISPR.

**9.** Construction génétique comprenant
au moins deux séquences de guidage CRISPR ;
une séquence de squelette qui recrute une endonucléase vers un acide nucléique cible ;
un élément de code à barres ; et
un site de reconnaissance de restriction situé entre chaque séquence de guidage CRISPR et entre l'élément de code à barres et la séquence de guidage CRISPR la plus proche de l'élément de code à barres.

**10.** Construction génétique comprenant
une pluralité d'éléments d'ADN, chacun comprenant
une séquence de guidage CRISPR et
une séquence de squelette qui recrute une endonucléase vers un acide nucléique cible ;
un élément de code à barres ; et
une séquence de promoteur situé en amont de chacun des éléments d'ADN de la pluralité d'éléments d'ADN.

**11.** Construction génétique selon la revendication 9 ou 10, dans laquelle l'élément de code à barres est situé au niveau de l'extrémité 5' de la construction génétique.

**12.** Construction génétique selon la revendication 9 ou 10, dans laquelle l'élément de code à barres est situé au niveau de l'extrémité 3' de la construction génétique.

**13.** Vecteur comprenant l'une quelconque des constructions génétiques selon les revendications 9 à 12.

**14.** Procédé de génération d'un vecteur combinatoire, comprenant

(a) la fourniture d'un vecteur comprenant :

une pluralité de séquence de guidage CRISPR ;
un élément de code à barres, dans lequel l'élément de code à barres est situé en aval de la pluralité de séquences de guidage CRISPR ;
facultativement une séquence de promoteur située en amont d'au moins l'une de la pluralité de séquences de guidage CRISPR ; et
une pluralité de sites de reconnaissance pour une pluralité d'enzymes de restriction, dans lequel chacun de la pluralité de sites de reconnaissance est situé en aval d'une de la pluralité de séquences de guidage CRISPR ;

(b) le clivage du vecteur au niveau d'au moins l'un de la pluralité de sites de reconnaissance avec au moins l'une de la pluralité d'enzymes de restriction, pour donner un premier élément terminal compatible et un deuxième élément terminal compatible ;
(c) la fourniture d'un premier élément de squelette comprenant :

une séquence de squelette qui recrute une endonucléase vers un acide nucléique cible,
facultativement une séquence de promoteur, et

un troisième élément terminal compatible et un quatrième élément terminal compatible flanquant le premier élément de squelette, dans lequel le troisième élément terminal compatible est capable de s'hybrider au premier élément terminal compatible du vecteur clivé et le quatrième élément terminal compatible est capable de s'hybrider au deuxième élément terminal compatible du vecteur clivé ;

(d) l'hybridation du premier élément de squelette au vecteur clivé, dans lequel l'hybridation a lieu au niveau des éléments terminaux compatibles à l'intérieur du premier élément de squelette et du vecteur clivé, et dans lequel après l'hybridation, le premier élément de squelette est intégré en aval de l'une de la pluralité de séquences de guidage CRISPR, pour ainsi produire un vecteur combinatoire,
facultativement dans lequel le procédé est itératif.

**15.** Procédé de génération d'un vecteur combinatoire, comprenant

(a) la fourniture d'un vecteur comprenant :

une pluralité de séquence de guidage CRISPR,
un élément de code à barres, dans lequel l'élément de code à barres est situé en amont de la pluralité de séquences de guidage CRISPR ;
facultativement une séquence de promoteur située en amont d'au moins l'une de la pluralité de séquences de guidage CRISPR ; et
une pluralité de sites de reconnaissance pour une pluralité d'enzymes de restriction, dans lequel chacun de la pluralité de sites de reconnaissance est situé en amont d'une de la pluralité de séquences de guidage CRISPR ;

(b) le clivage du vecteur au moins à l'un de la pluralité de sites de reconnaissance avec au moins l'une de la pluralité d'enzymes de restriction, pour donner un premier élément terminal compatible et un deuxième élément terminal compatible ;
(c) la fourniture d'un premier élément de squelette comprenant :

facultativement une séquence de squelette qui recrute une endonucléase vers un acide nucléique cible,
une séquence de promoteur, et
un troisième élément terminal compatible et un quatrième élément terminal compatible flanquant le premier élément de squelette, dans lequel le troisième élément terminal compatible est capable de s'hybrider au premier élément terminal compatible du vecteur clivé et le quatrième élément terminal compatible est capable de s'hybrider au deuxième élément terminal compatible du vecteur clivé ;

(d) l'hybridation du premier élément de squelette au vecteur clivé, dans lequel l'hybridation a lieu au niveau des éléments terminaux compatibles à l'intérieur du premier élément de squelette et du vecteur clivé, et dans lequel après l'hybridation, le premier élément de squelette est intégré en amont de l'une de la pluralité de séquences de guidage CRISPR, pour ainsi produire un vecteur combinatoire,
facultativement dans lequel le procédé est itératif.

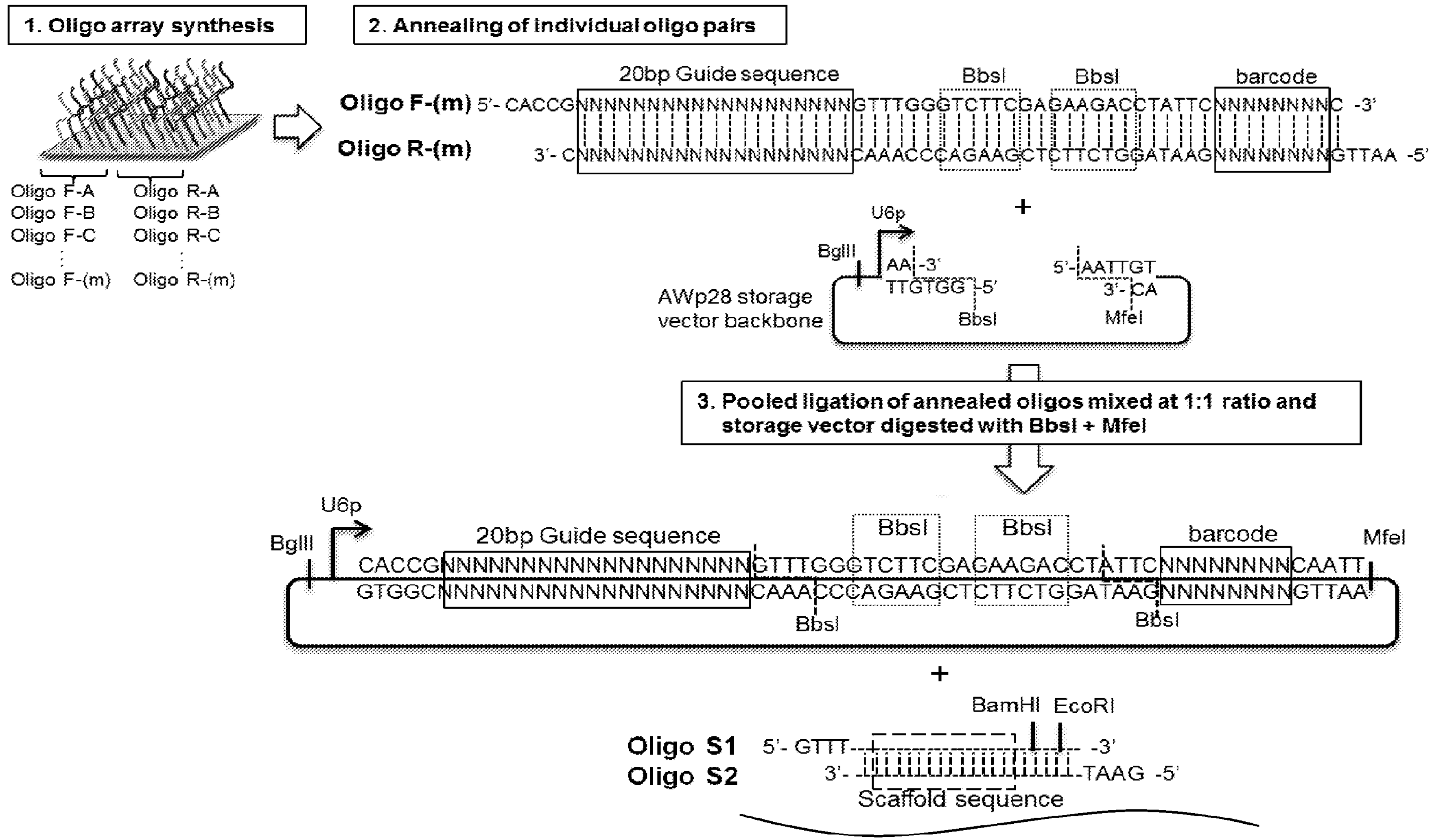


FIG. 1A

TO FIG. 1A (CONT.)

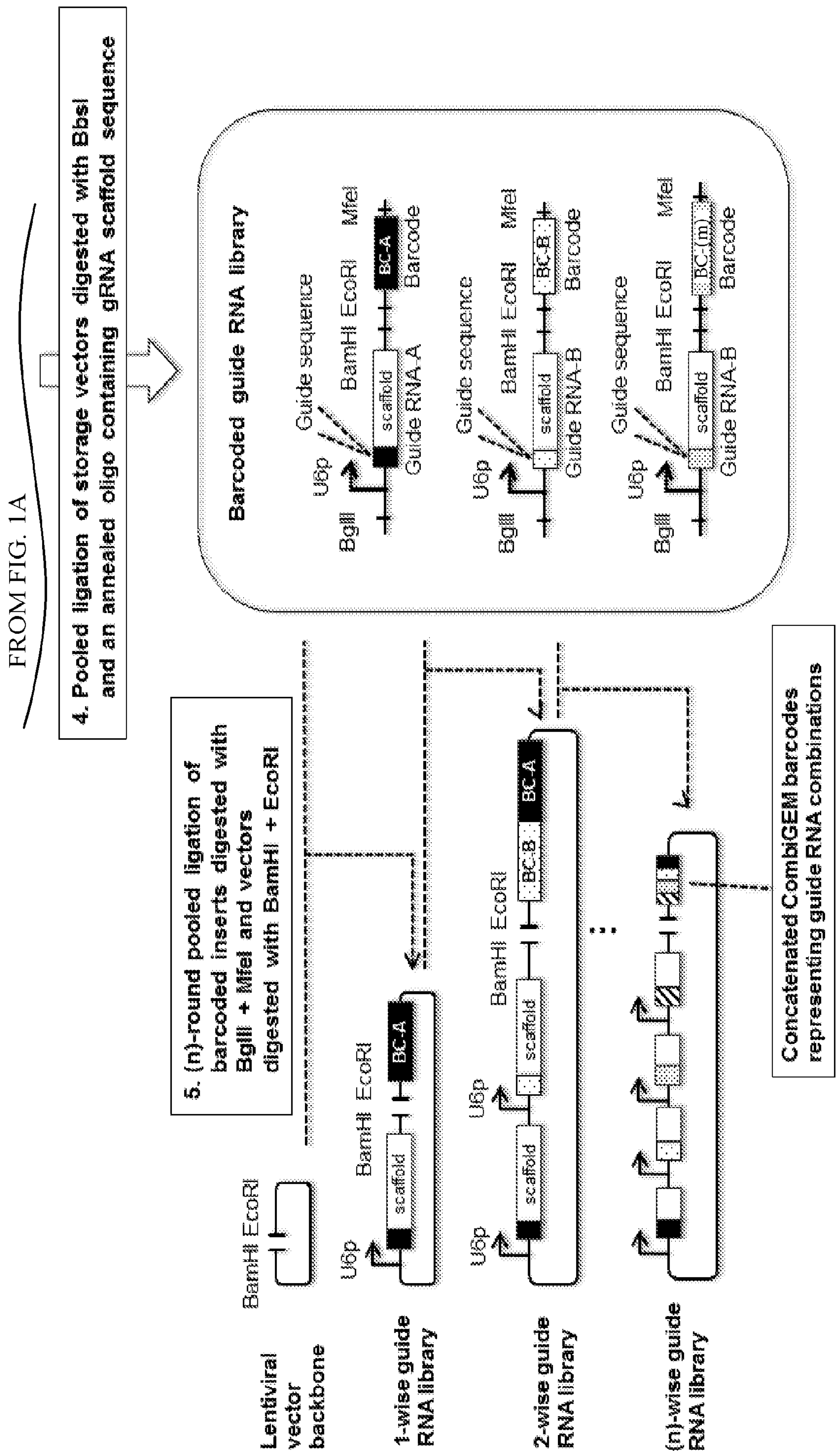
FROM FIG. 1A
4. Pooled ligation of storage vectors digested with BbsI and an annealed oligo containing gRNA scaffold sequence
Barcoded guide RNA library
BglII
U6p
Guide sequence
BamHI EcoRI
MfeI
scaffold
BC-A
Guide RNA-A
Barcode
BC-B
Guide RNA-B
BC-(m)
5. (n)-round pooled ligation of barcoded inserts digested with BglII + MfeI and vectors digested with BamHI + EcoRI
Lentiviral vector backbone
1-wise guide RNA library
2-wise guide RNA library
(n)-wise guide RNA library
Concatenated CombiGEM barcodes representing guide RNA combinations

FIG. 1A (CONT.)

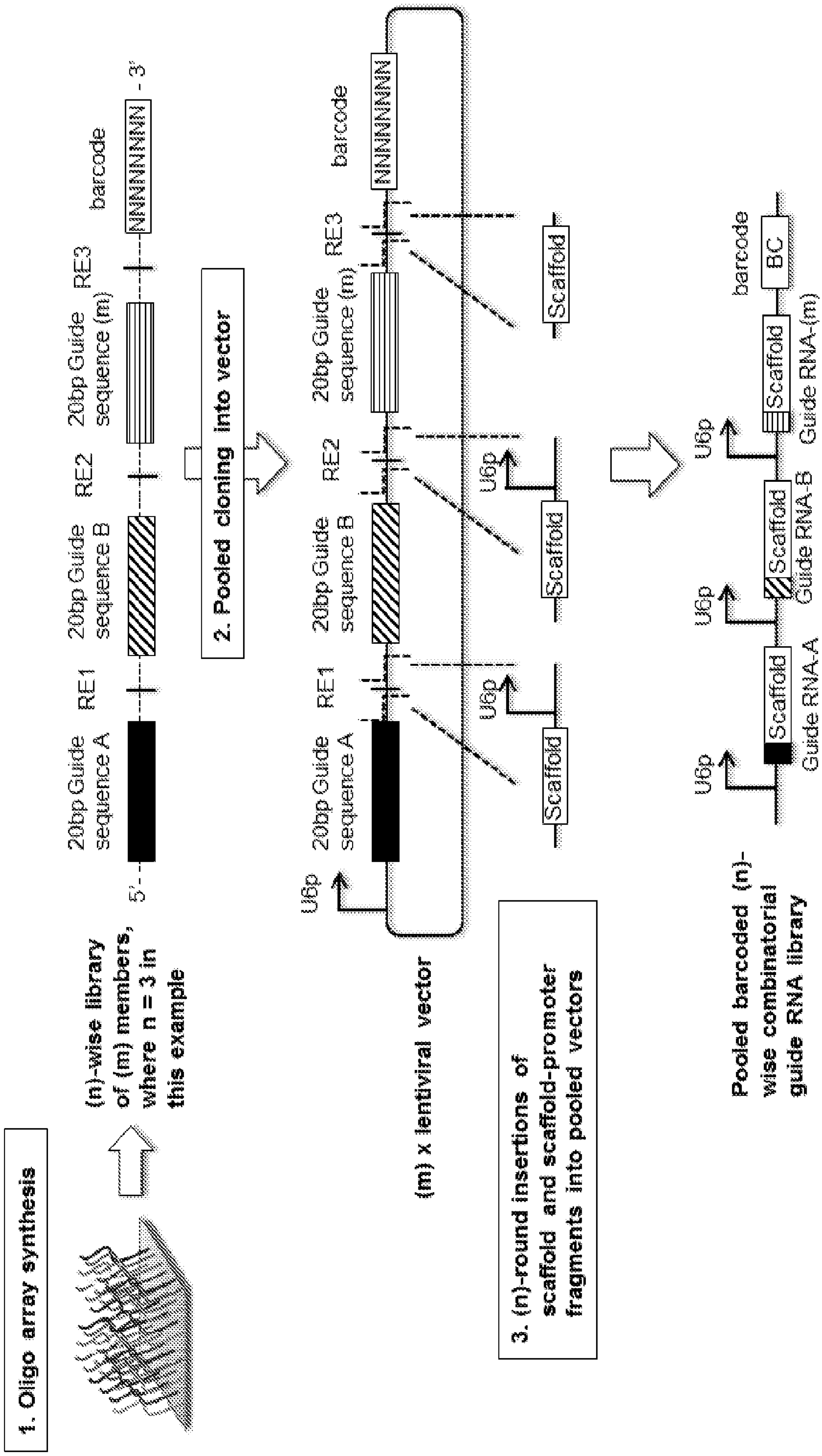
1. Oligo array synthesis
(n)-wise library of (m) members, where n = 3 in this example
5' -
20bp Guide sequence A
RE1
20bp Guide sequence B
RE2
20bp Guide sequence (m)
RE3
barcode
NNNNNNNNN
- 3'
2. Pooled cloning into vector
U6p
20bp Guide sequence A
RE1
20bp Guide sequence B
RE2
20bp Guide sequence (m)
RE3
barcode
NNNNNNNNN
(m) x lentiviral vector
Scaffold
U6p
Scaffold
U6p
Scaffold
3. (n)-round insertions of scaffold and scaffold-promoter fragments into pooled vectors
U6p
Scaffold
Guide RNA-A
U6p
Scaffold
Guide RNA-B
U6p
Scaffold
Guide RNA-(m)
barcode
BC
Pooled barcoded (n)-wise combinatorial guide RNA library

FIG. 2A

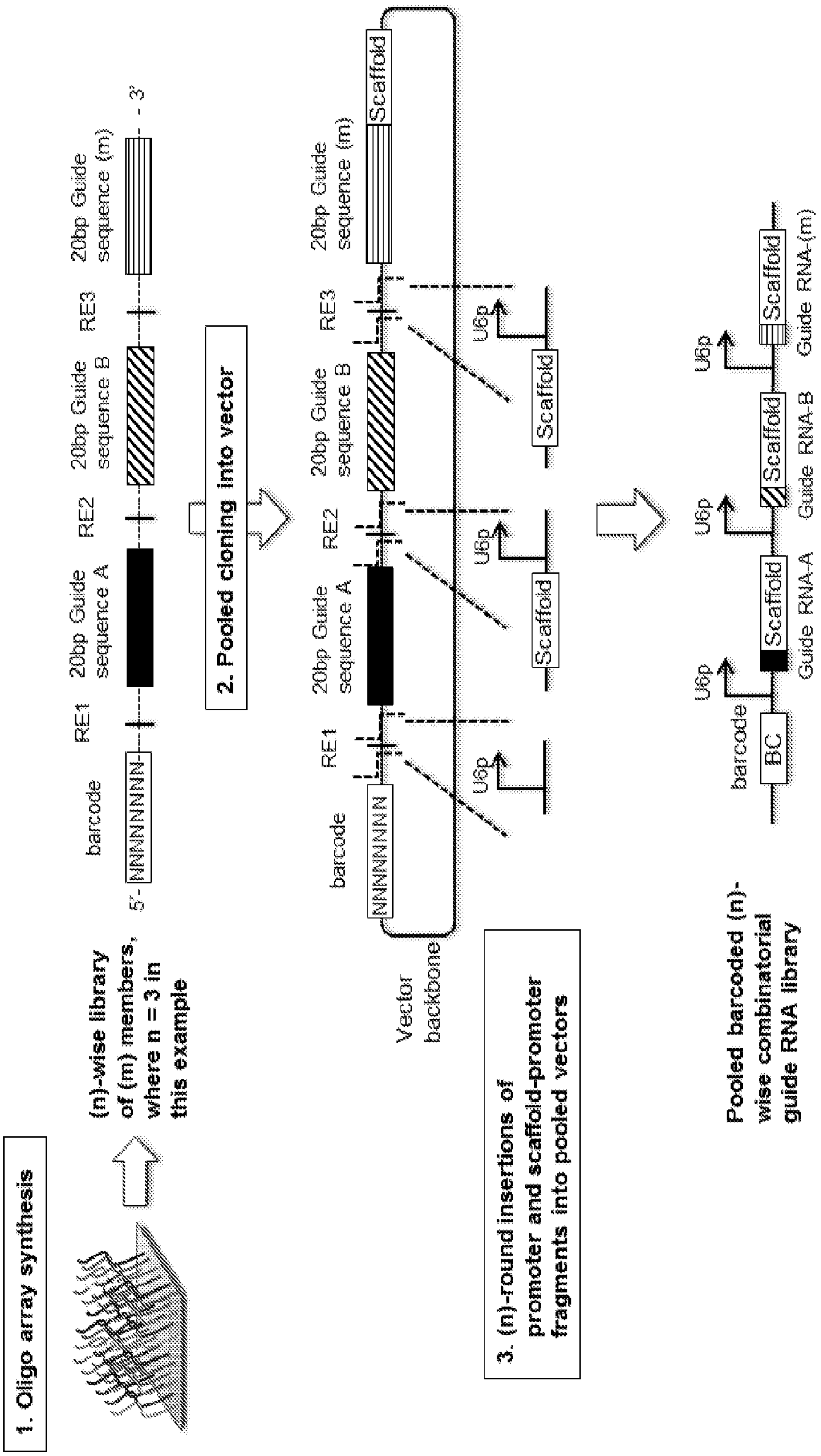
1. Oligo array synthesis
(n)-wise library of (m) members, where n = 3 in this example
barcode
NNNNNNNN
5'-
RE1
20bp Guide sequence A
RE2
20bp Guide sequence B
RE3
20bp Guide sequence (m)
- 3'
2. Pooled cloning into vector
Vector backbone
barcode
NNNNNNNN
RE1
20bp Guide sequence A
RE2
20bp Guide sequence B
RE3
20bp Guide sequence (m)
Scaffold
U6p
Scaffold
U6p
Scaffold
U6p
3. (n)-round insertions of promoter and scaffold-promoter fragments into pooled vectors
Pooled barcoded (n)-wise combinatorial guide RNA library
barcode
BC
U6p
Scaffold
Guide RNA-A
U6p
Scaffold
Guide RNA-B
U6p
Scaffold
Guide RNA-(m)

FIG. 2B

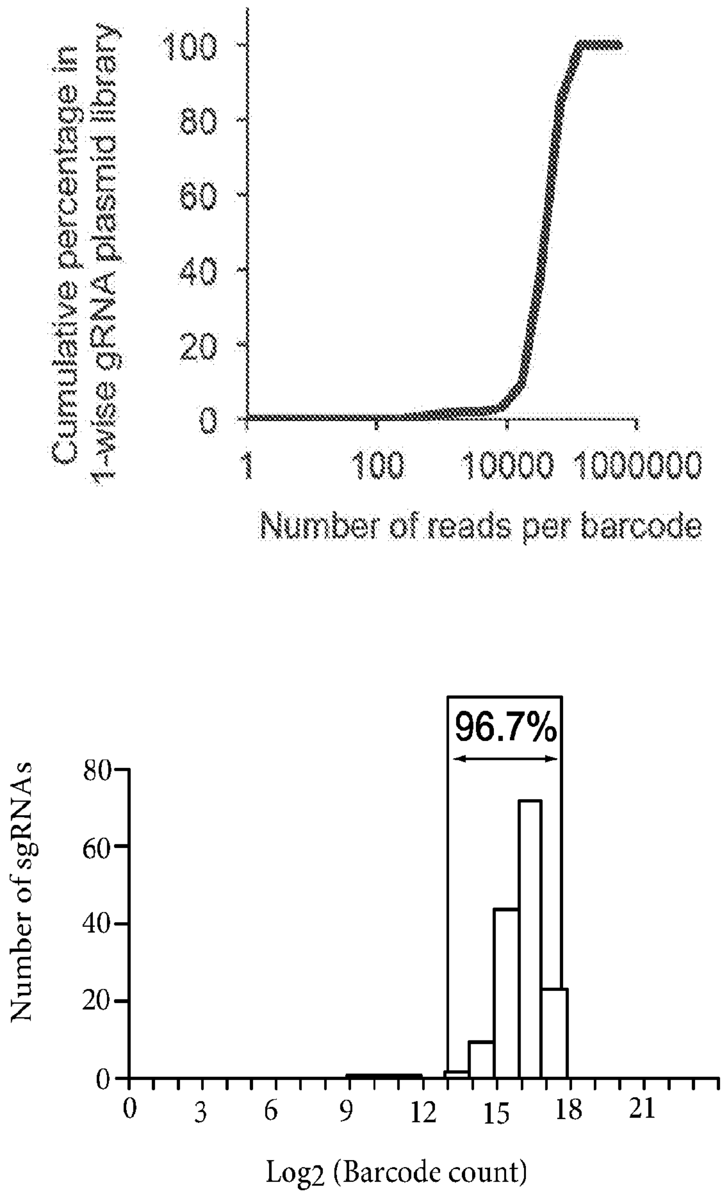
Cumulative percentage in
1-wise gRNA plasmid library
100
80
60
40
20
0
1
100
10000
1000000
Number of reads per barcode
96.7%
Number of sgRNAs
80
60
40
20
0
0
3
6
9
12
15
18
21
Log2 (Barcode count)

**FIG. 3A**

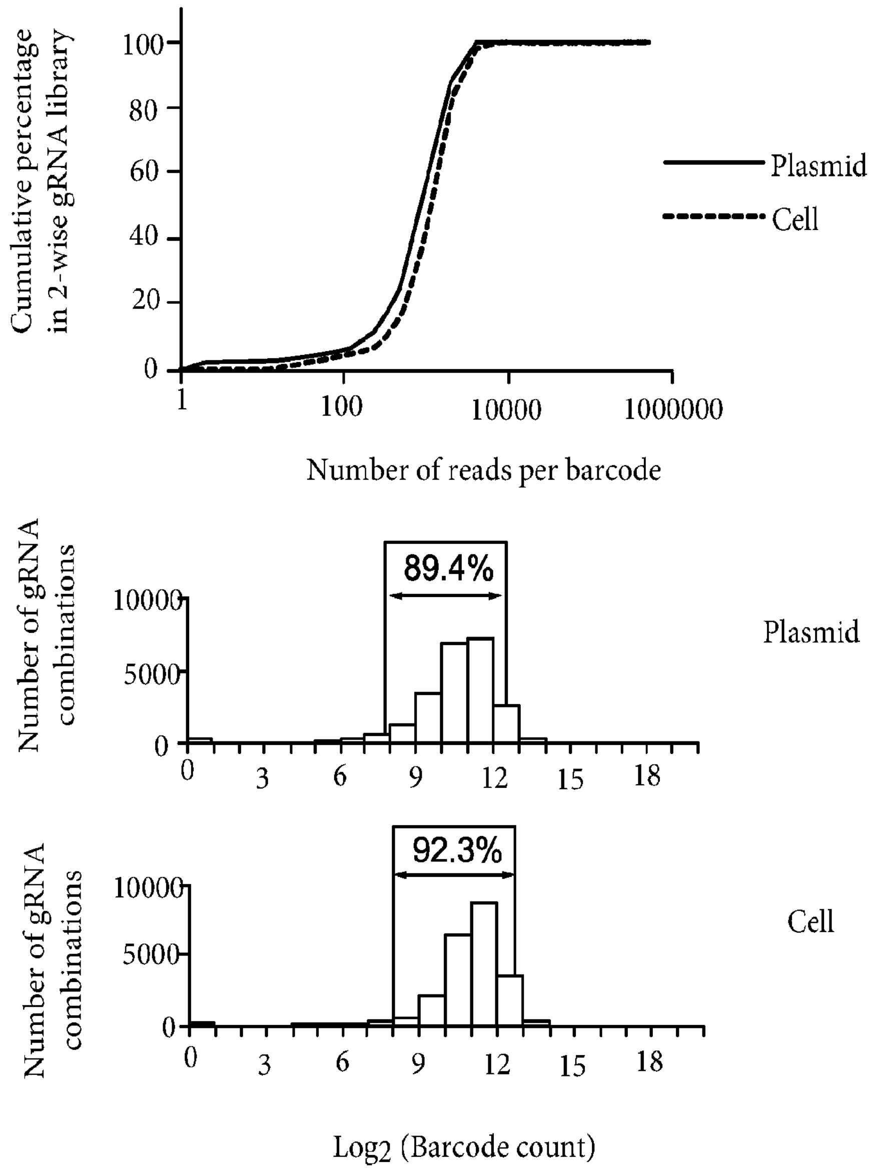
Cumulative percentage in 2-wise gRNA library
100
80
60
40
20
0
1
100
10000
1000000
Number of reads per barcode
Plasmid
Cell
Number of gRNA combinations
89.4%
10000
5000
0
0 3 6 9 12 15 18
Plasmid
Number of gRNA combinations
92.3%
10000
5000
0
0 3 6 9 12 15 18
Cell
Log2 (Barcode count)

**FIG. 3B**

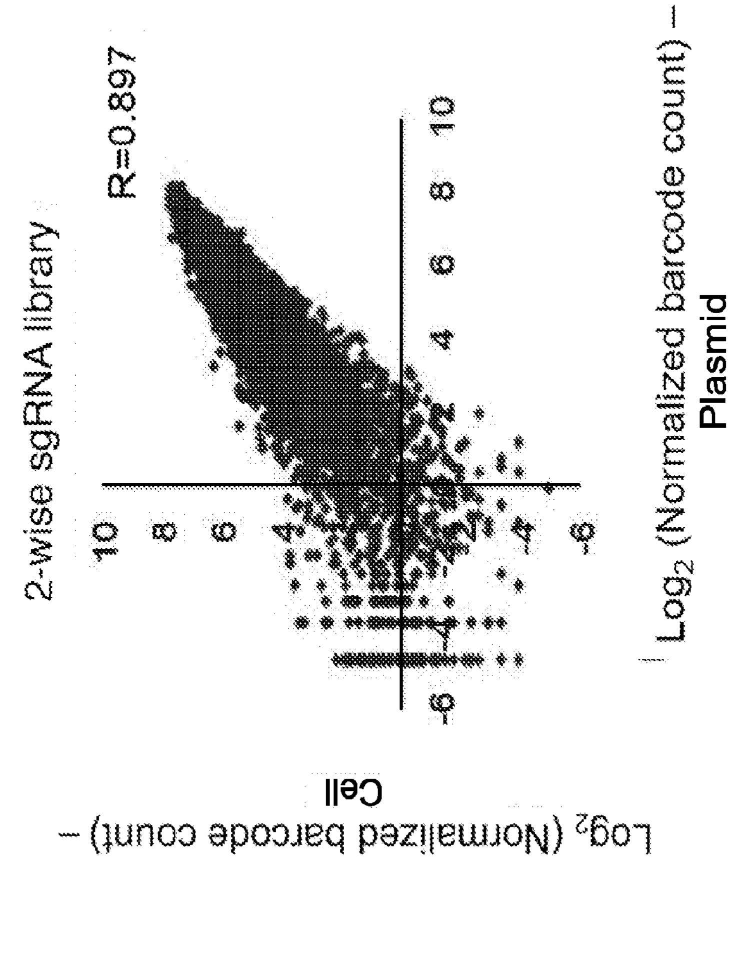
2-wise sgRNA library
R=0.897
Log2 (Normalized barcode count) – Plasmid
Log2 (Normalized barcode count) – Cell
10
8
6
4
2
-2
-4
-6
-6
-4
2
4
6
8
10

FIG. 3C

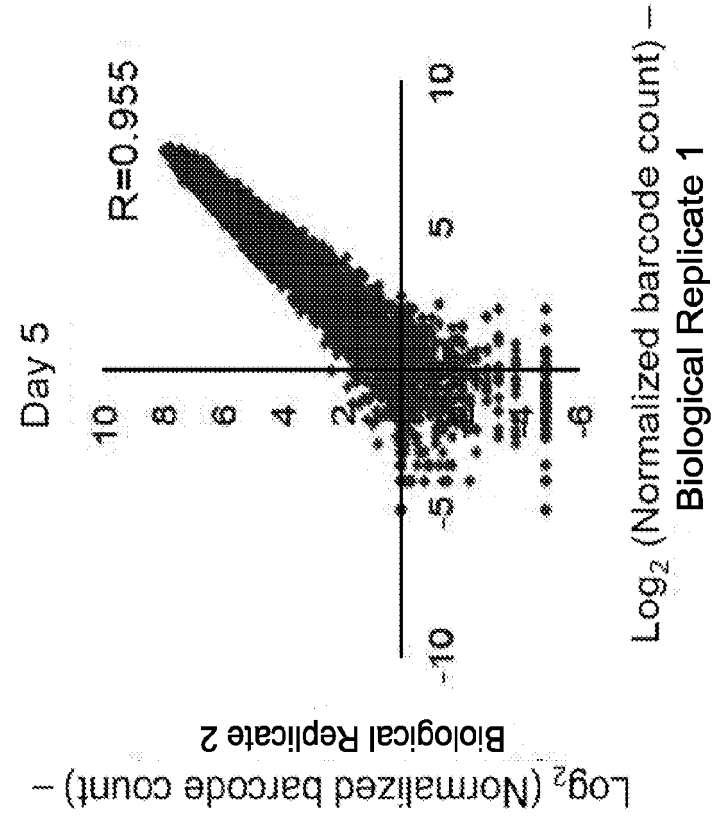
Day 5
R=0.955
Log2 (Normalized barcode count) – Biological Replicate 1
Log2 (Normalized barcode count) – Biological Replicate 2
10
8
6
4
2
-6
-10
-5
5
10

FIG. 3D

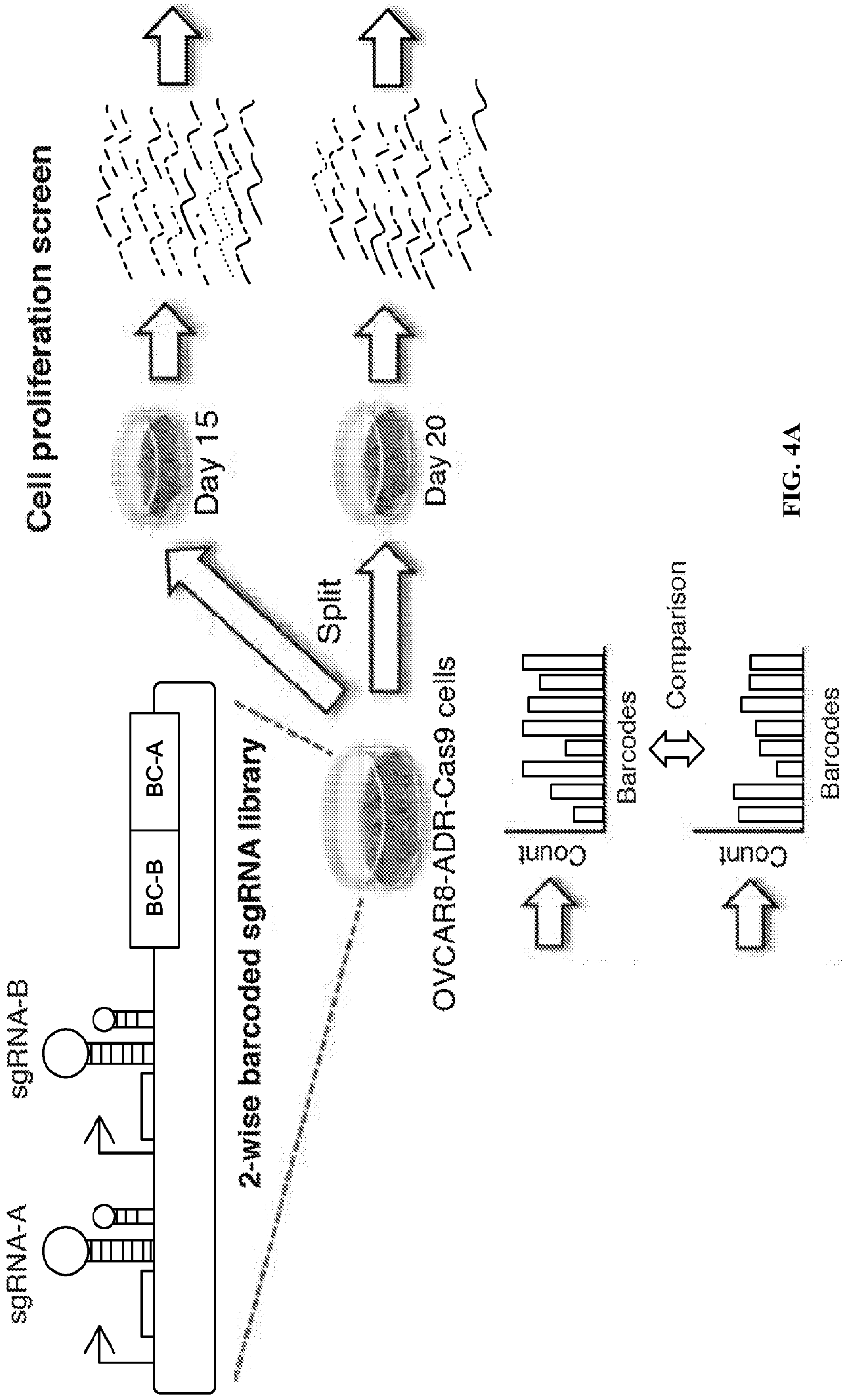
sgRNA-A
sgRNA-B
BC-B
BC-A
2-wise barcoded sgRNA library
OVCAR8-ADR-Cas9 cells
Split
Day 15
Day 20
Cell proliferation screen
Count
Barcodes
Comparison
Count
Barcodes

FIG. 4A

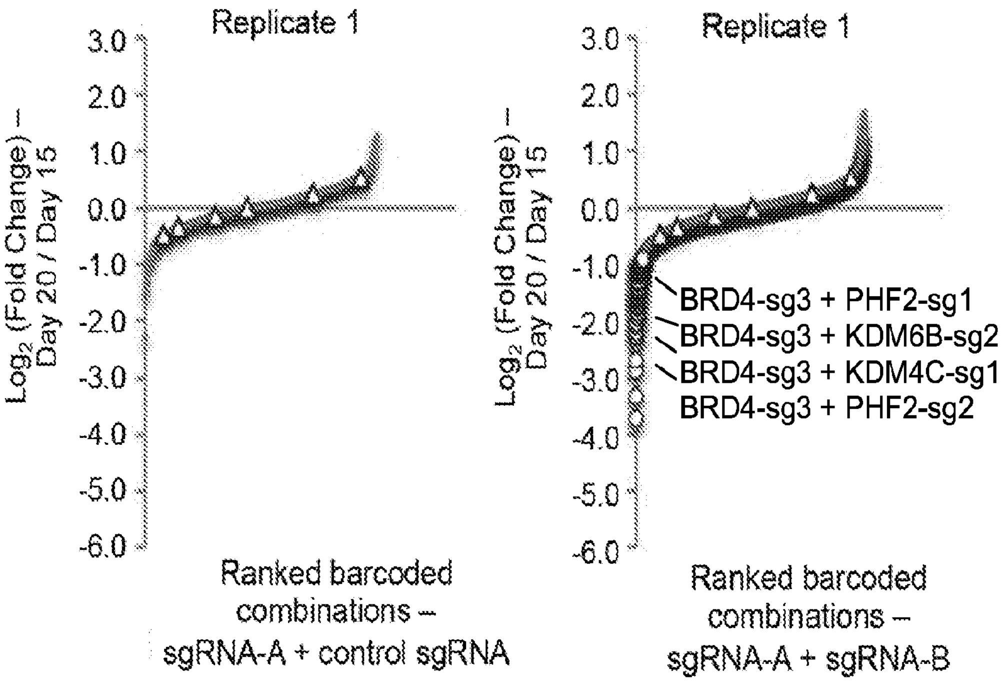
Replicate 1
3.0
2.0
1.0
0.0
-1.0
-2.0
-3.0
-4.0
-5.0
-6.0
Log2 (Fold Change) – Day 20 / Day 15
Ranked barcoded combinations – sgRNA-A + control sgRNA
Replicate 1
3.0
2.0
1.0
0.0
-1.0
-2.0
-3.0
-4.0
-5.0
-6.0
Log2 (Fold Change) – Day 20 / Day 15
BRD4-sg3 + PHF2-sg1
BRD4-sg3 + KDM6B-sg2
BRD4-sg3 + KDM4C-sg1
BRD4-sg3 + PHF2-sg2
Ranked barcoded combinations – sgRNA-A + sgRNA-B

FIG. 4B

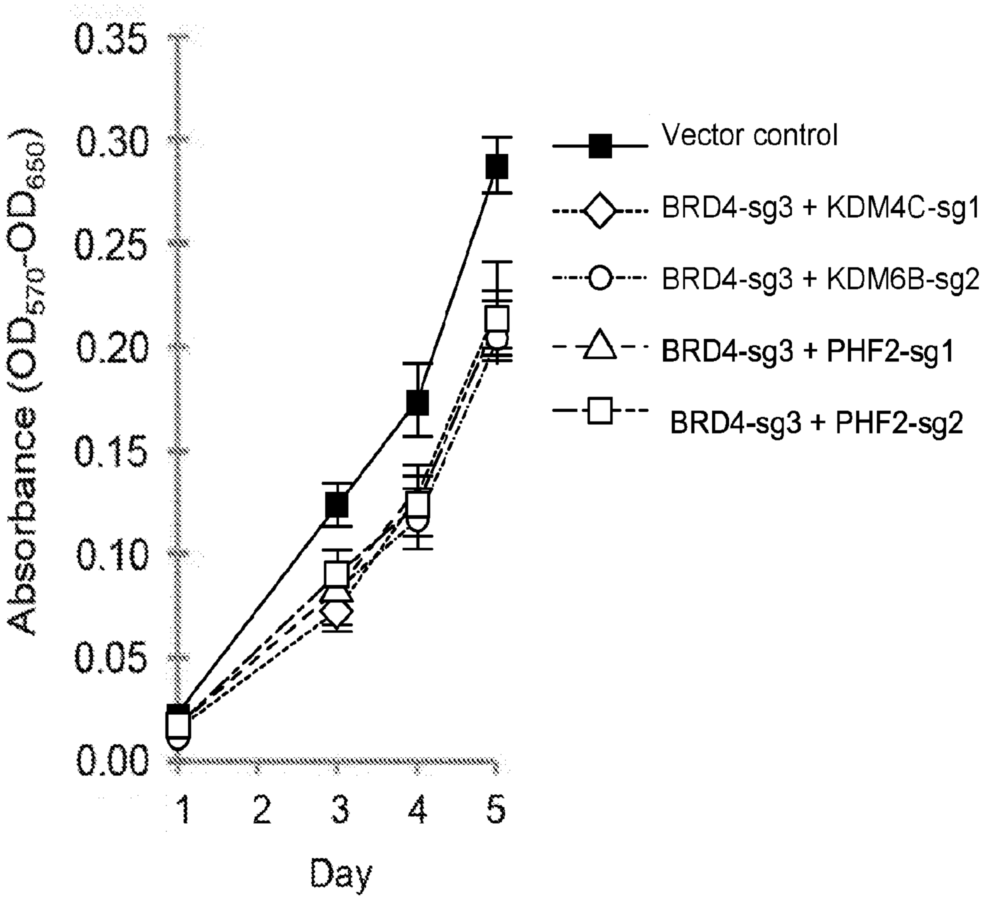
0.35
0.30
0.25
0.20
0.15
0.10
0.05
0.00
Absorbance (OD570-OD650)
1
2
3
4
5
Day
Vector control
BRD4-sg3 + KDM4C-sg1
BRD4-sg3 + KDM6B-sg2
BRD4-sg3 + PHF2-sg1
BRD4-sg3 + PHF2-sg2

FIG. 4C

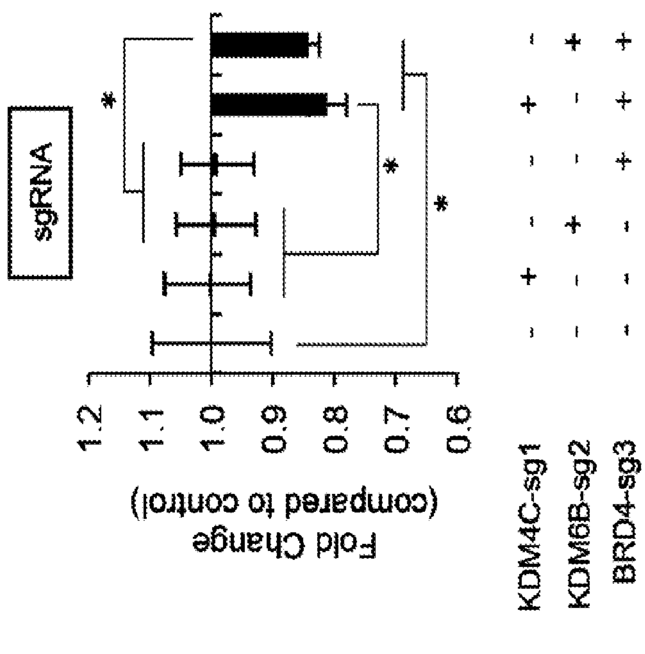
sgRNA
Fold Change
(compared to control)
1.2
1.1
1.0
0.9
0.8
0.7
0.6
*
*
*
KDM4C-sg1
KDM6B-sg2
BRD4-sg3

FIG. 5A

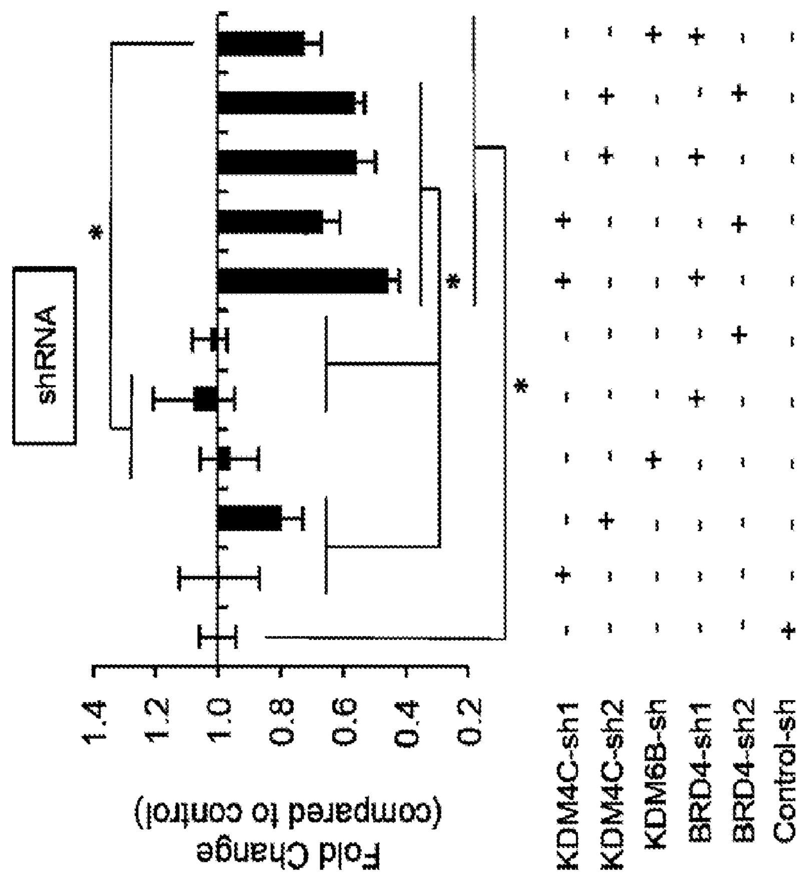
shRNA
Fold Change
(compared to control)
1.4
1.2
1.0
0.8
0.6
0.4
0.2
*
*
*
KDM4C-sh1
KDM4C-sh2
KDM6B-sh
BRD4-sh1
BRD4-sh2
Control-sh

FIG. 5B

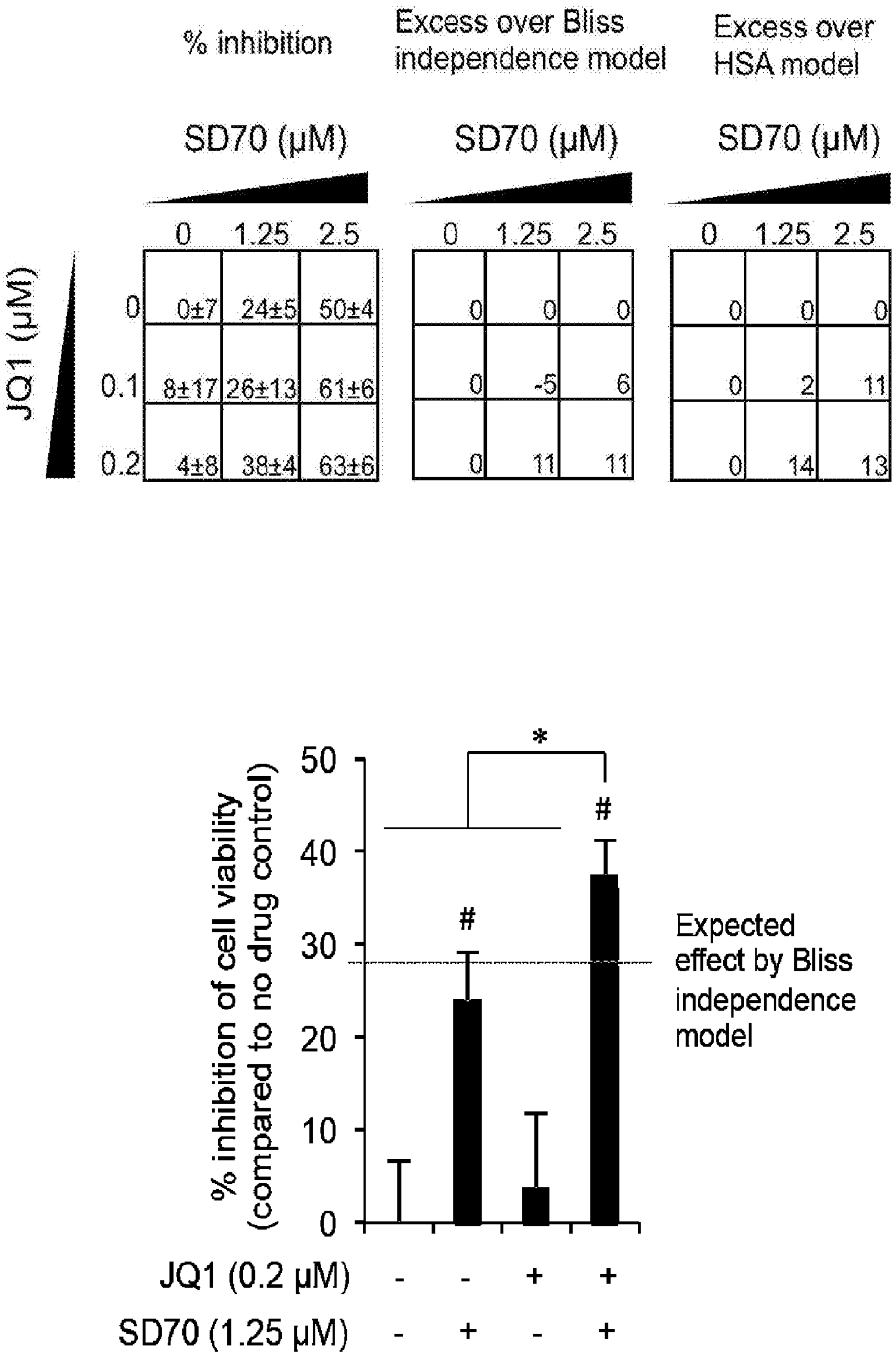
% inhibition
Excess over Bliss independence model
Excess over HSA model
SD70 (μM)
0 1.25 2.5
JQ1 (μM)
0 0.1 0.2
0±7 24±5 50±4
8±17 26±13 61±6
4±8 38±4 63±6
0 0 0
0 -5 6
0 11 11
0 0 0
0 2 11
0 14 13
% inhibition of cell viability (compared to no drug control)
50 40 30 20 10 0
*
#
#
Expected effect by Bliss independence model
JQ1 (0.2 μM) - - + +
SD70 (1.25 μM) - + - +

FIG. 5C

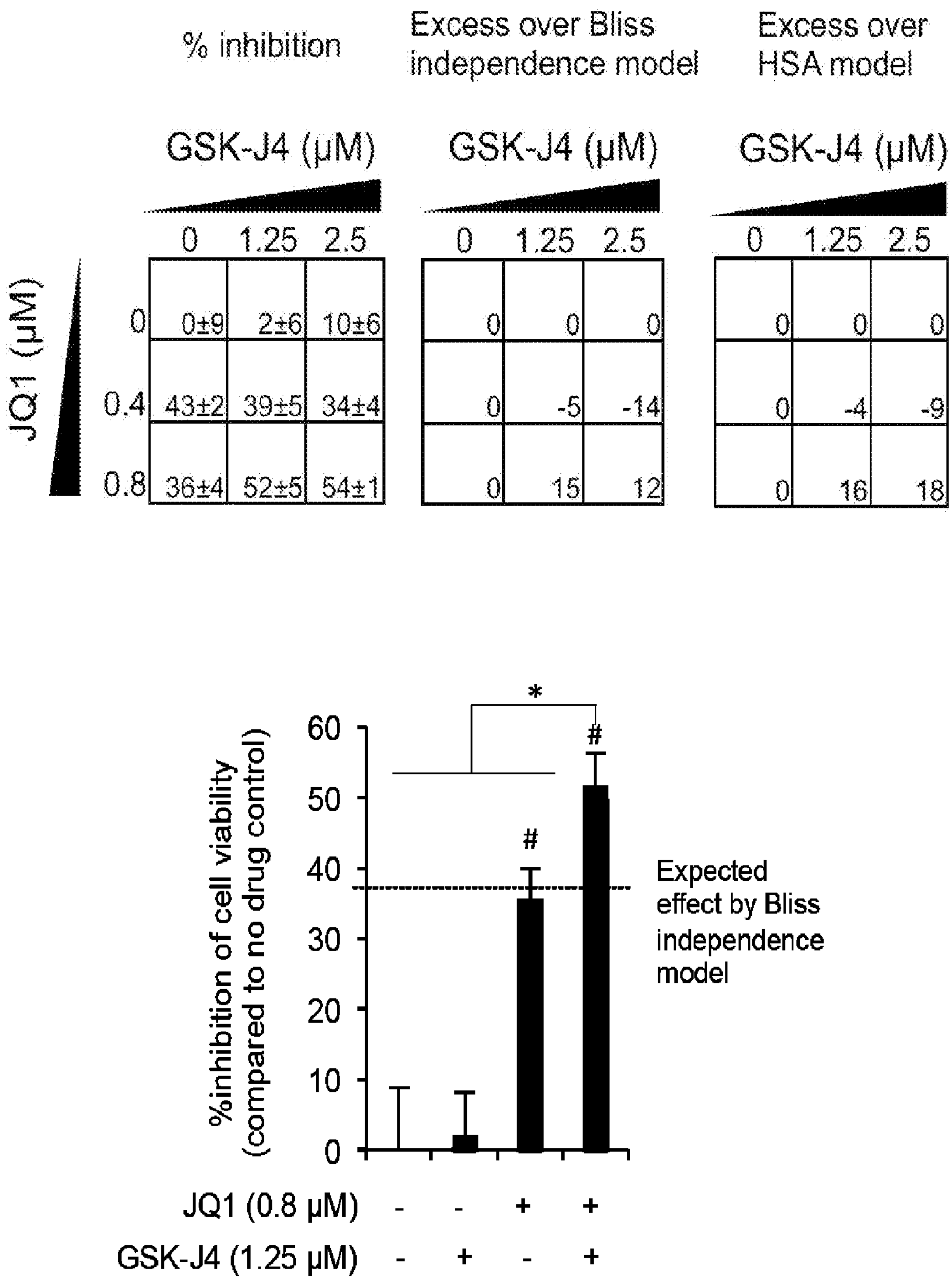
% inhibition
Excess over Bliss independence model
Excess over HSA model
GSK-J4 (μM)
0 1.25 2.5
JQ1 (μM)
0 0.4 0.8
0±9 2±6 10±6
43±2 39±5 34±4
36±4 52±5 54±1
0 0 0
0 -5 -14
0 15 12
0 0 0
0 -4 -9
0 16 18
%inhibition of cell viability (compared to no drug control)
0 10 20 30 40 50 60
Expected effect by Bliss independence model
JQ1 (0.8 μM) - - + +
GSK-J4 (1.25 μM) - + - +
*
#

FIG. 5D

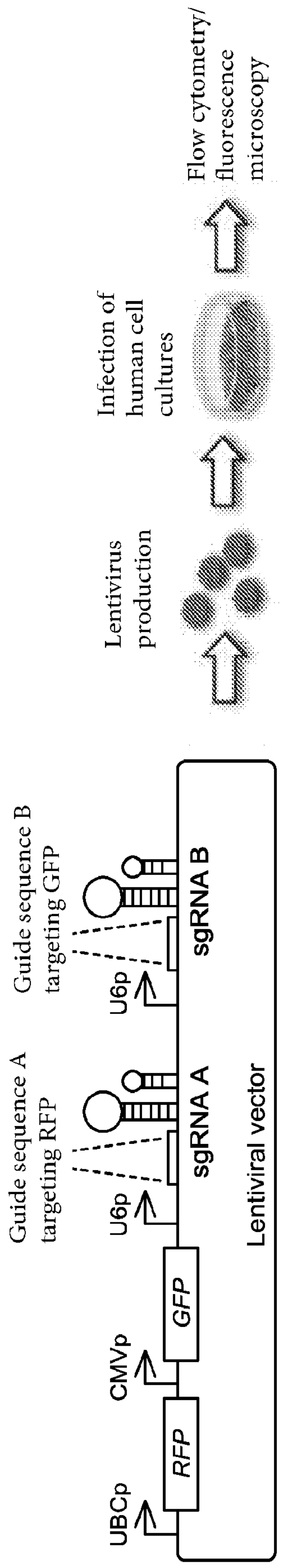
UBCp
RFP
CMVp
GFP
U6p
Guide sequence A targeting RFP
sgRNA A
U6p
Guide sequence B targeting GFP
sgRNA B
Lentiviral vector
Lentivirus production
Infection of human cell cultures
Flow cytometry/ fluorescence microscopy

FIG. 6A

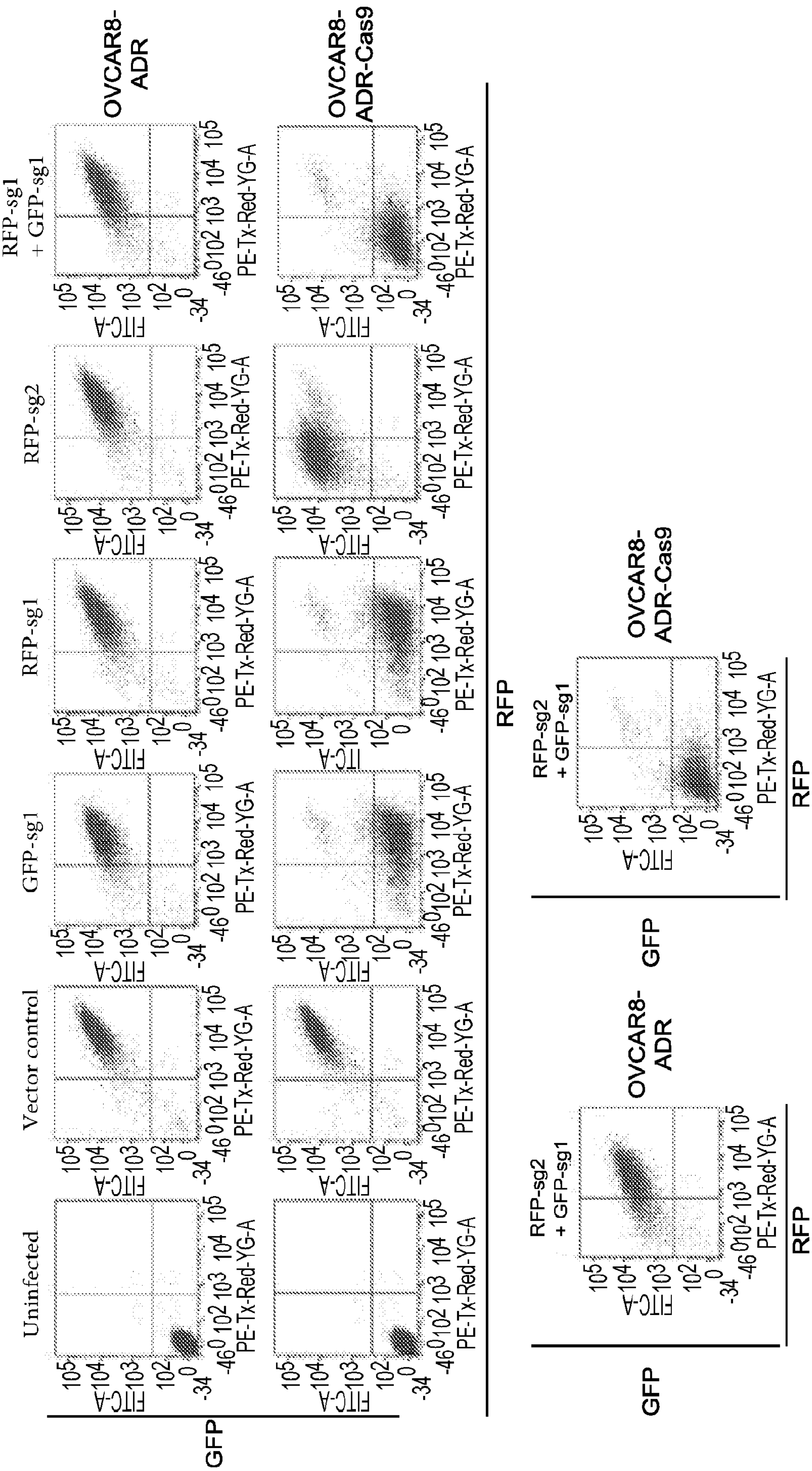
Uninfected
Vector control
GFP-sg1
RFP-sg1
RFP-sg2
RFP-sg1
+ GFP-sg1
OVCAR8-
ADR
OVCAR8-
ADR-Cas9
FITC-A
PE-Tx-Red-YG-A
GFP
RFP
RFP-sg2
+ GFP-sg1
OVCAR8-
ADR
OVCAR8-
ADR-Cas9

FIG. 6B

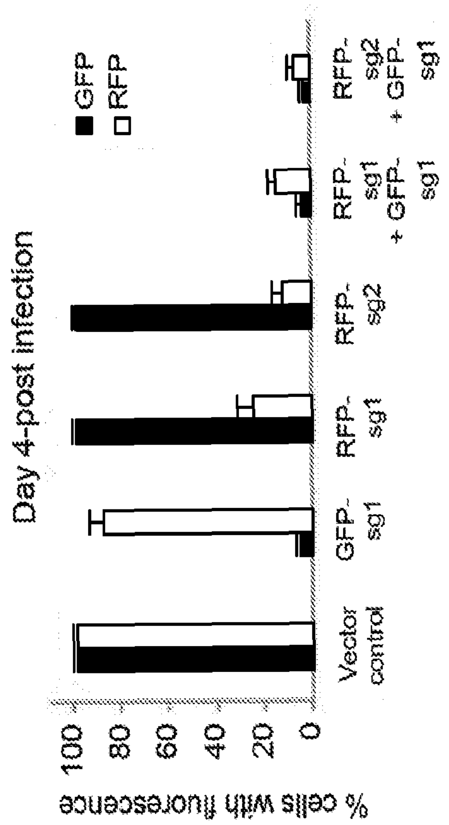
Day 4-post infection
% cells with fluorescence
100
80
60
40
20
0
Vector control
GFP-sg1
RFP-sg1
RFP-sg2
RFP-sg1 + GFP-sg1
RFP-sg2 + GFP-sg1
GFP
RFP

FIG. 6C

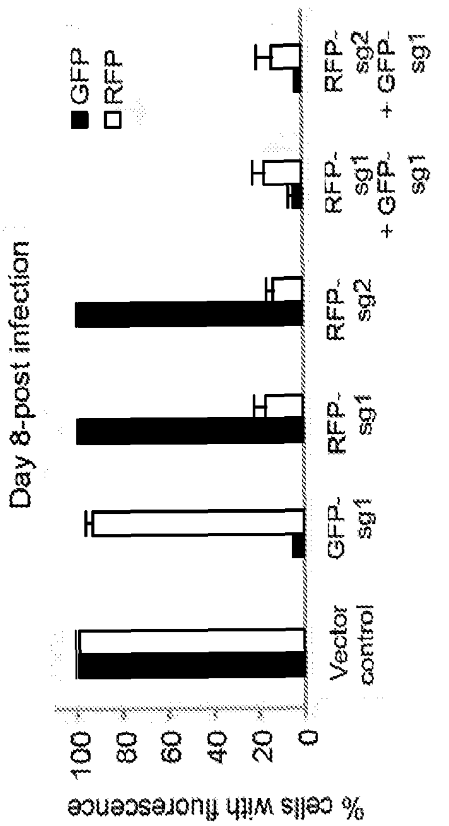
Day 8-post infection
% cells with fluorescence
100
80
60
40
20
0
Vector control
GFP-sg1
RFP-sg1
RFP-sg2
RFP-sg1 + GFP-sg1
RFP-sg2 + GFP-sg1
GFP
RFP

FIG. 6D

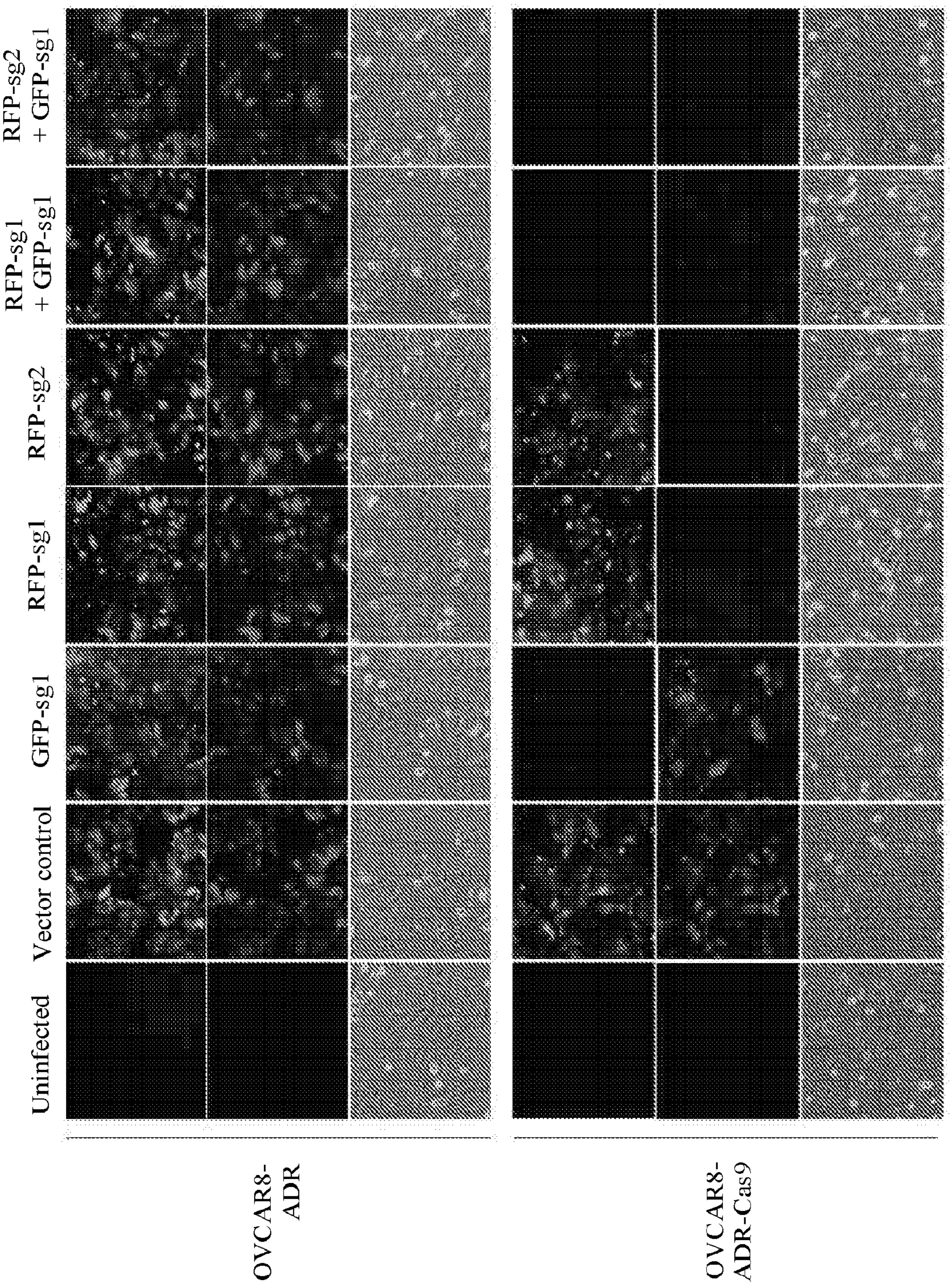
Uninfected
Vector control
GFP-sg1
RFP-sg1
RFP-sg2
RFP-sg1 + GFP-sg1
RFP-sg2 + GFP-sg1
OVCAR8-ADR
OVCAR8-ADR-Cas9

FIG. 6E

| sgRNA | Uncleaved band | Cleaved bands |
|---|---|---|
| DNMT1-sg1 | 600 | 450/150 |
| DNMT3B-sg1 | 544 | 400/150 |
| PRMT2-sg3 | 559 | 370/170 |
| HDAC2-sg1 | 871 | 500/300 |
| ING4-sg1 | 502 | 300/200 |
| KDM1B-sg3 | 499 | 350/150 |
| KDM2A-sg3 | 455 | 300/150 |
| PRMT6-sg1 | 849 | 550/300 |
| KDM4C-sg1 | 419 | 264/155 |
| KDM6B-sg2 | 489 | 273/216 |
| BRD4-sg3 | 541 | 341/200 |

FIG. 7A

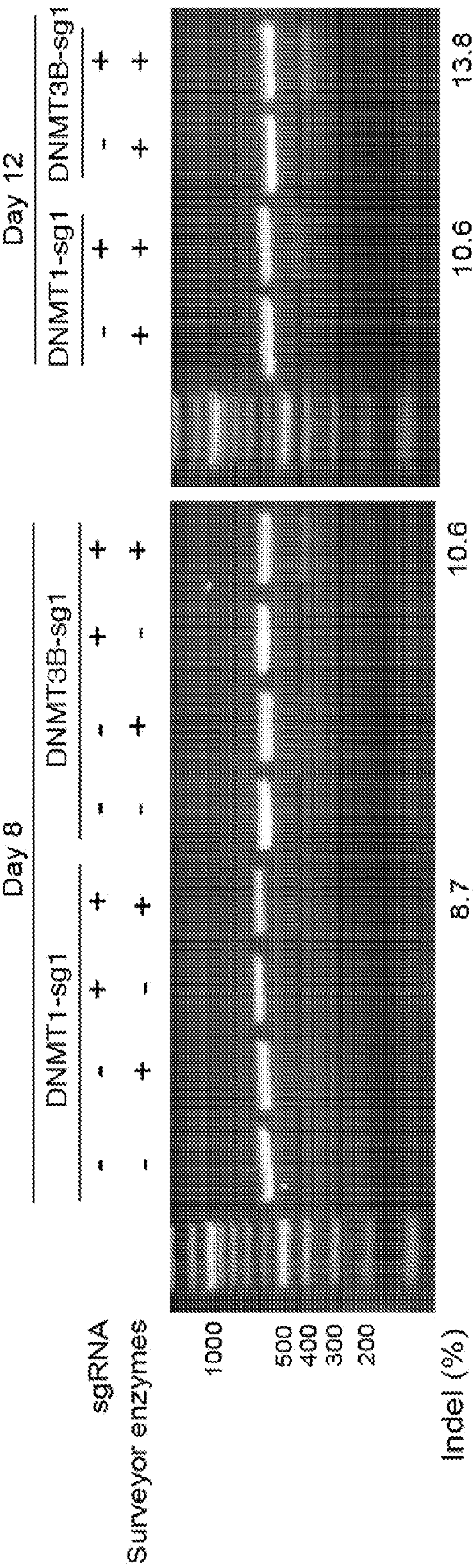
Day 8
Day 12
DNMT1-sg1
DNMT3B-sg1
DNMT1-sg1
DNMT3B-sg1
sgRNA
Surveyor enzymes
1000
500
400
300
200
Indel (%)
8.7
10.6
10.6
13.8

FIG. 7B

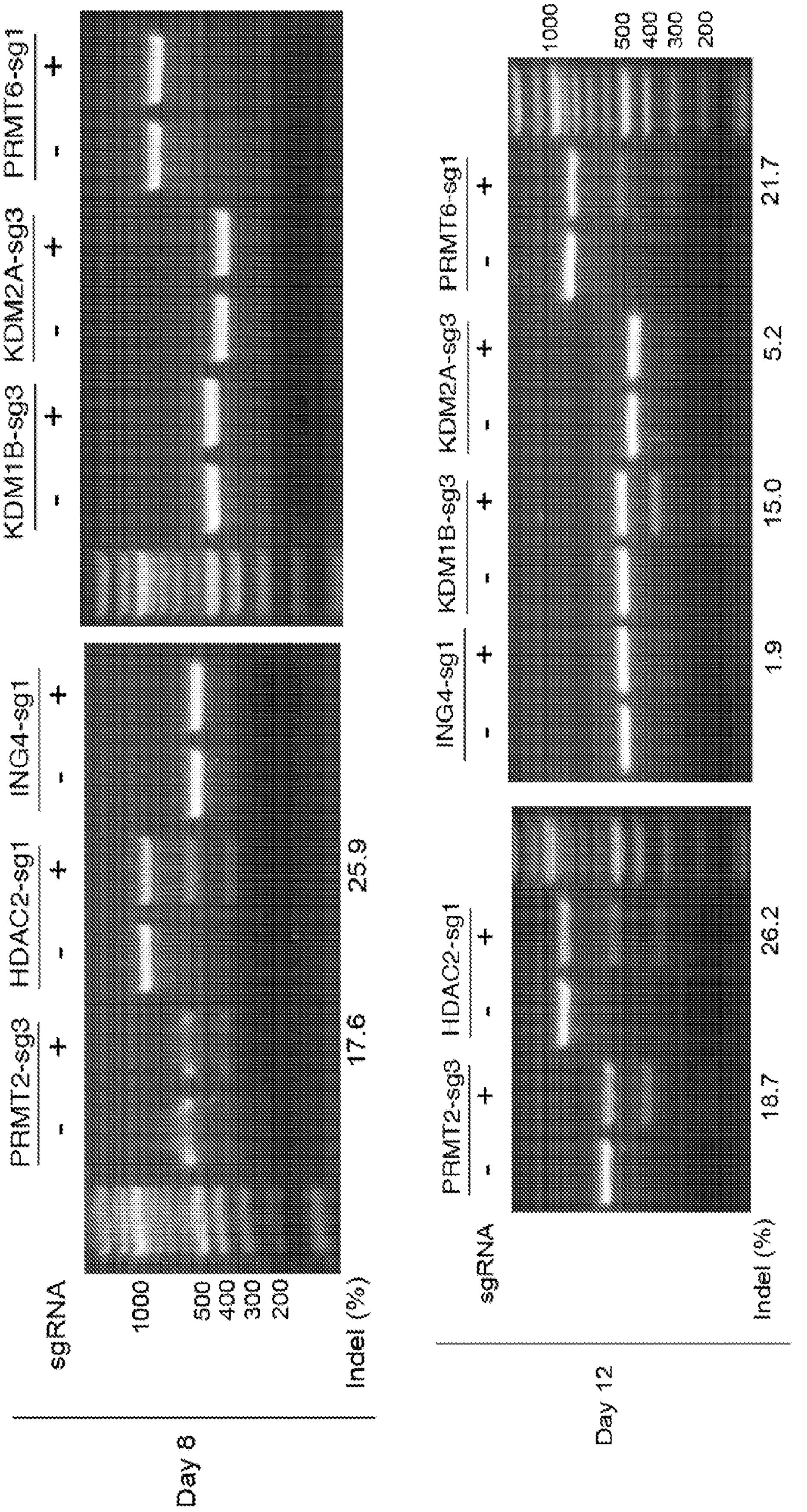
Day 8
sgRNA
PRMT2-sg3
HDAC2-sg1
ING4-sg1
KDM1B-sg3
KDM2A-sg3
PRMT6-sg1
- + - + - + - + - + - +
1000
500
400
300
200
Indel (%)
17.6
25.9
Day 12
sgRNA
PRMT2-sg3
HDAC2-sg1
ING4-sg1
KDM1B-sg3
KDM2A-sg3
PRMT6-sg1
- + - + - + - + - + - +
Indel (%)
18.7
26.2
1.9
15.0
5.2
21.7
1000
500
400
300
200

FIG. 7C

| sgRNA | Uncleaved band | Cleaved bands |
|---|---|---|
| KDM4C-sg1 | 419 | 264/155 |
| KDM6B-sg2 | 489 | 273/216 |
| BRD4-sg3 | 541 | 341/200 |

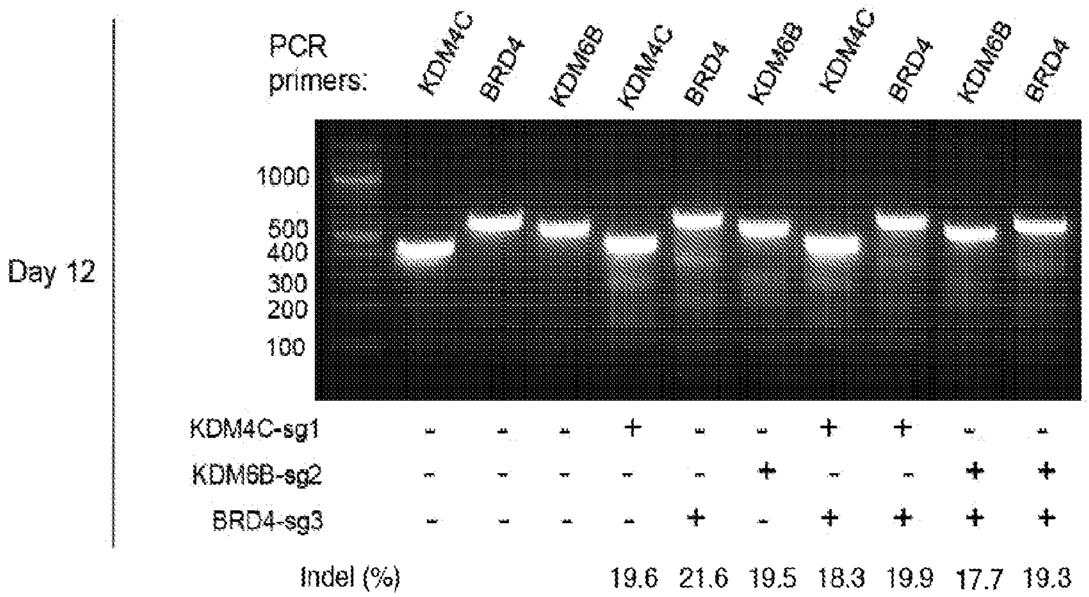


FIG. 8A

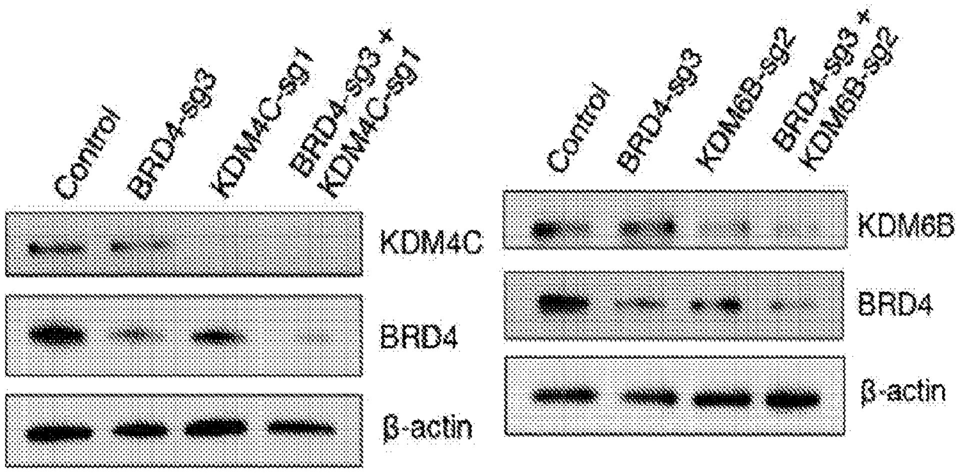


FIG. 8B

## BMI1-sg2 + PHF8-sg2

| | Sequence | | Gene |
|---|---|---|---|
| WT | 5'- TTTCAACAGTTTCCTACCTTATATTCAGTAGTGGTCTGGTCTTGTGAACTTGGACATCACAA -3' | | BMI1 |
| | 5'- TGACTCACAATCTCTCATGTTTTTCTGGCTTAGTGAAAAAACGCCGTGGATCTTCAAAGGGG -3' | | PHF8 |
| Single cell-derived clone #1 | 5'- TTTCAACAGTTTCCTACCTTATATTCAGTAGTGGTCTGGTCTTGTGAACTTGGACATCACAA -3' | WT | BMI1 |
| | 5'- TTTCAACAGTTTCCTACCTTATATTCAGTAGTGGTCTGGTCTTGTGAACTTGGACATCACAA -3' | WT | |
| | 5'- TGACTCACAATCTCTCATGTTTTTCTGGCTTAGTGAAAAAACGCCGTGGATCTTCAAAGGGG -3' | WT | PHF8 |
| | 5'- TGACTCACAATCTCTCATGTTTTTCTGGCTTAGTGAAAAAACGCCGTGGATCTTCAAAGGGG -3' | WT | |
| Single cell-derived clone #2 | 5'- TTTCAACAGTTTCCTACCTTATATTCAGTAGTGGTCTGGTCTTGTGAACTTGGACATCACAA -3' | WT | BMI1 |
| | 5'- TTTCAACAGTTTCCTACCTTATATT------------------GTGAACTTGGACATCACAA -3' | -18 bp | |
| | 5'- TGACTCACAATCTCTCATGTTTTTCTGGCTTAGTGAAAAAACGGCCGTGGATCTTCAAAGGGG -3' | +1 bp | PHF8 |
| | 5'- TGACTCACAATCTCTCATGTTTTTCTGGCTTAGTGAAAAAACGGCCGTGGATCTTCAAAGGGG -3' | +1 bp | |
| Single cell-derived clone #3 | 5'- TTTCAACAGTTTCCTACCTTATATTCAGTAGTGGTCTGGTCTTGTGAACTTGGACATCACAA -3' | WT | BMI1 |
| | 5'- TTTCAACAGTTTCCTACCTTATATTCAGTAGTGGTCTGGTCTTGTGAACTTGGACATCACAA -3' | WT | |
| | 5'- TGACTCACAATCTCTCATGTTTTTCTGGCTTAGTGAAAAAACGCCGTGGATCTTCAAAGGGG -3' | WT | PHF8 |
| | 5'- TGACTCACAATCTCTCATGTTTTTCTGGCTTAGTGAAAAAA---CGTGGATCTTCAAAGGGG -3' | -3 bp | |
| Single cell-derived clone #4 | 5'- TTTCAACAGTTTCCTACCTTATATTC---AGTGGTCTGGTCTTGTGAACTTGGACATCACAA -3' | -3 bp | BMI1 |
| | 5'- TTTCAAC------------------------------------------------ATCACAA- 3' | -48 bp | |
| | 5'- TGACTCACAATCTCTCATGTTTTTCTGGCTTAGTGAAAAAACGCCGTGGATCTTCAAAGGGG -3' | WT | PHF8 |
| | 5'- TGACTCACAATCTCTCATGTTTTTCTGGCTTAGTGAAAAAA---CGTGGATCTTCAAAGGGG -3' | -3 bp | |
| Single cell-derived clone #5 | 5'- TTTCAACAGTTTCCTACCTTATATTC---AGTGGTCTGGTCTTGTGAACTTGGACATCACAA -3' | -3 bp | BMI1 |
| | 5'- TTTCAACAGTTTCCTACCTTATATTC---AGTGGTCTGGTCTTGTGAACTTGGACATCACAA -3' | -3 bp | |
| | 5'- TGACTCACAATCTCTCATGTTTTTCTGGCTTAGTGAAAAAA---CGTGGATCTTCAAAGGGG -3' | -3 bp | PHF8 |
| | 5'- TGACTCACAATCTCTCATGTTTTTCTGGCTTAGTGAAAAAA---CGTGGATCTTCAAAGGGG -3' | -3 bp | |

FIG. 9A

## BMI1-sg2 + PHF8-sg2

| | | | |
|---|---|---|---|
| Single cell-derived clone #6 | 5'- TTTCAACAGTTTCCTACCTTATATTCAGTAGTGGTCTGGTCTTGTGAACTTGGACATCACAA -3' | WT | BMI1 |
| | 5'- TTTCAACAGTTTCCTACCTTATATTCAGT-------------TGTGAACTTGGACATCACAA -3' | -13 bp | |
| | 5'- TGACTCACAATCTCTCATGTTTTTCTGGCTTAGTGAAAAAACGGCCGTGGATCTTCAAAGGGG -3' | +1 bp | PHF8 |
| | 5'- TGACTCACAATCTCTCATGTTTTTCTGGCTTAGTGAAAAAACGGCCGTGGATCTTCAAAGGGG -3' | +1 bp | |
| Single cell-derived clone #7 | 5'- TTTCAACAGTTTCCTACCTTATATTC---AGTGGTCTGGTCTTGTGAACTTGGACATCACAA -3' | -3 bp | BMI1 |
| | 5'- TTTCAACAGTTTCCTACCTTATATTC---AGTGGTCTGGTCTTGTGAACTTGGACATCACAA -3' | -3 bp | |
| | 5'- TGACTCACAATCTCTCATGTTTTTCTGGCTTAGTGAAAAAACAAAACCGTGGATCTTCAAAGGGG -3' | 1 mut.; +3 bp | PHF8 |
| | 5'- TGACTCACAATCTCTCATGTTTTTCTGGCTTAGTGAAAAAACAAAACCGTGGATCTTCAAAGGGG -3' | 1 mut.; +3 bp | |
| Single cell-derived clone #8 | 5'- TTTCAACAGTTTCCTACCTTAT------TAGTGGTCTGGTCTTGTGAACTTGGACATCACAA -3' | -6 bp | BMI1 |
| | 5'- TTTCAACAGTTTCCTACCTTATAT----------TCTGGTCTTGTGAACTTGGACATCACAA -3' | -10 bp | |
| | 5'- TGACTCACAATCTCTCATGTTTTTCTGGCTTAGTGAAAAAACGCCGTGGATCTTCAAAGGGG -3' | WT | PHF8 |
| | 5'- TGACTCACAATCTCTCATGTTTTTCTGGCTTAGTGAAAAAACGCCGTGGATCTTCAAAGGGG -3' | WT | |
| Single cell-derived clone #9 | 5'- TTTCAACAGTTTCCTACCTTATATTC--TAGTGGTCTGGTCTTGTGAACTTGGACATCACAA -3' | -2 bp | BMI1 |
| | 5'- TTTCAACAGTTTCCTACCTTT-------TAGTGGTCTGGTCTTGTGAACTTGGACATCACAA -3' | 1 mut.; -7 bp | |
| | 5'- TGACTCACAATCTCTCATGTTTTTCTGGCTTAGTGAAAAAACGCCGTGGATCTTCAAAGGGG -3' | WT | PHF8 |
| | 5'- TGACTCACAATCTCTCATGTTTTTCTGGCTTAGTGAAAAAACGCCGTGGATCTTCAAAGGGG -3' | WT | |
| Single cell-derived clone #10 | 5'- TTTCAACAGTTTCCTACCTTATATTCAGTAGTGGTCTGGTCTTGTGAACTTGGACATCACAA -3' | WT | BMI1 |
| | 5'- TTTCAACAGTTTCCTACCTTATATTCAGTAGTGGTCTGGTCTTGTGAACTTGGACATCACAA -3' | WT | |
| | 5'- TGACTCACAATCTCTCATGTTTTTCTGGCTTAGTGAAAAAACGCCGTGGATCTTCAAAGGGG -3' | WT | PHF8 |
| | 5'- TGACTCACAATCTCTCATGTTTTTCTGGCTTAGTGAAAAAACGCCGTGGATCTTCAAAGGGG -3' | WT | |

FIG. 9A (CONT.)

**BRD4-sg2 + KDM4C-sg1**

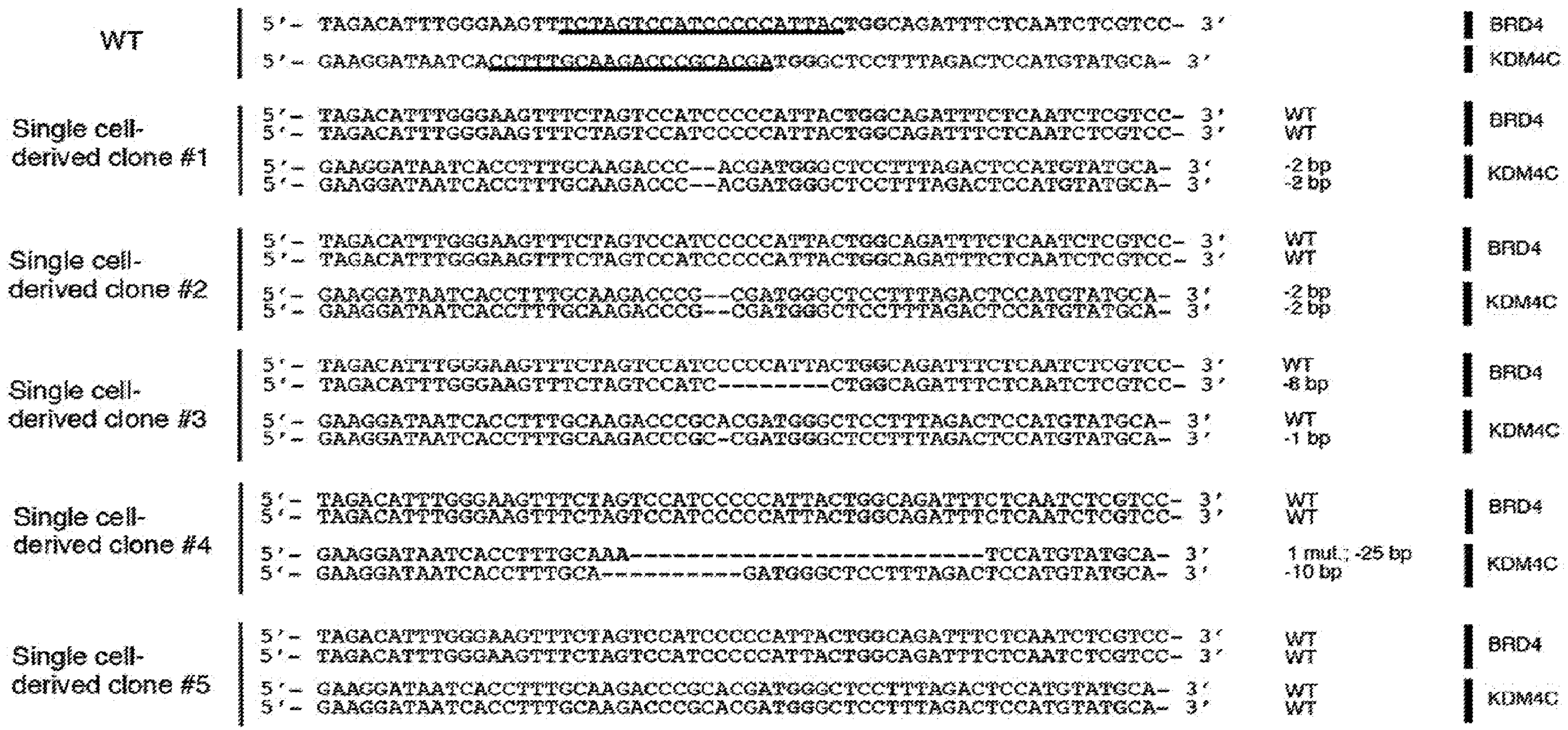


**FIG. 9B**

**BRD4-sg2 + KDM4C-sg1**

| Clone | Sequence | Mutation | Gene |
|---|---|---|---|
| Single cell-derived clone #6 | 5'- TAGACATTTGGGAAGTTTCTAGTCCATCCCCCATTACTGGCAGATTTCTCAATCTCGTCC- 3' | WT | BRD4 |
| | 5'- TAGACATTTGGGAAGTTTCTAGTCCATCCCCCATTACTGGCAGATTTCTCAATCTCGTCC- 3' | WT | |
| | 5'- GAAGGATAATCACCTTTGCAAGACCCGCAAAGATGGGCTCCTTTAGACTCCATGTATGCA- 3' | 1 mut.; +1 bp | KDM4C |
| | 5'- GAAGGATAATCACCTTTGCAAGACCCGCAACGATGGGCTCCTTTAGACTCCATGTATGCA- 3' | +1 bp | |
| Single cell-derived clone #7 | 5'- TAGACATTTGGGAAGTTTCTAGTCCATCCCCCATTACTGGCAGATTTCTCAATCTCGTCC- 3' | WT | BRD4 |
| | 5'- TAGACATTTGGGAAGTTTCTAGTCCATCCCCCATTACTGGCAGATTTCTCAATCTCGTCC- 3' | WT | |
| | 5'- GAAGGATAATCACCTTTGCAAGACCCGCAACGATGGGCTCCTTTAGACTCCATGTATGCA- 3' | +1 bp | KDM4C |
| | 5'- GAAGGATAATCACCTTTGCAAGAC-----CGATGGGCTCCTTTAGACTCCATGTATGCA- 3' | -5 bp | |
| Single cell-derived clone #8 | 5'- TAGACATTTGGGAAGTTTCTAGTCCATCCCCCATTACTGGCAGATTTCTCAATCTCGTCC- 3' | WT | BRD4 |
| | 5'- TAGACATTTGGGAAGTTTCTAGTCCATCCCCCATTACTGGCAGATTTCTCAATCTCGTCC- 3' | WT | |
| | 5'- GAAGGATAATCACCTTTGCAAGACCCGCACGATGGGCTCCTTTAGACTCCATGTATGCA- 3' | WT | KDM4C |
| | 5'- GAAGGATAATCACCTTTGCAAGACCCGCACGATGGGCTCCTTTAGACTCCATGTATGCA- 3' | WT | |
| Single cell-derived clone #9 | 5'- TAGACATTTGGGAAGTTTCTAGTCCATCCCCCATTACTGGCAGATTTCTCAATCTCGTCC- 3' | WT | BRD4 |
| | 5'- TAGACATTTGGGAAGTTTACATTGGGAAGAAGAAATACTGGCAGATTTCTCAATCTCGTCC- 3' | 16 bp mut.; +1bp | |
| | 5'- GAAGGATAATCACCTTTGCAAGACCCGCAACGATGGGCTCCTTTAGACTCCATGTATGCA- 3' | +1 bp | KDM4C |
| | 5'- GAAGGATAATCACCTTTGCAAGACCCGCATACGATGGGCTCCTTTAGACTCCATGTATGCA- 3' | +2 bp | |
| Single cell-derived clone #10 | 5'- TAGACATTTGGGAAGTTTCTAGTCCATCCCCCATTACTGGCAGATTTCTCAATCTCGTCC- 3' | WT | BRD4 |
| | 5'- TAGACATTTGGGAAGTTTCTAGTCCATCCCCCATTACTGGCAGATTTCTCAATCTCGTCC- 3' | WT | |
| | 5'- GAAGGATAATCACCTTTGCAAGACCCGCACCCGATGGGCTCCTTTAGACTCCATGTATGCA- 3' | +2 bp | KDM4C |
| | 5'- GAAGGATAATCACCTTTGCAAGACCCGCAACGATGGGCTCCTTTAGACTCCATGTATGCA- 3' | +1 bp | |
| Single cell-derived clone #11 | 5'- TAGACATTTGGGAAGTTTCTAGTCCATCCCCCATTACTGGCAGATTTCTCAATCTCGTCC- 3' | WT | BRD4 |
| | 5'- TAGACATTTGGGAAGTTTCTAGTCCATCCCCCATTACTGGCAGATTTCTCAATCTCGTCC- 3' | WT | |
| | 5'- GAAGGATAATCACCTTTGC---------------------TTTAGACTCCATGTATGCA- 3' | -21 bp | KDM4C |
| | 5'- GAAGGATAATCACCTTTGCAAGACCCGCAACGATGGGCTCCTTTAGACTCCATGTATGCA- 3' | +1 bp | |

FIG. 9B (CONT.)

BMI1-sg2 + PHF8-sg2

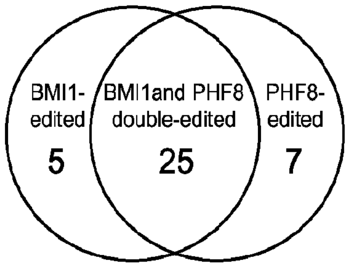


FIG. 9C

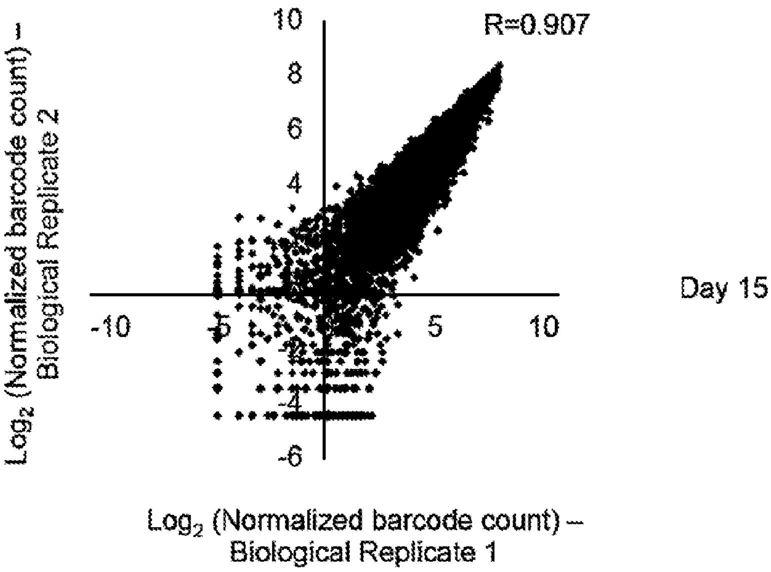


FIG. 10A

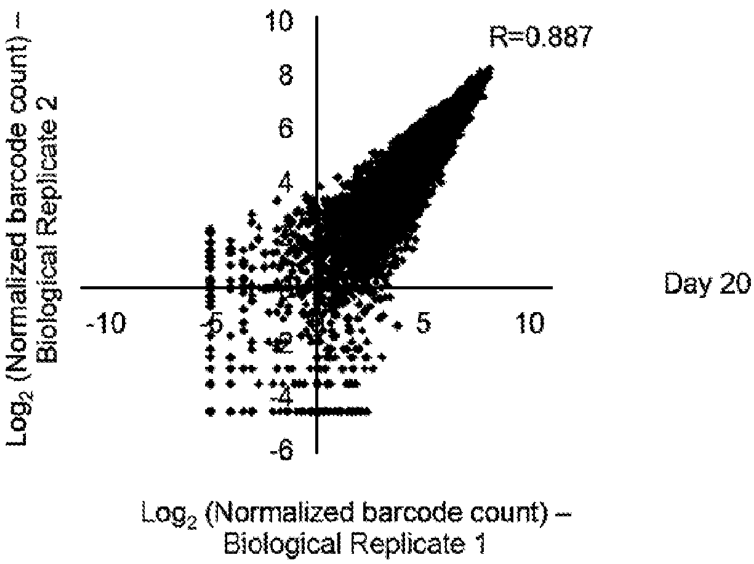


FIG. 10B

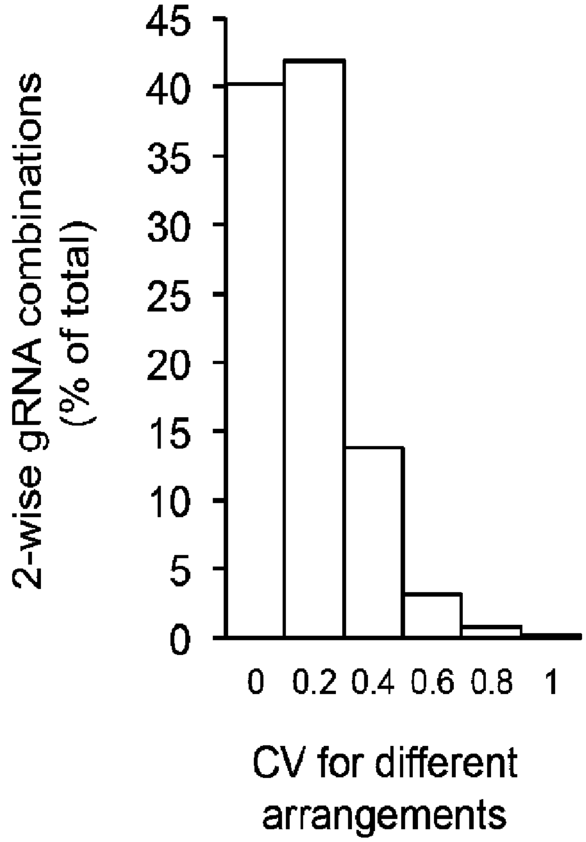
2-wise gRNA combinations
(% of total)
45
40
35
30
25
20
15
10
5
0
0 0.2 0.4 0.6 0.8 1
CV for different arrangements

FIG. 11

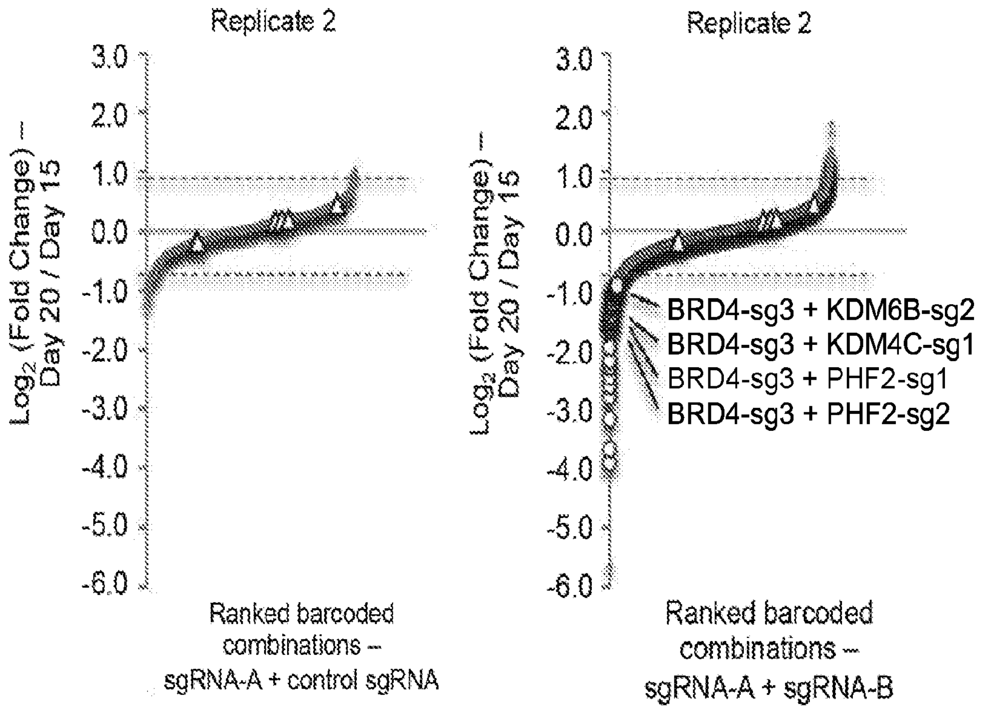


FIG. 12A

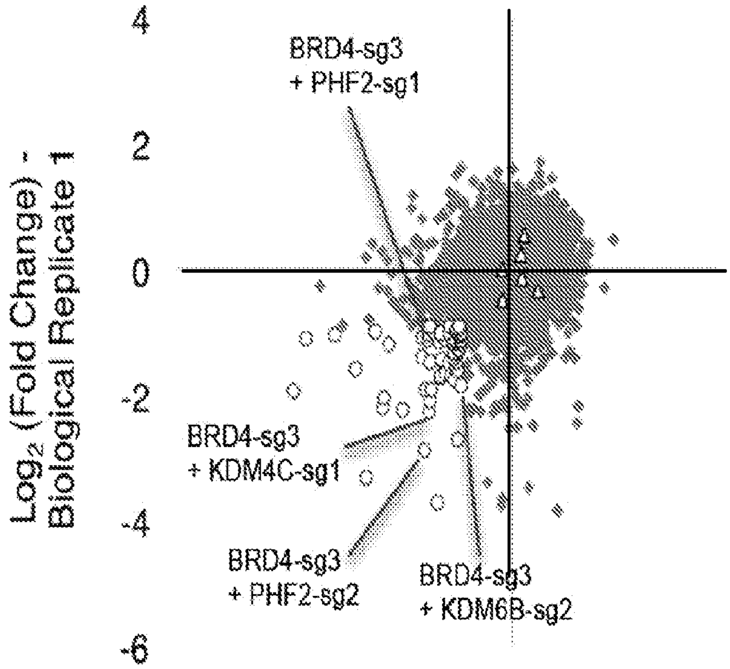


FIG. 12B

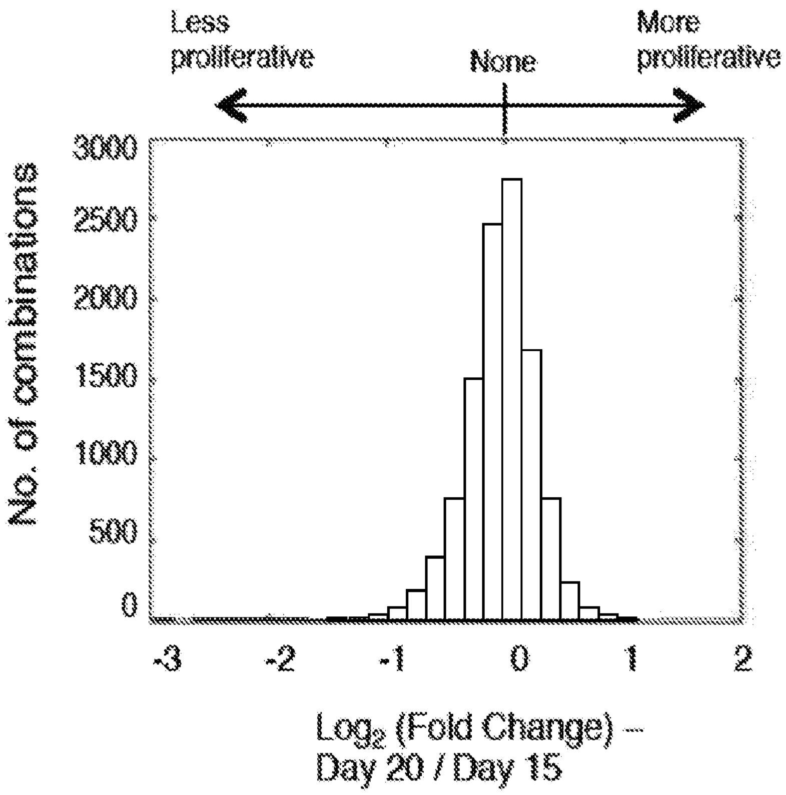
Less proliferative
None
More proliferative
No. of combinations
3000
2500
2000
1500
1000
500
0
-3
-2
-1
0
1
2
Log2 (Fold Change) –
Day 20 / Day 15

FIG. 12C

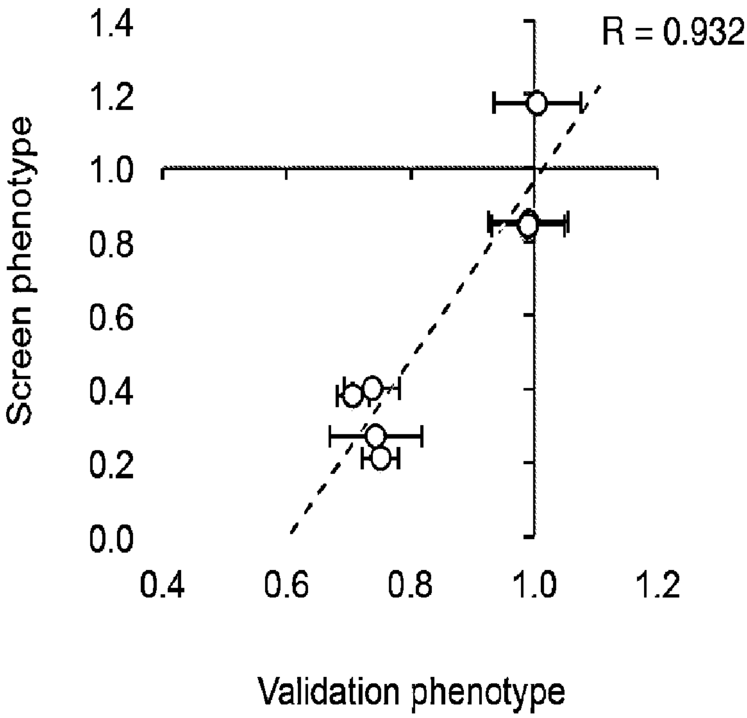
R = 0.932
Screen phenotype
1.4
1.2
1.0
0.8
0.6
0.4
0.2
0.0
0.4
0.6
0.8
1.0
1.2
Validation phenotype

FIG. 13

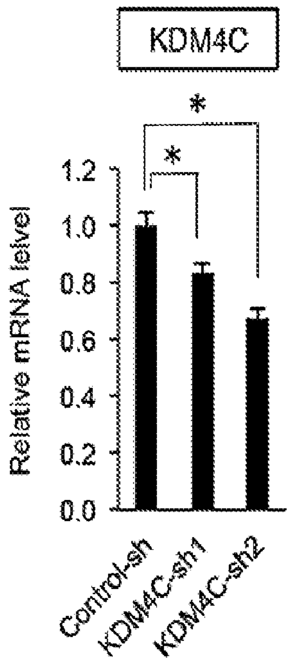


FIG. 14A

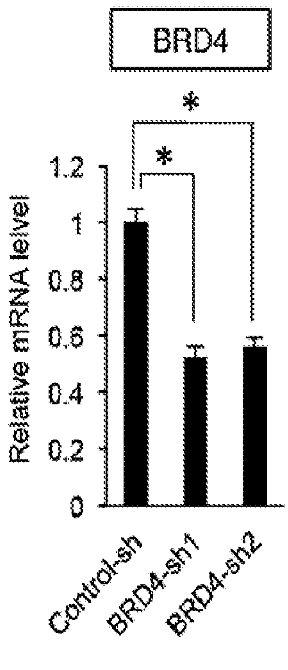


FIG. 14B

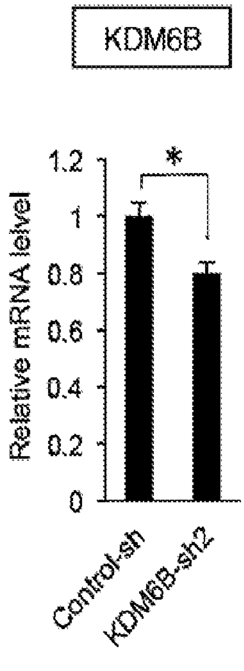


FIG. 14C

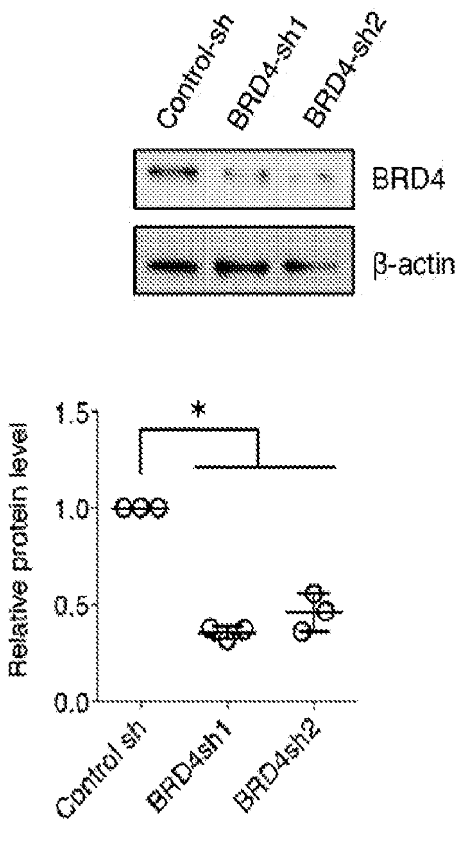


FIG. 14D

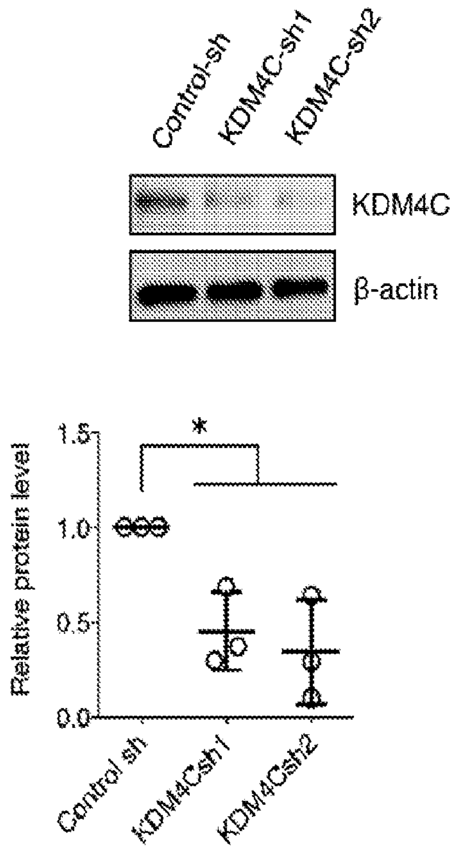


FIG. 14E

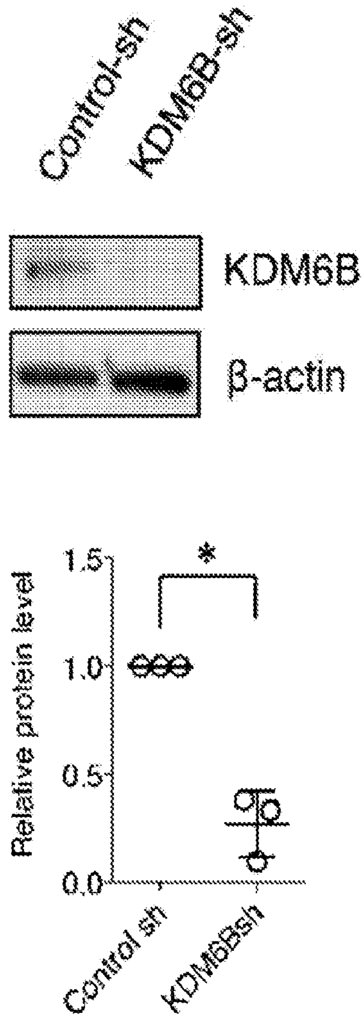
Control-sh
KDM6B-sh
KDM6B
β-actin
Relative protein level
1.5
1.0
0.5
0.0
*
Control sh
KDM6Bsh

FIG. 14F

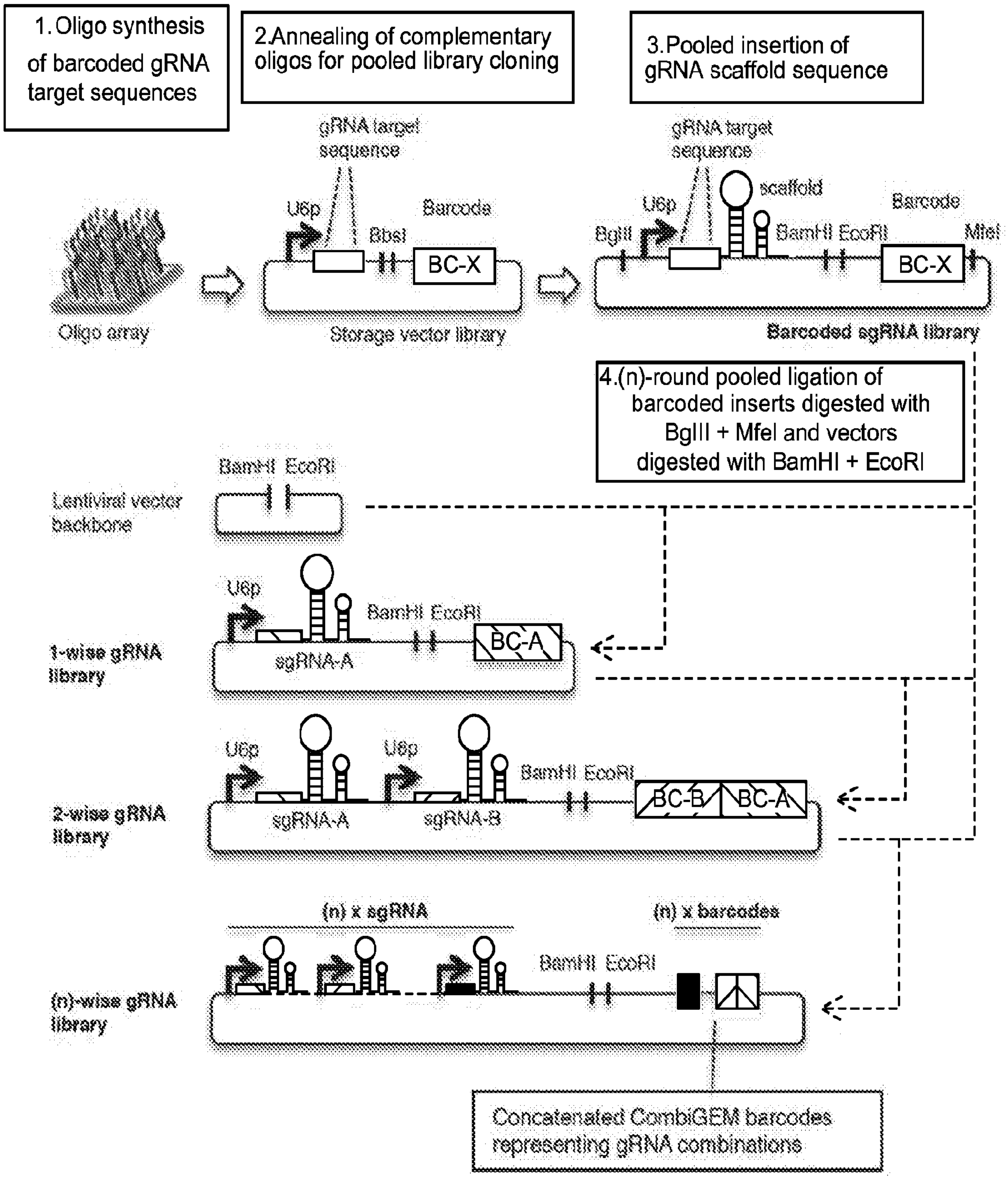
1. Oligo synthesis of barcoded gRNA target sequences
2.Annealing of complementary oligos for pooled library cloning
3.Pooled insertion of gRNA scaffold sequence
gRNA target sequence
U6p
BbsI
Barcode
BC-X
Oligo array
Storage vector library
scaffold
BglII
BamHI EcoRI
MfeI
Barcoded sgRNA library
4.(n)-round pooled ligation of barcoded inserts digested with BglII + MfeI and vectors digested with BamHI + EcoRI
Lentiviral vector backbone
1-wise gRNA library
sgRNA-A
BC-A
2-wise gRNA library
sgRNA-B
BC-B
(n) x sgRNA
(n) x barcodes
(n)-wise gRNA library
Concatenated CombiGEM barcodes representing gRNA combinations

FIG. 15

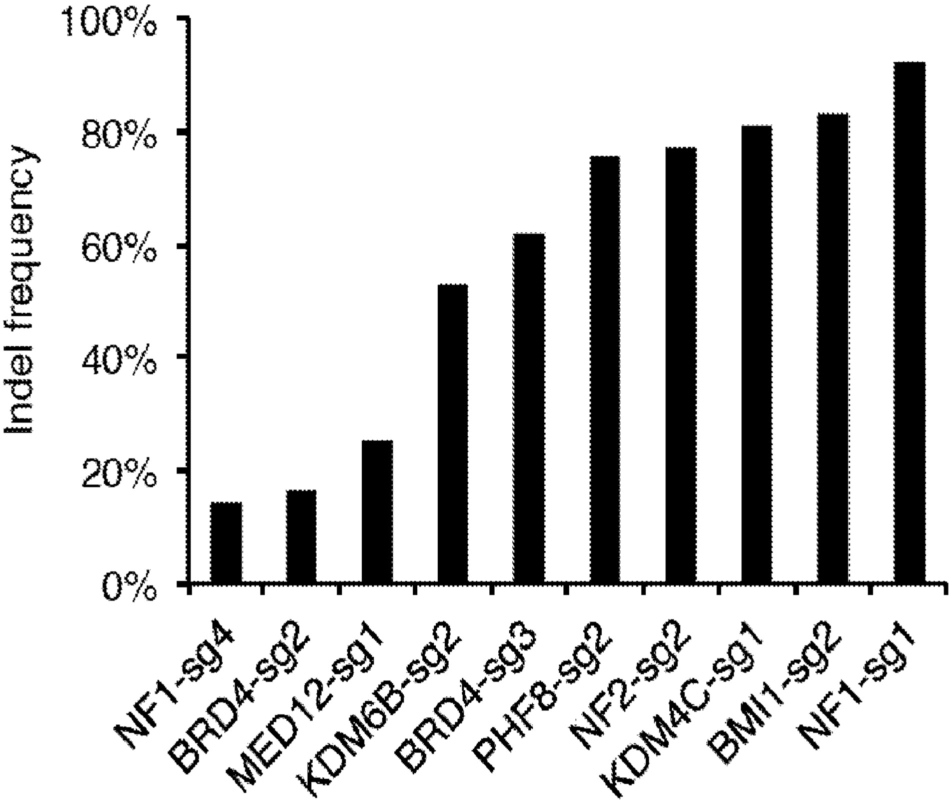


FIG. 16A

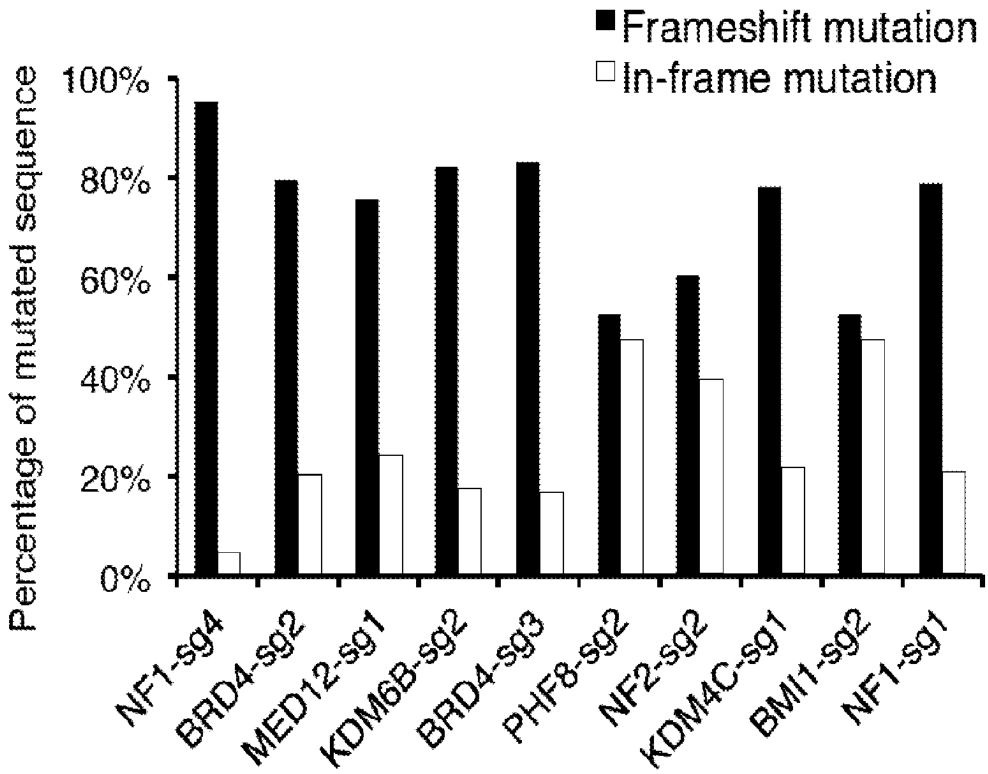


FIG. 16B

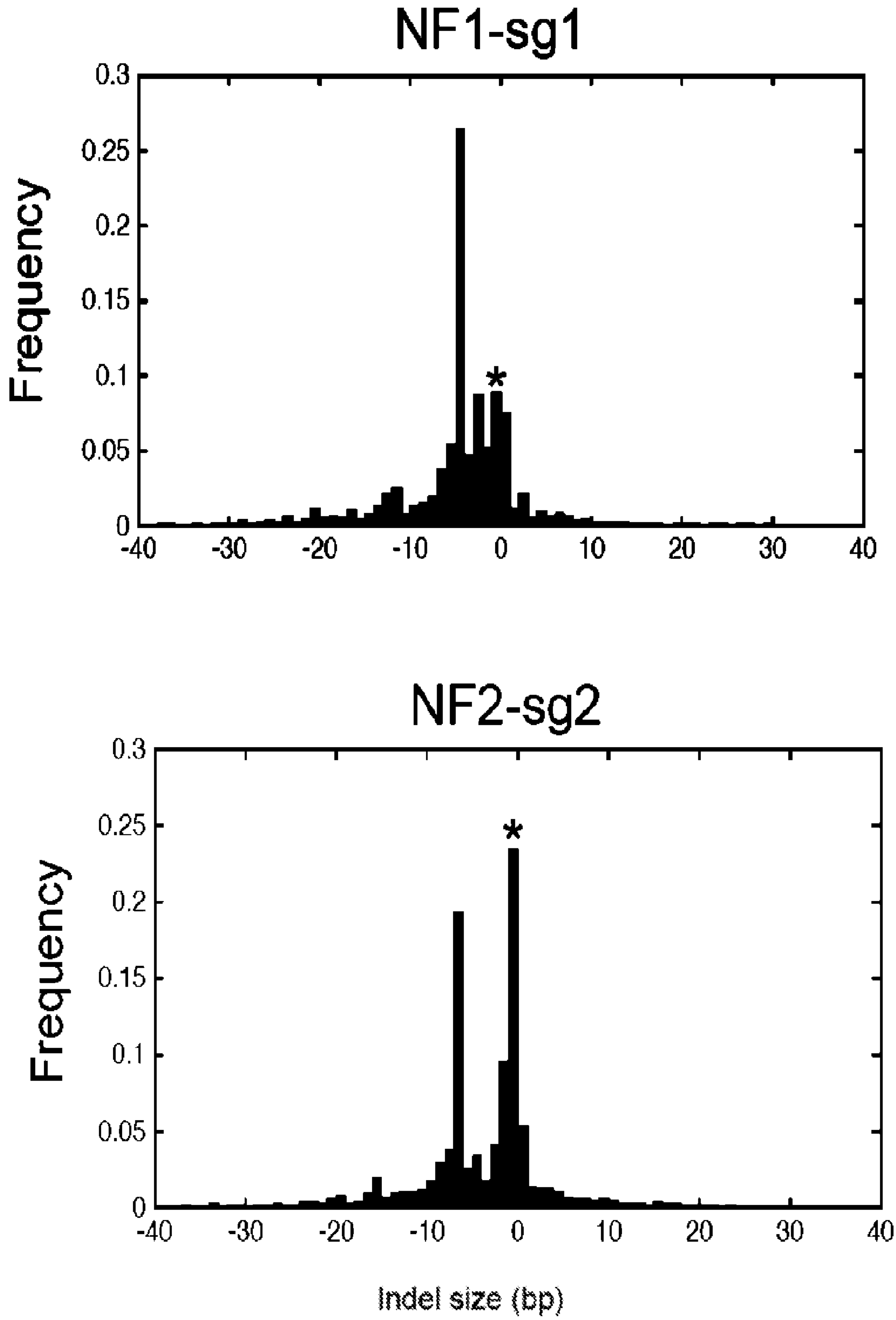
NF1-sg1
Frequency
0.3
0.25
0.2
0.15
0.1
0.05
0
-40
-30
-20
-10
0
10
20
30
40
*
NF2-sg2
Frequency
0.3
0.25
0.2
0.15
0.1
0.05
0
-40
-30
-20
-10
0
10
20
30
40
*
Indel size (bp)

FIG. 16C

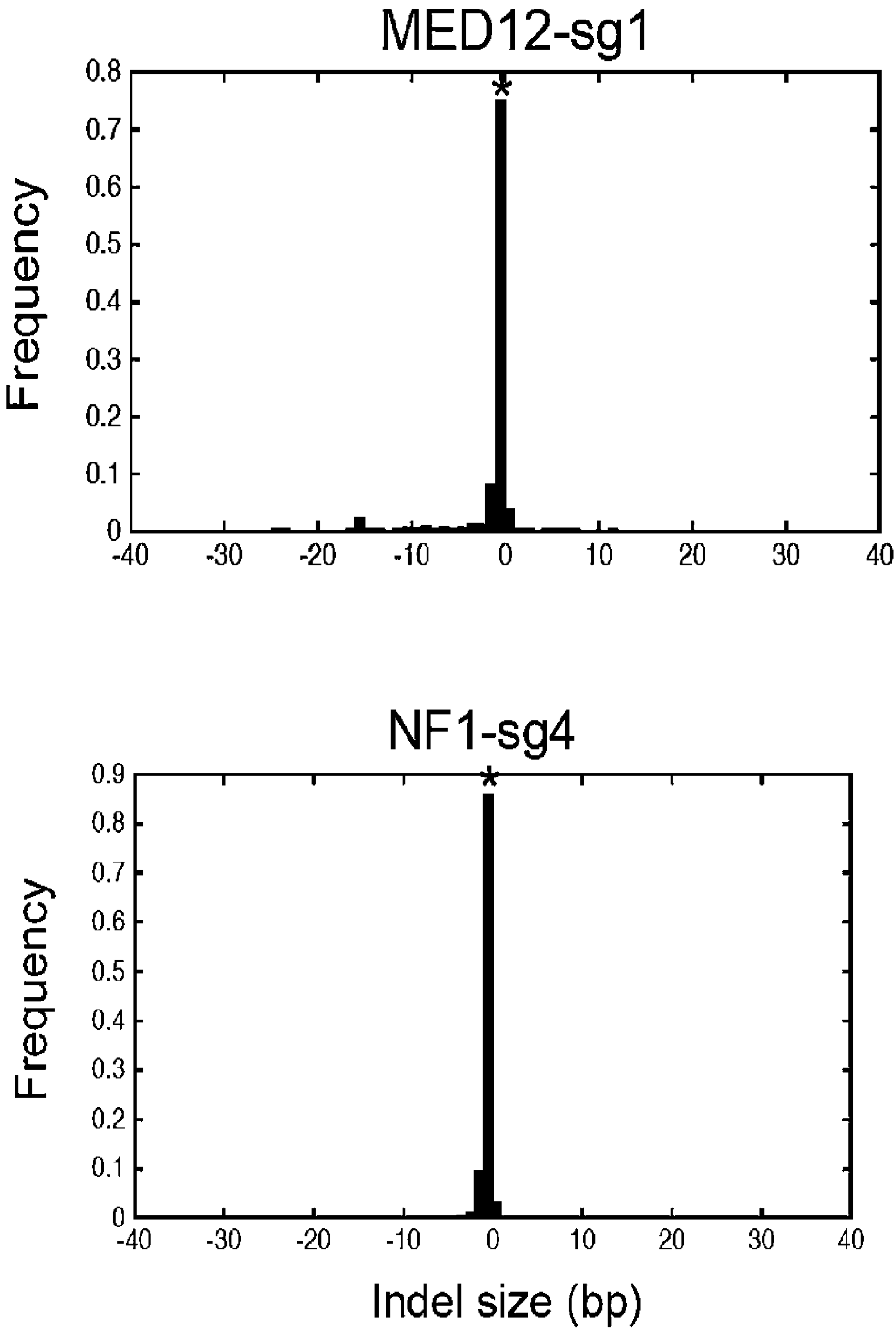
MED12-sg1
Frequency
0.8
0.7
0.6
0.5
0.4
0.3
0.2
0.1
0
-40
-30
-20
-10
0
10
20
30
40
*
NF1-sg4
Frequency
0.9
0.8
0.7
0.6
0.5
0.4
0.3
0.2
0.1
0
-40
-30
-20
-10
0
10
20
30
40
*
Indel size (bp)

FIG. 16C (CONT.)

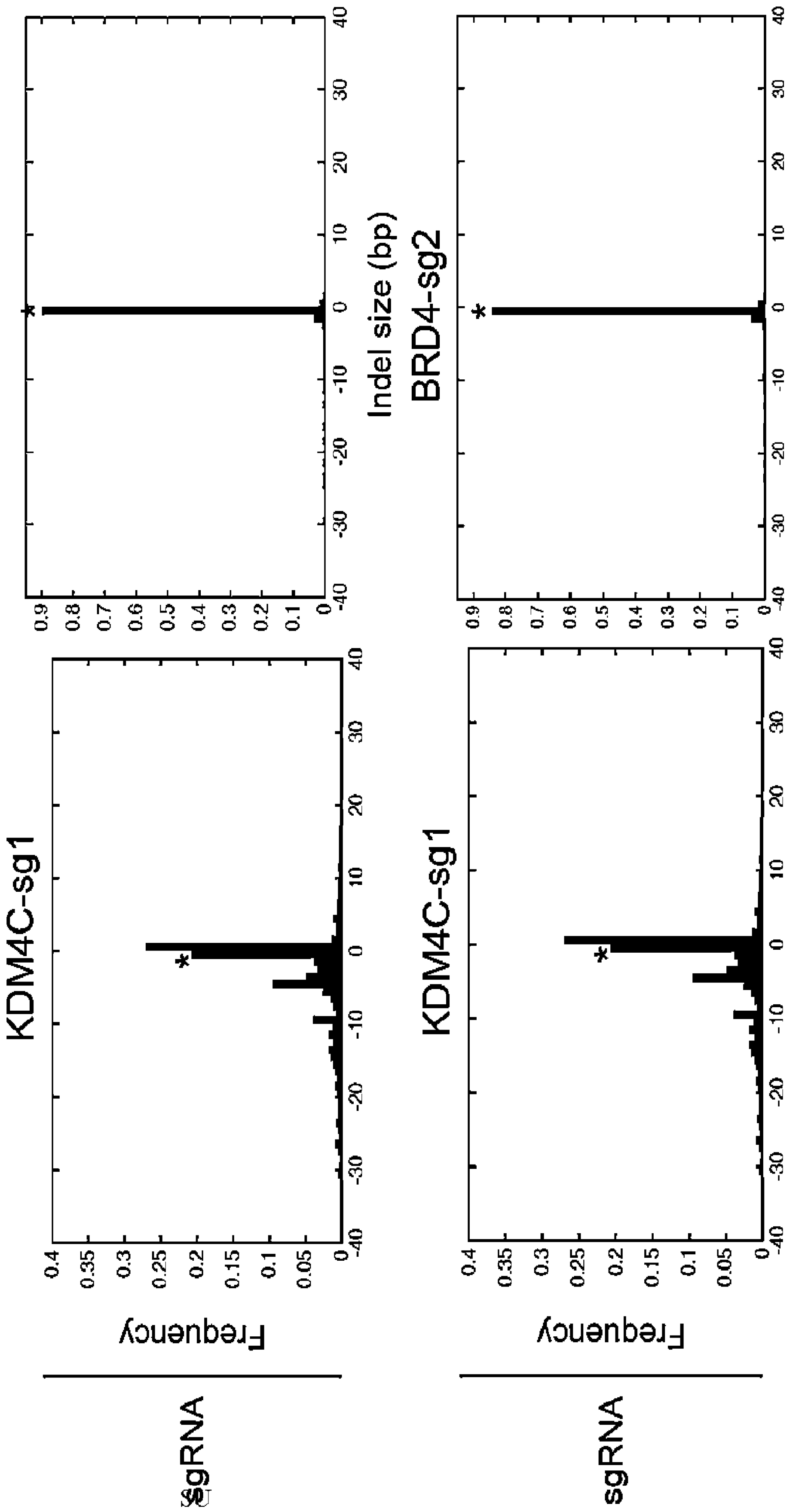
sgRNA
Frequency
KDM4C-sg1
Indel size (bp)
sgRNA
Frequency
KDM4C-sg1
BRD4-sg2
0.4
0.35
0.3
0.25
0.2
0.15
0.1
0.05
0
0.9
0.8
0.7
0.6
0.5
0.4
0.3
0.2
0.1
-40
-30
-20
-10
0
10
20
30
40
*

FIG. 16D

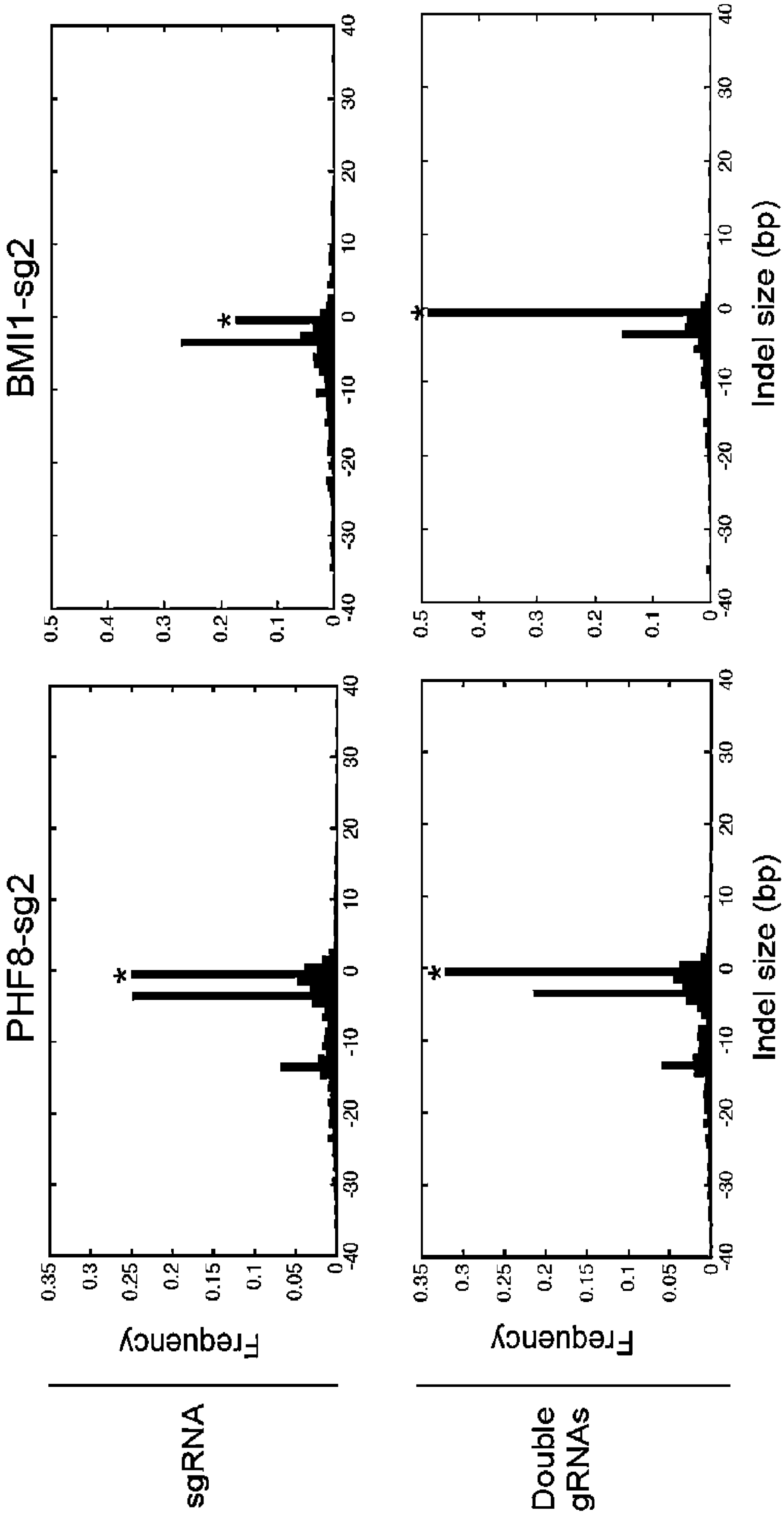

PHF8-sg2
BMI1-sg2
sgRNA
Double gRNAs
Frequency
Indel size (bp)
*
0.35
0.3
0.25
0.2
0.15
0.1
0.05
0
0.5
0.4
-40
-30
-20
-10
10
20
30
40


FIG. 16E

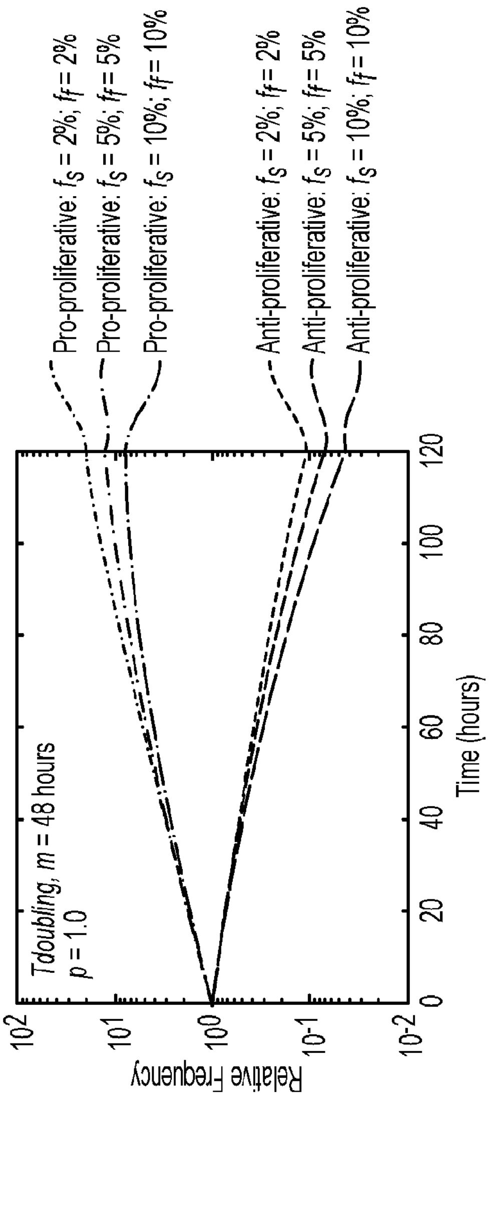

Tdoubling, m = 48 hours
p = 1.0
Pro-proliferative: $f_S$ = 2%; $f_f$ = 2%
Pro-proliferative: $f_S$ = 5%; $f_f$ = 5%
Pro-proliferative: $f_S$ = 10%; $f_f$ = 10%
Anti-proliferative: $f_S$ = 2%; $f_f$ = 2%
Anti-proliferative: $f_S$ = 5%; $f_f$ = 5%
Anti-proliferative: $f_S$ = 10%; $f_f$ = 10%
Relative Frequency
$10^2$
$10^1$
$10^0$
$10^{-1}$
$10^{-2}$
0
20
40
60
80
100
120
Time (hours)


FIG. 17A

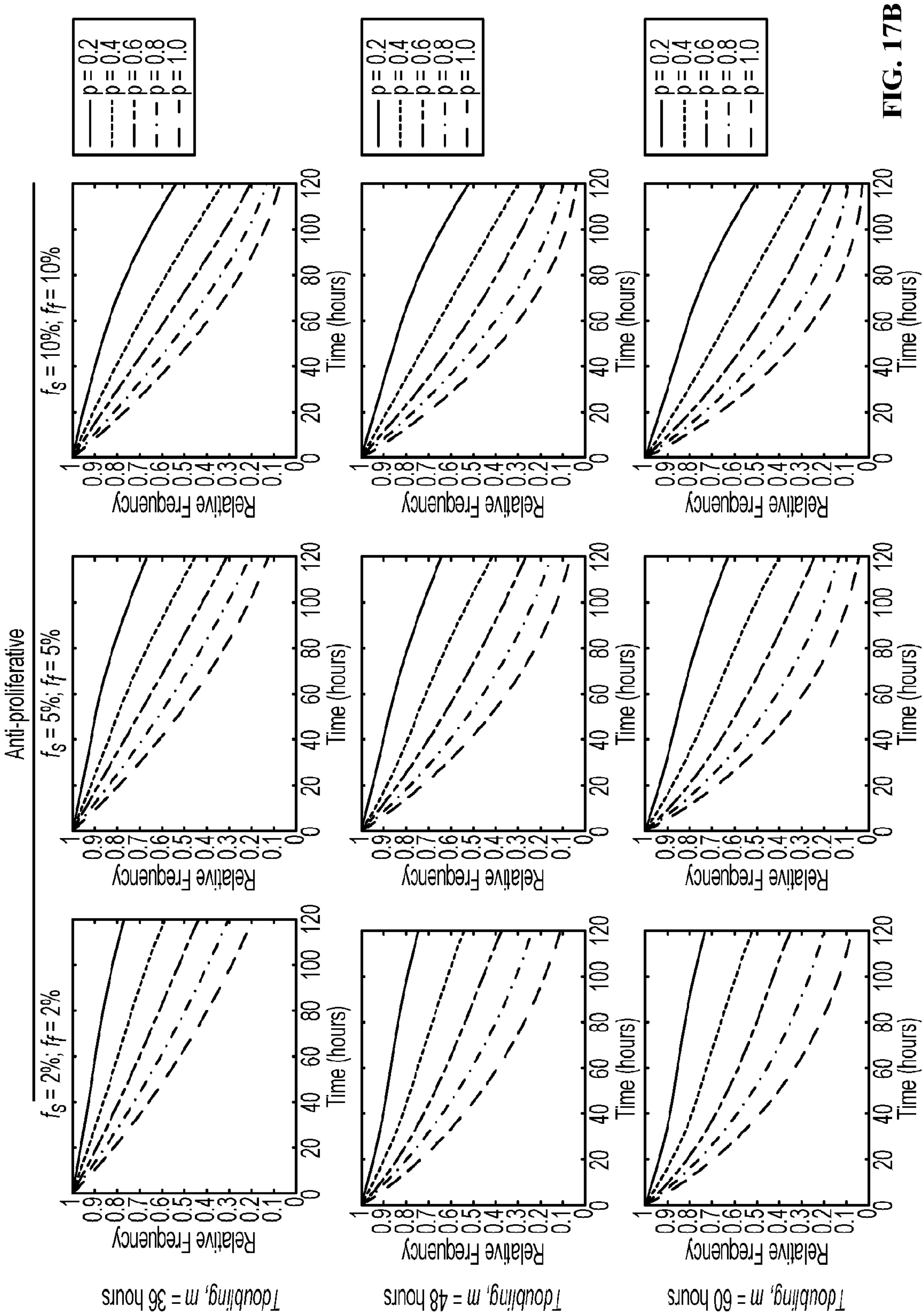
Anti-proliferative
$f_S$ = 2%; $f_f$ = 2%
$f_S$ = 5%; $f_f$ = 5%
$f_S$ = 10%; $f_f$ = 10%
Tdoubling, m = 36 hours
Tdoubling, m = 48 hours
Tdoubling, m = 60 hours
Relative Frequency
Time (hours)
p = 0.2
p = 0.4
p = 0.6
p = 0.8
p = 1.0

FIG. 17B

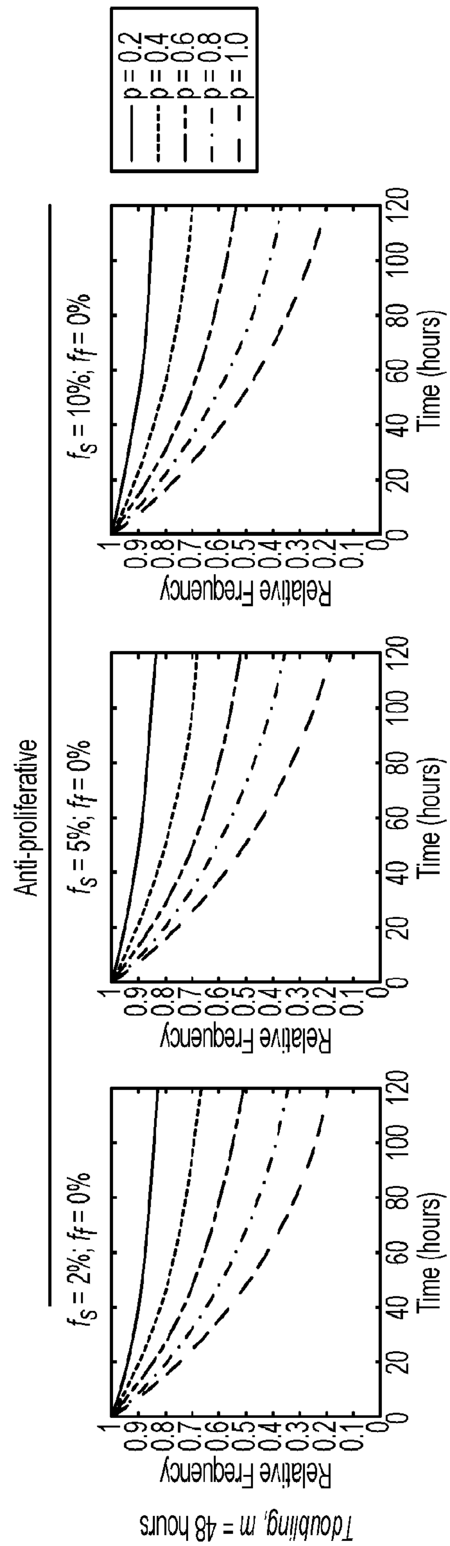

Anti-proliferative
fS = 2%; ff = 0%
fS = 5%; ff = 0%
fS = 10%; ff = 0%
Relative Frequency
Time (hours)
Tdoubling, m = 48 hours
p = 0.2
p = 0.4
p = 0.6
p = 0.8
p = 1.0


FIG. 17C

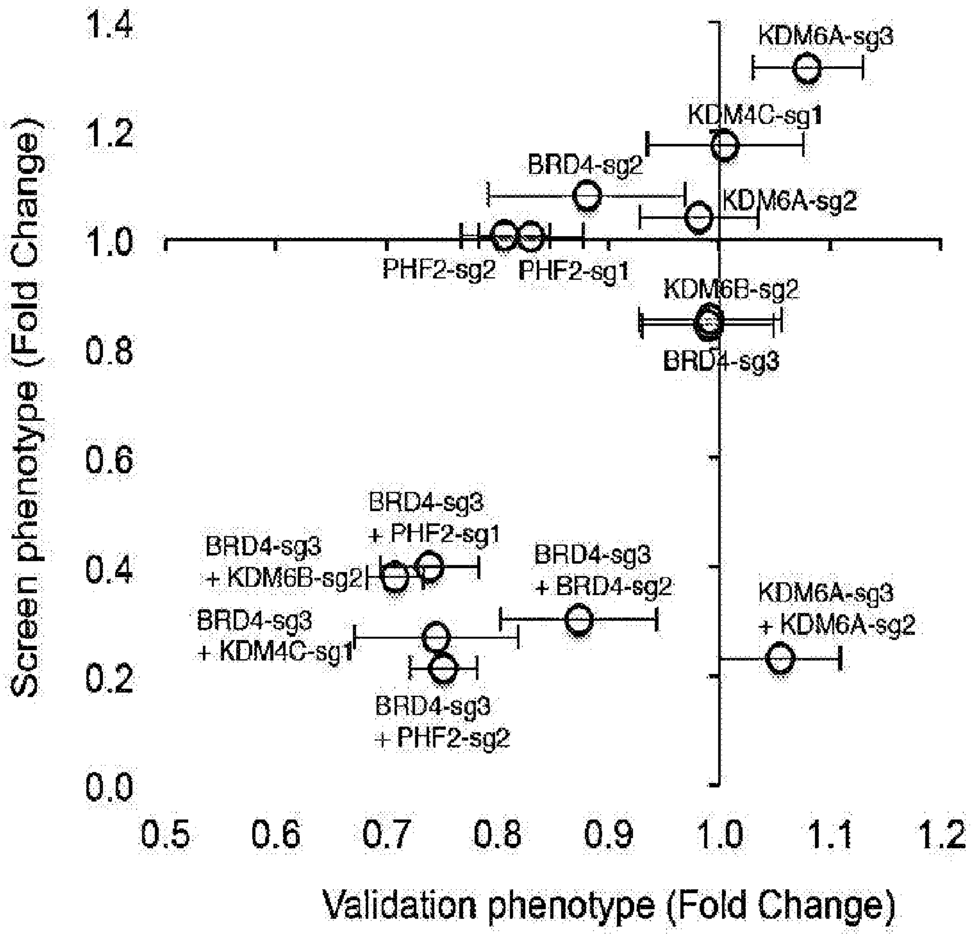
1.4
1.2
1.0
0.8
0.6
0.4
0.2
0.0
Screen phenotype (Fold Change)
0.5
0.6
0.7
0.8
0.9
1.0
1.1
1.2
Validation phenotype (Fold Change)
KDM6A-sg3
KDM4C-sg1
BRD4-sg2
KDM6A-sg2
PHF2-sg2
PHF2-sg1
KDM6B-sg2
BRD4-sg3
BRD4-sg3
+ PHF2-sg1
BRD4-sg3
+ KDM6B-sg2
BRD4-sg3
+ BRD4-sg2
KDM6A-sg3
+ KDM6A-sg2
BRD4-sg3
+ KDM4C-sg1
BRD4-sg3
+ PHF2-sg2

FIG. 18

| | Guide Sequence | PAM | | |
|---|---|---|---|---|
| KDM4C-sg1 | **CCTTTGCAAGACCCGCACGA** | TGG | 80.85% | |
| | GCTTTGCCCGAACCGCACGA | GAG | 0.29% | |
| | CCTAGGCCAGACCTGCACGA | TGG | 0.23% | |
| | GCTCTGGAAGACCCGCACCA | GGG | 0.21% | exonic |
| | CCTTATCAAGACCCACACCA | GAG | 0.15% | |
| KDM6B-sg2 | **ATCCCCCTCCTCGTAGCGCA** | **TGG** | 52.74% | |
| | TGCGCCCTCCTCCTAGCGCA | TGG | 3.74% | |
| | CTGCCCCTCCTGGTAGCGCC | TGG | 1.01% | |
| | CTGCTCCTCCTCGTAGCGCT | GGG | 0.43% | |
| | AACCAGCTCCTCGTAGCTCA | GGG | 0.32% | |
| | AGCCCGCTCCTCGTGGGGCA | CGG | 0.19% | exonic |
| | ACCGCCCTCCTCCTAGCTCA | GGG | 0.17% | |
| | CTCCTCCTCCGCGTAGCGCT | TGG | 0.14% | |
| | CTCCTCCTCCTCCTACCGCA | AGG | n.d. | |
| | ATTCCTCTCCTGGTACCGCA | AGG | n.d. | |
| | CTCACCCTCCTCCTAGCACA | TAG | n.d. | |
| BRD4-sg3 | **GGGAACAATAAAGAAGCGCT** | **TGG** | 61.97% | |
| | TGGAAAAACAAAGAAGAGCT | TGG | 0.38% | |
| | GGGAAGTATAAGGAAGAGCT | CAG | 0.26% | exonic |
| | GTGAGCAATAAAGCAGCCCT | AAG | 0.18% | |
| BRD4-sg2 | **TCTAGTCCATCCCCCATTAC** | **TGG** | 16.65% | |
| | AATATTCCATTCCCCATTAC | TGG | 0.33% | |
| | TGTTGTCCATACCTCATTAC | TGG | 0.19% | exonic |
| | TCTAGGTCATGCACCATTAC | TGG | 0.19% | |
| | TCCACACCCTCCCCCATTAC | TAG | 0.25% | intronic |
| | CCCATTCCTTCCCCCATTAC | CAG | 0.21% | |

FIG. 19

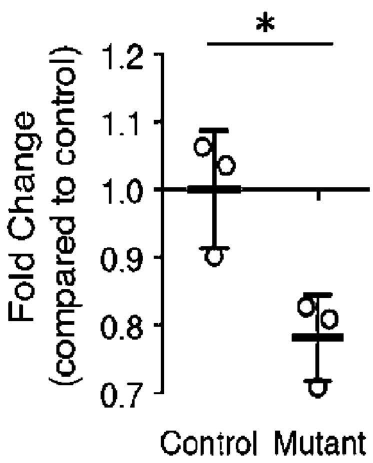


FIG. 20A

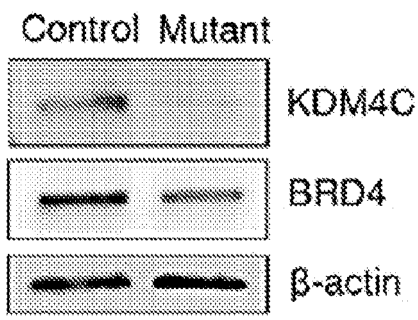


FIG. 20B

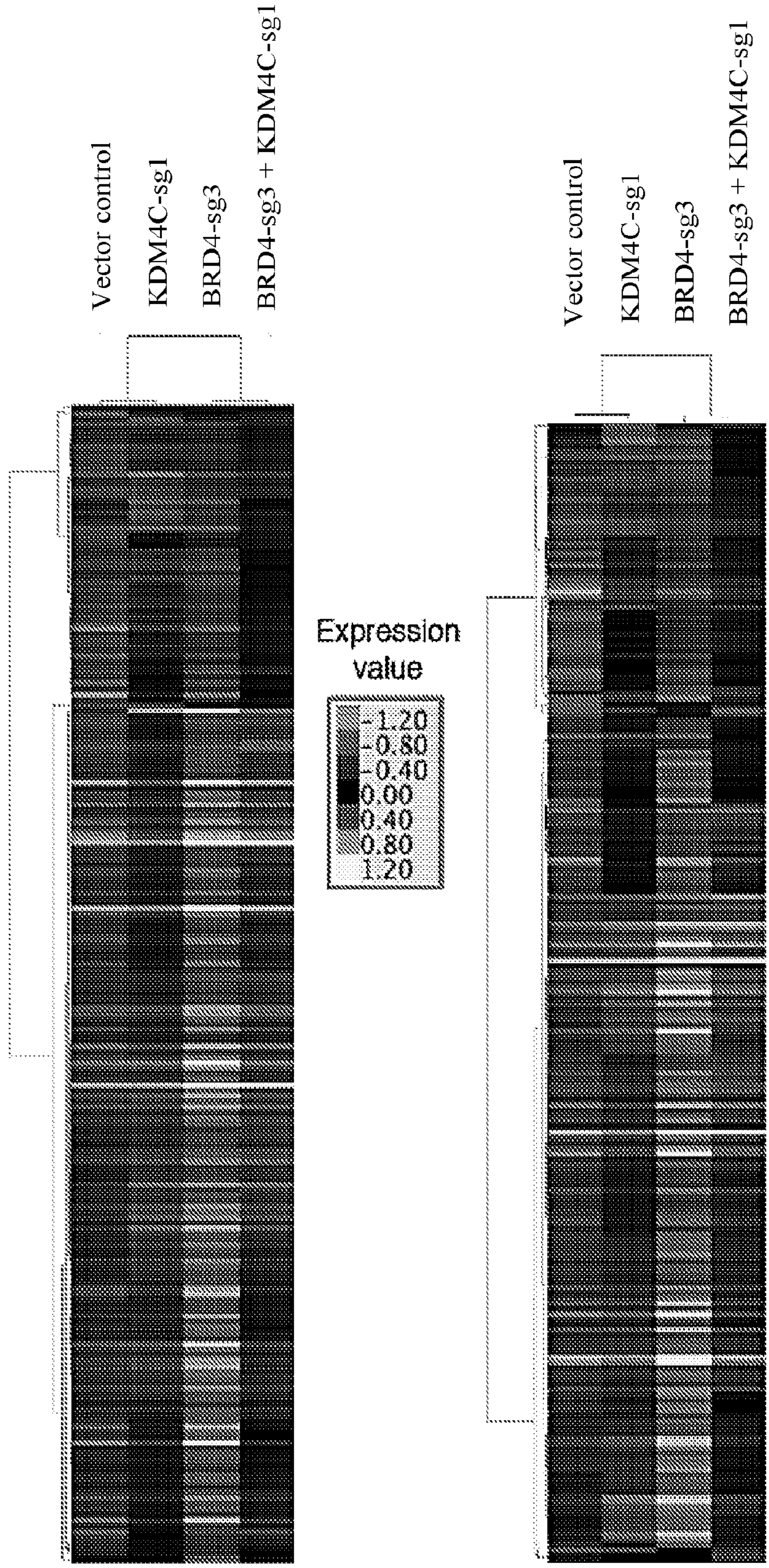
Vector control
KDM4C-sg1
BRD4-sg3
BRD4-sg3 + KDM4C-sg1
Expression value
-1.20
-0.80
-0.40
0.00
0.40
0.80
1.20
Vector control
KDM4C-sg1
BRD4-sg3
BRD4-sg3 + KDM4C-sg1

FIG. 21A

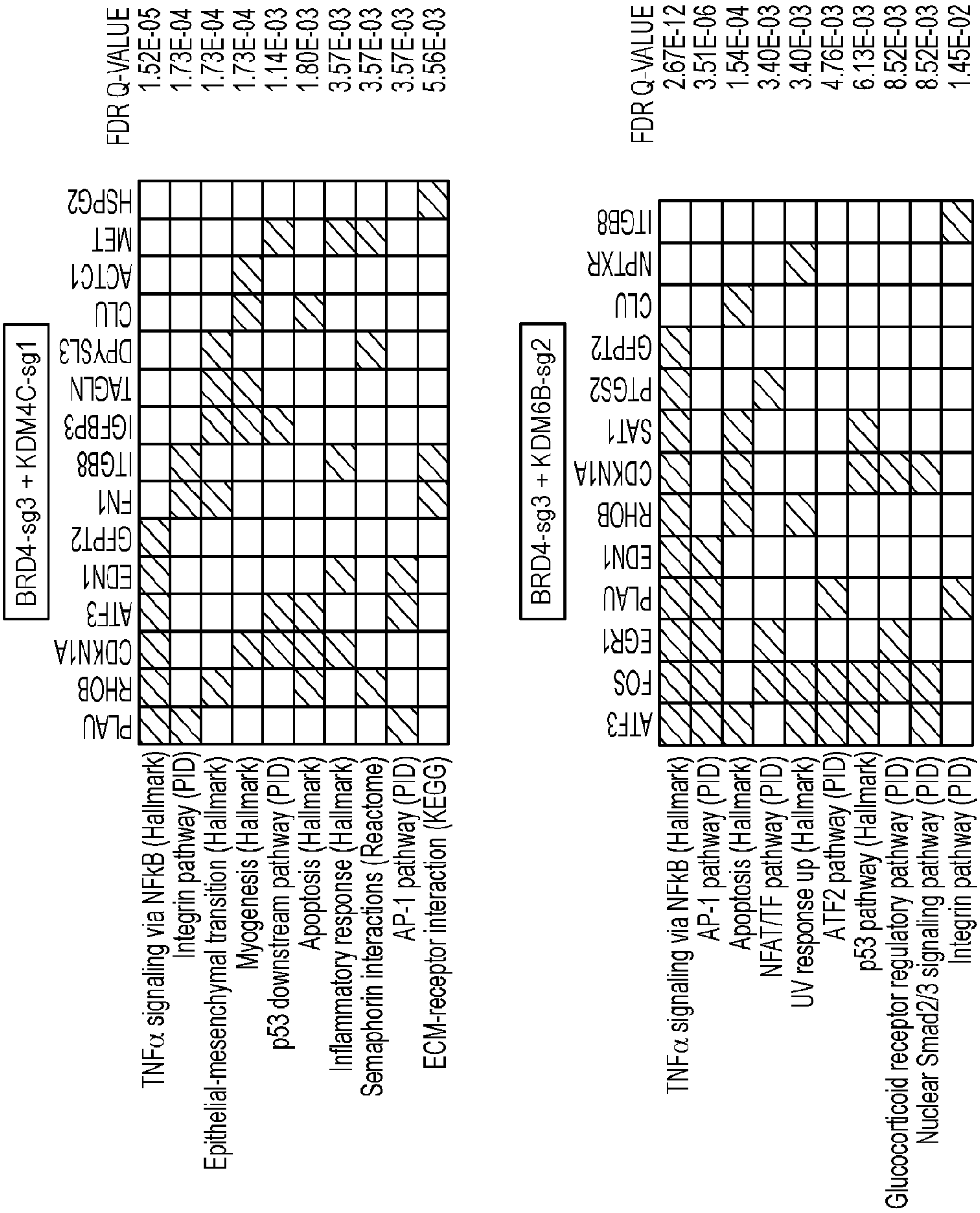
BRD4-sg3 + KDM4C-sg1
PLAU
RHOB
CDKN1A
ATF3
EDN1
GFPT2
FN1
ITGB8
IGFBP3
TAGLN
DPYSL3
CLU
ACTC1
MET
HSPG2
FDR Q-VALUE
TNFα signaling via NFkB (Hallmark) 1.52E-05
Integrin pathway (PID) 1.73E-04
Epithelial-mesenchymal transition (Hallmark) 1.73E-04
Myogenesis (Hallmark) 1.73E-04
p53 downstream pathway (PID) 1.14E-03
Apoptosis (Hallmark) 1.80E-03
Inflammatory response (Hallmark) 3.57E-03
Semaphorin interactions (Reactome) 3.57E-03
AP-1 pathway (PID) 3.57E-03
ECM-receptor interaction (KEGG) 5.56E-03
BRD4-sg3 + KDM6B-sg2
ATF3
FOS
EGR1
PLAU
EDN1
RHOB
CDKN1A
SAT1
PTGS2
GFPT2
CLU
NPTXR
ITGB8
FDR Q-VALUE
TNFα signaling via NFkB (Hallmark) 2.67E-12
AP-1 pathway (PID) 3.51E-06
Apoptosis (Hallmark) 1.54E-04
NFAT/TF pathway (PID) 3.40E-03
UV response up (Hallmark) 3.40E-03
ATF2 pathway (PID) 4.76E-03
p53 pathway (Hallmark) 6.13E-03
Glucocorticoid receptor regulatory pathway (PID) 8.52E-03
Nuclear Smad2/3 signaling pathway (PID) 8.52E-03
Integrin pathway (PID) 1.45E-02

FIG. 21B

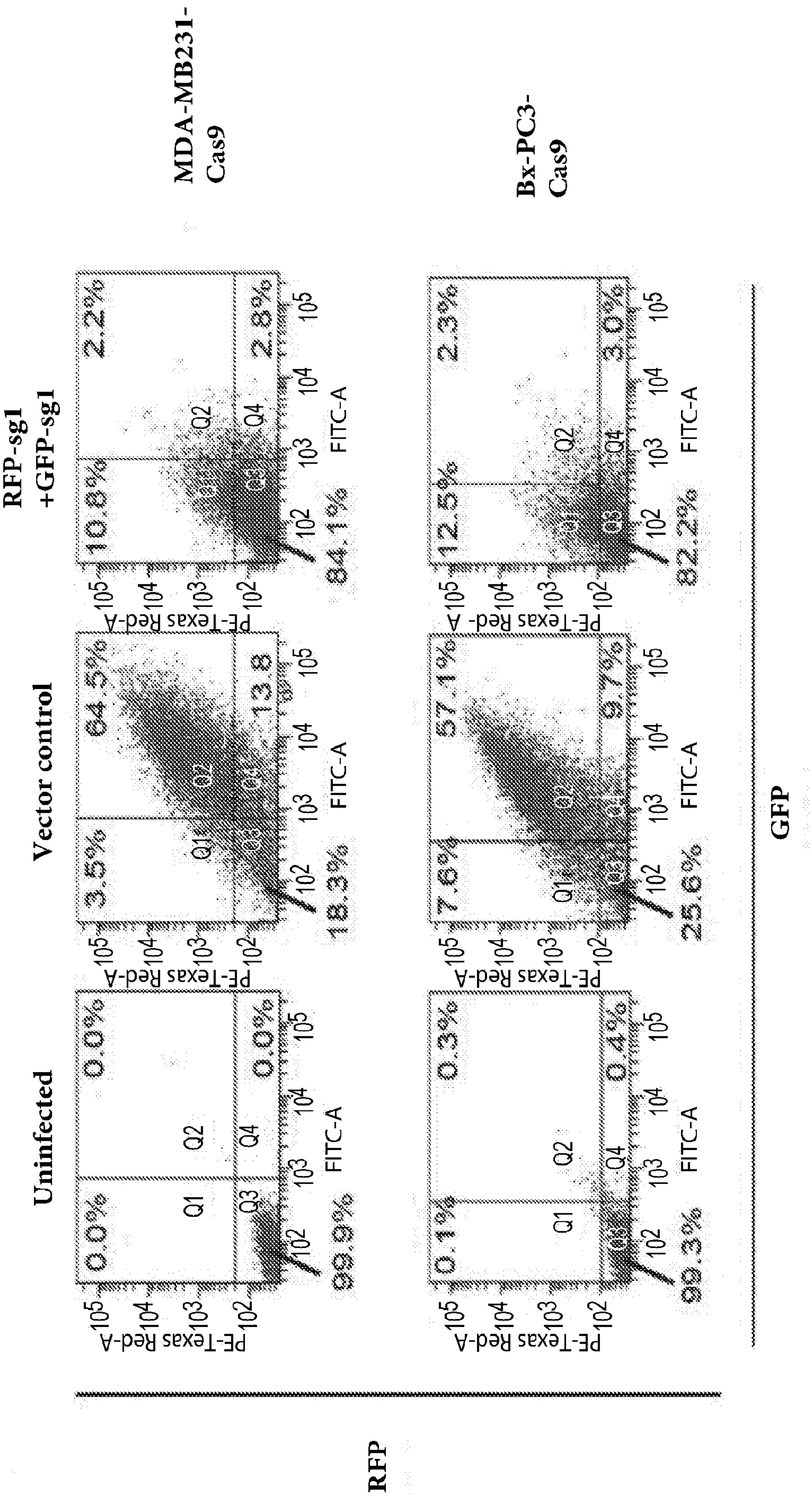
MDA-MB231-Cas9
Bx-PC3-Cas9
Uninfected
Vector control
RFP-sg1 +GFP-sg1
PE-Texas Red-A
FITC-A
Q1
Q2
Q3
Q4
0.0%
0.0%
0.0%
99.9%
3.5%
64.5%
13.8
18.3%
10.8%
2.2%
2.8%
84.1%
0.1%
0.3%
0.4%
99.3%
7.6%
57.1%
9.7%
25.6%
12.5%
2.3%
3.0%
82.2%
GFP
RFP

FIG. 22A

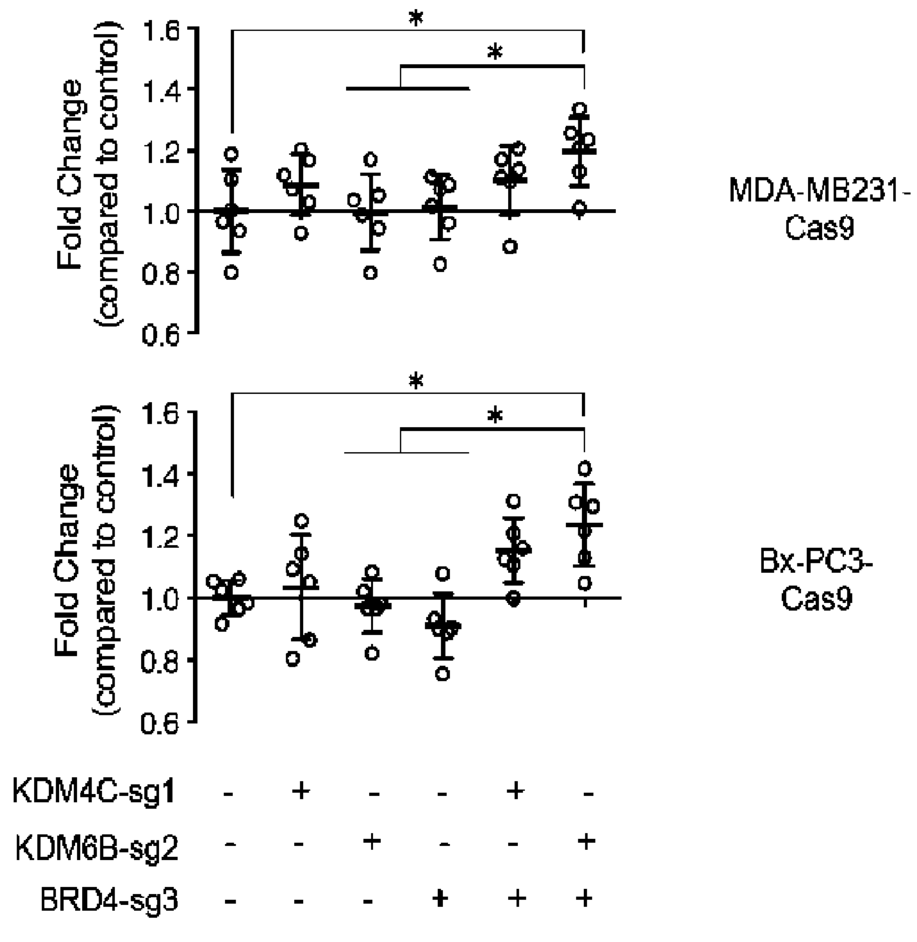
Fold Change (compared to control)
1.6
1.4
1.2
1.0
0.8
0.6
*
*
MDA-MB231-Cas9
Bx-PC3-Cas9
KDM4C-sg1 - + - - + -
KDM6B-sg2 - - + - - +
BRD4-sg3 - - - + + +

FIG. 22B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2014005042 A
- US 8697359 B
- WO 2014093694 A
- WO 2013176772 A
- WO 2014154760 A1
- US 62166302 B
- US 62073126 B

### Non-patent literature cited in the description


- **HSU et al.** *Cell,* 2014, vol. 157 (6), 1262-1278
- **JINEK et al.** *Science,* 2012, vol. 337 (6096), 816-821
- **YUEXIN ZHOU et al.** *Nature,* 09 April 2014, vol. 509 (7501), 487-491
- **T. WANG et al.** *Science,* 12 December 2013, vol. 343, 80-84
- **RAN et al.** *Nature Protocols,* 2013, vol. 8, 2281-2308
- **STEMMER.** *PLoS One,* 2015, vol. 10, e0124633
- **UPADHYAY et al.** *Genes Genome Genetics,* 2013, vol. 3 (12), 2233-2238
- **GIBSON et al.** *Nature Methods,* 2009, vol. 6, 343-5
- **ANDERSON et al.** *Journal of Biological Engineering,* 2010, vol. 4, 1
- **JAENISCH ; BIRD.** *Nat. Gene,* 2003, vol. 33, 245-254
- **DAWSON ; KOUZARIDES.** *Cell,* 2012, vol. 150 (1), 12-27
- **VIDLER et al.** *J. Med. Chem.,* 2013, vol. 56, 8073-8088
- **LEURS et al.** *Bioorg. & Med. Chem. Lett.,* 2012, vol. 22 (12), 5811-5813
- **HAMADA et al.** *Bioorg. & Med. Chem. Lett.,* 2009, vol. 19, 2852-2855
- **SAMBROOK et al.** Molecular Cloning: A Laboratory Manual. Cold Spring Harbor Laboratory Press, 2012
- **HONMA et al.** *Nat. Med.,* 2008, vol. 14, 939-948
- **WANG et al.** *Nat. Genet.,* 2008, vol. 40, 897-903
- **DAWSON et al.** *Cell,* 2012, vol. 150, 12-27
- **JUERGENS et al.** *Cancer Discov.,* 2011, vol. 1, 598-607
- **SHALEM et al.** *Science,* 2014, vol. 343, 84-87
- **SHALEM et al.** *Science,* 2014, vol. 343, 84-7
- **WU et al.** *Nat. Biotechnol.,* 2014, vol. 32, 670-6
- **GHEZRAOUI et al.** *Mol Cell,* 2014, vol. 55, 829-42
- **DONESCH et al.** *Nat Biotechnol,* 2014, vol. 32, 1262-7
- **JIN et al.** *PNAS,* 2014, vol. 111, 9235-9240
- **ASANGANI et al.** *Nature,* 2014, vol. 510, 278-282
- **KRUIDENIER et al.** *Nature,* 2012, vol. 488, 404-408
- **BLISS.** *Ann. Appl. Biol.,* 1939, vol. 6, 585-615
- **BORISY et al.** *PNAS,* 2003, vol. 100, 7977-7982
- **BLISS.** *Ann. Appl.Biol.,* 1939, vol. 26, 585-615
- **RAN et al.** *Nat. Protoc.,* 2013, vol. 8, 2281-2308
- **KIM et al.** *Genome Biol,* 2013, vol. 14, R36
- **LANGMEAD et al.** *Genome Biol,* 2009, vol. 10, R25
- **TRAPNELL et al.** *Nat Biotechnol,* 2013, vol. 31, 46-53
- **SUBRAMANIAN et al.** *Proc Natl Acad Sci USA,* 2005, vol. 102, 15545-50
- **SHALEM, O. et al.** Genome-scale CRISPR-Cas9 knockout screening in human cells. *Science,* 2014, vol. 343, 84-7
- **WANG, T. ; WEI, J. J. ; SABATINI, D. M. ; LANDER, E. S.** Genetic screens in human cells using the CRISPR-Cas9 system. *Science,* 2014, vol. 343, 80-4
- **ZHOU, Y. et al.** High-throughput screening of a CRISPR/Cas9 library for functional genomics in human cells. *Nature,* 2014, vol. 509, 487-491
- **KOIKE-YUSA, H. ; LI, Y. ; TAN, E.-P. ; VELASCO-HERRERA, M. D. C. ; YUSA, K.** Genome-wide recessive genetic screening in mammalian cells with a lentiviral CRISPR-guide RNA library. *Nat. Biotechnol.,* 2014, vol. 32, 267-73

- **GILBERT, L. A. et al.** Genome-Scale CRISPR-Mediated Control of Gene Repression and Activation. *Cell,* 2014, vol. 159, 647-661
- **KONERMANN, S. et al.** Genome-scale transcriptional activation by an engineered CRISPR-Cas9 complex. *Nature,* 2014
- **CONG, L. et al.** Multiplex genome engineering using CRISPR/Cas systems. *Science,* 2013, vol. 339, 819-23
- **MALI, P. et al.** RNA-guided human genome engineering via Cas9. *Science,* 2013, vol. 339, 823-6
- **GILBERT, L. A. et al.** CRISPR-mediated modular RNA-guided regulation of transcription in eukaryotes. *Cell,* 2013, vol. 154, 442-451
- **CHENG, A. A. ; DING, H. ; LU, T. K.** Enhanced killing of antibiotic-resistant bacteria enabled by massively parallel combinatorial genetics. *Proc. Natl. Acad. Sci.,* 2014, vol. 111, 12462-7
- **HONMA, K. et al.** RPN2 gene confers docetaxel resistance in breast cancer. *Nat. Med.,* 2008, vol. 14, 939-948
- **WANG, Z. et al.** Combinatorial patterns of histone acetylations and methylations in the human genome. *Nat. Genet.,* 2008, vol. 40, 897-903
- **DAWSON, M. A. ; KOUZARIDES, T.** Cancer epigenetics: From mechanism to therapy. *Cell,* 2012, vol. 150, 12-27
- **JUERGENS, R. A. et al.** Combination epigenetic therapy has efficacy in patients with refractory advanced non-small cell lung cancer. *Cancer Discov.,* 2011, vol. 1, 598-607
- **JONES, P. A. ; BAYLIN, S. B.** The Epigenomics of Cancer. *Cell,* 2007, vol. 128, 683-692
- **YOO, C. B. ; JONES, P. A.** Epigenetic therapy of cancer: past, present and future. *Nat. Rev. Drug Discov.,* 2006, vol. 5, 37-50
- **JIN, C. et al.** Chem-seq permits identification of genomic targets of drugs against androgen receptor regulation selected by functional phenotypic screens. *Proc. Natl. Acad. Sci. U. S. A.,* 2014, vol. 111, 9235-40
- **ASANGANI, I. A et al.** Therapeutic targeting of BET bromodomain proteins in castration-resistant prostate cancer. *Nature,* 2014, vol. 510, 278-82
- **KRUIDENIER, L. et al.** A selective jumonji H3K27 demethylase inhibitor modulates the proinflammatory macrophage response. *Nature,* 2012, vol. 488, 404-408
- **BLISS, C. I.** THE TOXICITY OF POISONS APPLIED JOINTLY1. *Ann. Appl. Biol.,* 1939, vol. 26, 585-615
- **BORISY, A. A. et al.** Systematic discovery of multicomponent therapeutics. *Proc. Natl. Acad. Sci. U. S. A.,* 2003, vol. 100, 7977-7982
- **PATTANAYAK, V. et al.** High-throughput profiling of off-target DNA cleavage reveals RNA-programmed Cas9 nuclease specificity. *Nat. Biotechnol.,* 2013, vol. 31, 839-43
- **KUSCU, C. ; ARSLAN, S. ; SINGH, R. ; THORPE, J. ; ADLI, M.** Genome-wide analysis reveals characteristics of off-target sites bound by the Cas9 endonuclease. *Nat. Biotechnol.,* 2014, vol. 32, 677-683
- **WU, X. et al.** Genome-wide binding of the CRISPR endonuclease Cas9 in mammalian cells. *Nat. Biotechnol.,* 2014, vol. 32, 670-676
- **DOENCH, J. G. et al.** Rational design of highly active sgRNAs for CRISPR-Cas9-mediated gene inactivation. *Nat. Biotechnol.,* 2014, vol. 32, 1262-1267
- **ESSLETZBICHLER, P. et al.** Megabase-scale deletion using CRISPR/Cas9 to generate a fully haploid human cell line. *Genome Res.,* 2014, vol. 24, 2059-2065
- **BLASCO, R. B. et al.** Simple and rapid in vivo generation of chromosomal rearrangements using CRISPR/Cas9 technology. *Cell Rep.,* 2014, vol. 9, 1219-1227
- **CHOI, P. S. ; MEYERSON, M.** Targeted genomic rearrangements using CRISPR/Cas technology. *Nat. Commun.,* 2014, vol. 5, 3728
- **MOFFAT, J. et al.** A Lentiviral RNAi Library for Human and Mouse Genes Applied to an Arrayed Viral High-Content Screen. *Cell,* 2006, vol. 124, 1283-1298
- **RAN, F. A. et al.** Genome engineering using the CRISPR-Cas9 system. *Nat. Protoc.,* 2013, vol. 8, 2281-308
- **KIM D et al.** TopHat2: accurate alignment of transcriptomes in the presence of insertions, deletions and gene fusions. *Genome Biol,* 2013, vol. 14, R36
- **LANGMEAD B ; TRAPNELL C ; POP M ; SALZBERG SL.** Ultrafast and memory-efficient alignment of short DNA sequences to the human genome. *Genome Biol,* 2009, vol. 10, R25
- **TRAPNELL C et al.** Differential analysis of gene regulation at transcript resolution with RNA-seq. *Nat Biotechnol,* 2013, vol. 31, 46-53
- **SUBRAMANIAN A et al.** Gene set enrichment analysis: a knowledge-based approach for interpreting genome-wide expression profiles. *Proc Natl Acad Sci U S A,* 2005, vol. 102, 15545-50
- **STEMMER M.** CCTop: An Intuitive, Flexible and Reliable CRISPR/Cas9 Target Prediction Tool. *PLoS One,* 2015, vol. 10 (4), e0124633